# EUROPEAN PATENT APPLICATION

(11) **EP 2 399 577 A1**
(43) Date of publication of application: **28.12.2011**
(21) Application number: 11178398.1
(22) Date of filing: 07.09.2007
(51) Int. Cl.: A61K 31/4025, A61K 31/453, A61K 31/4535, A61K 31/454, A61K 31/4525, A61K 31/4545, A61K 31/46, A61K 31/506, A61K 31/5377, A61K 31/55, A61K 31/4439, A61P 25/28, A61P 9/10, A61K 45/06

(54) **Use of N-containing spirocompounds for the enhancement of cognitive function**

(30) Priority: 12.09.2006 US 843979 P
(62) Divisional of application: 07811730.6
(71) Applicant: Adolor Corporation, Exton, PA 19341 (US)
(72) Inventor: Dolle, Roland, E., King of Prussia, PA Pennsylvania 19406 (US); LeBourdonnec, Bertrand, East Fallow Field, PA Pennsylvania 19320 (US)
(74) Representative: Hallybone, Huw George

(57) **Abstract**

Methods for enhancing cognitive function are disclosed. More particularly, methods are disclosed for enhancing cognitive function comprising the step of administering spirocyclic heterocyclic derivatives (including derivatives of spiro(2H-1-benzopyran-2,4'-piperidines) of the general formula (IV):

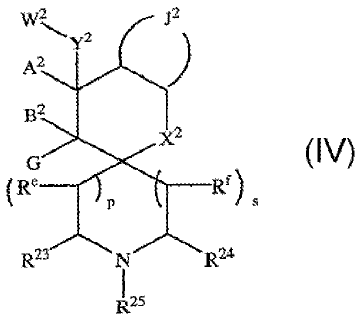

## Description

### FIELD OF THE INVENTION

The invention relates to methods for enhancing cognitive function. More particularly, the invention relates to the methods for enhancing cognitive function by administering spirocyclic heterocyclic derivatives, including derivatives of spiro(2*H*-1-benzopyran-2,4'-piperidines).

### BACKGROUND OF THE INVENTION

Various disorders and diseases exist that affect cognitive function. Cognitive function may be generally described as including at least three different components: attention, learning, and memory. Each of these components and their respective levels affect the overall level of a subject's cognitive ability. For instance, while Alzheimer's Disease patients suffer from a loss of overall cognition and thus deterioration of each of these characteristics, it is the loss of memory that is most often associated with the disease. In other diseases, patients suffer from cognitive dysfunction or impairment that is more predominately associated with different characteristics of cognition. Other conditions include general dementia associated with other neurological diseases, aging, and treatment of conditions that can cause deleterious effects on mental capacity, such as cancer treatments, stroke/ischemia, and mental retardation.

Cognitive dysfunction creates a variety of problems for today's society. Therefore, scientists have made efforts to develop cognitive enhancers or cognition activators. The cognition enhancers or activators that have been developed are generally classified to include nootropics, vasodilators, metabolic enhancers, psychostimulants, cholinergic agents, biogenic amine drugs, and neuropeptides.

There are at least three different opioid receptors (µ, δ and κ) that are present in both central and peripheral nervous systems of many species, including humans. Lord, J.A.H., et al., Nature, 1977, 267, 495. Activation of the δ opioid receptors induces analgesia in various animal models. Moulin, et al., Pain, 1985, 23, 213. Some work suggests that the analgesics working at δ opioid receptors do not have the attendant side effects associated with µ and κ opioid receptor activation. Galligan, et al., J. Pharm. Exp. Ther., 1985, 229, 641. The δ opioid receptor has also been identified as having a role in circulatory systems. Ligands for the δ receptor have also been shown to possess immunomodulatory activities. Dondio, et al., Exp. Opin. Ther. Patents, 1997, 10, 1075. Further, selective δ opioid receptor agonists have been shown to promote organ and cell survival. Su, T-P, Journal of Biomedical Science, 2000, 9(3), 195-199. Ligands for the δ opioid receptor may therefore find potential use as analgesics, as antihypertensive agents, as immunomodulatory agents, and/or agents for the treatment of cardiac disorders.

Numerous selective δ opioid ligands are peptidic in nature and thus are unsuitable for administration by systemic routes. For example, rubiscolin-6 (YPLDLF), a selective δ opioid peptide isolated from the enzymatic digest of spinach leaves, has been shown to enhance memory consolidation at sub-optimal analgesic doses of 3 nmol/mouse after i.c.v. administration and 100 mg/kg after oral administration. Yang, Shuzhang, et al., Peptides (2003), 24, 325-328.

Certain non-peptidic δ opioid receptor ligands have been developed. See, for example, E. J. Bilsky, et al., Journal of Pharmacology and Experimental Therapeutics, 1995, 273(1), 359-366; WO 93/15062, WO 95/04734, WO 95/31464, WO 96/22276, WO 97/10216, WO 01/46192, WO 02/094794, WO 02/094810, WO 02/094811, WO 02/094812, WO 02/48122, WO 03/029215, WO 03/033486, JP-4275288, EP-A-0,864,559, US-A-5,354,863, US-B-6,200,978, US-B-6,436,959 and US 2003/0069241. In particular, U.S. Patent Publication No. US 2005-0159438 A1 and U.S. Application No. 11/393,133 filed March 30, 2006 disclose spirocyclic heterocyclic derivatives (including derivatives of spiro(2*H*-1-benzopyran-2,4'-piperidines), which are ligands of the δ opioid receptor, pharmaceutical compositions containing these compounds, and methods for their pharmaceutical use for treating and/or preventing pain, gastrointestinal disorders, urogenital tract disorders including incontinence and overactive bladder, immunomodulatory disorders, inflammatory disorders, respiratory function disorders, anxiety, mood disorders, stress-related disorders, attention deficit hyperactivity disorders, sympathetic nervous system disorders, depression, tussis, motor disorders, traumatic injuries, especially to the central nervous system, stroke, cardiac arrhythmias, glaucoma, sexual dysfunctions, shock, brain edema, cerebral ischemia, cerebral deficits subsequent to cardiac bypass surgery and grafting, systemic lupus erythematosus, Hodgkin's disease, Sjogren's disease, epilepsy, rejections in organ transplants and skin grafts, and substance addiction. In certain other embodiments, the spirocyclic heterocyclic derivatives are ligands of the δ opioid receptor and may be useful in, *inter alia,* methods for improving organ and cell survival, methods for providing cardioprotection following myocardial infarction, methods for reducing the need for anesthesia, methods for producing and/or maintaining an anaesthetic state, and methods of detecting, imaging or monitoring degeneration or dysfunction of opioid receptors in a patient. However, these applications do not disclose or teach methods for enhancing cognitive function using non-peptidic δ opioid receptor modulators.

Thus, there is still an unfulfilled need for methods for enhancing cognitive function employing non-peptidic compounds. The present invention is directed to these, as well as other important ends.

### SUMMARY OF THE INVENTION

In one aspect, the invention is directed to methods for enhancing cognitive function, comprising the step of:
administering to a patient in need thereof an effective amount of a compound of formula IV: 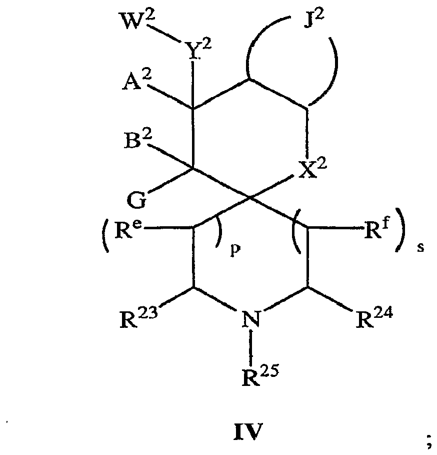 wherein:
   Y² is a single bond or -[C(R^{c})(R^{d})]ₖ-;
   each R^{c}, R^{d}, R^{e}, and R^{f} is independently H or alkyl;
   W² is aryl, alkaryl, heterocycloalkylaryl, heteroaryl, alkylheteroaryl, heteroarylaryl, or alkylheteroarylaryl;
   R²³ and R²⁴ are each independently H or alkyl;
   R²⁵ is H, alkyl, -(CH₂)-alkenyl" -(CH₂)-alkynyl, cycloalkyl, alkylcycloalkyl, aralkyl, or heteroarylalkyl;
   each k is independently 1, 2, or 3;
   pis 0, 1, 2 or 3;
   s is 0, 1, 2 or 3, provided that the sum of p and s is ≤ 4;
   A² and B² are each independently H or alkyl, or together form a double bond;
   G is H or alkyl;
   X² is -CH₂-, -O-, or -S-, -SO₂-;
   R²⁶ is H, alkyl, cycloalkyl, -(CH₂)-alkenyl, -(CH₂)-alkynyl, aryl, -C(=O)R^{d}, or -S(=O)₂R^{d}; and
   J² forms a 6- to 10-membered aryl when taken together with the carbon atoms to which it is attached;
or a stereoisomer, prodrug, pharmaceutically acceptable salt, hydrate, solvate, acid salt hydrate, or N-oxide thereof.

In another aspect, the invention is directed to compositions, comprising:
a. a compound of formula IV;
b. a second pharmaceutically active compound selected from the group consisting of cholinesterase inhibitor (including acetylcholinesterase inhibitor and butylcholinesterase inhibitor), NMDA receptor modulator, β amyloid modulator, β amyloid inhibitor, nicotinic cholinergic agent, nicotine receptor partial agonist (NRPA), estrogenic agent, selective estrogen receptor modulator (SERM), muscarinic agonist, neurotransmitter precursor, neurotransmitter reuptake inhibitor, Coenzyme Q10, *Ginkgo biloba,* phosphatidylserine, vitamin E, and combinations thereof; and
c. a pharmaceutically acceptable carrier.

These and other embodiments of the invention will become more apparent from the following detailed description.

### DETAILED DESGRIPTION OF ILLUSTRATIVE EMBODIMENTS

The invention relates to methods for enhancing cognitive function in patients by administering spirocyclic heterocyclic derivatives, pharmaceutical compositions containing these compounds. The spirocyclic heterocyclic derivatives useful in the methods of this invention and processes for their synthesis are disclosed in U.S. Provisional Application No. 60/507,864 filed October 1, 2003, U.S. Application No. 10/957,554 filed October 1, 2004, U.S. Provisional Application No. 60/667,177 filed March 31, 2005, U.S. Application No. 11/393,133 filed March 30, 2006, the entire disclosures of which are hereby incorporated herein by reference.

"Cognitive function," as used herein, refers to the function of the mental process of a human or other animal, who may be unhealthy or unhealthy, and includes at least three different components: attention, learning, and memory. See, *e.g.,* Mosby's Medical Dictionary, 5th edition (1998); Stedman's Medical Dictionary, 11th edition (1990). In certain preferred embodiments of the invention, the method of the invention are useful for enhancing cognitive function, particularly memory, including enhancing the cognitive function, particularly memory, of a healthy or unhealthy patient. As used herein, "memory" refers to the complex mental function having at least four distinct phases: (1) memorizing or learning, (2) retention, (3) recall, and (4) recognition, and includes immediate, recent, and remote memory. See, *e.g.,* Mosby's Medical Dictionary, 5th edition (1998); Stedman's Medical Dictionary, 11th edition (1990).

The term "enhancing," as used herein, refers to the ability of the compounds of the invention and/or composition containing them to increase the ability or capacity of an individual (healthy or unhealthy) to comprehend, judge, memorize, and reason; and can be measured by typically employed by those skilled in the art. Such an improvement can include at least about a 10% increase, preferably, at least about a 25% increase, and more preferably, at least about a 50% increase.

In other embodiments, the patient is suffering cognitive dysfunction. "Cognitive dysfunction," as used herein, refers to disturbances in the mental process related to thinking, reasoning, and judgment and includes, without limitation, memory loss. See, *e.g.,* Mosby's Medical Dictionary, 5th edition (1998); Stedman's Medical Dictionary, 11th edition (1990). The term "cognitive dysfunction" indicates disruptions in performance including one or more of the following signs:
(1) memory deficits (impaired ability to learn new information or recall previously learned information;
(2) one (or more) of the following disturbances:
   (a) aphasia (language disturbance) [
   (b) apraxia (impaired ability to carry out motor activities despite intact motor function)
   (c) agnosia (failure to recognize or identify objects despite in tact sensory function); and/or
   (d) disturbance in executive functioning (*i*.*e.* planning, organizing, sequencing, abstracting);
(3) memory disturbances causing significant impairment in social or occupational functioning, and representing a significant decline from a previous level of functioning; and
(4) impairment in cognitive functioning as evidenced by neuropsychological testing or quantified clinical assessment, accompanied by objective evidence of a systemic general medical condition or central nervous system dysfunction.
Cognitive dysfunction is exhibited in certain diseases and disorders, including, but not limited to Alzheimer's Disease, mild cognitive impairment, age-related cognitive decline, vascular dementia, Parkinson's Disease dementia, amyotrophic lateral sclerosis, Huntington's Disease, stroke, traumatic brain injury, AIDS-associated dementia, schizophrenia, Lewy-body variant of Alzheimer's Disease with or without association with Parkinson's Disease, Creutzfeld-Jakob Disease, Korsakoff's Disorder, learning disabilities caused by degenerative disorders, learning disabilities caused by non-degenerative disorders, genetic conditions (*e.g*., Rubenstein-Taybi Syndrome), cerebral senility, vascular dementia, electric shock induced amnesia, memory impairment associated with depression or anxiety, memory impairment associated with surgical procedures such as coronary artery bypass grafting (CABG), Down's syndrome, and combinations thereof.

In certain embodiments of the methods of the invention, the cognitive dysfunction is memory loss, including memory loss that is age-associated, caused by electro-convulsive therapy, the result of brain damage, or a combination thereof. The brain damage may be caused by a stroke, an anesthetic accident, head trauma, hypoglycemia, carbon monoxide poisoning, lithium intoxication, vitamin deficiency (such as B1, thiamine and B12), or a combination thereof.

Memory loss and impaired learning ability are features of a range of clinical conditions. For instance, loss of memory is the most common symptom of dementia states including Alzheimer's disease. Memory defects also occur with other kinds of dementia such as multi-infarct dementia (MID), a senile dementia caused by cerebrovascular deficiency, and the Lewy-body variant of Alzheimer's disease with or without association with Parkinson's disease, or Creutzfeld-Jakob disease. Loss of memory is a common feature of brain-damaged patients. Brain damage may occur, for example, after a classical stroke or as a result of an anesthetic accident, head trauma, hypoglycemia, carbon monoxide poisoning, lithium intoxication, vitamin (B1, thiamine and B12) deficiency, or excessive alcohol use or Korsakoff's disorder. Memory impairment may furthermore be age-associated; the ability to recall information such as names, places and words seems to decrease with increasing age. Transient memory loss may also occur in patients, suffering from a major depressive disorder, after electro-convulsive therapy (ECT).

As employed above and throughout the disclosure, the following terms, unless otherwise indicated, shall be understood to have the following meanings.

"Alkyl" refers to an optionally substituted, saturated straight, or branched, hydrocarbon having from about 1 to about 20 carbon atoms (and all combinations and subcombinations of ranges and specific numbers of carbon atoms therein), preferably from about 1 to about 8 carbon atoms, herein referred to as "lower alkyl," more preferably from about 1 to about 6, still more preferably from about 1 to about 4, with from about 2 to about 3 being most preferred. In certain alternative preferred embodiments, the alkyl group, more preferably, has one carbon atom. Alkyl groups include, but are not limited to, methyl, ethyl, n-propyl, isopropyl, n-butyl, isobutyl, t-butyl, n-pentyl, cyclopentyl, isopentyl, neopentyl, n-hexyl, isohexyl, 3-methylpentyl, 2,2-dimethylbutyl, and 2,3-dimethylbutyl.

As used herein, "alkylene" refers to an optionally substituted bivalent alkyl radical having the general formula -(CH₂)ₙ-, where n is 1 to 10, preferably 1 to 6, with 1 to 4 being most preferred. In alternative embodiments, n is preferably 4 to 6. Non-limiting examples include methylene, dimethylene, trimethylene, tetramethylene, pentamethylene, and hexamethylene.

"Cycloalkyl" refers to an optionally substituted alkyl group having one or more rings in their structures and having from about 3 to about 20 carbon atoms (and all combinations and subcombinations of ranges and specific numbers of carbon atoms therein), with from about 3 to about 10 carbon atoms being preferred. Multi-ring structures may be bridged or fused ring structures. Cycloalkyl groups include, but are not limited to, cyclopropyl, cyclobutyl, cyclopentyl, cyclohexyl, cyclooctyl, 2-[4-isopropyl-1-methyl-7-oxabicyclo[2.2.1]heptanyl], 2-[1,2,3,4-tetrahydro-naphthalenyl], and adamantyl.

"Alkylcycloalkyl" refers to an optionally substituted ring system comprising a cycloalkyl group having one or more alkyl substituents, wherein cycloalkyl and alkyl are each as previously defined. Exemplary alkylcycloalkyl groups include, for example, 2-methylcyclohexyl, 3,3-dimethylcyclopentyl, trans-2,3-dimethylcyclooctyl, and 4-methyldecahydronaphthalenyl.

"Heterocycloalkyl" refers to an optionally substituted ring system composed of a cycloalkyl radical wherein in at least one of the carbon atom ring members is independently replaced by a heteroatom group selected from the group consisting of O, S, N, and NH, wherein cycloalkyl is as previously defined. Heterocycloalkyl ring systems having a total of from about 5 to about 14 carbon atom ring members and heteroatom ring members (and all combinations and subcombinations of ranges and specific numbers of carbon and heteroatom ring members) are preferred. In other preferred embodiments, the heterocyclic groups may be fused to one or more aromatic rings. Exemplary heterocycloalkyl groups include, but are not limited to, tetrahydrofuranyl, tetrahydrothienyl, piperidinyl, pyrrolidinyl, isoxazolidinyl, isothiazolidinyl, pyrazolidinyl, oxazolidinyl, thiazolidinyl, piperazinyl, morpholinyl, piperadinyl, decahydroquinolyl, octahydrochromenyl, octahydro-cyclopenta[c]pyranyl, 1,2,3,4,-tetrahydroquinolyl, octahydro-[2]pyrindinyl, decahydro-cycloocta[c]furanyl, tetrahydroquinolyl, and imidazolidinyl.

"Alkylheterocycloalkyl" refers to an optionally substituted ring system comprising a heterocycloalkyl group having one or more alkyl substituents, wherein heterocycloalkyl and alkyl are each as previously defined. Exemplary alkylheterocycloalkyl groups include, for example, 2-methylpiperidinyl, 3,3-dimethylpyrrolidinyl, *trans*-2,3-dimethylmorpholinyl, and 4-methyldecahydroquinolinyl.

"Alkenyl" refers to an optionally substituted alkyl group having from about 2 to about 10 carbon atoms and one or more double bonds (and all combinations and subcombinations of ranges and specific numbers of carbon atoms therein), wherein alkyl is as previously defined.

"Alkynyl" refers to an optionally substituted alkyl group having from about 2 to about 10 carbon atoms and one or more triple bonds (and all combinations and subcombinations of ranges and specific numbers of carbon atoms therein), wherein alkyl is as previously defined.

"Aryl" refers to an optionally substituted, mono-, di-, tri-, or other multicyclic aromatic ring system having from about 5 to about 50 carbon atoms (and all combinations and subcombinations of ranges and specific numbers of carbon atoms therein), with from about 6 to about 10 carbons being preferred. Non-limiting examples include, for example, phenyl, naphthyl, anthracenyl, and phenanthrenyl.

"Aralkyl" refers to an optionally substituted moiety composed of an alkyl radical bearing an aryl substituent and having from about 6 to about 50 carbon atoms (and all combinations and subcombinations of ranges and specific numbers of carbon atoms therein), with from about 6 to about 10 carbon atoms being preferred. Non-limiting examples include, for example, benzyl, diphenylmethyl, triphenylmethyl, phenylethyl, and diphenylethyl.

"Halo" refers to a fluoro, chloro, bromo, or iodo moiety, preferably fluoro.

"Heteroaryl" refers to an optionally substituted aryl ring system wherein in at least one of the rings, one or more of the carbon atom ring members is independently replaced by a heteroatom group selected from the group consisting of S, O, N, and NH, wherein aryl iso as previously defined. Heteroaryl groups having a total of from about 5 to about 14 carbon atom ring members and heteroatom ring members (and all combinations and subcombinations of ranges and specific numbers of carbon and heteroatom ring members) are preferred. Exemplary heteroaryl groups include, but are not limited to, pyrryl, furyl, pyridyl, 1,2,4-thiadiazolyl, pyrimidyl, thienyl, isothiazolyl, imidazolyl, tetrazolyl, pyrazinyl, pyrimidyl, quinolyl, isoquinolyl, thiophenyl, benzothienyl, isobenzofuryl, pyrazolyl, indolyl, purinyl, carbazolyl, benzimidazolyl, and isoxazolyl. Heteroaryl may be attached via a carbon or a heteroatom to the rest of the molecule.

"Heteroarylalkyl" and "heteroaralkyl" each refers to an optionally substituted, heteroaryl substituted alkyl radical where heteroaryl and alkyl are as previously defined Non-limiting examples include, for example, 2-(1H-pyrrol-3-yl)ethyl, 3-pyridylmethyl, 5-(2*H*-tetrazolyl)methyl, and 3-(pyrimidin-2-yl)-2-methylcyclopentanyl.

"Perhaloalkyl" refers to an alkyl group, wherein two or more hydrogen atoms are replaced by halo (F, Cl, Br, I) atoms, and alkyl is as previously defined. Exemplary perhaloalkyl groups include, for example, perhalomethyl, such as perfluoromethyl and difluoromethyl.

"Alkoxy" and "alkoxyl" refer to an optionally substituted alkyl-O- group wherein alkyl is as previously defined. Exemplary alkoxy and alkoxyl groups include, for example, methoxy, ethoxy, n-propoxy, i-propoxy, n-butoxy, and heptoxy.

"Alkenyloxy" refers to an optionally substituted alkenyl-O- group wherein alkenyl is as previously defined. Exemplary alkenyloxy and alkenyloxyl groups include, for example, allyloxy, butenyloxy, heptenyloxy, 2-methyl-3-buten-1-yloxy, and 2,2-dimethylallyloxy.

"Alkynyloxy" refers to an optionally substituted alkynyl-O- group wherein alkynyl is as previously defined. Exemplary alkynyloxy and alkynyloxyl groups include, for example, propargyloxy, butynyloxy, heptynyloxy, 2-methyl-3-butyn-1-yloxy, and 2,2-dimethylpropargyloxy.

"Aryloxy" and "aryloxyl" refer to an optionally substituted aryl-O- group wherein aryl is as previously defined. Exemplary aryloxy and aryloxyl groups include, for example, phenoxy and naphthoxy.

"Aralkoxy" and "aralkoxyl" refer to an optionally substituted aralkyl-O- group wherein aralkyl is as previously defined. Exemplary aralkoxy and aralkoxyl groups include, for example, benzyloxy, 1-phenylethoxy, 2-phenylethoxy, and 3-naphthylheptoxy.

"Cycloalkoxy" refers to an optionally substituted cycloalkyl-O- group wherein cycloalkyl is as previously defined. Exemplary cycloalkoxy groups include, for example, cyclopropanoxy, cyclobutanoxy, cyclopentanoxy, cyclohexanoxy, and cycloheptanoxy.

"Heteroaryloxy" refers to an optionally substituted heteroaryl-O- group wherein heteroaryl is as previously defined. Exemplary heteroaryloxy groups include, but are not limited to, pyrryloxy, furyloxyl, pyridyloxy, 1,2,4-thiadiazolyloxy, pyrimidyloxy, thienyloxy, isothiazolyloxy, imidazolyloxy, tetrazolyloxy, pyrazinyloxy, pyrimidyloxy, quinolyloxy, isoquinolyloxy, thiophenyloxy, benzothienyloxy, isobenzofuryloxy, pyrazolyloxy, indolyloxy, purinyloxy, carbazolyloxy, benzimidazolyloxy, and isoxazolyloxy.

"Heteroaralkoxy" refers to an optionally substituted heteroarylalkyl-O- group wherein heteroarylalkyl is as previously defined. Exemplary heteroaralkoxy groups include, but are not limited to, pyrrylethyloxy, furylethyloxy, pyridylmethyloxy, 1,2,4-thiadiazolylpropyloxy, pyrimidylmethyloxy, thienylethyloxy, isothiazolylbutyloxy, and imidazolyl-2-methylpropyloxy.

"Heterocycloalkylaryl" refers to an optionally substituted ring system composed of an aryl radical bearing a heterocycloalkyl substituent wherein heterocycloalkyl and aryl are as previously defined. Exemplary heterocycloalkylaryl groups include, but are not limited to, morpholinylphenyl, piperidinylnaphthyl, piperidinylphenyl, tetrahydrofuranylphenyl, and pyrrolidinylphenyl.

"Alkylheteroaryl" refers to an optionally substituted ring system composed of a heteroaryl radical bearing an alkyl substituent wherein heteroaryl and alkyl are as previously defined. Exemplary alkylheteroaryl groups include, but are not limited to, methylpyrryl, ethylfuryl, 2,3-dimethylpyridyl, N-methyl-1,2,4-thiadiazoly], propylpyrimidyl, 2-butylthienyl, methylisothiazolyl, 2-ethylimidazolyl, butyltetrazolyl, 5-ethylbenzothienyl, and N-methylindolyl. Alkyheteroaryl groups may be attached via a carbon or a heteroatom to the rest of the molecule.

"Heteroarylaryl" refers to an optionally substituted ring system composed of an aryl radical bearing a heteroaryl substituent wherein heteroaryl and aryl are as previously defined. Exemplary heteroarylaryl groups include, but are not limited to, pyrrylphenyl, furylnaphthyl, pyridylphenyl, 1,2,4-thiadiazolylnaphthyl, pyrimidylphenyl, thienylphenyl, isothiazolylnaphthyl, imidazolylphenyl, tetrazolylphenyl, pyrazinylnaphthyl, pyrimidylphenyl, quinolylphenyl, isoquinolylnaphthyl, thiophenylphenyl, benzothienylphenyl, isobenzofurylnaphthyl, pyrazolylphenyl; indolylnaphthyl, purinylphenyl, carbazolylnaphthyl, benzimidazolylphenyl, and isoxazolylphenyl. Heteroarylaryl may be attached via a carbon or a heteroatom to the rest of the molecule.

"Alkylheteroarylaryl" refers to an optionally substituted ring system composed of an aryl radical bearing an alkylheteroaryl substituent and have from about 12 to about 50 carbon atoms (and all combinations and subcombinations of ranges and specific numbers of carbon atoms therein), with from about 12 to about 30 carbon atoms being preferred wherein aryl and alkylheteroaryl are as previously defined. Exemplary heteroarylaryl groups include, but are not limited to, methylpyrrylphenyl, ethylfurylnaphthyl, methylethylpyridylphenyl, dimethylethylpyrimidylphenyl, and dimethylthienylphenyl.

Typically, substituted chemical moieties include one or more substituents that replace hydrogen. Exemplary substituents include, for example, halo (*e.g*., F, Cl, Br, I), alkyl, cycloalkyl, alkylcycloalkyl, alkenyl, alkynyl, aralkyl, aryl, heteroaryl, heteroaralkyl, spiroalkyl, heterocycloalkyl, hydroxyl (-OH), oxo (=O), alkoxyl, aryloxyl, aralkoxyl, nitro (-NO₂) cyano (-CN), amino (-NH₂), -N-substituted amino (-NHR"), -N,N-disubstituted amino (-N(R")R"), carboxyl (-COOH), -C(=O)R", -OR", -C(=O)OR", -C(=O)NHSO₂R", - NHC(=O)R", aminocarbonyl (-C(=O)NH₂), -N-substituted aminocarbonyl (-C(=O)NHR"), - N,N-disubstituted aminocarbonyl (-C(=O)N(R")R"), thiol, thiolato (SR"), sulfonic acid and its esters (SO₃R"), phosphonic acid and its mono-esters (P(=O)OR"OH) and di-esters (P(=O)OR"OR"), S(=O)₂R", S(=O)₂NH₂, S(=O)₂NHR", S(=O)₂NR"R", SO₂NHC(=O)R", NHS(=O)₂R", NR"S(=O)₂R", CF₃, CF₂CF₃, NHC(=O)NHR", NHC(=O)NR"R", NR"C(=O)NHR", NR"C(=O)NR"R", NR"C(=O)R", NR"C(=N-CN)NR"R", and the like. Aryl substituents may also include (CH₂)ₚSO₂NR"(CH₂)_{q} and (CH₂)ₚCO₂NR"(CH₂)_{q}, where p and q are independently integers from 0 to 3, where the methylene units are attached in a 1,2 arrangement yielding substituted aryls of the type:

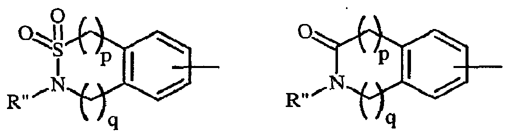

In relation to the aforementioned substituents, each moiety R" can be, independently, any of H, alkyl, cycloalkyl, alkenyl, aryl, aralkyl, heteroaryl, or heterocycloalkyl, or when (R"(R")) is attached to a nitrogen atom, R" and R" can be taken together to form a 4- to 8-membered nitrogen heterocycloalkyl ring, wherein said heterocycloalkyl ring is optionally interrupted by one or more additional -O-, -S-, -SO, -SO₂, -NH-, -N(alkyl)-, or -N(aryl)- groups, for example.

As used herein, an "*" denotes the presence of a non-racemic stereoisomeric center in a molecule, wherein one stereoisomeric form (R or S) predominates, but for which the absolute configuration at this center has not been conclusively established. This is equivalently expressed by saying that the molecule's configuration at the asterisked carbon atom is either greater than 50% R or greater than 50% S. More preferably the compound, or its stereoisomeric center, is "substantially enriched", and even more preferably is substantially enantiomerically pure.

As used herein, the term "substantially enriched", when referring to a stereoisomer or stereoisomeric center, denotes that at least about 60%, preferably about 705, more preferably about 805, still more preferably about 90% of one stereoisomer or one stereoisomeric center predominating in the mixture, with at least about 95% of one stereoisomer or one stereoisomeric center being even more preferred. In some preferred embodiments, the compound is "substantially enantiomerically pure", that is, at least about 97.5%, more preferably about 99%, even more preferably about 99.5% of one stereoisomeric forms predominates. For example, a compound having one stereoisomeric center may be represented by one of two stereoisomeric forms (R or S), differing only in the spatial arrangement of atoms about a single carbon atom. The "*" denotes non-equal amounts of the two isomers. When a compound has two or more stereoisomeric centers, each center denoted by an asterisk is evaluated individually. A predominance of one stereoisomeric form (R or S) occurring at least one center is considered non-racemic within the definition herein provided. The range of possible non-racemic compounds extends from the point at which a stereoisomeric form predominates at a single chiral center and includes all combinations and subcombinations up to and including the compound wherein all stereoisomeric centers in the compound are each individually R or S.

Use of the "*" can be expressed, for example in a compound's identification number such as 4*, and indicates that the stereochemical configuration of at least one chiral center of the identified compound has not been established. The specific center is identified within a structure by placing the "*" adjacent the chiral center in question, such as, for example, in the structure below.

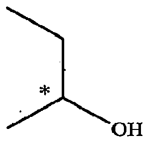

In some compounds, several chiral centers may be present. The presence of two asterisks "*" in a single structure indicates that two racemic pairs may be present, but that each pair is diastereomeric relative to the other pair. As such, the first pair of enantiomers having two chiral centers may have the configurations, for example, (R, R) and (S, S). The second pair then have configurations, for example, (R, S) and (S, R). Alternatively, where only two stereoisomers bearing an enantiomeric relationship to each other are initially provided, such as the (R, R) and (S,S) pair, the asterisks may indicate that the enantiomers have been enriched (partially resolved) or preferably fully resolved, into the individual enantiomers.

"Ligand" or "modulator" refers to a compound that binds to a receptor to form a complex, and includes, agonists, partial agonists, antagonists and inverse agonists.

"Agonist" refers to a compound that may bind to a receptor to form a complex that may elicit a full pharmacological response, which is typically peculiar to the nature of the receptor involved and which may alter the equilibrium between inactive and active receptor.

"Partial agonist" refers to a compound that may bind to a receptor to form a complex that may elicit only a proportion of the full pharmacological response, typically peculiar to the nature of the receptor involved, even if a high proportion of the receptors are occupied by the compound.

"Antagonist" refers to a compound that may bind to a receptor to form a complex that may not elicit any response, typically in the same manner as an unoccupied receptor, and which preferably does not alter the equilibrium between inactive and active receptor.

"Inverse agonist" refers to a compound that may bind to a receptor to form a complex that may preferentially stabilize the inactive conformation of the receptor.

"Prodrug" refers to compounds specifically designed to maximize the amount of active species that reaches the desired site of reaction that are themselves typically inactive or minimally active for the activity desired, but through biotransformation are converted into biologically active metabolites.

"Stereoisomers" refers to compounds that have identical chemical constitution, but differ as regards the arrangement of the atoms or groups in space.

"N-oxide" refers to compounds wherein the basic nitrogen atom of either a heteroaromatic ring or tertiary amine is oxidized to give a quaternary nitrogen bearing a positive formal charge and an attached oxygen atom bearing a negative formal charge.

"Hydrate" refers to a compound as described herein which is associated with water in the molecular form, i.e., in which the H-OH bond is not split, and may be represented, for example, by the formula R-H₂O, where R is a compound as described herein. A given compound may form more than one hydrate including, for example, monohydrates (R-H₂O), dihydrates (R-2H₂O), trihydrates (R-3H₂O), and the like.

As used herein, "haloalkoxy" refers to an alkoxy group, wherein one, preferably two or more, hydrogen(s) of the alkyl moiety of said alkoxy are replaced by halo atoms, and alkoxy, alkyl, and halo are each as previously defined.

As used herein, "solvate" refers to a compound of the present invention that is associated with solvent in the molecular form, *i.e*., in which the solvent is coordinatively bound, and may be represented, for example, by the formula R-(solvent), where R is a compound of the invention. A given compound may form more than one solvate including, for example, monosolvates (R·(solvent)) or polysolvates (R·n(solvent)) wherein n is an integer > 1) including, for example, disolvates (R·2(solvent)), trisolvates (R·3(solvent)), and the like, or hemisolvates, such as, for example, R·n/2(solvent), R·n/3(solvent), R·n/4(solvent) and the like wherein n is an integer. Solvents herein include mixed solvents, for example, methanol/water, and as such, the solvates may incorporate one or more solvents within the solvate.

As used herein, "acid hydrate" refers to a complex that may be formed through association of a compound having one or more base moieties with at least one compound having one or more acid moieties or through association of a compound having one or more acid moieties with at least one compound having one or more base moieties, said complex being further associated with water molecules so as to form a hydrate, wherein said hydrate is as previously defined and R represents the complex herein described above.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. These physiologically acceptable salts are prepared by methods known in the art, *e.g*., by dissolving the free amine bases with an excess of the acid in aqueous alcohol, or neutralizing a free carboxylic acid with an alkali metal base such as a hydroxide, or with an amine.

Compounds described herein throughout can be used or prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

Certain acidic or basic compounds as described herein may exist as zwitterions. All forms of the compounds, including free acid, free base and zwitterions, are contemplated to be within the scope of the present invention. It is well known in the art that compounds containing both basic nitrogen atom and acidic groups often exist in equilibrium with their zwitterionic forms. Thus, any of the compounds described herein throughout that contain, for example, both basic nitrogen and acidic groups, also include reference to their corresponding zwitterions.

"Effective amount" refers to an amount of a compound as described herein that may be therapeutically effective to inhibit, prevent, or treat the symptoms of particular disease, disorder, condition, or side effect or enhance a desired condition or effect (such as enhanced cognitive function in a healthy patient). Such diseases, disorders, conditions, and side effects include, but are not limited to, those pathological conditions associated with cognitive dysfunction, such as Alzheimer's Disease, mild cognitive impairment, age-related cognitive decline, vascular dementia, Parkinson's Disease dementia, amyotrophic lateral sclerosis, Huntington's Disease, stroke, traumatic brain injury, AIDS-associated dementia, schizophrenia, Lewy-body variant of Alzheimer's Disease with or without association with Parkinson's Disease, Creutzfeld-Jakob Disease, Korsakoff's Disorder, and combinations thereof. The term "effective amount," when used in connection with compounds active against cognitive dysfunction, refers to the treatment and/or prevention of symptoms, diseases, disorders, and conditions typically associated with cognitive dysfunction.

As used herein, "pharmaceutically acceptable" refers to those compounds, materials, compositions, and/or dosage forms that are, within the scope of sound medical judgment, suitable for contact with the tissues of human beings and animals without excessive toxicity, irritation, allergic response, or other problems or complications commensurate with a reasonable benefit/risk ratio. The term specifically encompasses veterinary uses.

As used herein, "in combination with," "combination therapy," and "combination products" refer, in certain embodiments, to the concurrent administration to a patient of a compound as described herein including, for example, a compound of formula IV and VI to XXXIV, and one or more additional agents including, for example, an opioid, an anaesthetic agent (such as for example, an inhaled anesthetic, hypnotic, anxiolytic, neuromuscular blocker and opioid), an anti-Parkinson's agent (for example, in the case of treating or preventing a motor disorder, particularly Parkinson's disease), an antidepressant (for example, in the case of treating or preventing a mood disorder, particularly depression), an agent for the treatment of incontinence (for example, in the case of treating or preventing a urogenital tract disorder), an agent for the treatment of pain, including neuralgias or neuropathic pain, and/or other optional ingredients (including, for example, antibiotics, antivirals, antifungals, anti-inflammatories, anesthetics and mixtures thereof). When administered in combination, each component may be administered at the same time or sequentially in any order at different points in time. Thus, each component may be administered separately but sufficiently closely in time so as to provide the desired therapeutic effect.

As used herein, "dosage unit" refers to physically discrete units suited as unitary dosages for the particular individual to be treated. Each unit may contain a predetermined quantity of active compound(s) calculated to produce the desired therapeutic effect(s) in association with the required pharmaceutical carrier. The specification for the dosage unit forms of the invention may be dictated by (a) the unique characteristics of the active compound(s) and the particular therapeutic effect(s) to be achieved, and (b) the limitations inherent in the art of compounding such active compound(s).

"Stroke" refers to a condition due to the lack of oxygen to the brain.

"Anaesthetic state" refers to the state of the loss of feeling or sensation, including not only the loss of tactile sensibility or of any of the other senses, but also to the loss of sensation of pain, as it is induced to permit performance of surgery or other painful procedures, and specifically including amnesia, analgesia, muscle relaxation and sedation.

"Improving organ and cell survival" refers to the maintenance and/or improvement of a minimally-acceptable level of organ or cell survival.

"Patient" refers to animals, including mammals, preferably humans.

"Side effect" refers to a consequence other than the one(s) for which an agent or measure is used, as the adverse effects produced by a drug, especially on a tissue or organ system other then the one sought to be benefited by its administration. In the case, for example, of opioids, the term "side effect" may refer to such conditions as, for example, constipation, nausea, vomiting, dyspnea and pruritus.

When any variable occurs more than one time in any constituent or in any formula, its definition in each occurrence is independent of its definition at every other occurrence. Combinations of substituents and/or variables are permissible only if such combinations result in stable compounds.

It is believed the chemical formulas and names used herein correctly and accurately reflect the underlying chemical compounds. However, the nature and value of the present invention does not depend upon the theoretical correctness of these formulae, in whole or in part. Thus it is understood that the formulas used herein, as well as the chemical names attributed to the correspondingly indicated compounds, are not intended to limit the invention in any way, including restricting it to any specific tautomeric form or to any specific optical or geometric isomer, except where such stereochemistry is clearly defined.

In certain preferred embodiments, the compounds, pharmaceutical compositions and methods of the present invention may involve a peripheral δ opioid modulator compound. The term "peripheral" designates that the compound acts primarily on physiological systems and components external to the central nervous system. In preferred form, the peripheral δ opioid modulator compounds employed in the methods of the present invention exhibit high levels of activity with respect to peripheral tissue, such as, gastrointestinal tissue, while exhibiting reduced, and preferably substantially no, CNS activity. The phrase "substantially no CNS activity," as used herein, means that less than about 50% of the pharmacological activity of the compounds employed in the present methods is exhibited in the CNS, preferably less than about 25%, more preferably less than about 10%, even more preferably less than about 5% and most preferably 0% of the pharmacological activity of the compounds employed in the present methods is exhibited in the CNS.

Furthermore, it is preferred in certain embodiments of the invention that the δ opioid modulator compound does not substantially cross the blood-brain barrier. The phrase "does not substantially cross," as used herein, means that less than about 20% by weight of the compound employed in the present methods crosses the blood-brain barrier, preferably less than about 15% by weight, more preferably less than about 10% by weight, even more preferably less than about 5% by weight and most preferably 0% by weight of the compound crosses the blood-brain barrier. Selected compounds can be evaluated for CNS penetration, for example, by determining plasma and brain levels following i.v. administration.

Accordingly, in one embodiment, the invention is directed to methods for enhancing cognitive function, comprising the step of:
administering to a patient in need thereof an effective amount of a compound of formula IV: 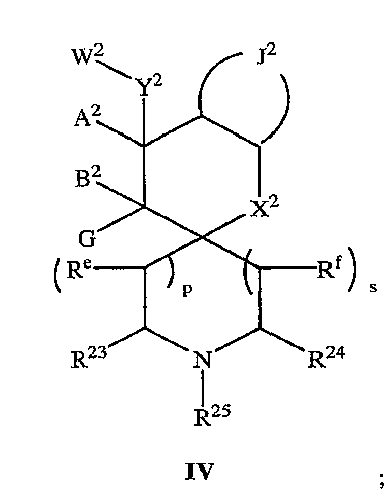 wherein:
   Y² is a single bond or -[C(R^{c})(R^{d})]ₖ-;
   each R^{c}, R^{d}, R^{e}, and R^{f} is independently H or alkyl;
   W² is aryl, alkaryl, heterocycloalkylaryl, heteroaryl, alkylheteroaryl, heteroarylaryl, or alkylheteroarylaryl;
   R²³ and R²⁴ are each independently H or alkyl;
   R²⁵ is H, alkyl, -(CH₂)-alkenyl, -(CH₂)-alkynyl, cycloalkyl, alkylcycloalkyl, aralkyl, or heteroarylalkyl, or R²³ and R²⁵ when taken together with the atoms through which they are connected, form a 4- to 8-membered heterocycloalkyl ring, or R²⁴ and R²⁵ when taken together with the atoms through which they are connected, form a 4- to 8-membered heterocycloalkyl ring;
   each k is independently 1, 2, or 3;
   p is 0, 1, 2 or 3;
   s is 0, 1, 2 or 3, provided that the sum of p and s is ≤ 4;
   A² and B² are each independently H or alkyl, or together form a double bond;
   G is H or alkyl;
   X² is -C(R^{c})(R^{d})-, -O-, or -S-;
   R²⁶ is H, alkyl, cycloalkyl, -(CH₂)-alkenyl, -(CH₂)-alkynyl, aryl, -C(=O)R^{d}, or -S(=O)₂R^{d}; and
   J² forms a 6- to 10-membered aryl when taken together with the carbon atoms to which it is attached;
or a stereoisomer, prodrug, pharmaceutically acceptable salt, hydrate, solvate, acid salt hydrate, or N-oxide thereof.
In certain embodiments of the method of the invention, when
(a) J² taken together with the carbon atoms to which it is attached forms a 6- to 10-membered aryl ring substituted with 0-3 groups selected from the group consisting of:
   halogen,
   hydroxy,
   -SH,
   -C(=O)-H
   -S-C₁₋₄alkyl,
   -NHS(=O)₂-C₁₋₄alkyl,
   -NHS(=O)₂-H,
   -N(C₁₋₄ alkyl)S(=O)₂-H,
   C₁₋₄alkyl, and
   C₁₋₄ alkoxy, the latter two optionally substituted with one or more halogens or with C₁₋₄ alkoxy;
   W² is phenyl substituted with 0-3 groups selected from the group consisting of:
      halogen,
      cyano,
      hydroxy,
      C₁₋₆ alkyl optionally substituted with one or more halogens,
      C₁₋₆ alkoxy optionally substituted with one or more halogens or with C₃₋₆ cycloalkyl,
      C₂₋₆ alkenyloxy,
      C₂₋₆ alkynyloxy,
      C₃₋₆ cycloalkyloxy,
      C₆₋₁₂ aryloxy,
      aralkoxy,
      heteroaryloxy,
      heteroaralkoxy,
      heterocycloalkyl substituted with alkoxy,
      -SH,
      -S-C₁₋₄ alkyl,
      -NH₂,
      -N=C(ary)₂,
      -N(H)C₁₋₄ alkyl,
      -N(C₁₋₄ alkyl)₂,
      -OS(=O)₂-C₁₋₄ alkyl optionally substituted with one or more halogens,
      -OS(=O)₂-C₆₋₁₂ aryl optionally substituted with C₁₋₄ alkyl,
      -NHS(=O)₂-C₁₋₄ alkyl,
      -N(C₁₋₄ alkyl)S(=O)₂-C₁₋₄ alkyl,
      -NHS(=O)₂-H, and
      -N(C₁₋₄ alkyl)S(=O)₂-H;
   p and s are each 1,
   R^{e}, R^{f}, R²³, R²⁴, and G are each H,
   A² and B² together form a double bond,
   Y² is a single bond; and
   X² is -O-;
   then R²⁵ is other than: 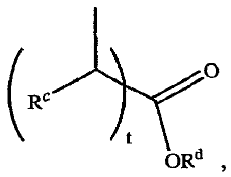 wherein t is an integer from 1 to 20; and
(b) J² taken together with the carbon atoms to which it is attached forms a phenyl ring substituted with 0-3 groups selected from the group consisting of:
   halogen,
   hydroxy,
   -S-C₁₋₄alkyl,
   C₁₋₄ alkyl, and
      C₁₋₄ alkoxy, the latter two optionally substituted with one or more halogens or with C₁₋₄ alkoxy;
      W² is unsubstituted naphthyl, or phenyl substituted with 0-3 groups selected from the group consisting of:
   halogen,
   C₁₋₆ alkyl,
      C₁₋₆ alkoxy,
      phenyl,
   phenoxy,
   1,3-benzodioxazolyl, or 2,2-difluoro-1,3-benzodioxazolyl fluoro,
   -NH₂,
   -N(C₁₋₄ alkyl)₂, and
   pyrrolyl;
   p and s are each 1,
   R^{e}, R^{f}, R²³, R²⁴, and G are each H,
   A² and B² together form a double bond,
   Y² is a single bond; and
   X² is -O-;
   then R²⁵ is other than H or methyl; and
   provided that when:
(c) J² taken together with the carbon atoms to which it is attached forms unsubstituted phenyl,
   W² is phenyl substituted with 0-3 groups selected from the group consisting of:
      fluoro,
      hydroxy,
      C₁₋₆ alkoxy optionally substituted with one or more fluoro, C₂₋₆ alkenyloxy, and
      -S-C₁₋₄ alkyl,
   p and s are each 1,
   R^{e}, R^{f}, R²³, R²⁴, and G are each H,
   A² and B² together form a double bond,
   Y² is a single bond; and
   X² is -O-;
   then R²⁵ is other than H or benzyl.

In certain preferred embodiments of compounds of formula IV, Y² is a single bond.

In certain preferred embodiments of compounds of formula IV, X² is -CH₂- or -O-, and, more preferably, X² is -O-.

In certain preferred embodiments of compounds of formula IV, W² is aryl, alkaryl, heteroaryl, alkylheteroaryl, heteroarylaryl, or alkylheteroarylaryl, more preferably, W² is aryl, alkaryl, heteroaryl, alkylheteroaryl, heteroarylaryl, or alkylheteroarylaryl. In certain embodiments of compounds of formula IV, said aryl, alkaryl, heteroaryl, alkylheteroaryl, heteroarylaryl, or alkylheteroarylaryl in W² is optionally substituted with at least one of alkyl, aryl, hydroxyl, carboxyl, *N,N-*dialkylaminocarbonyl, -S(=O)₂-N(alkyl)₂, -N(H)S(=O)₂-alkyl, and -N(alkyl)C(=O)-alkyl. In certain preferred embodiments of compounds of formula IV, W² is: 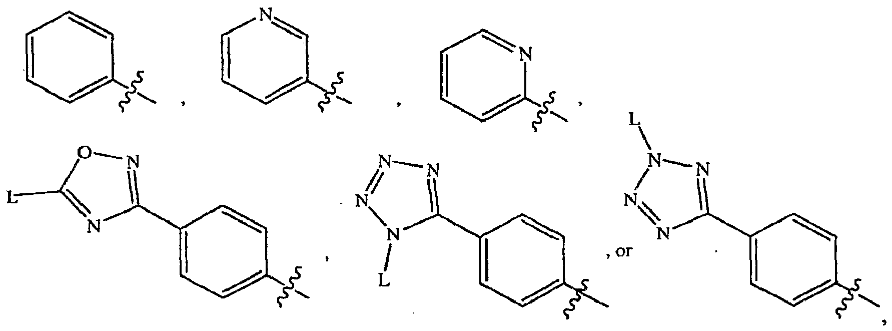 wherein W² is optionally substituted with at least one of alkyl, aryl, hydroxyl, carboxyl, *N,N* dialkylaminocarbonyl, -S(=O)₂-N(alkyl -N(H)S(=O)₂-alky and -N(alkyl)C(=O)-alkyl; and L is H or alkyl. In certain embodiments of compounds of formula IV, W² is: 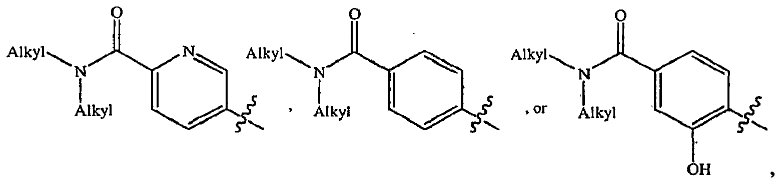 preferably, W² is:

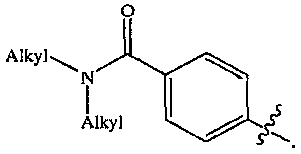

In certain preferred embodiments of compounds of formula IV, R²³ and R²⁴ are each independently H or C₁-C₃ alkyl. In certain preferred embodiments of compounds of formula IV, at least one of R²³ and R²⁴ is H, preferably, R²³ and R²⁴ are each H.

In certain preferred embodiments of compounds of formula IV, R²⁵ is H, alkyl, - (CH₂)-alkenyl, -(CH₂)-alkynyl, cycloalkyl, alkylcycloalkyl, aralkyl, or heteroarylalkyl, preferably, R²⁵ is H or alkyl.

In certain preferred embodiments of compounds of formula IV, R^{c}, R^{d}, R^{e} and R^{f} are each independently H or C₁-C₃ alkyl, preferably, R^{c}, R^{d}, R^{e} and R^{f} are each independently H or methyl, more preferably, at least one of R^{c}, R^{d}, R^{e} and R^{f} is H, and even more preferably, R^{c}, R^{d}, R^{e} and R^{f} are each H.

In certain preferred embodiments of compounds of formula IV, p is 1 or 2, preferably, p is 1.

In certain preferred embodiments of compounds of formula IV, s is 1.

In certain preferred embodiments of compounds of formula IV, J² forms a 6- to 10-membered aryl ring when taken together with the carbon atoms to which it is attached, preferably, J² forms a 6-membered aryl ring when taken together with the carbon atoms to which it is attached.

In certain preferred embodiments of compounds of formula IV, G is H.

In certain preferred embodiments of compounds of formula IV, A² and B² are each independently H or alkyl, preferably, A² and B² are each H.

In certain preferred embodiments of compounds of formula IV, A² and B² taken together form a double bond between the carbon atoms to which they are attached.

In certain preferred embodiments, the compound of formula IV is a compound of formula VI: 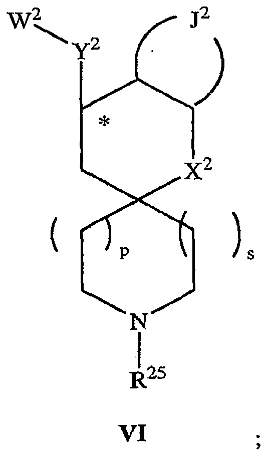 or a stereoisomer, prodrug, pharmaceutically acceptable salt, hydrate, solvate, acid salt hydrate, or N-oxide thereof;
wherein:
p is 0, 1, or 2;
s is 0, 1, or 2; and
X² is O or CH₂.

In certain preferred embodiments, the compound of formula IV is a compound of formula VH: 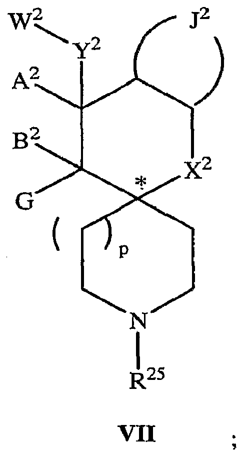 or a stereoisomer, prodrug, pharmaceutically acceptable salt, hydrate, solvate, acid salt hydrate, or N-oxide thereof;
wherein:
p is 0 or 2; and
X² is O or CH₂.

In certain preferred embodiments, the compound of formula IV is a compound of formula VIII: 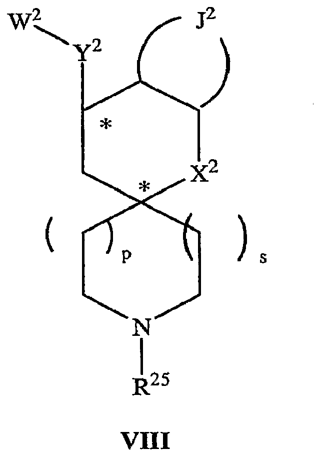 or a stereoisomer, prodrug, pharmaceutically acceptable salt, hydrate, solvate, acid salt hydrate, or N-oxide thereof;
wherein:
p is 0 or 2; and
X² is O or CH₂.

In certain preferred embodiments, the compound of formula IV is a compound of formula IX: 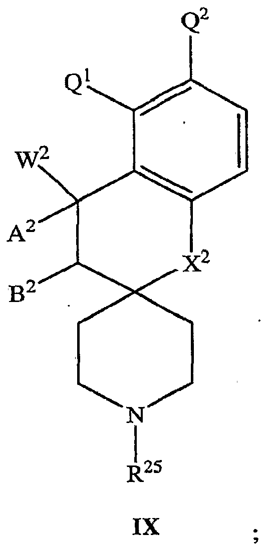 or a stereoisomer, prodrug, pharmaceutically acceptable salt, hydrate, solvate, acid salt hydrate, or N-oxide thereof;
wherein:
X² is O or CH₂;
Q¹ and Q² is H, alkyl, alkoxy, OCF₂H, OCF₃, hydroxy, chloro, fluoro, SO₂-alkyl, SO₂NR^{g}R^{h}, where at least one of Q¹ or Q² is H;
R²⁵ is H or methyl;
A² and B² are H or taken together to form a double bond;
W² is phenyl or pyridinyl ring, each substituted with 1 to 3 substitutions selected from the group consisting of alkyl, alkoxy, carboxy, -COO-alkyl, -CONR^{g}R^{h}, SO₂NR^{g}R^{h}, NRⁱSO₂R^{j};
R^{g} and R^{h} are H or alkyl, or taken together with the nitrogen to which they are attached form a 4 to 7 membered heterocyclalkyl; and
Rⁱ and R^{j} are H or alkyl.

In certain preferred embodiments; the compound of formula IV is a compound of formula X: 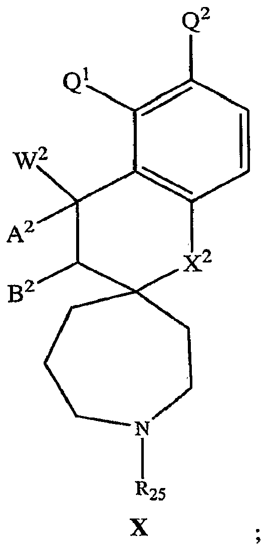 or a stereoisomer, prodrug, pharmaceutically acceptable salt, hydrate, solvate, acid salt hydrate, or N-oxide thereof;
wherein:
X² is O or CH₂;
Q¹ and Q² is H, alkyl, alkoxy, OCF₂H, OCF₃, hydroxy, chloro, fluoro, SO₂-alkyl, SO₂NR^{g}R^{h}, where at least one of Q¹ or Q² is H;
R²⁵ is H or methyl;
A² and B² are H or taken together to form a double bond;
W² is phenyl or pyridinyl ring, each substituted with 1 to 3 substitutions selected from the group consisting of alkyl, alkoxy, carboxy, -COO-alkyl, -CONR^{g}R^{h}, SO₂NR^{g}R^{h}, NRⁱSO₂R^{j};
R^{g} and R^{h} are H or alkyl, or taken together with the nitrogen to which they are attached form a 4 to 7 membered heterocyclalkyl; and
Rⁱ and R^{j} are H or alkyl.

In certain preferred embodiments, the compound of formula IV is a compound of formula XI:

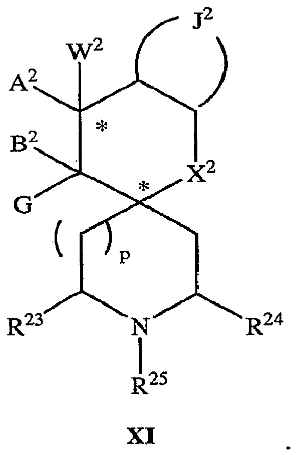

In certain preferred embodiments, the compound of formula IV is a compound of formula XII: 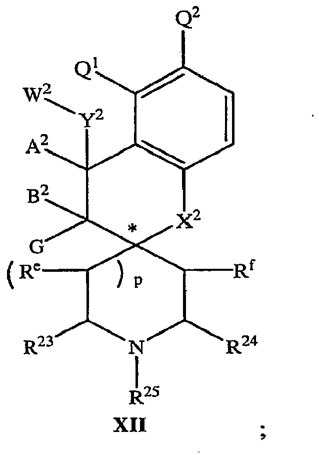 wherein:
Q¹ and Q² are each independently H, halo, alkyl, hydroxyl, alkoxyl, cycloalkyl substituted alkoxyl, aminocarbonyl, -S(=O)₂-alkyl, -S(=O)₂-N(H)alkyl, -S(=O)₂-N(H)cycloalkylalkyl, or -N(H)S(=O)₂-alkyl.

In certain preferred embodiments, the compound of formula IV is a compound of formula XIII: 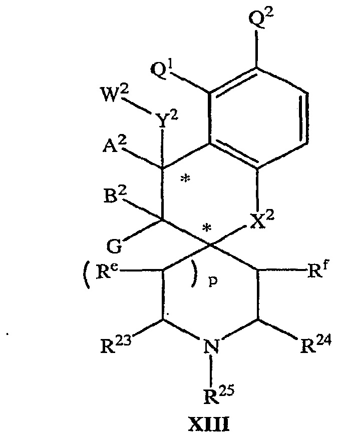 wherein:
A² and B² are each independently H or alkyl.

In certain preferred methods, the compounds of formula IV are compounds of formula XIV: 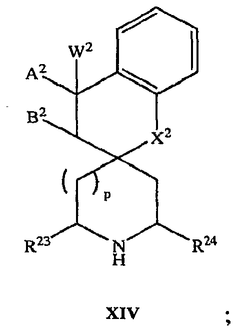 wherein:
W² is aryl or heteroaryl, wherein the aryl or heteroaryl is substituted with 0-3 groups selected independently from hydroxy, aminocarbonyl (-C(=O)₋NH₂), N-alkylaminocarbonyl (-C(=O)-NH(alkyl)), and N,N-dialkylaminocarbonyl (-C(=O)-N(alkyl)(alkyl));
R²³ and R²⁴ are each independently H or alkyl, provided that at least one of R²³ and R²⁴ is alkyl;
p is 1 or 2;
A² and B² are each H, or together form a double bond; and
X² is -CH₂- or -O-;
or a stereoisomer, prodrug, pharmaceutically acceptable salt, hydrate, solvate, acid salt hydrate, or N-oxide thereof.

In preferred embodiments of formula XIV compounds, W² is aryl or heteroaryl. When W² is aryl, the aryl ring is preferably phenyl. When W² is heteroaryl, the heteroaryl ring is preferably pyridyl.

As set forth above, W² is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). In preferred embodiments, W² is substituted with 1-2 groups, selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). More preferably, W² is substituted with N,N-dialkylaminocarbonyl and/or hydroxyl.

In preferred embodiments of formula XIV compounds, W² is: 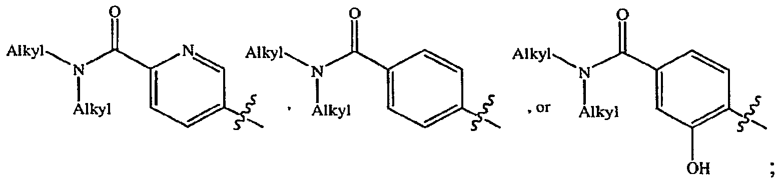 more preferably:

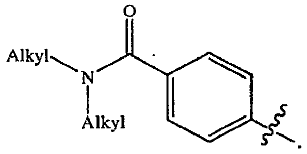

In embodiments in which W² is substituted with N-alkylaminocarbonyl (-C(=O)-NH(alkyl)) or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XIV compounds, p is 1.

In preferred embodiments of formula XIV compounds, A² and B² together form a double bond.

In preferred embodiments of formula XIV compounds, X² is -O-.

In preferred embodiments of formula XIV compounds, R²³ and R²⁴ are each independently H or alkyl, preferably H or C₁-C₃ alkyl, more preferably H or methyl. In certain preferred embodiments, one of R²³ and R²⁴ is H and the other is alkyl.

In preferred embodiments, the compounds of formula XIV have the following formula XV: 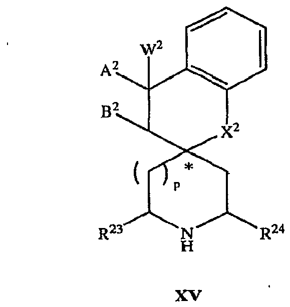 with compounds of formula XVI being even more preferred:

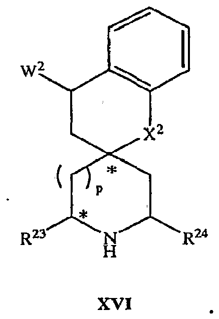

In preferred embodiments, the compound of formula XIV is: 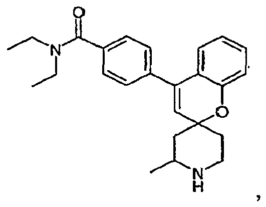 more preferably:

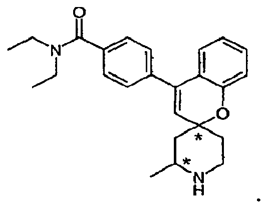

In an alternative embodiment, the invention is directed to compounds of formula XVII: 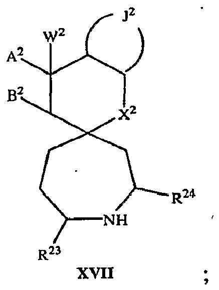 wherein:
W² is aryl or heteroaryl, wherein the aryl or heteroaryl is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl));
R²³ and R²⁴ are each independently H or alkyl;
A² and B² are each H, or together form a double bond;
X² is -CH₂- or -O-; and
J² when taken together with the carbon atoms to which it is attached forms a 6-membered aryl ring substituted with 0-3 groups selected independently from halo, hydroxy, and -S(=O)₂-alkyl;
or a stereoisomer, prodrug, pharmaceutically acceptable salt, hydrate, solvate, acid salt hydrate, or N-oxide thereof.
In certain preferred embodiments of compounds of formula XVII:
when W² is *para*-diethylaminocarbonylphenyl, X² is O, and A² and B² together form a double bond, then the aryl ring of J² is substituted with at least one group selected independently from halo, and -S(=O)₂-alk in which the alkyl group is C₂-C₆ alkyl;
when W² is *para*-diethylaminocarbonylphenyl, X² is O, and A² and B² are each H, then the aryl ring of J² is substituted with 1-3 groups selected independently from halo, hydroxy, and -S(=O)₂-alkyl; and
the compound of formula XVII is other than:

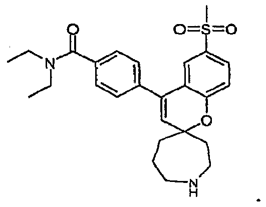

In preferred embodiments of compounds of formula XVII, W² is aryl or heteroaryl. When W² is aryl, the aryl ring is preferably phenyl. When W² is heteroaryl, the heteroaryl ring is preferably pyridyl. -

As set forth above, W² is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). In preferred embodiments, W² is substituted with 1-2 groups, selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). More preferably, W² is substituted with N,N-dialkylaminocarbonyl and/or hydroxyl.

In preferred embodiments of compounds of formula XVII, W² is:

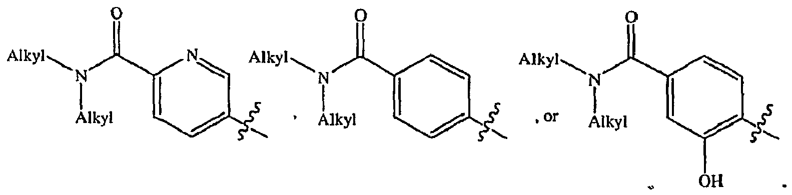

In embodiments in which W² is substituted with N-alkylaminocarbonyl (-C(=O)-NH(alkyl)) or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of compounds of formula XVII, X² is -O-.

In preferred embodiments of compounds of formula XVII, A² and B² together form a double bond.

In preferred embodiments of compounds of formula XVII, R²³ and R²⁴ are each independently H or alkyl, preferably H or C₁-C₃ alkyl, more preferably H or methyl, yet more preferably H.

In preferred embodiments of compounds of formula XVII, J² when taken together with the carbon atoms to which it is attached forms a 6-membered aryl ring, preferably phenyl.

As set forth above, J² is substituted with 0-3 groups, preferably 0-2, more preferably 0-1 groups, selected independently from halo, hydroxy, and -S(=O)₂-alkyl. In embodiments wherein J² is substituted with halo, the halo group is preferably fluoro. In embodiments wherein J² is substituted with -S(=O)₂-alkyl, the alkyl group is preferably lower alkyl, with alkyl groups of 1 to 3 carbons being more preferred, still more preferably alkyl groups of 1 to 2 carbons, yet more preferably methyl or ethyl.

In preferred embodiments, the compounds of formula XVII have the following formula XVIII: 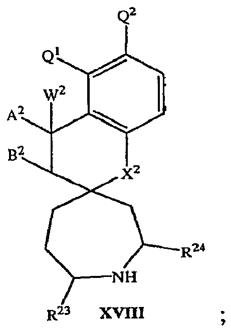 wherein:
Q¹ and Q² are each independently H, halo, hydroxy, or -S(=O)₂-alkyl. In embodiments wherein Q¹ or Q² is halo, the halo group is preferably fluoro.
In embodiments wherein Q¹ or Q² is-S(=O)₂-alkyl, the alkyl group is preferably lower alkyl, with alkyl groups of 1 to 3 carbons being more preferred, still more preferably alkyl groups of 1 to 2 carbons, yet more preferably methyl or ethyl. Still more preferably, the compounds of formula XVIII have the following formula XIX:

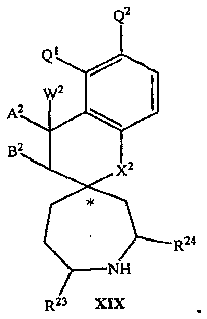

In preferred embodiments, the compounds of formula XVII have the following structures: 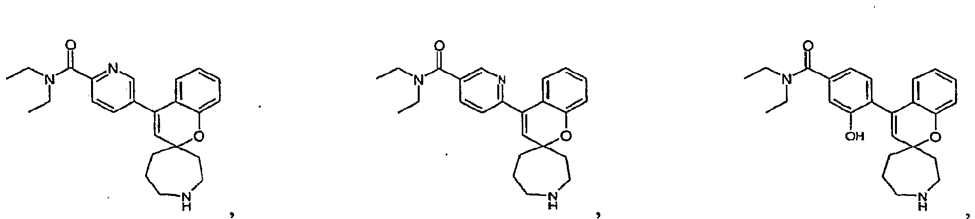 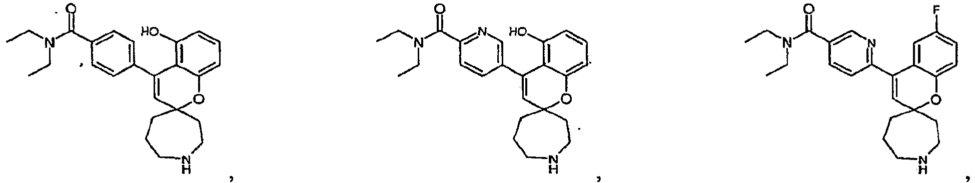 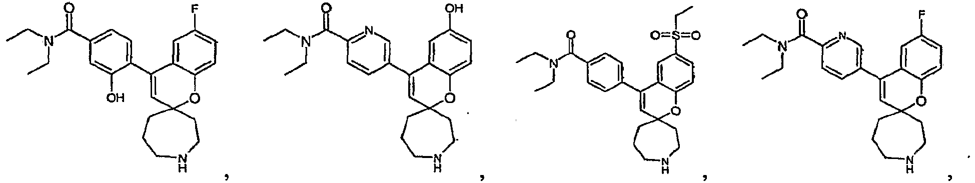 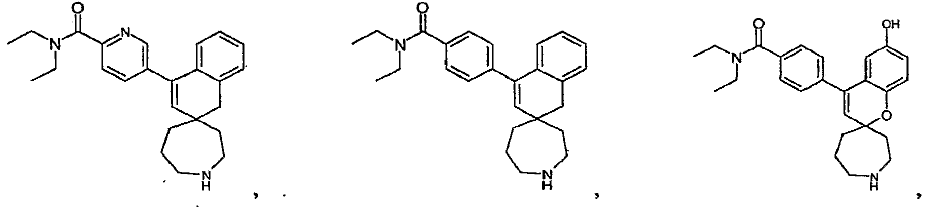 or

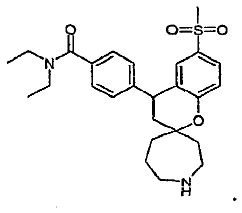

In certain embodiments, the above compounds may be resolved into any of their R and S, or (R,R), (S,S), (R,S), and (S,R) enantiomers, or partially resolved into any of their non-racemic mixtures.

More preferably, the compounds of formula XVII have the following structures: 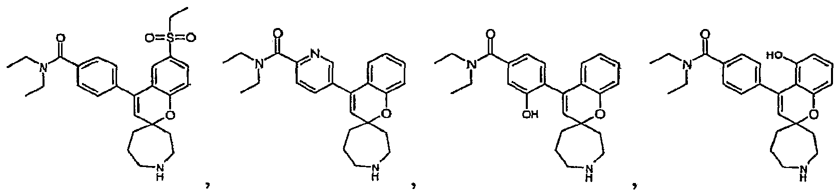 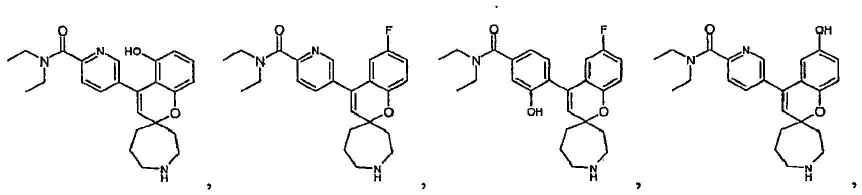 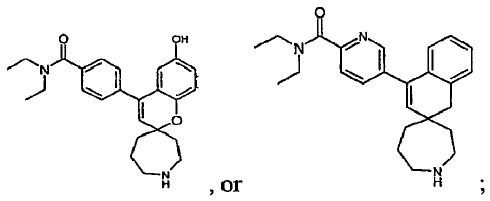 yet more preferably:

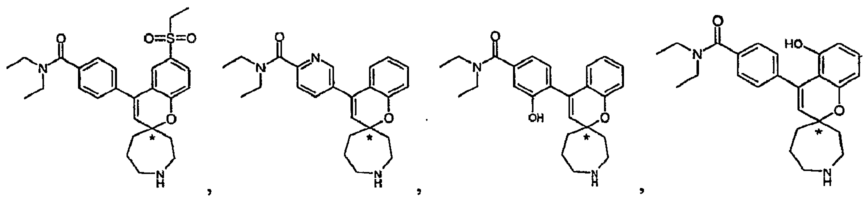

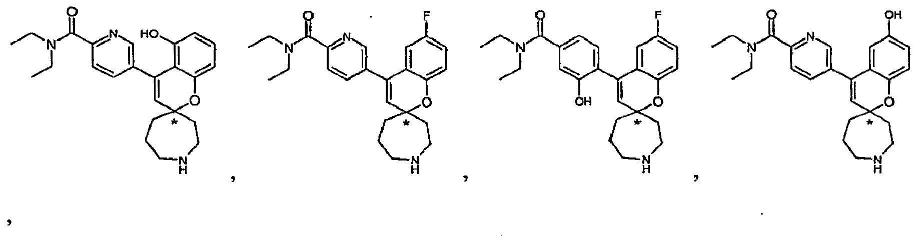

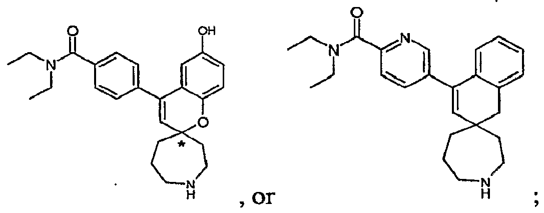

In certain embodiments, the invention is directed to compounds of formula XX: 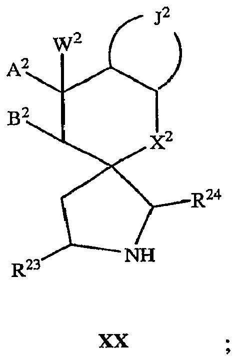 wherein:
W² is aryl or heteroaryl, wherein the aryl or heteroaryl is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl));
R²³ and R²⁴ are each independently H or alkyl;
A² and B² are each H, or together form a double bond;
X² is -CH₂- or -O-; and
J² when taken together with the carbon atoms to which it is attached forms a 6-membered aryl ring substituted independently with 0-3 hydroxy or halo groups;
or a stereoisomer, prodrug, pharmaceutically acceptable salt, hydrate, solvate, acid salt hydrate, or N-oxide thereof.
In preferred embodiments, the compound of formula XX is other than 4-[(4-N,N-diethylaminocarbonyl)phenyl]-spiro[2H,1-benzopyran-2,3'-pyrrolidine].

In preferred embodiments of compounds of formula XX, W² is aryl or heteroaryl. When W² is aryl, the aryl ring is preferably phenyl. When W² is heteroaryl, the heteroaryl ring is preferably pyridyl.

As set forth above, W² is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). In preferred embodiments, W² is substituted with 1-2 groups, selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). More preferably, W² is substituted with N,N-dialkylaminocarbonyl and/or hydroxyl.

In preferred embodiments of compounds of formula XX, W² is:

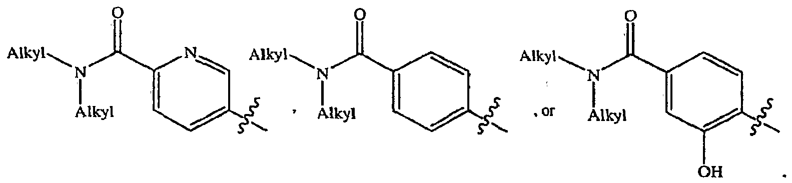

In embodiments in which W² is substituted with N-alkylaminocarbonyl (-C(=O)-NH(alkyl)) or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XX compounds, A² and B² together form a double bond.

In preferred embodiments of formula XX compounds, X² is -O-.

In preferred embodiments of formula XX compounds, R²³ and R²⁴ are each independently H or alkyl, preferably H or C₁-C₃ alkyl, more preferably H or methyl, yet more preferably H.

In preferred embodiments of formula XX compounds, J² when taken together with the carbon atoms to which it is attached forms a 6-membered aryl ring, preferably phenyl.

As set forth above, J² is substituted independently with 0-3, preferably 0-2, more preferably 0-1, hydroxy or halo groups, more preferably still, 0-1 hydroxy groups.

In preferred embodiments, the compounds of formula XX have the following formula XXI: 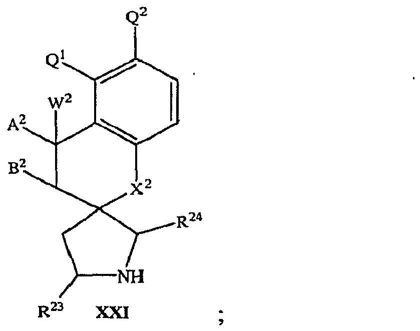 wherein:
Q¹ and Q² are each independently H, hydroxy, or halo.
In embodiments wherein at least one of Q¹ and Q² is halo, the halo is preferably fluoro.

In preferred embodiments, the compounds of formula XX have the following structures:

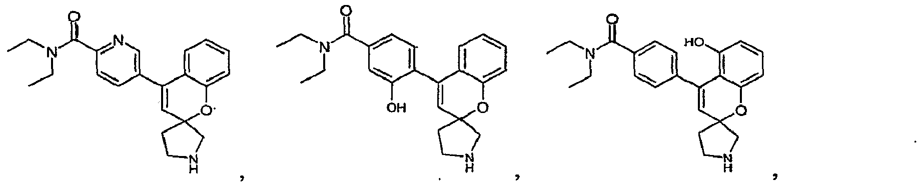

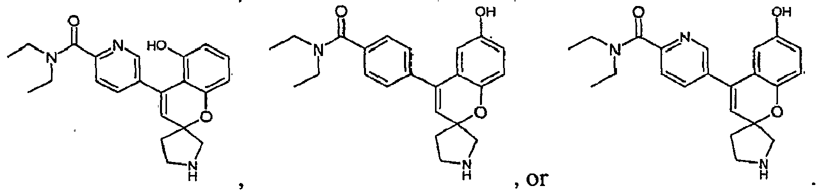

In an alternative embodiment, the invention is directed to compounds of formula XXII: 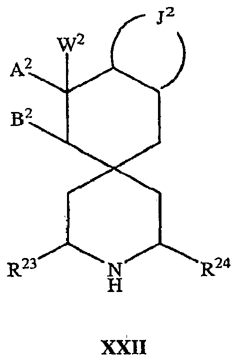 wherein:
W² is aryl or heteroaryl, wherein the aryl or heteroaryl is substituted with 0-3 groups selected independently from heteroaryl, hydroxy, carboxy (-COOH), -C(=O)-alkyl, -C(=O)-aryl, -C(=O)-O-alkyl, -S(=O)₂-N(alkyl)(alkyl); aminocarbonyl (-C(=O)-NH₂), N-alkylaminocarbonyl (-C(=O)-NH(alkyl)), and N,N-dialkylaminocarbonyl (-C(=O)-N(alkyl)(alkyl));
R²³ and R²⁴ are each independently H or alkyl;
A² and B² are each H, or together form a double bond; and
J² when taken together with the carbon atoms to which it is attached forms a 6-membered aryl ring substituted with 0-3 groups selected independently from halo, heterocycloalkyl, hydroxy, alkoxy, -S(=O)₂-alkyl, -S(=O)₂-NH₂, -S(=O)₂-NH(alkyl), -S(=O)₂-N(alkyl)(alkyl), carboxy (-COOH), -C(=O)-O-alkyl, and N,N-dialkylaminocarbonyl (-C(=O)-N(alkyl)(alkyl));
or a stereoisomer, prodrug, pharmaceutically acceptable salt, hydrate, solvate, acid salt hydrate, or N-oxide thereof.
In certain preferred embodiments of the compounds of formula XXII:
when W² is *para*-diethylaminocarbonylphenyl, *para*-prop-2-ylaminocarbonylphenyl, or *para*-pent-3-ylaminocarbonylphenyl, R²³ and R²⁴ are each H, and A and B are each H or together form a double bond, then J² is other than unsubstituted phenyl or anisyl; and
when W² is: 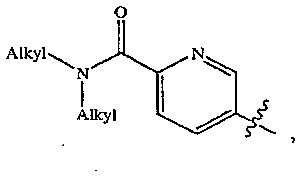
R²³ and R²⁴ are each H, and A and B together form a double bond; then J² is other than unsubstituted phenyl.

In preferred embodiments of formula XXII compounds, W² is aryl or heteroaryl. When W² is aryl, the aryl ring is preferably phenyl. When W² is heteroaryl, the heteroaryl ring is preferably pyridyl.

As set forth above, W² is substituted with 0-3 groups selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). In preferred embodiments, W² is substituted with 1-2 groups, selected independently from hydroxy, aminocarbonyl (-C(=O)-NH₂), N-alkylaminocarbonyl (-C(=O)-NH(alkyl)), and N,N-dialkylaminocarbonyl (-C(=O)-N(alkyl)(alkyl)). More preferably, W² is substituted with N,N-dialkylaminocarbonyl and/or hydroxyl.

In preferred embodiments of formula XXII compounds, W² is:

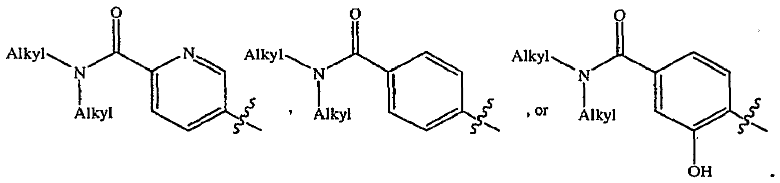

In embodiments in which W² is substituted with N-alkylaminocarbonyl (-C(=O)-NH(alkyl)) or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XXIX compounds, R²³ and R²⁴ are each independently H or alkyl, preferably H or C₁-C₃ alkyl, more preferably H or methyl, yet more preferably H.

In preferred embodiments of formula XXII compounds, J² when taken together with the carbon atoms to which it is attached forms a 6-membered aryl ring, preferably phenyl.

As set forth above, J² is substituted with 0-3 groups, preferably 0-2 groups, selected independently from halo, heterocycloalkyl, hydroxy, alkoxy, -S(=O)₂-alkyl, -S(=O)₂-NH₂, -S(=O)₂-NH(alkyl), -S(=O)₂-N(alkyl)(alkyl), carboxy (-COOH), -C(=O)-O-alkyl, and N,N-dialkylaminocarbonyl (-C(=O)-N(alkyl)(alkyl)). In embodiments wherein J² is substituted with alkoxy, -S(=O)₂-alkyl, -S(=O)₂-NH(alkyl), -S(=O)₂-N(alkyl)(alkyl), -C(=O)-O-alkyl, or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 1 to 2 carbons being still more preferred. In particularly preferred embodiments, the alkyl group is methyl or ethyl. In embodiments wherein J² is substituted with halo, the halo group is preferably fluoro.

In preferred embodiments of formula XXII compounds, the compounds have the following formula XXIII: 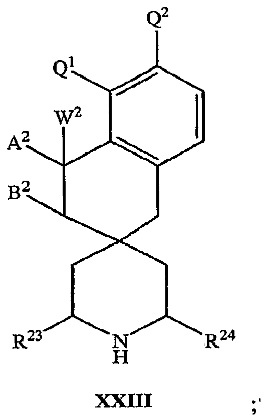 wherein:
Q¹ and Q² are each independently H, halo, heterocycloalkyl, hydroxy, alkoxy, -S(=O)₂-alkyl, -S(=O)₂-NH₂, -S(=O)₂-NH(alkyl), -S(=O)₂-N(alkyl)(alkyl), carboxy (-COOH), -C(=O)-O-alkyl, or N,N-dialkylaminocarbonyl (-C(=O)-N(alkyl)(alkyl)). In certain preferred embodiments, at least one of Q¹ or Q² is H, more preferably, one of Q¹ or Q² is H. In embodiments wherein Q¹ or Q² is alkoxy, -S(=O)₂-alkyl, -S(=O)₂-NH(alkyl), -S(=O)₂-N(alkyl)(alkyl), -C(=O)-O-alkyl, or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 1 to 2 carbons being still more preferred. In particularly preferred embodiments, the alkyl group is methyl or ethyl. In embodiments wherein Q¹ or Q² is halo, the halo group is preferably fluoro. In certain preferred embodiments, one of Q¹ or Q² is H.

In preferred embodiments, the compounds of formula XXII have the following formula XXIV:

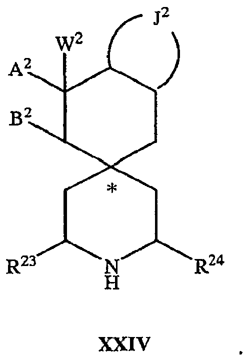

In preferred embodiments, the compounds of formula XXII have the following structure: 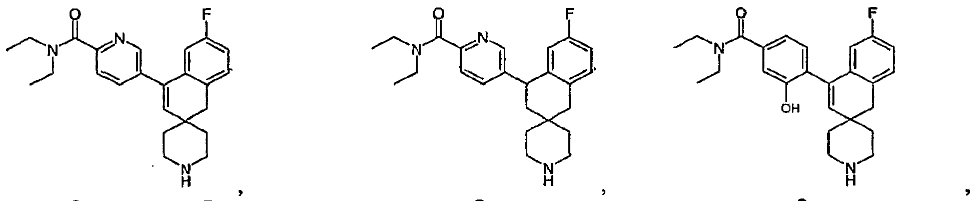 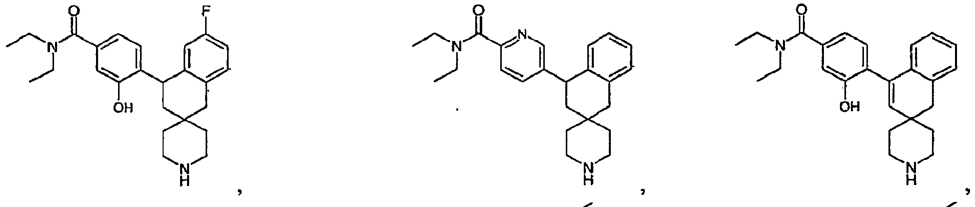 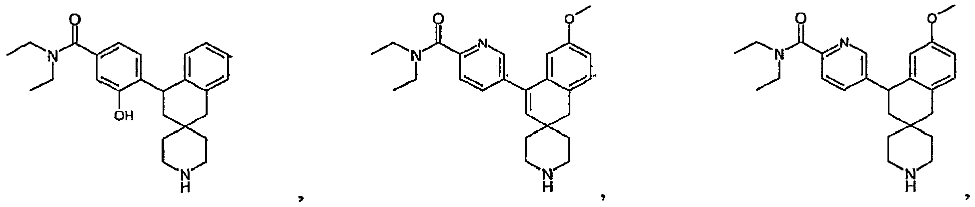 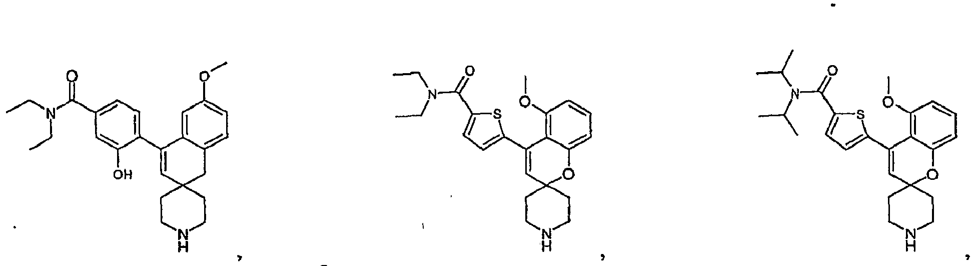 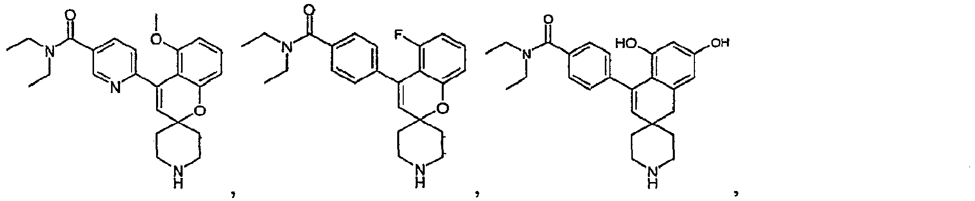 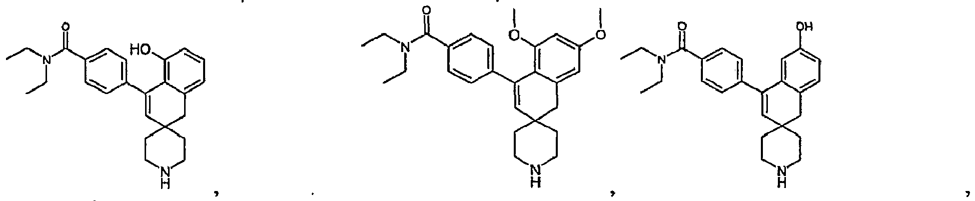 or

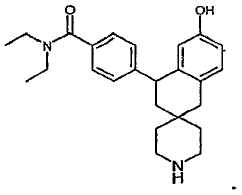

In certain embodiments, the above compounds may be resolved into any of their R and S, or (R,R), (S,S), (R,S), and (S,R) enantiomers, or partially resolved into any of their non-racemic mixtures.

In an alternative embodiment, the invention is directed to compounds of formula XXV: 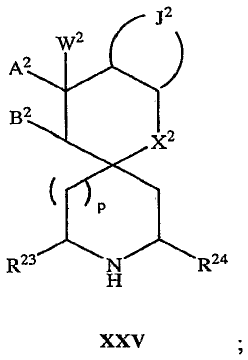 wherein:
W² is aryl optionally substituted with -C(=O)-alkyl or -C(=O)-aryl;
R²³ and R²⁴ are each independently H or alkyl;
p is 1 or 2;
A² and B² are each H, or together form a double bond;
X² is -CH₂- or -O-; and
J² when taken together with the carbon atoms to which it is attached forms a 6-membered aryl ring substituted with 0-3 groups selected independently from hydroxy, alkoxy, -S(=O)₂-alkyl, -S(=O)₂-NH₂, -S(=O)₂-NH(alkyl), -S(=O)₂-N(alkyl)(alkyl), -C(=O)-N(alkyl)(alkyl), carboxy (-COOH), and -C(=O)-O-alkyl;
or a stereoisomer, prodrug, pharmaceutically acceptable salt, hydrate, solvate, acid salt hydrate, or N-oxide thereof.
In certain preferred embodiments, the compound of formula XXV is other than 4-phenylspiro[2H,1-benzopyran-2,4'-piperidine].

In preferred embodiments of compounds of formula XXV, W² is aryl more preferably phenyl.

In embodiments wherein W² is substituted with -C(=O)-alkyl, the alkyl group is preferably lower alkyl, with alkyl groups of 1 to 3 carbons being more preferred, and with alkyl groups of 1 carbon being still more preferred. In particularly preferred embodiments, the alkyl group is methyl.

In embodiments wherein W² is substituted with or -C(=O)-aryl, preferably the aryl group is a 6-membered aryl ring, more preferably phenyl.

In preferred embodiments of formula XXV compounds, A² and B² together form a double bond.

In preferred embodiments of formula XXV compounds, X² is -O-.

In preferred embodiments of formula XXV compounds, p is 1.

In preferred embodiments of formula XXV compounds, R²³ and R²⁴ are each independently H or alkyl, preferably H or C₁-C₃ alkyl, more preferably H or methyl, yet more preferably H.

In preferred embodiments of formula XXV compounds, J² when taken together with the carbon atoms to which it is attached forms a 6-membered aryl ring, preferably phenyl.

As set forth above, J² is substituted with 0-3 groups, preferably 0-2, yet more preferably 0-1 groups, selected independently from halo, heterocycloalkyl, hydroxy, alkoxy, - S(=O)₂-alkyl, -S(=O)₂-NH₂, -S(=O)₂-NH(alkyl), -S(=O)₂-N(alkyl)(alkyl), carboxy (-COOH), -C(=O)-O-alkyl, and N,N-dialkylaminocarbonyl (-C(=O)-N(alkyl)(alkyl)). In embodiments wherein J² is substituted with alkoxy, -S(=O)₂-alkyl, -S(=O)₂-NH(alkyl), -S(=O)₂-N(alkyl)(alkyl), -C(=O)-O-alkyl, or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 1 to 2 carbons being still more preferred. In particularly preferred embodiments, the alkyl group is methyl or ethyl. In embodiments wherein J² is substituted with halo, the halo group is preferably fluoro.

In preferred embodiments, the compounds of formula XXV have the following formula XXVI: 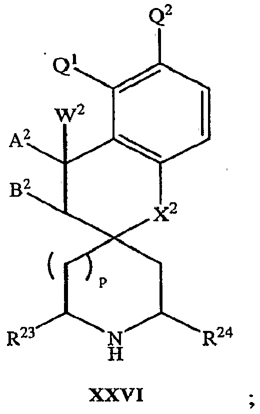 wherein:
Q¹ and Q² are each independently H, hydroxy, alkoxy, -S(=O)₂-alkyl, -S(=O)₂-NH₂, -S(=O)₂-NH(alkyl), -S(=O)₂-N(alkyl)(alkyl), -C(=O)-N(alkyl)(alkyl), carboxy (-COOH), or -C(=O)-O-alkyl. In embodiments wherein Q¹ or Q² is alkoxy, -S(=O)₂-alkyl, -S(=O)₂-NH(alkyl), -S(=O)₂-N(alkyl)(alkyl), -C(=O)-O-alkyl, or N,N-dialkylaminocarbonyl (-C(=O)-N(alkyl)(alkyl)), the alkyl group is preferably lower alkyl, with alkyl groups of 1 to 3 carbons being more preferred, and with alkyl groups of 1 to 2 carbons being still more preferred.

In particularly preferred embodiments, the alkyl group is methyl or ethyl. In embodiments wherein Q¹ or Q² is halo, the halo group is preferably fluoro.

In preferred embodiments, the compounds of formula XXV have the following structure: 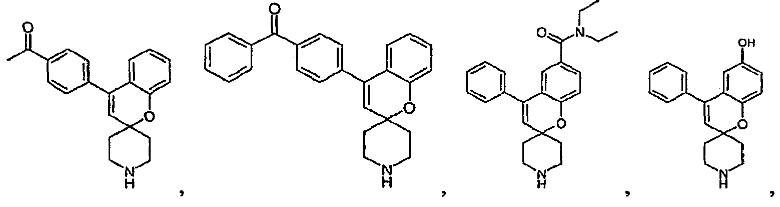 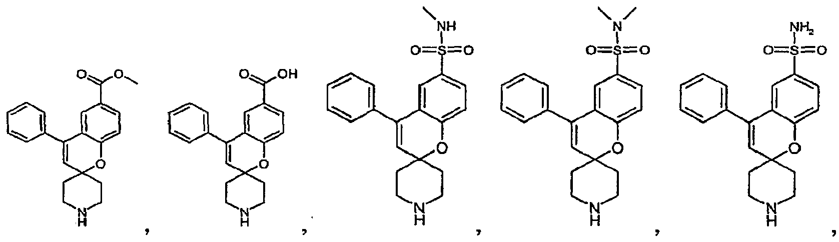 or

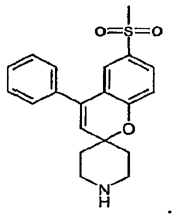

In an alternative embodiment, the invention is directed to compounds of formula XXVII: 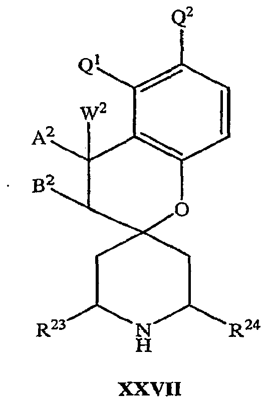 wherein:
W² is *para*-dialkylaminocarbonylphenyl, the phenyl group of which is further optionally substituted with 1-2 groups independently selected from tetrazolyl, N-alkyltetrazolyl, hydroxy, carboxy (-COOH), and aminocarbonyl (-C(=O)-NH₂);
R²³ and R²⁴ are each independently H or alkyl;
A² and B² are each H, or together form a double bond;
Q¹ and Q² are each independently H, hydroxy, alkoxy, haloalkoxy, halo, or heterocycloalkyl;
or a stereoisomer, prodrug, pharmaceutically acceptable salt, hydrate, solvate, acid salt hydrate, or N-oxide thereof;
In certain embodiments of compounds of formula XXVII:
when one of Q¹ and Q² is hydroxy and the other is H, or both Q¹ and Q² are hydroxy, then the phenyl group of W² is further substituted with 1-2 groups selected from tetrazolyl, N-alkyltetrazolyl, hydroxy, carboxy (-COOH), and aminocarbonyl (-C(=O)-NH₂);
when Q¹, Q², R²³, and R²⁴ are each H and the phenyl group of W² is further substituted with one hydroxy, then A² and B² are each H;
when W² is *para*-dialkylaminocarbonylphenyl, then at least one of Q¹, Q², R²³, and R²⁴ is other than H;
when W² is *para*-dialkylaminocarbonylphenyl, R²³ and R²⁴ are each H, and Q² is halo, then Q¹ is other than H or hydroxy;
when W² is *para*-dialkylaminocarbonylphenyl, R²³ and R²⁴ are each H, Q¹ is methoxy, cyclopropylmethoxy, cyclobutoxy, or cyclopentoxy, and Q² is H, then A² and B² are each H; and
when W² is *para*-dialkylaminocarbonylphenyl, R²³ and R²⁴ are each H, and Q¹ is H or OH, then Q² is other than methoxy, cyclopropylmethoxy, cyclobutoxy, or cyclopentoxy.

In preferred embodiments of formula XXVII compounds, W² is *para-*dialkylaminocarbonylphenyl. As set forth above, W² is further optionally substituted with 1-2 groups independently selected from tetrazolyl, N-alkyltetrazolyl, hydroxy, carboxy (-COOH), and aminocarbonyl (-C(=O)-NH₂). In preferred embodiments wherein W² is substituted with 1-2 groups, W² is preferably substituted with hydroxy.

In preferred embodiments of compounds of formula XXVII, W² is:

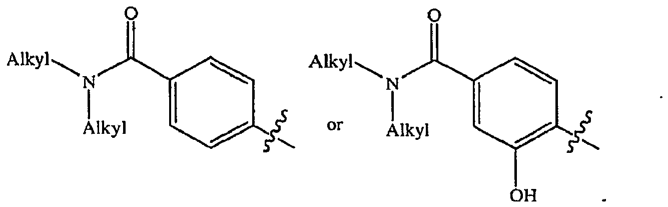

In embodiments in which W² is substituted with N,N-dialkylaminocarbonyl (-C(=O)-N(alkyl)(alkyl)), the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XXVII compounds, R²³ and R²⁴ are each independently H or alkyl, preferably H or C₁-C₃ alkyl, more preferably H or methyl, yet more preferably H.

In preferred embodiments of formula XXVII compounds, Q¹ and Q² are each independently H, hydroxy, alkoxy, haloalkoxy, halo, or heterocycloalkyl. In embodiments where Q¹ or Q² is alkoxy, the alkoxy is preferably C₁-C₃ alkoxy, more preferably C₁ alkoxy, yet more preferably, methoxy. In embodiments where Q¹ or Q² is halo, the halo is preferably fluoro. In embodiments where Q¹ or Q² is heterocycloalkyl, the heterocycloalkyl is preferably a 5- or 6-membered ring heterocycloalkyl, more preferably pyrrolidinyl or morpholinyl. In embodiments where Q¹ or Q² is haloalkoxy, the alkoxy is substituted with one or more, preferably two or more, fluoro atoms.

In preferred embodiments, the compounds of formula XXVII, the compounds have the structures: 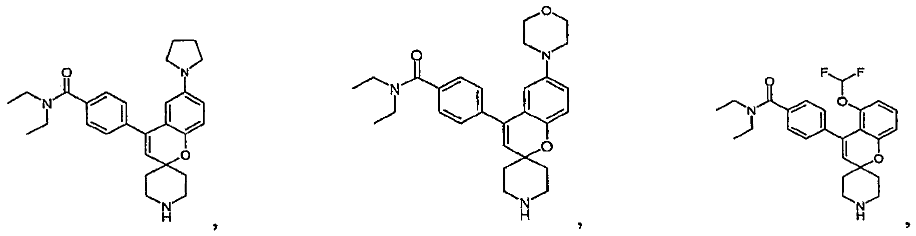 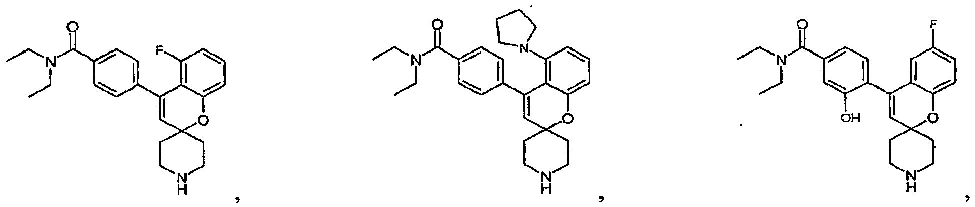 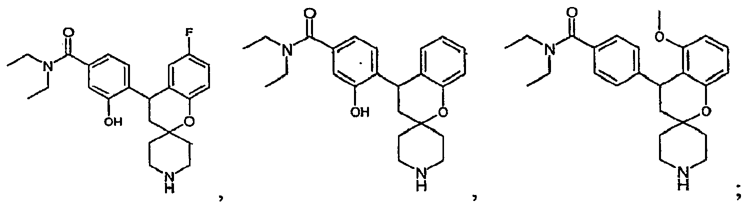 or

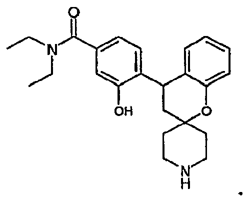

In some preferred embodiments, the compound of formula XXVII has the structure: 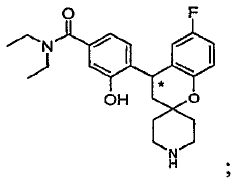 wherein denotes "*" a chiral center, as described herein above. In certain preferred embodiments, the compound is substantially enantiomerically pure.

In an alternative embodiment, the invention is directed to compounds of formula XXVIII: 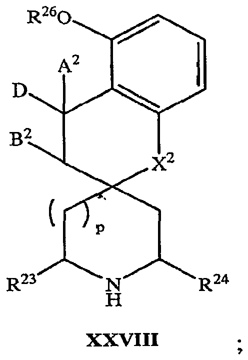 wherein:
D is: 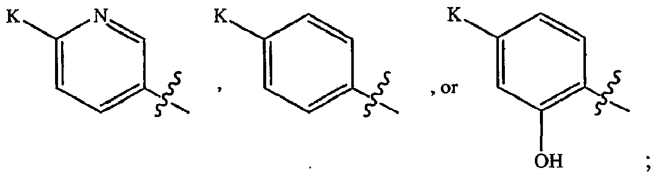
K is carboxy (-COOH), -C(=O)-O-alkyl, -S(=O)₂-N(alkyl)(alkyl), heteroaryl, alkylheteroaryl, aminocarbonyl (-C(=O)-NH₂), or N-alkylaminocarbonyl (-C(=O)-NH(alkyl));
R²³, R²⁴, and R²⁶ are each independently H or alkyl;
p is 1 or 2;
A² and B² are each H, or together form a double bond; and
X² is -CH₂- or -O-;
or a stereoisomer, prodrug, pharmaceutically acceptable salt, hydrate, solvate, acid salt hydrate, or N-oxide thereof.

In preferred embodiments of formula XXVIII compounds, D is: 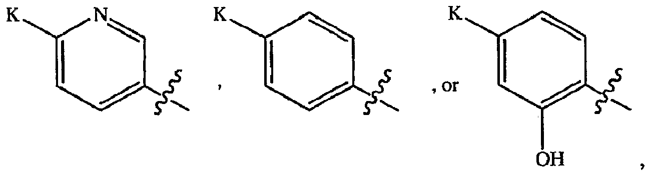 more preferably:

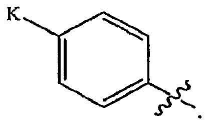

In preferred embodiments of formula XXVIII compounds wherein K is -S(=O)₂-N(alkyl)(alkyl), or N-alkylaminocarbonyl (-C(=O)-NH(alkyl)), the alkyl group independently is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XXVIII compounds wherein K is -C(=O)-O-alkyl or alkyltetrazolyl, the alkyl group independently is preferably lower alkyl, with alkyl groups of 1 to 3 carbons being more preferred, and with alkyl groups of 1-2 carbon being still more preferred. In particularly preferred embodiments, the alkyl group is methyl or ethyl.

In preferred embodiments of formula XXVIII compounds wherein K is heteroaryl or alkylheteroaryl, the heteroaryl group is preferably a 5-membered ring heteroaryl, more preferably a tetrazolyl ring.

In preferred embodiments of formula XXVIII compounds, A² and B² together form a double bond.

In preferred embodiments of formula XXVIII compounds, p is 1.

In preferred embodiments of formula XXVIII compounds, X² is -O-.

In preferred embodiments of formula XXVTII compounds, R²³, R²⁴, and R²⁶ are each independently H or alkyl, preferably H or C₁-C₃ alkyl, more preferably H or methyl, yet more preferably H. In certain preferred embodiments, one of R²³ and R²⁴ is H and the other is alkyl.

In preferred embodiments, the compounds of formula XXVIII, have the structures:

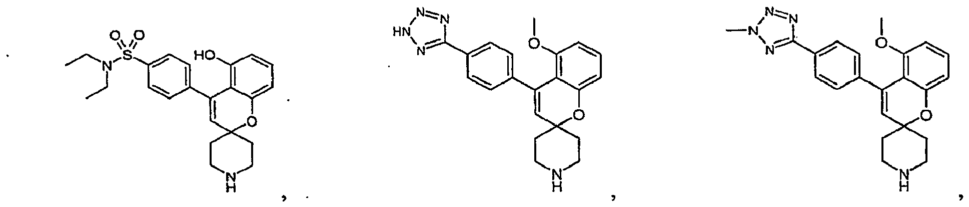

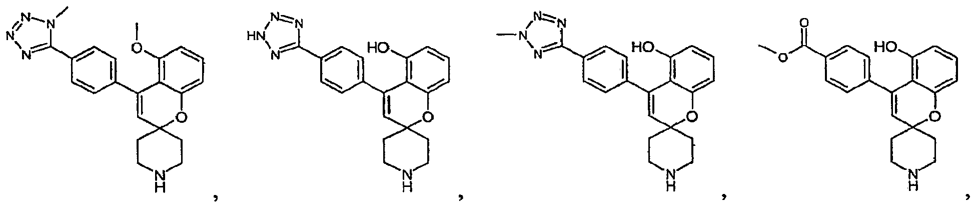

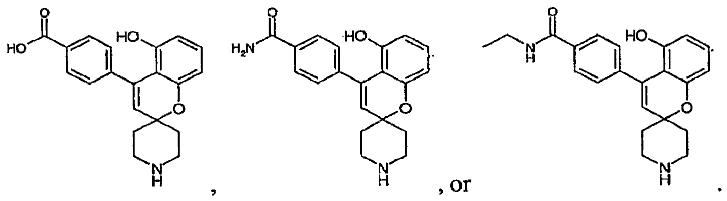

In an alternative embodiment, the invention is directed to compounds of formula XXIX: 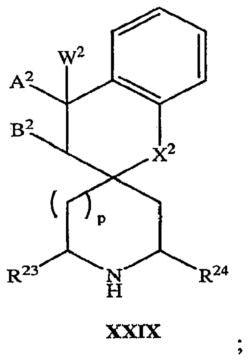 wherein:
W² is *para*-N(alkyl),N(alkyl-Z)aminocarbonylaryl or *para*-N(alkyl),N(alkyl-Z)aminocarbonylheteroaryl, wherein the aryl or heteroaryl ring of W² is substituted with 0-2 groups selected independently from hydroxy and alkoxy;
R²³ and R²⁴ are each independently H or alkyl;
p is 1 or 2;
A² and B² are each H, or together form a double bond; and
X² is -CH₂- or -O-;
or a stereoisomer, prodrug, pharmaceutically acceptable salt, hydrate, solvate, acid salt hydrate, or N-oxide thereof.

In preferred embodiments of formula XXIX compounds, W² is *para*-N(alkyl),N(alkyl-Z)-aminocarbonylaryl or *para*-N(alkyl),N(alkyl-Z)-aminocarbonylheteroaryl. When W² is *para*-N(alkyl),N(alkyl-Z)aminocarbonylaryl, the aryl ring is preferably phenyl. When *para*-N(alkyl),N(alkyl-Z)aminocarbonylheteroaryl, the heteroaryl ring is preferably pyridyl.

As set forth above, W² is substituted with 0-2 groups selected independently from selected independently from hydroxy and alkoxy. In preferred embodiments, W² is substituted with 0-1 groups, selected independently from hydroxy and alkoxy, more preferably hydroxy.

In preferred embodiments of formula XXIX compounds, W² is: 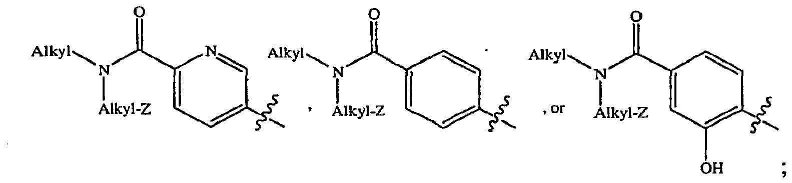 more preferably: 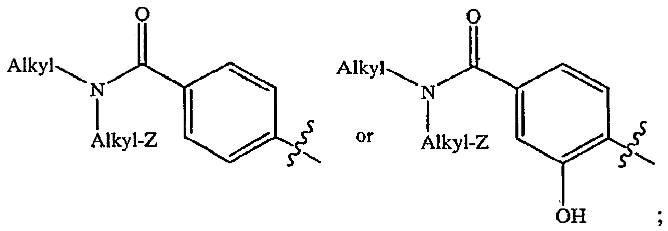 more preferably still:

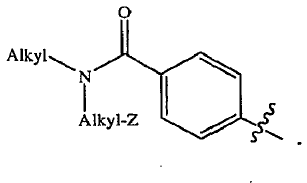

In embodiments in which W² is *para*-N(alkyl),N(alkyl-Z)aminocarbonylaryl or *para*-N(alkyl),N(alkyl-Z)aminocarbonylheteroaryl, the alkyl group is preferably lower alkyl, with alkyl groups of 1 to 3 carbons being more preferred, and with alkyl groups of 2 carbons being still more preferred. In particularly preferred embodiments, the alkyl group is ethyl.

In preferred embodiments of formula XXIX compounds, p is 1.

In preferred embodiments of formula XXIX compounds, A² and B² together form a double bond.

In preferred embodiments of formula XXIX compounds, X² is -O-.

In preferred embodiments of formula XXIX compounds, R²³ and R²⁴ are each independently H or alkyl, preferably H or C₁-C₃ alkyl, more preferably H or methyl. In certain preferred embodiments, one of R²³ and R²⁴ is H and the other is alkyl.

In certain embodiments, the compounds of formula XXIX have the structures:

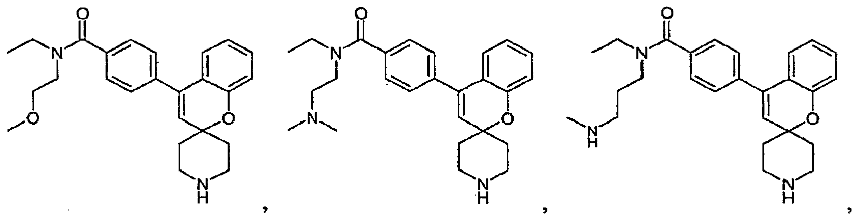

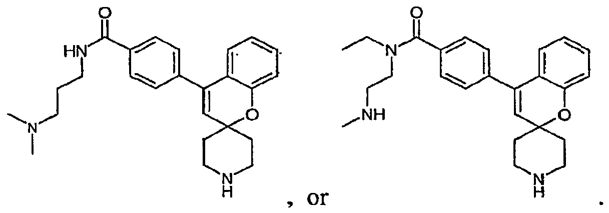

In an alternative embodiment, the invention is directed to compounds of formula XXX: 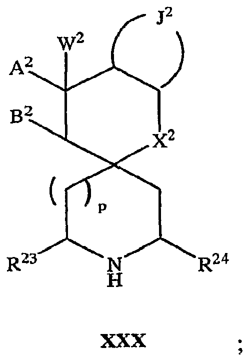 wherein:
W² is: 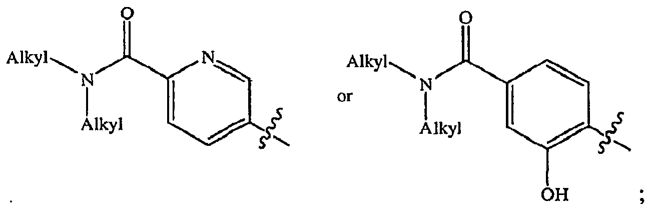
R²³ and R²⁴ are each independently H or alkyl;
p is 1 or 2;
A² and B² are each H, or together form a double bond;
X² is -CH₂- or -O-; and
J² when taken together with the carbon atoms to which it is attached forms a 6-membered aryl ring substituted with 1-3 groups selected independently from halo or haloalkoxy;
or a stereoisomer, prodrug, pharmaceutically acceptable salt, hydrate, solvate, acid salt hydrate, or N-oxide thereof;

In certain preferred embodiments of compounds of formula XXX, when W² is: 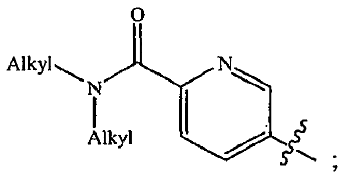 then the aryl ring of J² is substituted with at least one haloalkoxy.

In preferred embodiments of compounds of formula XXX, W² is:

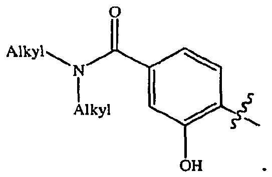

In preferred embodiments of formula XXIX compounds, p is 1.

In preferred embodiments of formula XXX compounds, R²³ and R²⁴ are each independently H or alkyl, preferably H or C₁-C₃ alkyl, more preferably H or methyl, yet more preferably H. In certain preferred embodiments, one of R²³ and R²⁴ is H and the other is alkyl.

In preferred embodiments of formula XXX compounds, J² when taken together with the carbon atoms to which it is attached forms a 6-membered aryl ring, preferably a phenyl ring. In embodiments wherein J² is substituted with 1-3 groups selected independently from halo or haloalkoxy, the halo group of halo or haloalkoxy is preferably fluoro.

In preferred embodiments, the compounds of formula XXX have the following formula XXXI: 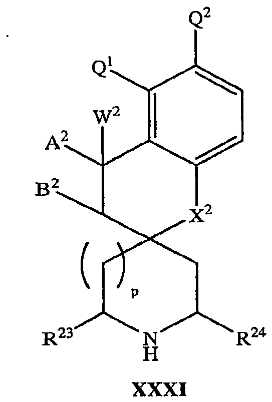 wherein:
Q¹ and Q² are each independently H, halo, or haloalkoxy, provided that at least one of Q¹ and Q² is other than H. In embodiments wherein Q¹ or Q² is halo or haloalkoxy, the halo group of halo or haloalkoxy is preferably fluoro.

In preferred embodiments, the compounds of formula XXX have the structures:

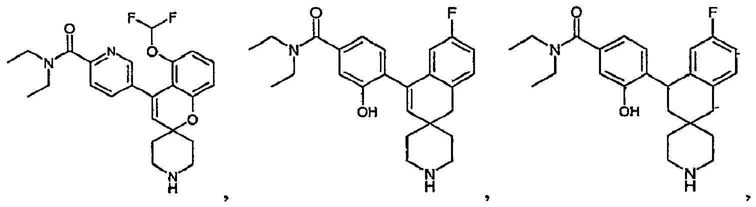

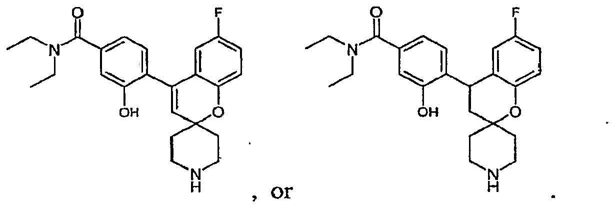

In an alternative embodiment, the invention is directed to compounds of formula XXXII: 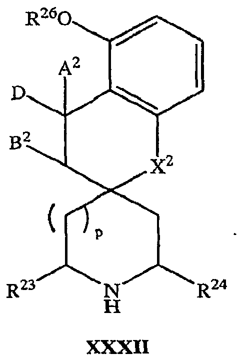 wherein:
D is N(alkyl),N(alkyl}aminocarbonylheteroaryl;
R²³ , R²⁴, and R²⁶ are each independently H or alkyl;
p is 1 or 2;
A² and B² are each H, or together form a double bond; and
X² is -CH₂- or -O-;
or a stereoisomer, prodrug, pharmaceutically acceptable salt, hydrate, solvate, acid salt hydrate, or N-oxide thereof.
In certain preferred embodiments of the compounds of formula XXXII , when D is: 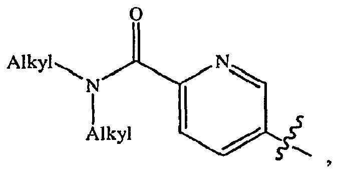 and X² is -O-, then A² and B² are each H.

In embodiments of formula XXXII compounds wherein D is N(alkyl),N(alkyl)aminocarbonylheteroaryl, the heteroaryl group is preferably pyridyl or thienyl.

In embodiments of formula XXXII compounds wherein D is N(alkyl),N(alkyl)aminocarbonylheteroaryl, the alkyl group is preferably lower alkyl, with alkyl groups of 1 to 3 carbons being more preferred, and with alkyl groups of 2-3 carbons being still more preferred. In particularly preferred embodiments, the alkyl group is ethyl or isopropyl.

In preferred embodiments of formula XXXII compounds, A² and B² are each H.

In preferred embodiments of formula XXXII compounds, X² is -O-.

In preferred embodiments of formula XXXII compounds, R²³ , R²⁴ , and R²⁶ are each independently H or alkyl, preferably H or C₁-C₃ alkyl, more preferably H or methyl, yet more preferably H. In certain preferred embodiments, one of R²³ and R²⁴ is H and the other is alkyl.

In certain embodiments, the compounds of formula XXXII, have the structures: 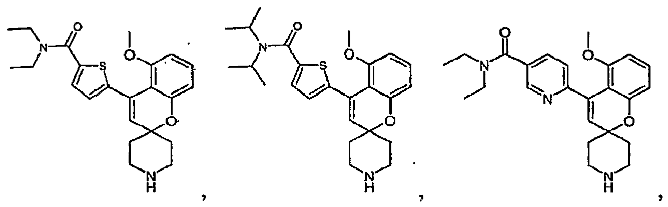 or 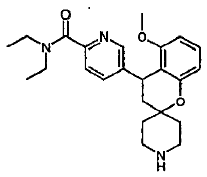 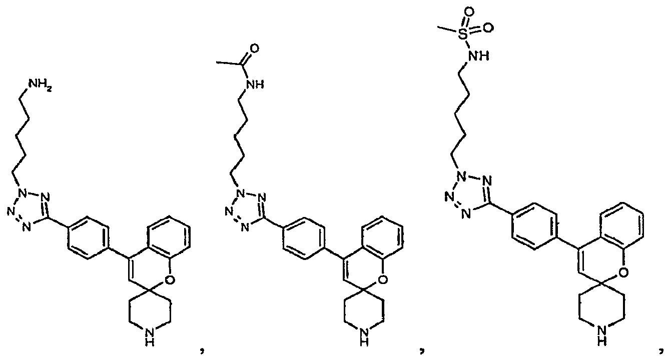 and

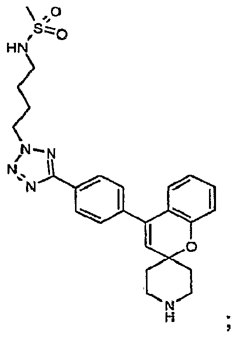

In preferred embodiments of the methods of the invention, the compounds of formula IV are preferable selected from the group consisting of:
4-[(4-*N*,*N*-diethylaminocarbonyl)phenyl]-spiro[2H,1-benzopyran-2,4'-piperidine];
4-[(2-*N*,*N*-diethylaminocarbonyl)pyrid-5-yl]-spiro[6-fluoro-2H,1-benzopyran-2,4'-piperidine];
4-[(2-*N*,*N*-diethylaminocarbonyl)pyrid-5-yl]-spiro[5-methoxy-2H,1-benzopyran-2,4'-piperidine];
4-[(4-*N*,*N*-diethylaminocarbonyl)phenyl]-spiro[5-hydroxy-2H,1-benzopyran-2,4'-piperidine];
4-[(4-*N*,*N*-diethylaminocarbonyl)phenyl]-spiro[2H,1-benzopyran-2,4'-azepane];
4-[(4-*N*,*N*-diethylaminocarbonyl)phenyl]-spiro[6-cyclopropylmethylamitiosulfonyl-2H,1-benzopyran-2,4'-azepane];
4-[(4-*N*,*N*-diethylaminocarbonyl)phenyl]-spiro[3,4-dihydro-2H,1-benzopyran-2,4'-piperidine];
4-[(4-*N*,*N*-diethylaminocarbonyl)phenyl]-spiro[1,2-dihydronaphthalene-2,4'-piperidine];
4-[(4-*N*,*N*-diethylanfnocarbonyl-2-hydroxy)phenyl]-spiro[2H,1-benzopyran-2,4'-piperidine];
4-[(4-*N*,*N*-diethylaminocarbonyl-3-hydroxy)phenyl]-spiro[2H,1-benzopyran-2,4'-piperidine] ;
4-[(4-*N*,*N*-diethylaminocarbonyl)phenyl]-3-methyl-spiro[2H,1-benzopyran-2,4'-piperidine] ;
4-[(2-*N*,*N*-diethylaminocarbonyl)pyrid-5-yl]-spiro[2H,1-benzopyran-2,4'-piperidine];
4-[(4-*N*,*N*-diethylaminocarbonyl)phenyl]-spiro[6-cyclopropylmethoxy-2H,1-benzopyran-2,4'-piperidine];
4-[(2-*N*,*N*-diethylaminocarbonyl)pyrid-5-yl]-spiro[-6-cyclopropylmethoxy-2H,1-benzopyran-2,4'-piperidine];
4-[(4-*N*,*N*-diethylaminocarbonyl)phenyl]-spiro[6-aminocarbonyl-2H,1-benzopyran-2,4'-piperidine];
4-[(4-*N*,*N*-diethylaminocarbonyl)phenyl]-spiro[6-propylaminosulfonyl-2H,1-benzopyran-2,4'-azepane];
4-[(4-*N,N*-diethylaminocarbonyl)phenyl]-spiro[6-methanesulfonyl-2H,1-benzopyran-2,4'-azepane];
4-[(2-*N*,*N*-diethylaminocarbonyl)pyrid-5-yl]-spiro[3,4-dihydro-2H,1-benzopyran-2,4'-piperidine];
4-[(2-*N*,*N*-diethylaminocarbonyl)pyrid-5-yl]-spiro[6-fluoro-3,4-dihydro-2H,1-benzopyran-2,4'-piperidine];
4-[(5-*N*,*N*-diisopropylaminocarbonyl)pyrid-2-yl]-spiro[2H,1-benzopyran-2,4'-piperidine
-4-[(4-*N,N-*diethylaminocarbonyl)phenyl]-spiro[6-ethylsulfonylamino-2H,1-benzopyran-2,4'-piperidine];
4-[(4-*N*,*N*-diethylaminocarbonyl)phenyl]-spiro[6-methylsulfonylamino-2H,1-benzopyran-2,4'-piperidine];
4-[(4-*N*,*N*-diethylaminocarbonyl)phenyl]-spiro[5-methyl-2H,1-benzopyran-2,4'-piperidine];
4-[4-(2*H*-tetrazol-5-yl)phenyl]-spiro[2H,1-benzopyran-2,4'-piperidine];
4-[4-(2-methyl-tetrazol-5-yl)phenyl]-spiro[2H,1-benzopyran-2,4'-piperidine];
4-[3-(2-(3-carboxyprop-1-yl)-tetrazol-5-yl)phenyl]-spiro[2H,1-benzopyran-2,4'-piperidine];
4-[4-(5-methyl-[1,2,4]oxadiazol-3-yl)phenyl]-spiro[2H,1-benzopyran-2,4'-piperidine];
4-[(4-*N*,*N*-diethylaminocarbonyl)phenyl]-spiro[2H,1-benzopyran-2,4'-(1'-methyl-piperidine)];
4-[(4-*N*,*N*-diethylaminosulfonyl)phenyl]-spiro[2H,1-benzopyran-2,4'-piperidine]; and
4-[(4-(*N*-methyl-*N*-(3-methylbutanoyl)-anfino)phenyl]-spiro[2H,1-benzopyran-2,4'-piperidine];
4*-[(4-*N*,*N*-diethylaminocarbonyl)phenyl]-spiro[3,4-dihydro-2H,1-benzopyran-2,4'-piperidine];
4*-[(2-*N*,*N*-diethylaminocarbonyl)pyrid-5-yl]-spiro[3,4-dihydro-2H,1-benzopyran-2,4'-piperidine];
4-[(4-*N*,*N*-diethylaminocarbonyl)phenyl]-spiro*[2H,1-benzopyran-2,4'-azepane];
4-[(4-*N*,*N*-diethylaminocarbonyl)phenyl]-spiro*[6-cyclopropylmethylaminosulfonyl-2H,1-benzopyran-2,4'-azepane];
4-[(4-*N*,*N*-diethylaminocarbonyl)phenyl]-spiro*[6-propylaminosulfonyl-2H,1-benzopyran-2,4'-azepane]; and
4-[(4-*N*,*N*-diethylaminocarbonyl)phenyl]-spiro*[6-methanesulfonyl-2H,1-benzopyran-2,4'-azepane]; and
a stereoisomer, partial stereoisomer, prodrug, pharmaceutically acceptable salt, hydrate, solvate, acid salt hydrate, and N-oxide thereof.

In any of the above teachings, a compound as described herein may be either a compound of one of the formulae herein described, or a stereoisomer, prodrug, pharmaceutically acceptable salt, hydrate, solvate, acid salt hydrate, N-oxide or isomorphic crystalline form thereof.

The compounds employed in the methods and compositions of the present invention may exist in prodrug form. As used herein, "prodrug" is intended to include any covalently bonded carriers which release the active parent drug, for example, as according to a compound of formula IV and VI to XXXIV. Since prodrugs are known to enhance numerous desirable qualities of pharmaceuticals (*e.g*., solubility, bioavailability, manufacturing, etc.), the compounds described herein may, if desired, be delivered in prodrug form. Thus the present invention contemplates compositions and methods involving prodrugs. Prodrugs of the compounds employed in the present invention, for example of formula IV and VI to XXXIV, may be prepared by modifying functional groups present in the compound in such a way that the modifications are cleaved, either in routine manipulation or *in vivo,* to the parent compound.

Accordingly, prodrugs include, for example, compounds described herein in which a hydroxy, amino, or carboxy group is bonded to any group that, when the prodrug is administered to a mammalian subject, cleaves to form a free hydroxyl, free amino, or carboxylic acid, respectively. Examples include, but are not limited to, acetate, formate and benzoate derivatives of alcohol and amine functional groups; and alkyl, carbocyclic, aryl, and alkylaryl esters such as methyl, ethyl, propyl, iso-propyl, butyl, isobutyl, sec-butyl, tert-butyl, cyclopropyl, phenyl, benzyl, and phenethyl esters, and the like.

Compounds described herein may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

The compounds as herein described may be prepared in a number of ways well known to those skilled in the art. The compounds can be synthesized, for example, by the methods described herein, in U.S. Patent Publication No. US 2005-0159438 A1 and U.S. Application No. 11/393,133 filed March 30, 2006, and variations thereon as appreciated by the skilled artisan. All processes disclosed in association with the present invention are contemplated to be practiced on any scale, including milligram, gram, multigram, kilogram, multikilogram or commercial industrial scale.

As will be readily understood, functional groups present may contain protecting groups during the course of synthesis. Protecting groups are known *per se* as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred protecting groups include the benzyloxycarbonyl group and the tert-butyloxycarbonyl group. Other preferred protecting groups that may be employed in accordance with the present invention may be described in Greene, T.W. and Wuts, P.G.M., Protective Groups in Organic Synthesis 2d. Ed., Wiley & Sons, 1991, the disclosures of which are hereby incorporated herein by reference, in their entirety.

The δ agonist compounds as described herein may be administered by any means that results in the contact of the active agent with the agent's site of action in the body of a patient. The compounds may be administered by any conventional means available for use in conjunction with pharmaceuticals, either as individual therapeutic agents or in a combination of therapeutic agents. For example, they may be administered as the sole active agent in a pharmaceutical composition, or they can be used in combination with other therapeutically active ingredients including, for example, opioid analgesic agents. In such combinations, selected compounds as described herein may provide equivalent or even enhanced therapeutic activity such as, for example, pain ameliorization, while providing reduced adverse side effects associated with opioids, such as addiction or pruritus, by lowering the amount of opioid required to achieve a therapeutic effect.

The compounds are preferably combined with a pharmaceutical carrier selected on the basis of the chosen route of administration and standard pharmaceutical practice as described, for example, in Remington's Pharmaceutical Sciences (Mack Publishing Co., Easton, PA, 1980), the disclosures of which are hereby incorporated herein by reference, in their entirety.

In addition to the pharmaceutical carrier, the compounds of formula IV and VI to XXXIV, may be co-administered with at least second pharmaceutically active compound selected from the group consisting of cholinesterase inhibitor (including acetylcholinesterase inhibitor and butylcholinesterase inhibitor), NMDA receptor modulator (including NAMENDA (memantine hydrochloride)), β amyloid modulator, β amyloid inhibitor, nicotinic cholinergic agent, nicotine receptor partial agonist (NRPA)(including those disclosed, for example, in WO 01/85145, incorporated herein by reference), estrogenic agent, selective estrogen receptor modulator (SERM), muscarinic agonist, neurotransmitter precursor (such as phenylalanine, tyrosine, and/or tryptophan, as described in WO 01/26623, incorporated herein by reference), neurotransmitter reuptake inhibitor (such as serotonin reuptake inhibitor (SRI), norepinephrine reuptake inhibitor (NRI), dual NRI/SRI, and dopamine reuptake inhibitor), Coenzyme Q10, *Ginkgo biloba,* phosphatidylserine, vitamin E, and other agents as described in WO 2005/019157, incorporated by reference, and combinations thereof. These second pharmaceutically active compounds may be coadministered at levels consistent with levels when used alone, such as those disclosed in WO 01/85145, incorporated herein by reference.

Suitable acetylcholinesterase or butylcholinesterase inhibitors include donepizil (Aricept^{™}), tacrine (Cognex^{™}), rivastigmine (Exelon^{™}), physostigmine (Synapton^{™}), galantamine (Reminyl) ^{™}, metrifonate (Promem^{™}), , quilostigmine, tolserine, thiatolserine, cymserine, thiacymserine, neostigmine, eseroline, zifrosilone, mestinon, huperzine A and icopezil.

Suitable estrogenic agents include include estradiol or a pharmaceutically acceptable form of estradiol, estrogen, lasofoxifene, droloxifene, tamoxifen and raloxifene (Evista).

Suitable SERMs include estradiol, estrogen, lasofoxifene, droloxifene, tamoxifen and raloxifene (Evista).

Suitable muscarinic agonists include milameline, xanomeline, sabcomeline, arecoline, oxotremorine and pilocarpine.

Compounds as described herein can be administered to a mammalian host in a variety of forms adapted to the chosen route of administration, *e.g*., orally or parenterally. Parenteral administration in this respect includes administration by the following routes: intravenous, intramuscular, subcutaneous, rectal, intraocular, intrasynovial, transepithelial including transdermal, ophthalmic, sublingual and buccal; topically including ophthalmic, dermal, ocular, rectal, and nasal inhalation via insufflation aerosol.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should preferably contain at least 0.1% of active compound. The percentage of the compositions and preparations may, of course, be varied and may conveniently be, for example, from about 2 to about 6% of the weight of the unit. The amount of active compound in such therapeutically useful compositions is preferably such that a suitable dosage will be obtained. Preferred compositions or preparations according to the present invention may be prepared so that an oral dosage unit form contains from about 0.1 to about 1000 mg of active compound.

The tablets, troches, pills, capsules and the like may also contain one or more of the following: a binder, such as gum tragacanth, acacia, corn starch or gelatin; an excipient, such as dicalcium phosphate; a disintegrating agent, such as corn starch, potato starch, alginic acid and the like; a lubricant, such as magnesium stearate; a sweetening agent such as sucrose, lactose or saccharin; or a flavoring agent, such as peppermint, oil of wintergreen or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring, such as cherry or orange flavor. Of course, any material used in preparing any dosage unit form is preferably pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or a pharmacologically acceptable salt can be prepared in water suitably mixed with a surfactant, such as hydroxypropylcellulose. A dispersion can also be prepared in glycerol, liquid polyethylene glycols and mixtures thereof and in oils. Under ordinary conditions of storage and use, these preparations may contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. In all cases, the form is preferably sterile and fluid to provide easy syringability. It is preferably stable under the conditions of manufacture and storage and is preferably preserved against the contaminating action of microorganisms such as bacteria and fungi. The carrier may be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating, such as lecithin, by the maintenance of the required particle size in the case of a dispersion, and by the use of surfactants. The prevention of the action of microorganisms may be achieved by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions may be achieved by the use of agents delaying absorption, for example, aluminum monostearate and gelatin.

Sterile injectable solutions may be prepared by incorporating the active compound in the required amount, in the appropriate solvent, with various of the other ingredients enumerated above, as required, followed by filtered sterilization. Generally, dispersions may be prepared by incorporating the sterilized active ingredient into a sterile vehicle that contains the basic dispersion medium and the required other ingredients from those enumerated above. In the case of sterile powders for the preparation of sterile injectable solutions, the preferred methods of preparation may include vacuum drying and the freeze-drying technique that yield a powder of the active ingredient, plus any additional desired ingredient from the previously sterile-filtered solution thereof.

The therapeutic compounds of this invention may be administered to a patient alone or in combination with a pharmaceutically acceptable carrier. As noted above, the relative proportions of active ingredient and carrier may be determined, for example, by the solubility and chemical nature of the compound, chosen route of administration and standard pharmaceutical practice.

The dosage of the compounds as described herein that will be most suitable for prophylaxis or treatment will vary with the form of administration, the particular compound chosen and the physiological characteristics of the particular patient under treatment. Generally, small dosages may be used initially and, if necessary, increased by small increments until the desired effect under the circumstances is reached. The therapeutic human dosage, based on physiological studies using rats, may generally range from about 0.01 mg to about 100 mg/kg of body weight per day, and all combinations and subcombinations of ranges and specific dosages therein. Alternatively, the therapeutic human dosage may be from about 0.4 mg to about 10 g or higher, and may be administered in several different dosage units from once to several times a day. Generally speaking, oral administration may require higher dosages.

It will be further appreciated that the amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

The desired dose may conveniently be presented in a single dose or as divided doses administered at appropriate intervals, for example, as two, three, four or more sub-doses per day. The sub-dose itself may be further divided, *e.g*., into a number of discrete loosely spaced administrations; such as multiple inhalations from an insufflator or by application of a plurality of drops into the eye.

The dose may also be provided by controlled release of the compound, by techniques well known to those in the art.

The compounds as described herein may also be formulated with other optional active ingredients, in addition to or instead of the optional opioids, and in addition to the optional pharmaceutical-acceptable carriers. Other active ingredients include, but are not limited to, antibiotics, antivirals, antifungals, anti-inflammatories, including steroidal and non-steroidal anti-inflammatories, anesthetics and mixtures thereof. Such additional ingredients include any of the following:

### a. Antibacterial agents

Aminoglycosides, such as Amikacin, Apramycin, Arbekacin, Bambermycins, Butirosin, Dibekacin, Dihydrostreptomycin, Fortimicin(s), Fradiomycin, Gentamicin, Ispamicin, Kanamycin, Micronomicin, Neomycin, Neomycin Undecylenate, Netilmicin, Paromomycin, Ribostamycin, Sisomicin, Spectinomycin, Streptomycin, Streptonicozid and Tobramycin;
Amphenicols, such as Azidamfenicol, Chloramphenicol, Chloramphenicol Palmirate, Chloramphenicol Pantothenate, Florfenicol, Thiamphenicol;
Ansamycins, such as Rifamide, Rifampin, Rifamycin and Rifaximin;
β -Lactams;
Carbapenems, such as Imipenem;
Cephalosporins, such as 1-Carba (dethia) Cephalosporin, Cefactor, Cefadroxil, Cefamandole, Cefatrizine, Cefazedone, Cefazolin, Cefixime, Cefmenoxime, Cefodizime, Cefonicid, Cefoperazone, Ceforanide, Cefotaxime, Cefotiam, Cefpimizole, Cefpirimide, Cefpodoxime Proxetil, Cefroxadine, Cefsulodin, Ceftazidime, Cefteram, Ceftezole, Ceftibuten, Ceftizoxime, Ceftriaxone, Cefuroxime, Cefuzonam, Cephacetrile Sodium, Cephalexin, Cephaloglycin, Cephaloridine, Cephalosporin, Cephalothin, Cephapirin Sodium, Cephradine and Pivcefalexin;
Cephamycins such as Cefbuperazone, Cefmetazole, Cefminox, Cefetan and Cefoxitin;
Monobactams such as Aztreonam, Carumonam and Tigemonan;
Oxacephems such as Flomoxef and Moxolactam;
Penicillins such as Amidinocillin, Amdinocillin, Pivoxil, Amoxicillin, Ampicillan, Apatcillin, Aspoxicillin, Azidocillan, Azlocillan, Bacampicillin, Benzylpenicillinic Acid, Benzylpenicillin, Carbenicillin, Carfecillin, Carindacillin, Clometocillin, Cloxacillin, Cyclacillin, Dicloxacillin, Diphenicillin, Epicillin, Fenbenicillin, Floxicillin, Hetacillin, Lenampicillin, Metampicillin, Methicillin, Mezlocillin, Nafcillin, Oxacillin, Penamecillin" Penethamate Hydriodide, Penicillin G Benethamine, Penicillin G Benzathine, Penicillin G Benzhydrylamine, Penicillin G Calcium, Penicillin G Hydragamine, Penicillin G Potassium, Penicillin G. Procaine, Penicillin N, Penicillin O, Penicillin V, Penicillin V Benzathine, Penicillin V Hydrabamine, Penimepicycline, Phenethicillin, Piperacillin, Pivapicillin, Propicillin, Quinacillin, Sulbenicillin, Talampicillin, Temocillin and Ticarcillin;
Lincosumides such as Clindamycin and Lincomycin;
Macrolides such as Azithromycin, Carbomycin, Clarithromycin, Erythromycin(s) and Derivatives, Josamycin, Leucomycins, Midecamycins, Miokamycin, Oleandomycin, Primycin, Rokitamycin, Rosaramicin, Roxithromycin, Spiramycin and Troleandomyein;
Polypeptides such as Amphomycin, Bacitracin, Capreomycin, Colistin, Enduracidin, Enviomycin, Fusafungine, Gramicidin(s), Gramicidin S, Mikamycin, Polymyxin, Polymyxin β-Methanesulfonic Acid, Pristinamycin, Ristocetin, Teicoplanin, Thiostrepton, Tuberactinomycin, Tyrocidine, Tyrothricin, Vancomycin, Viomycin(s), Virginiamycin and Zinc Bacitracin;
Tetracyclines such as Spicycline, Chlortetracycline, Clomocycline, Demeclocycline, Doxycycline, Guamecycline, Lymecycline, Meclocycline, Methacycline, Minocycline, Oxytetracycline, Penimepicycline, Pipacycline, Rolitetracycline, Sancycline, Senociclin and Tetracycline; and
others such as Cycloserine, Mupirocin, Tuberin.

### b. Synthetic Antibacterials

2,4-Diaminopyrimidines such as Brodimoprim, Tetroxoprim and Trimethoprim;
Nitrofurans such as Furaltadone, Furazolium, Nifuradene, Nifuratel, Nifurfoline, Nifurpirinol, Nifurprazine, Nifurtoinol and Nitrofurantoin;
Quinolones and analogs thereof, such as Amifloxacin, Cinoxacin, Ciprofloxacin, Difloxacin, Enoxacin, Fleroxacin, Flumequine, Lomefloxacin, Miloxacin, Nalidixic Acid, Norfloxacin, Ofloxacin, Oxolinic Acid, Perfloxacin, Pipemidic Acid, Piromidic Acid, Rosoxacin, Temafloxacin and Tosufloxacin;
Sulfonamides such as Acetyl Sulfamethoxypyrazine, Acetyl Sulfisoxazole, Azosulfamide, Benzylsulfamide, Chloramine-β, Chloramine-T, Dichloramine-T, Formosulfathiazole, N.sup.2 -Formyl-sulfisomidine, N.sup.4 -β-D-Glucosylsulfanilamide, Mafenide, 4'-(Methyl-sulfamoyl)sulfanilanilide, p-Nitrosulfathiazole, Noprylsulfamide, Phthalylsulfacetamide, Phthalylsulfathiazole, Salazosulfadimidine, Succinylsulfathiazole, Sulfabenzamide, Sulfacetamide, Sulfachlorpyridazine, Sulfachrysoidine, Sulfacytine, Sulfadiazine, Sulfadicramide, Sulfadimethoxine, Sulfadoxine, Sulfaethidole, Sulfaguanidine, Sulfaguanol, Sulfalene, Sulfaloxic Acid, Sulfamerazine, Sulfameter, Sulfamethazine, Sulfamethizole, Sulfamethomidine, Sulfamethoxazole, Sulfamethoxypyridazine, Sulfametrole, sulfamidochrysoidine, Sulfamoxole, Sulfanilamide, Sulfanilamidomethanesulfonic Acid Triethanolamine Salt, 4-Sulfanilamidosalicyclic Acid, N⁴ -Sulfanilylsulfanilamide, Sulfanilylurea, N-Sulfanilyl-3,4-xylamide, Sulfanitran, Sulfaperine, Sulfaphenazole, Sulfaproxyline, Sulfapyrazine, Sulfapyridine, Sulfasomizole, Sulfasymazine, Sulfathiazole, Sulfathiourea, Sulfatolamide, Sulfisomidine and Sulfisoxazole;
Sulfones, such as Acedapsone, Acediasulfone, Acetosulfone, Dapsone, Diathymosulfone, Glucosulfone, Solasulfone, Succisulfone, Sulfanilic Acid, p-Sulfanilylbenzylamine, p,p'-sulfonyldianiline-N,N'-digalactoside, Sulfoxone and Thiazolsulfone;
Others such as Clofoctol, Hexedine, Magainins, Methenamine, Methenamine Anhydromethylene-citrate, Methenamine Hippurate, Methenamine Mandelate, Methenamine Sulfosalicylate, Nitroxoline, Squalamine and Xibomol.

### c. Antifungal (antibiotics)

Polyenes such as Amphotericin-B, Candicidin, Dermostatin, Filipin, Fungichromin, Hachimycin, Hamycin, Lucensomycin, Mepartricin, Natamycin, Nystatin, Pecilocin, Perimycin; and others, such as Azaserine, Griseofulvin, Oligomycins, Pyrrolnitrin, Siccanin, Tubercidin and Viridin.

### d. Antifungal (synthetic)

Allylamines such as Naftifine and terbinafine;
Imidazoles such as Bifonazole, Butoconazole, Chlordantoin, Chlormidazole, Cloconazole, Clotrimazole, Econazole, Enilconazole, Finticonazole, Isoconazole, Ketoconazole, Miconazole, Omoconazole, Oxiconazole Nitrate, Sulconazole and Tioconazole;
Triazoles such as Fluconazole, Itraconazole, Terconazole;
Others such as Acrisorcin, Amorolfine, Biphenamine, Bromosalicylchloranilide, Buclosamide, Chlophenesin, Ciclopirox, Cloxyquin, Coparaffinate, Diamthazole, Dihydrochloride, Exalamide, Flucytosine, Halethazole, Hexetidine, Loflucarban, Nifuratel, Potassium Iodide, Propionic Acid, Pyrithione, Salicylanilide, Sulbentine, Tenonitrozole, Tolciclate, Tolindate, Tolnaftate, Tricetin, Ujothion, and Undecylenic Acid.

### e. Antiglaucoma agents

Antiglaucoma agents, such as Dapiprazoke, Dichlorphenamide, Dipivefrin and Pilocarpine.

### f. Anti-inflammatory agents

Corticosteroids, aminoarylcarboxylic Acid Derivatives such as Etofenamate, Meclofenamic Acid, Mefanamic Acid, Niflumic Acid;
Arylacetic Acid Derivatives such as Acemetacin, Amfenac Cinmetacin, Clopirac, Diclofenac, Fenclofenac, Fenclorac, Fenclozic Acid, Fentiazac, Glucametacin, Isozepac, Lonazolac, Metiazinic Acid, Oxametacine, Proglumetacin, Sulindac, Tiaramide and Tolmetin;
Arylbutyric Acid Derivatives such as Butibufen and Fenbufen;
Arylcarboxylic Acids such as Clidanac, Ketorolac and Tinoridine;
Arylpropionic Acid Derivatives such as Bucloxic Acid, Carprofen, Fenoprofen, Flunoxaprofen, Ibuprofen, Ibuproxam, Oxaprozin, Piketoprofen, Pirprofen, Pranoprofen, Protizinic Acid and Tiaprofenic Add;
Pyrazoles such as Mepirizole;
Pyrazolones such as Clofezone, Feprazone, Mofebutazone, Oxyphenbutazone, Phenylbutazone, Phenyl Pyrazolidininones, Suxibuzone and Thiazolinobutazone;
Salicylic Acid Derivatives such as Bromosaligenin, Fendosal, Glycol Salicylate, Mesalamine, 1-Naphthyl Salicylate, Olsalazine and Sulfasalazine;
Thiazinecarboxamides such as Droxicam, Isoxicam and Piroxicam;
Others such as e-Acetamidocaproic Acid, S-Adenosylmethionine, 3-Amino-4-hydroxybutyric Acid, Amixetrine, Bendazac, Bucolome, Carbazones, Difenpiramide, Ditazol, Guaiazulene, Heterocyclic Aminoalkyl Esters of Mycophenolic Acid and Derivatives, Nabumetone, Niniesulide, Orgotein, Oxaceprol, Oxazole Derivatives, Paranyline, Pifoxime, 2-substituted-4,6-di-tertiary-butyl-s-hydroxy-1,3-pyrimidines, Proquazone and Tenidap.

### g. Antiseptics

Guanidines such as Alexidine, Ambazone, Chlorhexidine and Picloxydine;
Halogens/Halogen Compounds such as Bomyl Chloride, Calcium Iodate, Iodine, Iodine Monochloride, Iodine Trichloride, Iodoform, Povidone-Iodine, Sodium Hypochlorite, Sodium Iodate, Symclosene, Thymol Iodide, Triclocarban, Triclosan and Troclosene Potassium;
Nitrofurans such as Furazolidone, 2-(Methoxymethyl)-5-Nitrofuran, Nidroxyzone, Nifuroxime, Nifurzide and Nitrofurazone;
Phenols such as Acetomeroctol, Chloroxylenol, Hexachlorophene, 1-Naphthyl Salicylate, 2,4,6-Tribromo-m-cresol and 3',4';5-Trichlorosalicylanilide;
Quinolines such as Aminoquinuride, Chloroxine, Chlorquinaldol, Cloxyquin, Ethylhydrocupreine, Halquinol, Hydrastine, 8-Hydroxyquinoline and Sulfate; and
others, such as Boric Acid, Chloroazodin, m-Cresyl Acetate, Cupric sulfate and Ichthammol.

### h. Antivirals

Purines/Pyrimidinones, such as 2-Acetyl-Pyridine 5-((2-pyridylamino)thiocarbonyl) Thiocarbonohydrazone, Acyclovir, Dideoxyadenosine, Dideoxycytidine, Dideoxyinosine, Edoxudine, Floxuridine, Ganciclovir, Idoxuridine, MADU, Pyridinone, Trifluridine, Vidrarbine and Zidovudline;
others such as Acetylleucine Monoethanolamine, Acridinamine, Alkylisooxazoles, Amantadine, Amidinomycin, Cuminaldehyde Thiosemicarbzone, Foscamet Sodium, Kethoxal, Lysozyme, Methisazone, Moroxydine, Podophyllotoxin, Ribavirin, Rimantadine, Stallimycin, Statolon, Thymosins, Tromantadine and Xenazoic Acid.

### i. Agents for Neuralgia/Neuropathic Pain

Mild OTC (over the counter) analgesics, such as aspirin, acetaminophen, and ibuprophen.
Narcotic analgesics, such as codeine.
Anti seizure medications, such as carbamazepine, gabapentin, lamotrigine and phenytoin.
Anti-depressants, such as amitryptiline.

### j. Agents for the Treatment of Depression

Selective serotonin re-uptake inhibitors (SSRIs), such as Fluoxetine, Paroxetine, Fluvoxamine, Citaprolam, and Sertraline.
Tricyclics, such as Imipramine, Amitriptyline, Desipramine, Nortriptyline Protriptyline, Trimipramine, Doxepin, Amoxapine, and Clomipramine.
Monoamine Oxidase Inhibitors (MAOIs), such as Tranylcypromine, Phenelzine, and Isocarboxazid.
Heterocyclics, such as Amoxipine, Maprotiline and Trazodone.
others such as Venlafaxine, Nefazodone and Mirtazapine.

### k. Agents for the treatment of Incontinence

Anticholinergic agents such as propantheline.
Antispasmodic medications such as oxybutynin, tolterodine, and flavoxate.
Tricyclic antidepressants such as imipramine, and doxepin.
Calcium channel blockers such as tolterodine.
Beta agonists such as terbutaline.

### 1. Anti-Parkinson's Agents

Deprenyl, Amantadine, Levodopa, and Carbidopa.

### EXAMPLES

The present invention will now be illustrated by reference to the following specific, non-limiting examples. Those skilled in the art of organic synthesis may be aware of still other synthetic routes to the invention compounds. The reagents and intermediates used herein are commercially available or may be prepared according to standard literature procedures.

The examples listed in Tables 1, 2, and 3 may be prepared according to ***Schemes 1-57*** and as further described in U.S. Patent Publication No. US 2005-0159438 A1 and U.S. Application No. 11/393,133 filed March 30, 2006, the entire disclosures of which are herein incorporated by reference.

The synthesis of compounds **1A-1U** is outlined in ***Scheme** 1.* The 2'-hydroxyacetophenone derivatives **1.1a-1.1m** were condensed with 1-Boc-4-piperidone **1.2** in neat pyrrolidine (method 1A) at room temperature or in refluxing methanol in the presence of pyrrolidine (method 1B) to provide *N*-B oc-spiro[2*H*-1-benzopyran-2,4'-piperidine]-4(3*H*)-one derivatives **1.3**. Conversion of the ketones **1.3** to the enol triflate derivatives **1.5** was achieved using *N*-phenylbis(trifluoromethanesulphonimide) **1.4** as triflating reagent. Suzuki type coupling of the enol triflate derivatives **1.5** with either 4-(*N,N-*diethylaminocarbonyl)phenyl boronic acid **1.6** (commercially available from Combi-Blocks Inc.) or 2-(*N*,*N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0) (method 1C) or palladium, 10 wt.% (dry basis) on activated carbon (method 1D), lithium chloride, and an aqueous solution of sodium carbonate afforded compounds **1.8** which were converted to the final products (compounds **1A-1T)** under acidic conditions (method 1E: anhydrous HCl, diethyl ether, room temperature or method 1F: neat trifluoroacetic acid, room temperature). Demethylation of compound **1G** using boron tribromide provided the corresponding phenolic derivative (compound **1U**). The boronate derivative **1.7** was prepared in 4 steps from 2,5-dibromopyridine **1.9.** Treatment of 2,5-dibromopyridine with n-butyllithium provided the corresponding lithiated derivative, which reacted with carbon dioxide to provide 5-bromopyridine-2-carboxylic acid **1.10.** Treatment of the carboxylic acid derivative **1.10** with oxalyl chloride furnished the acyl chloride **1.11,** which reacted with diethylamine **1.12** to provide 5-bromo-2-(*N*,*N* diethylaminocarbonyl)-pyridine **1.13.** Conversion of the aryl bromide **1.13** to the corresponding boron derivative **1.7** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct, abbreviated as [Pd(dppf)Cl₂•CH₂Cl₂].

The synthesis of compounds **2A-2F** is outlined in ***Scheme* 2.** The 2'-5'-dihydroxyacetophenone derivative **2.1** was condensed with 1-Boc-4-piperidone **1.2** in refluxing methanol in the presence of pyrrolidine to provide *N*-Boc-spiro[2*H*-1-benzopyran-2,4'-piperidine]-4(3*H*)-one derivative **2.2** which was converted to the silyl ether derivative **2.4** using *tert*-butyldimethylsilyl chloride **2.3.** Conversion of the ketone **2.4** to the enol triflate derivative **2.5** was achieved using *N*-phenylbis(trifluoromethanesulphonimide) **1.4** as triflating reagent. Suzuki type coupling of the enol triflate derivative **2.5** with either 4-(*N*,*N-*diethylaminocarbonyl)-phenyl boronic acid **1.6** or 2-(*N*,*N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0) (method 1C) or palladium, 10 wt.% (dry basis) on activated carbon (method 1D), lithium chloride, and an aqueous solution of sodium carbonate afforded compounds **2.6.** Removal of the silyl protecting group of **2.6** using a solution of tetrabutylammonium fluoride (TBAF) in tetrahydrofuran gave the phenolic derivatives **2.7** which were converted to the final products compounds **2A** and **2B** under acidic conditions. Preparation of each of the ether derivatives **2.9** from the phenols **2.7** was achieved by alkylation reaction using the appropriate alkyl bromide **(2.8a, 2.8b)** (method 2A) or alkyl iodide **(2.8c)** reagent (method 2C). In some instances, the ether derivatives **2.9** were also obtained from the phenols **2.7** using the Mitsunobu conditions, i.e., condensation of the phenols **2.7** with the appropriate alcohol **(2.8d, 2.8e)** in the presence of triphenylphosphine and diisopropyl azodicarboxylate (DIAD) (method 2B). Treatment of the Boc derivatives **2.9** with hydrochloric acid provided the final compounds **2C-F.**

The synthesis of compounds **3A-AC** is outlined in Scheme 3. Conversion of the phenols **2.7** to the triflate derivatives **3.1** was achieved using the triflating reagent *N-*phenylbis(trifluoromethanesulphonimide) **1.4.** Palladium catalyzed carbonylation of **3.1,** conducted in methanol or in a mixture dimethylsulfoxide/methanol using palladium (II) acetate, 1,1'-bis(diphenylphosphino)ferrocene (dppf) and carbon monoxide, provided the methyl esters **3.2** which were hydrolyzed under basic conditions to give the carboxylic acid derivatives **3.3.** Coupling of the carboxylic acids **3.3** with various amines **(3.4a-3.4q)** using *O*-benzotriazol-1-yl-*N*,*N*,*N*',*N*'-tetramethyluronium tetrafluoroborate (TBTU) as coupling agent afforded the primary, secondary, and tertiary amides **3.5.** Treatment of the Boc derivatives **3.2, 3.3** and **3.5** with hydrochloric acid provided the final compounds **3A-3Y.** Suzuki type coupling of the triflate derivative **3.1a** (X = CH) with various organoboron reagents **(3.6a-3.6d)** in ethylene glycol dimethyl ether in the presence of tetrakis triphenylphosphine palladium (0), and/or dichloro[1,1'-bis(diphenylphosphino) ferrocene]palladium(II)dichloromethane, [Pd(dppf)Cl₂ CH₂Cl₂], lithium chloride, and an aqueous solution of sodium carbonate afforded compounds **3.7** which were converted to the final products (compounds **3Z-3AC)** under acidic conditions.

The synthesis of compounds **4A-4I** is outlined in Scheme 4. Treatment of compound **1A** with trifluoroacetic anhydride in tetrahydrofuran in the presence of triethylamine provided the trifluoroacetamide derivative **4.2** which was converted to the sulfonyl chloride **4.4** using sulfur trioxide *N,N*-dimethylformamide complex **(4.3)** as sulfating agent. Condensation of **4.4** with various primary and secondary amines **(3.4, 4.5)** afforded the sulfonamide derivatives **4.6** which were converted to the compounds **4A-4G** under basic conditions. Treatment of the sulfonyl chloride **4.4** with ammonium hydroxide in acetonitrile provided the sulfonamide compound **4H,** which was further protected as its *tert-*butyloxycarbonyl (Boc) derivative **4.8** buy treatment with *tert*-butyloxycarbonyl anhydride **(4.7).** Acetylation of **4.8** using acetic anhydride **(4.9)** gave the acetylsulfonamide derivative **4.10** which was converted to compound **41** by treatment with iodotrimethylsilane.

The synthesis of compound **5A** is described in Scheme 5. Condensation of hydrazine hydrate **(5.1)** with the sulfonyl chloride derivative **4.4** provided the sulfonyl hydrazide **5.2,** which was converted to the sulfone **5.3** by treatment with methyl iodide **(2.8c)** in the presence of sodium acetate. Deprotection of the trifluoroacetamide protecting group of **5.3** under basic conditions (potassium carbonate, methanol/tetrahydrofuran/water) provided the final compound **5A.**

The synthesis of compounds **6A-6E** is described in Scheme 6. Nitration of the trifluoroacetamide **4.2** using nitronium tetrafluoroborate complex **(6.1)** as nitrating reagent provided predominantly the *mono*-nitro isomer **6.2.** Reduction of the nitro functionality of **6.2** using tin(II) chloride dihydrate **(6.3)** gave the aniline derivative **6.4,** which reacted with the sulfonyl chloride derivatives **6.5** or with acetyl chloride **(6.7)** to provide the sulfonamides **6.6** or the acetamide **6.8,** respectively. Deprotection of the trifluoroacetamide protecting group of **6.2, 6.4, 6.6** and **6.8** under basic conditions (potassium carbonate, methanol/tetrahydrofuran/water) provided the final compounds (compounds **6A-6E).**

The synthesis of compounds **7A-7E** is described in Scheme 7. Buchwald type coupling of the triflate derivative **3.1a** with diphenylmethanimine **(7.1)** in toluene in the presence of tris(dibenzylideneacetone)dipalladium (0) [Pd₂(dba)₃], 1,1'-bis(diphenylphosphino)ferrocene (dppf) and sodium *tert*-butoxide afforded the benzophenone imine derivative **7.2,** which was converted to the aniline **7.3** by treatment with hydroxylamine hydrochloride in the presence of sodium acetate. Treatment of **7.3** with methanesulfonyl chloride **(7.4)** in dichloromethane in the presence of triethylamine provided the *bis-*methanesulfonamide **7.5,** which was hydrolyzed to the *mono* methanesulfonamide derivative **7.6** under basic conditions. Deprotection of the *tert*-butyloxycarbonyl protecting group of **7.6** under acidic conditions provided the final compound **7A.** Compound **7B** was obtained in two steps from **7.6.** Alkylation of **7.6** with methyl iodide **(2.8c)** in tetrahydrofuran in the presence of sodium hydride provided the *N*-methylsulfonamide **7.7,** which was converted to compound **7B** under acidic conditions. Treatment of the aniline derivative **6.4** with methanesulfonyl chloride **(7.4)** in dichloromethane in the presence of triethylamine provided the *bis-*methanesulfonamide **7.8,** which was hydrolyzed to the *mono*-methanesulfonamide derivative compound **7A** under basic conditions. During the course of this reaction, the *N*-methyl piperidine derivative compound **7C** was identified as a side product. The separation of the mixture containing compounds **7A** and **7C** was achieved by first treating the mixture of compounds **7A/7C** with *tert*-butyloxycarbonyl anhydride **(4.7)** which provided the Boc derivative **7.6** and unreacted compound **7C,** followed by purification of compound **7C** using flash colum chromatography. Buchwald type coupling of the triflate derivative **3.1a** with pyrrolidine **(3.4k)** or morpholine **(3.4p)** in ethylene glycol dimethyl ether in the presence of tris(dibenzylideneacetone)dipalladium (0) [Pd₂(dba)₃], the phosphine ligand 2-(di-*t-*butylphosphino)biphenyl **7.9** and potassium phosphate afforded the derivatives **7.10,** which were converted to compounds **7D,E** under acidic conditions.

The synthesis of compounds **8A-8F** is outlined in Scheme 8. The 2'-3'-dihydroxyacetophenone derivative **8.1** was condensed with 1-Boc-4-piperidone **1.2** in refluxing methanol in the presence of pyrrolidine to provide the *N*-Boc-spiro[2*H*-1-benzopyran-2,4'-piperidine]-4(3*H*)-one derivative **8.2** which was converted to the silyl ether derivative **8.3** using *tert*-butyldimethylsilyl chloride **2.3.** The ketone **8.3** was converted to the enol triflate derivative **8.4** using the triflating reagent *N-*phenylbis(trifluoromethanesulphonimide) **1.4.** Suzuki type coupling of the enol triflate derivative **8.4** with either 4-(*N*,*N*-diethylaminocarbonyl)phenyl boronic acid **1.6** or 2-(*N,N-*diethylaminocarbonyl)-5-(4,4,5,5-tetramethyl-1,3,2-dioxoborolan-2-yl)pyridine **1.7** in ethylene glycol dimethyl ether in the presence of palladium, 10 wt.% (dry basis) on activated carbon, lithium chloride, and an aqueous solution of sodium carbonate afforded compounds **8.5.** Removal of the silyl protecting group of **8.5** using a solution of tetrabutylammonium fluoride (TBAF) in tetrahydrofuran gave the phenolic derivatives **8.6** which were converted to the final products (compounds **8A** and **8B)** under acidic conditions. Preparation of the ether derivatives **8.7** from the phenols **8.6** was achieved by alkylation using the appropriate alkyl bromide **(2.8a)** or methyl iodide **(2.8c)** reagent. Treatment of the Boc derivatives **8.7** with hydrochloric acid provided the final compounds **8C-8F.**

The synthesis of compounds **9A-9B** is outlined in Scheme 9. The 2'-4'-dihydroxyacetophenone derivative **9.1** was condensed with 1-Boc-4-piperidone **1.2** in refluxing methanol in the presence of pyrrolidine to provide the *N*-Boc-spiro[2*H*-1-benzopyran-2,4'-piperidine]-4(3*H*)-one derivative **9.2** which was converted to the silyl ether derivative **9.3** using *tert*-butyldimethylsilyl chloride **2.3.** Conversion of the ketone **9.3** to the enol triflate derivative **9.4** was achieved using *N*-phenylbis(trifluoromethanesulphonimide) **1.4** as triflating reagent. Suzuki type coupling of the enol triflate derivative **9.4** with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the phenolic derivative **9.5** (simultaneous removal of the silyl protecting group occurred under the Suzuki coupling conditions). Alkylation of the phenol **9.5** with (bromomethyl)cyclopropane **(2.8a)** in acetone in the presence of potassium carbonate provided the ether derivative **9.6** which was converted to compound **9A** under acidic conditions. Treatment of the phenol **9.5** with methyl chlorodifluoroacetate **(9.7)** in *N*,*N*-dimethylformarnide in the presence of cesium carbonate provided the ether derivative **9.8** which was converted to compound **9B** under acidic conditions.

The synthesis of compounds **10A-10J** is outlined in Scheme 10. Conversion of the phenol **9.5** to the triflate derivative **10.1** was achieved using *N-*phenylbis(trifluoromethanesulphonimide) **1.4** as triflating reagent. Palladium catalyzed carbonylation of **10.1,** conducted in a mixture *N*,*N*-dimethylformamide/methanol using palladium (II) acetate, 1,1'-bis(diphenylphosphino)ferrocene (dppf), and carbon monoxide, provided the methyl ester **10.2** which was hydrolyzed under basic conditions to give the carboxylic acid derivative **10.3.** Coupling of the carboxylic acid **10.3** with various amines **(3.4a,c,j,k,p; 1.12)** using either *O*-(7-azabenzotriazol-1-yl)-*N*,*N*,*N*',*N*'-tetramethyluronium hexafluorophosphate (HATU) (method 10B) or *O*-benzotriazol-1-yl-*N*,*N*,*N*',*N*'-tetramethyluronium tetrafluoroborate (TBTU) (method 10A) as coupling agents afforded the primary, secondary, and tertiary amides **10.4.** The dimethylamide derivative **10.4b** (R₁ = H, R₂ = CH₃) was obtained by heating a mixture of the ester **10.2** with methylamine **(3.4b)** in methanol in a sealed tube. Treatment of the Boc derivatives **10.2, 10.3** and **10.4** with hydrochloric acid provided the final compounds **10A-10I.** Treatment of the ester **10.2** with lithium borohydride in tetrahydrofuran provided the primary alcohol **10.5** which was converted to the compound **10J** under acidic conditions.

The synthesis of compounds **11A-11I** is outlined in Scheme 11. The 2'-6'-dihydroxyacetophenone derivative **11.1** was condensed with 1-Boc-4-piperidone **1.2** in refluxing methanol in the presence of pyrrolidine to provide the *N*-Boc-spiro[2*H*-1-benzopyran-2,4'-piperidine]-4(3*H*)-one derivative **11.2** which was converted to the methoxymethyl (MOM) ether derivative **11.4** using chloro(methoxy)methane **(11.3).** Conversion of the ketone **11.4** to the enol triflate derivative **11.5** was achieved using *N-*phenylbis(trifluoromethanesulphonimide) **1.4** as triflating reagent. Suzuki type coupling of the enol triflate derivative **11.5** with either 4-(*N*,*N*-diethylaminocarbonyl)phenyl boronic acid **1.6** or 2-(*N*,*N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compounds **11.6.** Removal of the MOM and the Boc protecting groups of **11.6** in methanol at room temperature in the presence of hydrochloric acid (anhydrous solution in dioxane) afforded the phenolic compounds **11A** and **11B** which were converted to the corresponding Boc derivatives **11.7** by treatment with *tert-*butyloxycarbonyl anhydride **(4.7).** Preparation of the ether derivatives **11.9a** [X=CH; R = CH₂*c*(C₃H₅)], **11.9b** [X=N; R = CH₂*c*(C₃H₅)] and **11.9d** [X=N; R = *c*(C₅H₉)] from the corresponding phenols **11.7a** [X=CH] or **11.7b** [X=N] was achieved using the Mitsunobu conditions, i.e., condensation of the phenols **11.7a** or **11.7b** with cyclopropylmethanol **(2.8e)** or cyclopentanol **(11.10)** in dichloromethane in the presence of triphenylphosphine and diethyl azodicarboxylate (DEAD). The cyclobutyl ether **11.9c** [X=CH; R = *c*(C₄H₇)] was obtained by alkylation of the corresponding phenol **11.7a** [X=CH] with bromocyclobutane in acetone in the presence of potassium carbonate. Treatment of the Boc derivatives **11.9** with hydrochloric acid provided the final compounds **11C-11F.** Treatment of the phenol **11.2** with methyl chlorodifluoroacetate **(9.7)** in *N*,*N* dimethylformamide in the presence of cesium carbonate provided the ether derivative **11.11.** Conversion of the ketone **11.11** to the enol triflate derivative **11.12** was achieved using *N*-phenylbis(trifluoromethanesulphonimide) **1.4** as triflating reagent. Suzuki type coupling of the enol triflate derivative **11.12** with either 4-(*N*,*N*-diethylaminocarbonyl)phenyl boronic acid **1.6** or 2-(*N*,*N*-diethylaminocarbonyl)-5-(4,4,5,5-tetramethyl-1,3,2-dioxoborolan-2-yl)pyridine **1.7** in dioxane in the presence of dichloro[1,1'-bis(diphenylphosphino)ferrocene]palladium(II) dichloromethane adduct, potassium phosphate, and potassium bromide, afforded compounds **11.13.** Removal of the Boc protecting group of **11.13** in dichloromethane at room temperature in the presence of hydrochloric acid (anhydrous solution in diethyl ether) afforded the compounds **11G** and **11H.** Conversion of the aryl bromide **32.2b** to the corresponding boron derivative **11.14** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct. Suzuki type coupling of the enol triflate derivative **11.5** with the boron derivative **11.14** in ethylene glycol dimethyl ether in the presence of tetrakis triphenylphosphine palladium (0), potassium bromide and potassium phosphate afforded compound **11.15.** Removal of the Boc and the MOM protecting groups of **11.15** in methanol at room temperature in the presence of hydrochloric acid (anhydrous solution in diethyl ether) afforded the compound **11I.**

The synthesis of compounds **12A-12L** is outlined in Scheme **12.** Conversion of the phenol **11.2** to the triflate derivative **12.1** was achieved using *N-*phenylbis(trifluoromethanesulphonimide) **1.4** as triflating reagent. Palladium catalyzed Negishi-type coupling of **12.1** with methylzinc chloride **(12.2a),** propylzinc bromide **(12.2b),** or butylzinc bromide **(12.2c),** conducted in tetrahydrofuran using tetrakis triphenylphosphine palladium (0) as catalyst, provided the ketones **12.3.** Conversion of the ketones **12.3** to the enol triflate derivatives **12.4** was achieved using *N*-phenylbis(trifluoromethanesulphonimide) **1.4** as triflating reagent. Suzuki type coupling of the enol triflate derivative **12.4** with 4-(*N,N-*diethylaminocarbonyl)phenyl boronic acid **1.6** or 2-(*N*,*N*-diethylaminocarbonyl)-5-(4,4,5,5-tetramethyl-1,3,2-dioxoborolan-2-yl)pyridine **1.7** using either method 1C (tetrakis triphenylphosphine palladium (0), lithium chloride, aqueous solution of sodium carbonate, ethylene glycol dimethyl ether) or method 12A (tetrakis triphenylphosphine palladium (0), potassium bromide, potassium phosphate, dioxane) afforded compounds **12.5.** Removal of the Boc protecting group of **12.5** in dichloromethane at room temperature in the presence of hydrochloric acid (anhydrous solution in diethyl ether) afforded compounds **12A** and **12H-12L.** Palladium catalyzed carbonylation of **12.1,** conducted in a mixture *N*,*N-*dimethylformamide/methanol using palladium (II) acetate, 1,3-bis(diphenylphosphino)propane (dppp) and carbon monoxide, provided the methyl ester **12.6** which was hydrolyzed under basic conditions (lithium hydroxide, methanol/tetrahydrofuran) to give the carboxylic acid derivative **12.7.** Coupling of the carboxylic acid **12.7** with dimethylamine **(3.4j)** using *O*-benzotriazol-1-yl-*N*,*N*,*N*',*N*'-tetramethyluronium tetrafluoroborate (TBTU) as coupling agent afforded the dimethylaminocarbonyl derivative **12.8.** Conversion of **12.8** to the enol triflate derivative **12.9** was achieved using *N-*phenylbis(trifluoromethanesulphonimide) **1.4** as triflating reagent. Suzuki type coupling of the enol triflate derivative **12.9** with 4-(*N*,*N*-diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded compound **12.10.** Removal of the Boc protecting group of **12.10** in dichloromethane at room temperature in the presence of hydrochloric acid (anhydrous solution in diethyl ether) afforded compound **12G** (R₁ = R₂ = CH₃). Conversion of **12.6** to the enol triflate derivative **12.11** was achieved using *N-*phenylbis(trifluoromethanesulphonimide) **1.4** as triflating reagent. Suzuki type coupling of the enol triflate derivative **12.11** with 4-(*N*,*N* diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the ester **12.12** which was hydrolyzed under basic conditions (potassium *tert*-butoxide, diethyl ether, water) to give the carboxylic acid **12.13.** Coupling of the carboxylic acid **12.13** with various amines **(12.15** or **3.4b-3.4d)** using *O*-benzotriazol-1-yl-*N*,*N*,*N*',*N*'-tetramethyluronium tetrafluoroborate (TBTU) as coupling agent afforded the primary and secondary aminocarbonyl derivatives **12.14.** Treatment of the Boc derivatives **12.13** and **12.14** with hydrochloric acid provided the final compounds **12B-12F.**

The synthesis of compounds **13A-13S** is outlined in Scheme 13. The 2'-hydroxyacetophenone derivative **1.1a** was condensed with 1-Boc-4-piperidone **1.2** in refluxing methanol in the presence of pyrrolidine to provide *N*-Boc-spiro[2*H*-1-benzopyran-2,4'-piperidine]-4(3*H*)-one **1.3a.** Conversion of **1.3a** to the enol triflate derivative **1.5a** was achieved using *N*-phenylbis(trifluoromethanesulphonimide) **1.4** as triflating reagent. Suzuki type coupling of the enol triflate derivative **1.5a** with 4-(methoxycarbonyl)phenylboronic acid **(13.1)** in ethylene glycol dimethyl ether in the presence of tetrakis triphenylphosphine palladium (0), lithium chloride, and an aqueous solution of sodium carbonate afforded the ester **13.2** which was hydrolyzed under basic conditions (lithium hydroxide, methanol/tetrahydrofuran/water) to give the carboxylic acid **13.3.** Coupling of the carboxylic acid **13.3** with various amines **(3.4a-3.4c, 3.4e, 3.4j-3.4k, 3.4o-3.4q; 13.4a-13.4h)** using *O-*benzotriazol-1-yl-*N*,*N*,*N*',*N*'-tetramethyluronium tetrafluoroborate (TBTU) as coupling agent afforded the primary, secondary, and tertiary aminocarbonyl derivatives **13.5.** Treatment of the Boc derivatives **13.3** and **13.5** with hydrochloric acid provided the final compounds **13A-13R.** Hydrolysis of compound **130** under basic conditions (sodium hydroxide, ethanol/tetrahydrofuran) provided the carboxylic acid compound **13S.**

The synthesis of compounds **14A-14C** is outlined in Scheme 14. Suzuki type coupling of the enol triflate derivative **1.5a** with 4-cyanophenylboronic acid **(14.1)** in ethylene glycol dimethyl ether in the presence of tetrakis triphenylphosphine palladium (0), lithium chloride, and an aqueous solution of sodium carbonate afforded the cyanide **14.2** which was converted to the tetrazole **14.4** using sodium azide **(14.3)** and zinc bromide in a solution isopropanol/water. Alkylation of **14.4** with methyl iodide **(2.8c)** in N,N-dimethylformamide in the presence of triethylamine afforded the two regioisomers **14.5** (major isomer) and **14.6** (minor isomer) separated by silica gel column chromatography. The Boc protecting group of **14.4, 14.5,** and **14.6** was removed using hydrochloric acid to generate the compounds **14A-14C.** Alternatively, the Boc protecting group of **14.4** was also removed using trifluoroacetic acid to give **14A.**

The synthesis of compounds **15A-15N** is outlined in Scheme 15. Alkylation of **14.4** with the alkyl bromide derivatives **15.1a-15.1e** in *N*,*N*-dimethylformamide in the presence of triethylamine afforded the regioisomers **15.2** (major isomers) and **15.3** (minor isomers) separated by silica gel column chromatography. The Boc protecting group of **15.2** and **15.3** was removed using hydrochloric acid to generate the compounds **15A-15J.** Hydrolysis of compounds **15A** or **15C-15E** under basic conditions (sodium hydroxide, methanol (or ethanol) /tetrahydrofuran/water) provided the corresponding carboxylic acids compounds **15K-15N,** respectively. In some instances, compounds **15K-15N** were also obtained in two steps from **15.2,** i.e. by basic hydrolysis of the ester functionality of **15.2** followed by deprotection of the Boc derivatives **15.4** under acidic conditions.

The synthesis of compounds **16A-16C** is outlined in Scheme 16. Suzuki type coupling of the enol triflate derivative **1.5a** with 3-cyanophenylboronic acid **(16.1)** in ethylene glycol dimethyl ether in the presence of tetrakis triphenylphosphine palladium (0), lithium chloride, and an aqueous solution of sodium carbonate afforded the cyanide **16.2** which was converted to the tetrazole **16.3** using sodium azide **(14.3)** and zinc bromide in a solution isopropanol/water. Alkylation of **16.3** with methyl iodide **(2.8c)** in *N,N-*dimethylformamide in the presence of triethylamine afforded the two regioisomers **16.4** (major isomer) and **16.5** (minor isomer) separated by silica gel column chromatography. The Boc protecting group of **16.3, 16.4,** and **16.5** was removed using hydrochloric acid to generate the compounds **16A-16C.**

The synthesis of compounds **17A-17F** is outlined in Scheme 17. Alkylation of **16.3** with the alkyl bromide derivatives **15.1a** or **15.1c** in *N*,*N*-dimethylformamide in the presence of triethylamine afforded the regioisomers **17.1** (major isomers) and **17.2** (minor isomers) separated by silica gel column chromatography. Alkylation of **16.3** with 4-(2-bromoethyl)morpholine **(17.3)** in *N*,*N*-dimethylformamide in the presence of triethylamine afforded the isomer **17.4.** The Boc protecting group of **17.1, 17.2,** and **17.4** was removed using hydrochloric acid to generate the compounds **17A-17D.** Hydrolysis of compounds **17A** and **17B** under basic conditions (sodium hydroxide, methanol/ tetrahydrofuran/water) provided the corresponding carboxylic acids compound **17E** and compound **17F,** respectively. In some instances compounds **17E** and **17F** could also be obtained in two steps from **17.1,** i.e. by basic hydrolysis of the ester functionality of **17.1** followed by deprotection of the Boc derivatives **17.5** under acidic conditions.

The synthesis of compounds **18A-18C** is outlined in Scheme 18. Coupling of the carboxylic acid **13.3** with ammonium chloride **(3.4a)** in acetonitrile in the presence of diisopropylethylamine using *O*-benzotriazol-1-yl-*N*,*N*,*N*',*N*'-tetramethyluronium tetrafluoroborate (TBTU) as coupling agent afforded the primary aminocarbonyl derivative **13.5a** which was converted to the thioamide **18.2** using the Lawesson's reagent **(18.1)** [2,4-bis(4-methoxyphenyl)-1,3-dithia-2,4-diphosphetane-2,4-disulfide]. Condensation of the thioamide **18.2** with 1-bromo-3,3-dimethylbutan-2-one **(18.3a)** or 2-bromo-1-phenylethanone **(18.3b)** afforded the thiazole derivatives **18.4** which were converted to the final compounds (compounds **18A** and **18B)** under acidic conditions. Condensation of the nitrile derivative **14.2** with hydroxylamine hydrochloride **(18.5)** in ethanol in the presence of triethylamine afforded the *N*-hydroxybenzamidine derivative **18.6** which reacted with acetyl chloride **(6.7)** in refluxing pyridine to give the 1,2,4-oxadiazole derivative **18.7.** Deprotection of the Boc functionality of **18.7** under acidic conditions afforded compound **18C.**

The synthesis of compound **19A-19D** is outlined in Scheme 19. The 2'-hydroxyacetophenone **1.1a** was condensed with benzyl 4-oxopiperidine-1-carboxylate **(19.1)** in refluxing methanol in the presence of pyrrolidine to provide *N*-Cbz-spiro[2*H*-1-benzopyran-2,4'-piperidine]-4(3*H*)-one **(19.2).** Conversion of the ketone **19.2** to the enol triflate derivative **19.3** was achieved using *N*-phenylbis(trifluoromethanesulphonimide) **1.4** as triflating reagent. Conversion of the enol triflate **19.3** to the corresponding boron derivative **19.4** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct, abbreviated as [Pd(dppf)Cl₂•CH₂Cl₂]. Suzuki type coupling of the boronate derivative **19.4** with *tert*-butyl 4-bromophenylcarbamate **19.5** in ethylene glycol dimethyl ether in the presence of tetrakis triphenylphosphine palladium (0), lithium chloride, and an aqueous solution of sodium carbonate afforded the *tert*-butyloxycarbonyl (Boc) protected aniline derivative **19.6.** Acidic hydrolysis of **19.6** provided the aniline derivative **19.7** which reacted with acyl chlorides **19.8a, 19.8b,** isopropylsulfonyl chloride **(6.5b)** or ethyl isocyanate **(19.11)** to give the corresponding amide derivatives **19.9,** sulfonamide derivative **19.10** or urea derivative **19.12,** respectively. The derivatives **19.9, 19.10** and **19.12** were converted to compounds **19A-19D** by treatment with iodotrimethylsilane.

The synthesis of compounds **20A-20R** is outlined in Scheme 20. The tertiary amine derivatives compounds **20A-20R** were obtained from the secondary amines of general formula **20I,** by reductive amination methods (methods 20A or 20B) using the aldehydes **20.1a-20.1d** and sodium cyanoborohydride as reducing agent or by alkylation method (method 20C) using the bromides **2.8a, 20.2a-e** as the alkylating reagent.

The synthesis of compounds **21A-21F** is outlined in Scheme 21. Condensation of 1-Boc-4-piperidone **1.2** with ethyl diazoacetate **(21.1)** in the presence of boron trifluoride diethyl etherate provided 1-*tert*-butyl 4-ethyl 3-oxoazepane-1,4-dicarboxylate in equilibrium with its enol form **(21.2).** Ester hydrolysis followed by decarboxylation of **21.2** under acidic conditions provided the azepan-3-one **(21.3),** which was protected as its Boc derivative **21.4** by treatment with *tert*-butyloxycarbonyl anhydride **(4.7).** The 2'-hydroxyacetophenone **1.1a** was condensed with **21.4** in refluxing methanol in the presence of pyrrolidine to provide the racemic ketone **21.5.** Conversion of **21.5** to the enol triflate derivative **21.6** was achieved using the triflating reagent *N*-phenylbis(trifluoromethanesulphonimide) **1.4.** Suzuki type coupling of the enol triflate derivative **21.6** with 4-(*N*,*N*-diethylaminocarbonyl)phenyl boronic acid **(1.6)** in ethylene glycol dimethyl ether in the presence of tetrakis triphenylphosphine palladium (0), lithium chloride, and an aqueous solution of sodium carbonate afforded the racemic derivative **21.7,** which was hydrolyzed under acidic conditions to give the compound **21A** (racemic mixture). The two enantiomers derived from **21.7,** i.e. compounds **21.7a** and **21.7b,** were separated by chiral HPLC. The pure enantiomers **21.7a** and **21.7b** were converted to compounds **21B** and **21C,** respectively under acidic conditions. Palladium catalyzed hydrogenation of compounds **21B** and **21C** afforded compounds **21D** (diastereoisomeric mixture) and **21E** (diastereoisomeric mixture), respectively. Treatment of compound **21A** with benzyl chloroformate **(21.8)** in dichloromethane in the presence of triethylamine provided the Cbz-protected derivative **21.9,** which was converted to the sulfonyl chloride **21.10** using sulfur trioxide *N,N-*dimethylformamide complex **(4.3)** as sulfating agent. Condensation of **21.10** with ethylamine **(3.4c)** in dichloromethane in the presence of triethylamine, afforded the ethyl sulfonamide derivative **21.11** which was converted to compound **21F** by treatment with iodotrimethylsilane. Condensation of compound **21C** with 6-bromo-2-naphthoic acid **(21.12)** in acetonitrile in the presence of TBTU and diisopropylethylamine (Hunig's base) provided the amide derivative **21.13.** The absolute configuration of **21.13** was determined by X-ray crystallography, therefore establishing the absolute configuration of compound **21C,** and therefore by inference, its enantiomer, compound **21B.**

The synthesis of compounds **22A-22F** is outlined in Scheme 22. Treatment of compound **21B** (most active enantiomer) with trifluoroacetic anhydride **(4.1)** in tetrahydrofuran in the presence of triethylamine provided the trifluoroacetamide derivative **22.1** which was converted to the sulfonyl chloride **22.2** using sulfur trioxide *N,N-*dimethylformamide complex **(4.3)** as sulfating agent. Condensation of **22.2** with various primary amines **(3.4b, 3.4c, 3.4d, 3.4g)** afforded the sulfonamide derivatives **22.3** which were converted to compounds **22A-22D** under basic conditions. Condensation of hydrazine hydrate **(5.1)** with the sulfonyl chloride derivative **22.2** provided the sulfonyl hydrazide **22.4,** which was converted to the sulfones **22.5** and **22.7** by treatment with methyl iodide **(2.8c)** and ethyl iodide **(22.6),** respectively, in the presence of sodium acetate. Deprotection of the trifluoroacetamide protecting group of **22.5** and **22.7** under basic conditions (potassium carbonate, methanol, water) provided the corresponding methyl sulfone (compound **22E)** and ethyl sulfone (compound **22F)** derivatives.

The synthesis of compounds **23A-23C** is outlined in Scheme 23. The 2'-hydroxyacetophenone **1.1a** was condensed with *tert*-butyl 3-oxopyrrolidine-1-carboxylate **(23.1a)** or *tert*-butyl 3-oxopiperidine-1-carboxylate **(23.1b)** in refluxing methanol in the presence of pyrrolidine to provide the racemic ketones **23.2a** (n=0) and **23.2b** (n=1), respectively. Conversion of the ketones **23.2** to the enol triflate derivatives **23.3** was achieved using *N*-phenylbis(trifluoromethanesulphonimide) **1.4** as triflating reagent. Suzuki type coupling of the enol triflate derivatives **23.3** with 4-(*N*,*N*-diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the Boc derivatives **23.4** which were converted to the final products compounds **23A** and **23B** (racemic mixtures) under acidic conditions. The 2'-hydroxyacetophenone **1.1a** was also condensed with 1-Boc-4-nortropinone **(23.5)** in refluxing methanol in the presence of pyrrolidine to provide the ketone **23.6.** Conversion of the ketone **23.6** to the enol triflate derivative **23.7** was achieved using *N-*phenylbis(trifluoromethanesulphonimide) **1.4** as triflating reagent. Suzuki type coupling of the enol-triflate derivative **23.7** with 4-(*N*,*N*-diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the Boc derivative **23.8** which was converted to the final product compound **23C** under acidic conditions.

The synthesis of compounds **24A-24C** is outlined in Scheme 24. Treatment of the amine **24.1** with ethyl 2,2,2-trifluoroacetate **(59.3)** in methanol in the presence of triethylamine provided the trifluoroacetamide derivative **24.2.** Treatment of the alcohol **24.2** with methane sulfonyl chloride in dichloromethane in the presence of triethylamine provided the mesylate derivative **24.3.** Alkylation of 5-(4-bromophenyl)-2H-tetrazole **(59.6)** with the mesylate derivative **24.3,** provided the derivative **24.4.** Suzuki type coupling of the boronate derivative **32.1** with the aryl bromide derivative **24.4** in dioxane in the presence of tetrakis triphenylphosphine palladium (0) and an aqueous solution of potassium carbonate afforded the compound **24.5** which was converted to the derivative **24.6** under basic conditions. The Boc protecting group of **24.6** was removed using hydrochloric acid to generate the compound **24A.** Treatment of the amine **24.6** with acetic anhydride in dichloromethane in the presence of triethylamine provided the acetamide **24.7,** which was converted to the final products **24B** under acidic conditions. Treatment of the amine **24.6** with methane sulfonyl chloride in dichloromethane in the presence of triethylamine provided the sulfonamide **24.8,** which was converted to the final products **24C** under acidic conditions.

The synthesis of compounds **25A-25B** is outlined in Scheme 25. Chiral separation of the enantiomers derived from **25.1** (obtained by hydrogenation of **49.9**) provided compounds **25.2a** and **25.2b,** which were converted to the final products **25A** and **25B**, respectively, under acidic conditions.

The synthesis of compounds **26A-26B** is outlined in Scheme 26. Palladium catalyzed Negishi-type coupling of **1.5a** with 4-cyanobenzylzinc bromide (**26.1**) conducted in tetrahydrofuran using tetrakis triphenylphosphine palladium (0) as catalyst, provided the nitrile **26.2**. Acidic hydroysis of the nitrile **26.2** provided the carboxylic acid derivatives **26.3a** and **26.3b** (compounds **26.3a** and **26.3b** were separated by column chromatography; however, the following step was conducted using the mixture **26.3a** / **26.3b**). Treatment of **the** mixture **26.3a** / **26.3b** with methanol in the presence of hydrochloric acid afforded the piperidine esters **26.4a** / **26.4b** which were converted to the corresponding Boc derivatives **26.5a** / **26.5b** by treatment with *ter*t-butyloxycarbonyl anhydride (**4.7**)**.** Hydrolysis of the esters **26.5a** / **26.5b** in basic conditions gave the carboxylic acid derivatives **26.6a** / **26.6b.** Coupling of the carboxylic acid derivatives **26.6a** / **26.6b** with diethylamine (**1.12**) using *O-*benzotriazol-1-yl-*N,N,N',N*'-tetramethyluronium tetrafluoroborate (TBTU) as coupling agent afforded the dimethylaminocarbonyl derivatives **26.7a** / **26.7b.** Removal of the Boc protecting group of **26.7a** / **26.7b** in dichloromethane at room temperature in the presence of hydrochloric acid (anhydrous solution in dioxane) afforded compounds **26A** and **26.8** which were separated by column chromatography. Palladium catalyzed hydrogenation of compound **26.8** afforded compound **26B.**

The synthesis of compounds **27A-27W** is outlined in Scheme 27. The saturated derivatives (compounds **27A, 27D, 27G, 27H, 27K, 27N**, and **27W,** as their racemic mixtures) were obtained by hydrogenation of the unsaturated analogs (compounds 1A, 1D, **2C, 1N, 10, 1S,** and **1E**), respectively, in methanol in the presence of palladium, 10 wt.% (dry basis) on activated carbon (method 27A) or palladium hydroxide, 20 wt.% Pd (dry basis) on carbon (Pearlman's catalyst (method 27B)). Hydrogenation of **11.6a** in methanol in the presence of palladium hydroxide, 20 wt.% Pd (dry basis) on carbon (Pearlman's catalyst) provided the saturated derivative **27.1**. Acidic hydrolysis of **27.1** provided compound **27T.** Hydrolysis of **2.7a** in methanol in the presence of palladium, 10 wt.% (dry basis) on activated carbon, provided the saturated derivative **27.6**. Acidic hydrolysis of **27.6** provided the compound **27Q.** Chiral separation of the enantiomers derived from **27.1** provided compounds **27.4** and **27.5.** The enantiomers **27.4** and **27.5** were converted to compounds **27U** and **27V,** respectively under acidic conditions. Chiral separation of the enantiomers derived from each of the racemic compounds (compounds **27A, 27D**, **27G, 27H, 27K, 27N, 27Q** and **27W)** provided compounds **27B, 27E, 271, 27L, 270, 27R** (pure enantiomer) and compounds **27C, 27F, 27J, 27M, 27P, 27S** (pure enantiomer). Condensation of compound **27B** with (1S)-(+)-10-camphorsulfonyl chloride (**27.2**) (used as chiral resolving agent) in dichloromethane in the presence of triethylamine provided the chiral sulfonamide derivative **27.3.** The absolute configuration of **27.3** was determined by X-ray crystallography, therefore establishing the absolute configuration of compound **27B**, and therefore by inference, its enantiomer, compound **29C**.

The synthesis of compounds **28A-28E** is outlined in Scheme 28. Condensation of benzyl 4-oxopiperidine-1-carboxylate (**19.1**) with ethyl cyanoacetate (**28.1**) in the presence of acetic acid and ammonium acetate gave the unsaturated ester **28.2.** Compound **28.2** was subjected to conjugate addition by reaction with organo cuprate reagents derived from benzyl or methoxybenzyl magnesium chloride (**28.3a** and **28.3b**, respectively) and copper (I) cyanide to yield the cyano esters **28.4.** Treatment of the conjugate addition product **28.4a** (R^{V} =H) with concentrated sulfuric acid at 90 °C provided the amino ketone **28.5**. Treatment of **28.5** with benzyl chloroformate (21.8) in dichloromethane in the presence of triethylamine provided the corresponding Cbz-protected derivative **28.6a** (R^{v} =H). Decarboxylation of **28.4b (**R^{v} =OCH₃) by treatment with sodium chloride in dimethylsulfoxide containing small amount of water at 160°C afforded the nitrile **28.9.** Hydrolysis of the nitrile functionality of **28.9** to the methyl ester group by treatment with methanol in the presence of sulfuric acid provided the corresponding piperidine derivative (Cleavage of the Cbz protecting group of 28.9 occured during the course of the hydrolysis). Treatment of the piperidine derivative with benzyl chloroformate afforded the compound **28.10.** The ester **28.10** was hydrolyzed with lithium hydroxide to furnish the carboxylic acid **28.11.** Treatment of the acid **28.11** with oxalyl chloride followed by reaction of the resulting acyl chloride with aluminum chloride yielded the corresponding spiro piperidine derivative which was further protected as its CBz derivative **28.6b** (R^{V} =OCH₃) by treatment with benzylchloroformate. Conversion of the ketones **28.6** to the enol triflate derivatives **28.7** was achieved using N-phenylbis(trifluoromethanesulphonimide) 1.4 as triflating reagent. Suzuki type coupling of the enol triflate derivatives **28.7** with 4-(*N,N-*diethylaminocarbonyl)phenyl boronic acid 1.6 in ethylene glycol dimethyl ether in the presence of tetrakis triphenylphosphine palladium (0), lithium chloride, and an aqueous solution of sodium carbonate afforded the derivatives 28.8 which were converted to the compounds **28A** and **28B** by treatment with iodotrimethylsilane. The compounds **28C** and **28D** (racemic mixtures) were obtained by hydrogenation of unsaturated derivatives **28.8** in methanol in the presence of palladium, 10 wt.% (dry basis) on activated carbon. Suzuki type coupling of the enol triflate derivative **28.7a** (R^{v} =H) with 2-(*N,N-*diethylaminocarbonyl)-5-(4,4,5,5-tetramethyl-1,3,2-dioxoborolan-2-yl)pyridine 1.7 in ethylene glycol dimethyl ether in the presence of tetrakis triphenylphosphine palladium (0), lithium chloride, and an aqueous solution of sodium carbonate afforded the derivative **28.12** which was converted to the compound 28E by treatment with iodotrimethylsilane.

The synthesis of compounds **29A-29D** is outlined in Scheme 29. The Negishi coupling of the enol triflate **28.7a** with 4-(ethoxycarbonyl)phenylzinc iodide (**29.1**) in tetrahydrofuran in the presence of tetrakis triphenylphosphine palladium (0) gave the ester **29.2,** which was hydrolyzed with lithium hydroxide to afford the carboxylic acid **29.3**. Coupling of the carboxylic acid **29.3** with *iso*propylamine **(3.4h)** or 1-ethylpropylamine **(29.4)** using 2-chloro-1-methylpyridinium iodide (Mukaiyama acylating reagent) as coupling agent afforded the secondary aminocarbonyl derivatives **29.5**, which were converted to the compounds **29A** and **29B** by treatment with iodotrimethylsilane. Curtius rearrangement of the carboxylic acid **29.3** by reaction with diphenylphosphoryl azide (**29.6**) in the presence of *tert*-butyl alcohol provided the *tert*-butyloxycarbonyl (Boc) protected aniline derivative **29.7.** Acidic hydrolysis of **29.7** provided the aniline derivative **29.8** which reacted with propionyl chloride **29.9** or methanesulfonyl chloride (**7.4**) to give the corresponding amide derivative **29.10** or sulfonamide derivative **29.11,** respectively. The derivatives **29.10** and **29.11** were converted to compounds **29C** and **29D,** respectively, by treatment with iodotrimethylsilane.

The synthesis of compound **30A** is outlined in Scheme 30. Wittig type condensation of 1-benzoyl-4-piperidone (**30.1**) with methyl(triphenylphosphoranylidene)acetate (**30.2**) in toluene gave the unsaturated ester **30.3**. Compound **30.3** was subjected to conjugate addition by reaction with benzenethiol (**30.4**) to yield the thioether **30.5**. Treatment of the conjugate addition product **30.5** with concentrated sulfuric acid provided the cyclized product **30.6**, which was converted to the sulfone **30.7** by oxidation using a solution of hydrogen peroxide in glacial acetic acid. Acidic hydrolysis of **30.7** provided the amine **30.8,** which was treated with *tert*-butyloxycarbonyl anhydride (**4.7**) to give the Boc protected derivative **30.9.** Conversion of the ketone **30.9** to the enol triflate derivative **30.10** was achieved using *N-*phenylbis(trifluoromethanesulphonimide) **1.4** as triflating reagent. Suzuki type coupling of the enol triflate derivative **30.10** with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the derivative **30.11** which was converted to compound **30A** under acidic conditions.

The synthesis of compounds **31A-31AA** is outlined in Scheme 31. Suzuki type coupling of the enol triflate derivative **1.5a** with the commercially available boronic acid derivatives **13.1, 14.1, 16.1** or **31.1a-31.1u** in ethylene glycol dimethyl ether in the presence of tetrakis triphenylphosphine palladium (0), lithium chloride, and an aqueous solution of sodium carbonate afforded compounds **13.2, 14.2, 16.2** and **31.2,** respectively. Compounds **13.2, 14.2, 16.2** and **31.2** were converted to the final products compounds **31A-31X** under acidic conditions (method 1E: anhydrous HC1, diethyl ether, room temperature or method 1F: neat trifluoroacetic acid (with optional dichloromethane), room temperature or method 31A: anhydrous HC1, methanol, dioxane, reflux). Treatment of the nitrile **16.2** with lithium aluminum hydride in tetrahydrofuran provided the diamine compound **31Y**, which reacted with acetyl chloride (6.7) or methanesulfonyl chloride (**7.4**) to give the corresponding amide derivative compounds **31Z** or the sulfonamide derivative compound **31AA**, respectively.

The synthesis of compounds **32A-32Z** is outlined in Scheme 32. Conversion of the enol triflate **1.5a** to the corresponding boron derivative **32.1** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct, abbreviated as [Pd(dppf)Cl₂•CH₂Cl₂]. Suzuki type coupling of the boronate derivative 32.1 with various aryl bromide derivatives **32.2** under different conditions [method 1C: ethylene glycol dimethyl ether, tetrakis triphenylphosphine palladium (0), lithium chloride, aqueous solution of sodium carbonate; method 1D: ethylene glycol dimethyl ether, palladium, 10 wt.% (dry basis) on activated carbon, lithium chloride, aqueous solution of sodium carbonate; method 12A: tetrakis triphenylphosphine palladium (0), potassium bromide, potassium phosphate, dioxane] afforded the derivatives **32.3,** which were converted to compounds **32A-321** or **32K-32Z** under acidic conditions. The *tert*-butyl sulfonamide derivative compound **32.3b** was converted to the sulfonamide compound **32J** by treatment with trifluoroacetic acid. The derivatives **32.2** used in the Suzuki coupling step were prepared as follows. Coupling of the carboxylic acid **32.4** with diethylamine (**1.12**) using 2-chloro-1-methylpyridinium iodide (Mukaiyama acylating reagent) as coupling agent afforded 2-(4-bromophenyl)*-N,N-*diethylacetamide (**32.2a**). The sulfone derivatives **32.2j-32.2p** were obtained in two steps from 4-bromobenzenethiol (**32.7**). Alkylation of **32.7** with the alkyl bromide derivatives **20.2, 2.8** or **32.8** in acetonitrile in the presence of triethylamine (method 32A) or in *N,N-*dimethylformamide in the presence of sodium hydride (method 32B) provided the thioether derivatives **32.9**, which were oxidized to the sulfone derivatives **32.2j-32.2p** in glacial acetic acid in the presence of an aqueous solution of hydrogen peroxide. Coupling of 4-bromobenzene-1-sulfonyl chloride (**32.5**) with various amines (**3.4, 1.12, 13.4** or **32.6**) in tetrahydrofuran in the presence of triethylamine provided the sulfonamides **32.2b-32.2i.** Acylation of *N-*methyl-4-bromoaniline (**32.10**) with various acyl chloride derivatives ***(*19.8, 32.11** or **6.7**) in dichloromethane in the presence of triethylamine provided the amides **32.2q-32.2u, 32.2x, 32.2y**. The aryl bromides **32.2v** and **32.2w** are commercially available.

The synthesis of compounds **33A-33N** is outlined in Scheme 33. Suzuki type coupling of the boronate derivative **32.1** with various aryl bromide derivatives **33.1** under different conditions [method 1C: ethylene glycol dimethyl ether, tetrakis triphenylphosphine palladium (0), lithium chloride, aqueous solution of sodium carbonate; method 1D: ethylene glycol dimethyl ether, palladium, 10 wt.% (dry basis) on activated carbon, lithium chloride, aqueous solution of sodium carbonate; method 33A: ethylene glycol dimethyl ether, dichloro[1,1'-bis(diphenylphosphino)ferrocene]palladium(II) dichloromethane adduct, abbreviated as [Pd(dppf)C1₂•CH₂C1₂], lithium chloride, potassium phosphate; method 33B: dioxane, tetrakis triphenylphosphine palladium (0), potassium bromide, potassium phosphate] afforded the derivatives **33.2,** which were converted to compounds **33A-33K, 33M** and **33N** under acidic conditions. The derivatives **33.1** used in the Suzuki coupling step were either obtained from commercial sources (**33.1 a-e,l,m**) or prepared as follows. Coupling of 5-bromopyridine-3-carboxylic acid (**33.3**) or 6-bromopyridine-2-carboxylic acid (**33.4**) with diethylamine (**1.12**) using *O*-benzotriazol-1-yl-*N,N,N',N'*-tetramethyluronium tetrafluoroborate (TBTU) as coupling agent afforded the diethylaminocarbonyl derivative derivatives **33.1f** and **33.1g,** respectively. Treatment of 2,5-dibromopyridine (**1.9**) with *n-*butyllithium provided the corresponding lithiated derivative, which reacted with carbon dioxide to provide 5-bromopyridine-2-carboxylic acid **1.10.** The carboxylic acid **1.10** was also obtained by acidic hydrolysis of commercially available 5-bromopyridine-2-carbonitrile (**33.1e**). Treatment of the carboxylic acid derivative **1.10** with oxalyl chloride furnished the acyl chloride **1.11,** which reacted with dimethylamine (**3.4j**), ethylamine (**3.4c**) or methylamine (**3.4b**) to provide the corresponding aminocarbonyl derivatives **33.1h, 33.1i** and **33.1j,** respectively. Treatment of commercially available 5-bromo-2-iodopyrimidine (**33.5**) with *n*-butyllithium provided the corresponding lithiated derivative, which reacted with carbon dioxide to provide 5-bromopyrimidine-2-carboxylic acid (**33.6**). Treatment of the carboxylic acid derivative **33.6** with oxalyl chloride furnished the acyl chloride **33.7**, which reacted with diethylamine **1.12** to provide 5-bromo-2-(*N,N*-diethylaminoarbonyl)-pyrimidine **33.1k**.

Hydrolysis of the nitrile derivative **33.2a** under acidic conditions provided the carboxylic acid derivative compound **33E** and compound **33L**. Compound **33E** and compound **33L** were readily separated by column chromatography.

The synthesis of compounds **34A-34P** is outlined in Scheme 34. Suzuki type coupling of the boronate derivative **32.1** with various aryl bromide derivatives **34.1** in ethylene glycol dimethyl ether in the presence of tetrakis triphenylphosphine palladium (0), lithium chloride, and an aqueous solution of sodium carbonate afforded compounds **34.2** which were converted to the final products compounds **34A-34P** under acidic conditions. The derivatives **34.1** used in the Suzuki coupling step were prepared as follow. Coupling of 6-bromopyridine-3-carboxylic acid (**34.3**), 5-bromothiophene-2-carboxylic acid (**34.4**), 4-bromothiophene-2-carboxylic acid (**34.7**) or 5-bromofuran-2-carboxylic acid (**34.6**) with diethylamine (**1.12**) or diisopropylamine **(3.4o)** using O-benzotriazol-1-yl-*N,N,N',N'-*tetramethyluronium tetrafluoroborate (TBTU) as coupling agent afforded the diethylaminocarbonyl derivatives **34.1 a-d, f-i.** Coupling of 5-bromothiophene-2-sulfonyl chloride (**34.5**) with diethylamine (**1.12**) in acetonitrile in the presence of triethylamine provided the sulfonamide **34.1e.** Coupling of the commercially available carboxylic acid derivatives **34.8a-34.8f** and **34.9** with diethylamine (**1.12**) using *O*-benzotriazol-1-yl-N,N,N',N'-tetramethyluronium tetrafluoroborate (TBTU) as coupling agent afforded the corresponding diethylaminocarbonyl derivatives 34.1j-34.1o and 34.1p.

The synthesis of compounds 35A and 35B is outlined in Scheme 35. Iodination of 3-hydroxybenzoic acid (35.1) afforded 3-hydroxy-4-iodobenzoic acid (35.2), which was converted to the methyl ester 35.3 under standard esterification conditions. Alkylation of the phenolic derivative 35.3 with methyl iodide (2.8c) in acetone in the presence of potassium carbonate afforded the methyl ether 35.4, which was converted to the carboxylic acid 35.5 in the presence of lithium hydroxide. Coupling of the carboxylic acid derivatives 35.5 with diethylamine (1.12) using *O*-benzotriazol-1-yl-*N,N,N',N*'-tetramethyluronium tetrafluoroborate (TBTU) as coupling agent afforded the corresponding diethylaminocarbonyl derivative **35.6.** Demethylation of **35.6** using boron tribromide afforded the phenolic derivative **35.7** which was converted to the methyloxymethyl (MOM) ether derivative **35.8** using chloro(methoxy)methane **11.3**. Suzuki type coupling of the boronate derivative **32.1** with **35.6** in ethylene glycol dimethyl ether in the presence of tetrakis triphenylphosphine palladium (0), lithium chloride, and an aqueous solution of sodium carbonate afforded compound **35.9** which was converted to the final product compound **35A** under acidic conditions. Suzuki type coupling of the boronate derivative **32.1** with **35.8** in ethylene glycol dimethyl ether in the presence of palladium, 10 wt.% (dry basis) on activated carbon, lithium chloride, and an aqueous solution of sodium carbonate afforded compound **35.10** which was converted to the final product compound **35B** under acidic conditions.

The synthesis of compounds **36A** and **36B** is outlined in Scheme 36. Coupling of 4-bromo-2-hydroxybenzoic acid (**36.3**) [obtained from 4-amino-2-hydroxybenzoic acid (**36.1**) under Sandmeyer conditions] with diethylamine (**1.12**) using *O*-(7-azabenzotriazol-1-yl)-*N,N,N',N*'-tetramethyluronium hexafluorophosphate (HATU) as coupling agent afforded the corresponding diethylaminocarbonyl derivative **36.4.** Suzuki type coupling of the boronate derivative **32.1** with **36.4** in ethylene glycol dimethyl ether in the presence of tetrakis triphenylphosphine palladium (0), lithium chloride, and an aqueous solution of sodium carbonate afforded compound **36.5** which was converted to the final product (compound **36A)** under acidic conditions. Compound **36B** was obtained in 7 steps from 2-(3-methoxyphenyl)ethananaine (**36.6**). Coupling of **36.6** with ethyl chloroformate (**36.7**) afforded the ethyl carbamate derivative **36.8** which was cyclized to 3,4-dihydro-6-methoxyisoquinolin-1-(2*H*)-one (**36.9**) in the presence of polyphosphoric acid. Alkylation of **36.9** with ethyl iodide (**36.10**) in tetrahydrofuran in the presence of sodium hydride, afforded the methyl ether **36.11,** which was converted to the phenolic derivative **36.12** by treatment with boron tribromide. Condensation of **36.12** with trifluoromethanesulfonic anhydride (**36.13**) in dichloromethane in the presence of pyridine afforded the triftate derivative **36.14.** Suzuki type coupling of the boronate derivative **32.1** with **36.14** in *N,N*-dimethylformamide in the presence of dichloro[1,1'-bis(diphenylphosphino)ferrocene]palladium(II) dichloromethane adduct, abbreviated as [Pd(dppf)C1₂•CH₂C1₂], and potassium acetate afforded compound **36.15** which was converted to the final product (compound **36B**) under acidic conditions.

The synthesis of compounds **37A-37B** is outlined in Scheme 37. The 2'-hydroxyacetophenone **1.1a** was condensed with 1-benzyl-3-methylpiperidin-4-one (**37.1**) (racemic mixture) in refluxing methanol in the presence of pyrrolidine to provide the racemic ketones **37.2** and **37.3.** The diastereoisomers **37.2** and **37.3** were separated by column chromatography. Palladium catalyzed hydrogenation of **37.2** afforded the piperidine derivative **37.4,** which was converted to 37.5 by treatment with *tert*-butyloxycarbonyl anhydride (**4.7**). Conversion of the ketone **37.5** to the enol triflate derivative **37.6** was achieved using *N*-phenylbis(trifluoromethanesulphonimide) **1.4** as triflating reagent. Suzuki type coupling of the enol triflate derivative **37.6** with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the Boc derivative **37.7,** which was converted to the final product compound **37A** (racemic mixture) under acidic conditions. Similarly, palladium catalyzed hydrogenation of **37.3** afforded the piperidine derivative **37.8,** which was converted to **37.9** by treatment with *tert*-butyloxycarbonyl anhydride (**4.7**). Conversion of the ketone **37.9** to the enol triflate derivative **37.10** was achieved using *N-*phenylbis(trifluoromethanesulphonimide) **1.4** as triflating reagent. Suzuki type coupling of the enol triflate derivative **37.10** with 4-(*N,N*-diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded the Boc derivative **37.11**, which was converted to the final product compound **37B** (racemic mixture) under acidic conditions.

The synthesis of compounds **38A-38D** is outlined in Scheme 38. Condensation of benzyl 4-oxopiperidine-1-carboxylate (**19.1**) with 2,2-dimethyl-1,3-dioxane-4,6-dione (Meldrum's acid; **38.1**) in the presence of pyridine and piperidine gave the derivative **38.2**. Compound **38.2** was subjected to conjugate addition by reaction with organocuprate reagents derived from 4-fluorobenzyl magnesium chloride (**38.3**) and copper (I) iodide to yield, upon work-up, the sodium salt **38.4.** Hydrolysis and decarboxylation of the conjugate addition product **38.4** proceeded by heating a solution of **38.4** in *N,N*-dimethylformamide in the presence of water. Treatment of the corresponding carboxylic acid derivative **38.5** with oxalyl chloride followed by reaction of the resulting acyl chloride with aluminum chloride yielded the corresponding spiro piperidine derivative which was further protected as its *tert-*butyloxycarbonyl (Boc) derivative **38.6** by treatment with *tert*-butyloxycarbonyl anhydride (4.7). Conversion of the ketone **38.6** to the enol triflate derivative **38.7** was achieved using *N*-phenylbis(trifluoromethanesulphonimide) **1.4** as triflating reagent. Suzuki type coupling of the enol triflate derivative **38.7** with 2-(*N,N-*diethylaminocarbonyl)-5-(4,4,5,5-tetramethyl-1,3,2-dioxoborolan-2-yl)pyridine **1.7** in dioxane in the presence of dichloro[1,1'-bis(diphenylphosphino)ferrocene]palladium(II) dichloromethane adduct {[Pd(dppf)C1₂•CH₂C1₂]}, and an aqueous solution of potassium carbonate afforded the derivative **38.8** which was converted to the compound **38A** under acidic conditions. The compound **38B** (racemic mixture) was obtained by hydrogenation of the unsaturated derivative **38A** in methanol in the presence of palladium, 10 wt.% (dry basis) on activated carbon. Conversion of the enol triflate **38.7** to the corresponding boron derivative **38.9** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **38.9** with the aryl iodide derivative **35.8** in dioxane in the presence of dichloro[1,1'-bis(diphenylphosphino)ferrocene]palladium(II) dichloromethane adduct {[Pd(dppf)C1₂•CH₂C1₂]}, and an aqueous solution of potassium carbonate afforded the derivative **38.10** which was converted to the compound **38C** under acidic conditions. Hydrogenation of the unsaturated derivatives **38.10** in methanol in the presence of palladium, 10 wt.% (dry basis) on activated carbon provided the compound **38.11,** which was converted to the compound **38D** under acidic conditions.

The synthesis of compounds **39A-39G** is outlined in Scheme 39. Compound **38.2** was subjected to conjugate addition by reaction with organocuprate reagents derived from benzyl magnesium chloride (**28.3a**) and copper (I) iodide to yield, upon work-up, the sodium salt **39.1**. Hydrolysis and decarboxylation of the conjugate addition product **39.1** proceeded by heating a solution of **39.1** in *N,N*-dimethylformamide in the presence of water. Treatment of the corresponding carboxylic acid derivative **39.2** with oxalyl chloride followed by reaction of the resulting acyl chloride with aluminum chloride yielded the corresponding spiro piperidine derivative which was further protected as its CBz derivative **28.6a** (R^{V} =H) by treatment with benzylchloroformate. Conversion of the ketone **28.6a** to the enol triflate derivative **28.7a** was achieved using *N*-phenylbis(trifluoromethanesulphonimide) **1.4** as triflating reagent. Suzuki type coupling of the enol triflate derivatives **28.7a** 2-(*N,N-*diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded the derivative **28.12** which was converted to the compound **39A** by hydrogenation in methanol in the presence of palladium, 10 wt.% (dry basis) on activated carbon. Treatment of compound **39A** with *tert-*butyloxycarbonyl anhydride (**4.7**) provided the Boc derivative **39.3.** Chiral separation of the enantiomers derived from **39.3** provided compounds **39.4** and **39.5.** The enantiomers **39.4** and **39.5** were converted to compounds **39B** and **39C,** respectively under acidic conditions. Suzuki type coupling of the enol triflate derivatives **28.7a** with *N,N*-diethyl-3-(methoxymethoxy)-4-(4,4,5,5-tetramethyl-1,3,2-dioxaborolan-2-yl)benzamide **39.6** in ethylene glycol dimethyl ether in the presence of tetrakis triphenylphosphine palladium (0), lithium chloride, and an aqueous solution of sodium carbonate afforded the derivative **39.7** which was converted to the compound **39D** by treatment with iodotrimethylsilane followed by acidic cleavage of the MOM protecting group. Hydrogenation of **39.7** in methanol, in the presence of palladium, 10 wt.% (dry basis) on activated carbon, followed by acidic cleavage of the MOM protecting group, afforded compound **39E.** Treatment of compound **39E** with *tert*-butyloxycarbonyl anhydride (**4.7**) provided the Boc derivative **39.8.** Chiral separation of the enantiomers derived from **39.8** provided compounds **39.9** and **39.10.** The enantiomers **39.9** and **39.10** were converted to compounds **39F** and **39G,** respectively under acidic conditions.

The synthesis of compounds **40A-40C** is outlined in Scheme 40. Compound **38.2** was subjected to conjugate addition by reaction with organocuprate reagents derived from 4-methoxybenzyl magnesium chloride (**28.3b**) and copper (I) iodide to yield, upon work-up, the sodium salt **40.1.** Hydrolysis and decarboxylation of the conjugate addition product **40.1** proceeded by heating a solution of **40.1** in *N,N*-dimethylformamide in the presence of water. Treatment of the corresponding carboxylic acid derivative **28.11** with oxalyl chloride followed by reaction of the resulting acyl chloride with aluminum chloride yielded the corresponding spiro piperidine derivative which was further protected as its CBz derivative **28.6b** (R^{V} =OCH₃) by treatment with benzylchloroformate. Conversion of the ketone **28.6b** to the enol triflate derivative **28.7b** was achieved using *N-*phenylbis(trifluoromethanesulphonimide) **1.4** as triflating reagent. Suzuki type coupling of the enol triflate derivatives **28.7b** with 2-(*N,N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded the derivative **40.2** which was converted to the compound **40A** by treatment with iodotrimethylsilane. Hydrogenation of **40.2** in methanol in the presence of palladium, 10 wt.% (dry basis) on activated carbon afforded compound **40B** (racemic mixture). Suzuki type coupling of the enol triflate derivatives **28.7b** with *N,N*-diethyl-3-(methoxymethoxy)-4-(4,4,5,5-tetramethyl-1,3,2-dioxaborolan-2-yl)benzamide **39.6** in ethylene glycol dimethyl ether in the presence of tetrakis triphenylphosphine palladium (0), lithium chloride, and an aqueous solution of sodium carbonate afforded the derivative **40.3** which was converted to the compound **40C** by treatment with iodotrimethylsilane.

The synthesis of compounds **41A-41E** is outlined in Scheme 41. Coupling of the carboxylic acid **13.3** with *N*-ethyl-2-methoxyethanamine (**41.1**) using *O*-benzotriazol-1-yl-*N,N,N',N*'-tetramethyluronium tetrafluoroborate (TBTU) as coupling agent afforded the tertiary aminocarbonyl derivative **41.2,** which was converted to compound **41A** under acidic conditions. Coupling of the carboxylic acid **13.3** with *N*-ethyl-*N,N*-dimethylethane-1,2-diamine (**41.3**) using TBTU as coupling agent afforded the tertiary aminocarbonyl derivative **41.4,** which was converted to compound **41B** under acidic conditions. Coupling of the carboxylic acid **13.3** with *N*-(3-(ethylamino)propyl)-2,2,2-trifluoroacetamide (**41.7**) (obtained by treatment of *N*-ethylpxopane-1,3-diamine **41.5** with ethyl 2,2,2-trifluoroacetate **41.6**) using TBTU as coupling agent afforded the tertiary aminocarbonyl derivative **41.8,** which was converted to **41.9** under basic conditions. Treatment of **41.9** with 2-nitrobenzene-1-sulfonyl chloride (41.10) afforded the sulfonamide derivative. **41.11,** which was converted to **41.12** by treatment with methyl iodide. Thiophenol-mediated deprotection of **41.12** afforded the derivative **41.13,** which was converted to the final compound **41C** under acidic conditions. Coupling of the carboxylic acid **13.3** with *N,N-*dimethylpropane-1,3-diamine (**41.14**) using TBTU as coupling agent afforded the tertiary aminocarbonyl derivative **41.15,** which was converted to compound **41D** under acidic conditions. Coupling of the carboxylic acid **13.3** with *N*-(2-(ethylamino)ethyl)-2,2,2-trifluoroacetamide (**41.17**) (obtained by treatment of *N-*ethylethane-l,2-diamine **41.16** with ethyl 2,2,2-trifluoroacetate **41.6**) using TBTU as coupling agent afforded the tertiary aminocarbonyl derivative **41.18,** which was converted to **41.19** under basic conditions. Treatment of compound **41.19** with *tert-*butyloxycarbonyl anhydride **(4.7)** provided the bis-Boc derivative **41.20.** Treatment of **41.20** with methyl iodide in the presence of sodium hydride afforded the derivative **41.21,** which was converted to the compound **41E** under acidic conditions.

The synthesis of compounds **42A-42I** is outlined in Scheme 42. Suzuki type coupling of the enol triflate derivatives **21.6** with 2-(*N,N-*diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **42.1** (racemic mixture) which was converted to compound **42C** under acidic conditions. Chiral separation of the enantiomers derived from **42.1** provided compounds **42.2** and **42.3.** The enantiomers **42.2** and **42.3** were converted to compounds **42A** and **42B,** respectively under acidic conditions. Conversion of the enol triflate **21.6** to the corresponding boron derivative **42.4** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **42.4** with the aryl bromide **34.1a** in ethylene glycol dimethyl ether in the presence of tetrakis triphenylphosphine palladium (0), lithium chloride, and an aqueous solution of sodium carbonate afforded compound **42.5** (racemic mixture), which was converted to compound **42F** under acidic conditions. Chiral separation of the enantiomers derived from **42.5 provided** compounds **42.6** and **42.7**. The enantiomers **42.6** and **42.7** were converted to compounds **42D** and **42E**, respectively under acidic conditions. Suzuki type coupling of the boron derivative **42.4** with the aryl iodide **35.8** in ethylene glycol dimethyl ether in the presence of tetrakis triphenylphosphine palladium (0), lithium chloride, and an aqueous solution of sodium carbonate afforded compound **42.8** (racemic mixture), which was converted to compound **42I** under acidic conditions. Chiral separation of the enantiomers derived from **42.8** provided compounds **42.9** and **42.10**. The enantiomers **42.9** and **42.10** were converted to compounds **42G** and **42H**, respectively under acidic conditions.

The synthesis of compounds **43A-43F** is outlined in Scheme 43. The 2'-6'-dihydroxyacetophenone derivative **11.1** was condensed with *tert*-butyl 4-oxoazepane-1-carboxylate **21.4** in refluxing methanol in the presence of pyrrolidine to provide the derivative **43.1** which was converted to the methoxymethyl (MOM) ether derivative **43.2** using chloro(methoxy)methane (**11.3**). Conversion of the ketone **43.2** to the enol triflate derivative **43.3.** was achieved using *N*-phenylbis(trifluoromethanesulphonimide) **1.4** as triflating reagent. Suzuki type coupling of the enol triflate derivative 43.3 with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded compound **43.4** (racemic mixture). Removal of the MOM and the Boc protecting groups of **43.4** in methanol at room temperature in the presence of hydrochloric acid (anhydrous solution in dichloromethane) afforded compound **43C** (racemic mixture). Chiral separation of the enantiomers derived from **43.4** provided compounds **43.5** and **43.6**. The enantiomers **43.5** and **43.6** were converted to compounds **43A** and **43B,** respectively under acidic conditions. Suzuki type coupling of the enol triflate derivative **43.3** with 2-(*N,N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **43.7** (racemic mixture). Removal of the MOM and the Boc protecting groups of **43.7** in methanol at room temperature in the presence of hydrochloric acid (anhydrous solution in dichloromethane) afforded compound **43F** (racemic mixture). Chiral separation of the enantiomers derived from **43.7** provided compounds **43.8** and **43.9**. The enantiomers **43.8** and **43.9** were converted to compounds **43D** and **43E**, respectively under acidic conditions.

The synthesis of compounds **44A-44F** is outlined in Scheme 44. The 5'-fluoro-2'-hydroxy-acetophenone derivative **1.1d** was condensed with *tert*-butyl 4-oxoazepane-1-carboxylate **21.4** in refluxing methanol in the presence of pyrrolidine to provide the derivative **44.1.** Conversion of the ketone **44.1** to the enol triflate derivative **44.2** was achieved using *N*-phenylbis(trifluoromethanesulphonimide) **1.4** as triflating reagent. Suzuki type coupling of the enol triflate derivative 44.2 with 2-(*N,N-*diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **44.3** (racemic mixture) which was converted to compound **44C** under acidic conditions. Chiral separation of the enantiomers derived from **44.3** provided compounds **44.4** and **44.5.** The enantiomers **44.4** and **44.5** were converted to compounds **44A** and **44B,** respectively under acidic conditions. Conversion of the enol triflate 44.2 to the corresponding boron derivative **44.6** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **44.6** with the aryl bromide **34.1a** in ethylene glycol dimethyl ether in the presence of tetrakis triphenylphosphine palladium (0), lithium chloride, and an aqueous solution of sodium carbonate afforded compound **44.7** (racemic mixture), which was converted to compound **44F** under acidic conditions. Chiral separation of the enantiomers derived from **44.7** provided compounds **44.8** and **44.9.** The enantiomers **44.8** and **44.9** were converted to compounds **44D** and **44E**, respectively under acidic conditions.

The synthesis of compounds **45A-45F** is outlined in Scheme 45. Suzuki type coupling of the boron derivative **44.6** with the aryl iodide **35.8** in ethylene glycol dimethyl ether in the presence of tetrakis triphenylphosphine palladium (0), lithium chloride, and an aqueous solution of sodium carbonate afforded compound **45.1** (racemic mixture), which was converted to compound **45C** under acidic conditions. Chiral separation of the enantiomers derived from **45.1** provided compounds **45.2** and **45.3**. The enantiomers **45.2** and **45.3** were converted to compounds **45A** and **45B,** respectively under acidic conditions. The 2'-5'-dihydroxyacetophenone derivative **2.1** was condensed with *tert*-butyl 4-oxoazepane-1-carboxylate **21.4** in refluxing methanol in the presence of pyrrolidine to provide the derivative **45.4** which was converted to the silyl ether derivative **45.5** using *tert-*butyldimethylsilyl chloride 2.3. Conversion of the ketone **45.5** to the enol triflate derivative **45.6** was achieved using *N*-phenylbis(trifluoromethanesulphonimide) **1.4** as triflating reagent. Suzuki type coupling of the enol triflate derivative **45.6** with 2-(*N,N-*diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **45.7** (racemic mixture), which was converted to compound **45F** under acidic conditions. Chiral separation of the enantiomers derived from **45.7** provided compounds **45.8** and **45.9.** The enantiomers **45.8** and **45.9** were converted to compounds **45D** and **45E**, respectively under acidic conditions.

The synthesis of compounds **46A-46C** is outlined in Scheme 46. Suzuki type coupling of the enol triflate derivative **45.6** with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded compound **46.1** (racemic mixture), which was converted to compound **46C** under acidic conditions. Treatment of **46.1** with chloro(methoxy)methane (**11.3**) provided the methoxymethyl (MOM) ether derivative **46.2** (racemic mixture). Chiral separation of the enantiomers derived from **46.2** provided compounds **46.3** and **46.4.** The enantiomers **46.3** and **46.4** were converted to compounds **46A** and **46B,** respectively under acidic conditions.

The synthesis of compounds **47A-47F** is outlined in Scheme 47. Suzuki type coupling of the enol triflate derivative **23.3a** with 2-(*N,N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **47.1** (racemic mixture) which was converted to compound **47A** under acidic conditions. Conversion of the enol triflate **23.3a** to the corresponding boron derivative **47.2** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **47.2** with the aryl iodide 35.8 in ethylene glycol dimethyl ether in the presence of tetrakis triphenylphosphine palladium (0), lithium chloride, and an aqueous solution of sodium carbonate afforded compound **47.3** (racemic mixture), which was converted to compound **47B** under acidic conditions. The 2'-6'-dihydroxyacetophenone derivative **11.1** was condensed with *tert*-butyl 3-oxopyrrolidine-1-carboxylate **23.1a** in refluxing methanol in the presence of pyrrolidine to provide the derivative **47.4** which was converted to the methoxymethyl (MOM) ether derivative **47.5** using chloro(methoxy)methane (**11.3**). Conversion of the ketone **47.5** to the enol triflate derivative **47.6** was achieved using *N*-phenylbis(trifluoromethanesulphonimide) **1.4** as triflating reagent. Suzuki type coupling of the enol triflate derivative **47.6** with compound **1.6** in ethylene glycol dimethyl ether in the presence of tetrakis triphenylphosphine palladium (0), lithium chloride, and an aqueous solution of sodium carbonate afforded compound **47.7** (racemic mixture). Removal of the MOM and the Boc protecting groups of **47.7** in methanol at room temperature in the presence of hydrochloric acid afforded compound **47C** (racemic mixture). Suzuki type coupling of the enol triflate derivative **47.6** with 2-(*N,N*-diethylaminocarbonyl)-5-(4,4,5,5-tetramethyl-1,3,2-dioxoborolan-2-yl)pyridine 1.7 in ethylene glycol dimethyl ether in the presence of tetrakis triphenylphosphine palladium (0), lithium chloride, and an aqueous solution of sodium carbonate afforded compound **47.8** (racemic mixture). Removal of the MOM and the Boc protecting groups of **47.8** in methanol at room temperature in the presence of hydrochloric acid afforded compound **47D** (racemic mixture). The 2'-5'-dihydroxyacetophenone derivative **2.1** was condensed with *tert*-butyl 3-oxopyrrolidine-1-carboxylate **23.1a** in refluxing methanol in the presence of pyrrolidine to provide the derivative **47.9** which was converted to the silyl ether derivative **47.10** using *tert*-butyldimethylsilyl chloride 2.3. Conversion of the ketone **47.10** to the enol triflate derivative **47.11** was achieved using *N-*phenylbis(trifluoromethanesulphonimide) **1.4** as triflating reagent. Suzuki type coupling of the enol triflate derivative **47.11** with compound **1.6** in ethylene glycol dimethyl ether in the presence of tetrakis triphenylphosphine palladium (0), lithium chloride, and an aqueous solution of sodium carbonate afforded compound **47.12** (racemic mixture), which was converted to compound **47E** under acidic conditions. Suzuki type coupling of the enol triflate derivative **47.11** with 2-(*N,N*-diethylaminocarbonyl)-5-(4,4,5,5-tetramethyl-1,3,2-dioxoborolan-2-yl)pyridine **1.7** in ethylene glycol dimethyl ether in the presence of tetrakis triphenylphosphine palladium (0), lithium chloride, and an aqueous solution of sodium carbonate afforded compound **47.13** (racemic mixture), which was converted to compound **47F** under acidic conditions.

The synthesis of compounds **48A-48F** is outlined in Scheme 48. Suzuki type coupling of the enol triflate derivative **1.5f** with 4-cyanophenylboronic acid (**14.1**) in dioxane in the presence of dichloro[1,1'-bis(diphenylphosphino)ferrocene]palladium(II) dichloromethane adduct, and an aqueous solution of potassium carbonate afforded the cyanide **48.1** which was converted to the tetrazole **48.2** using sodium azide (**14.3**) and zinc bromide in a solution isopropanol/water. Alkylation of **48.2** with methyl iodide (**2.8c**) in *N,N-*dimethylformamide in the presence of triethylamine afforded the two regioisomers **48.3** (major isomer) and **48.4** (minor isomer) separated by silica gel column chromatography. The Boc protecting group of **48.2, 48.3,** and **48.4** was removed using hydrochloric acid to generate the compounds **48A-48C.** Conversion of the enol triflate **1.5f** to the corresponding boron derivative **48.5** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **48.5** with the aryl bromide **34.1a** in dioxane in the presence of tetrakis triphenylphosphine palladium (0), potassium bromide and potassium phosphate afforded compound **48.8** (racemic mixture), which was converted to compound **48F** under acidic conditions. Suzuki type coupling of the boron derivative **48.5** with the bromothiophene derivatives **34.1c** and **34.1d** in dioxane in the presence of and dichloro[1,1'-bis(diphenylphosphino)ferrocene]palladium(II) dichloromethane adduct, and an aqueous solution o potassium carbonate afforded compound **48.6** and **48.7** respectively. Compounds **48.6** and **48.7** were converted to compound **48D** and **48E,** respectively, under acidic conditions.

The synthesis of compounds **49A-49D** is outlined in Scheme 49. The 6'-fluoro-2'-hydroxy-acetophenone derivative **49.1** was condensed with 1-Boc-4-piperidone **1.2** in methanol at 0°C in the presence of pyrrolidine to provide the derivative **49.2.** Conversion of the ketone **49.2** to the enol triflate derivative **49.3** was achieved using *N-*phenylbis(trifluoromethanesulphonimide) 1.4 as triflating reagent. Suzuki type coupling of the enol triflate derivative **49.3** with 4-(*N,N*-diethylaminocarbonyl)phenyl boronic acid 1.6 in ethylene glycol dimethyl ether in the presence of tetrakis triphenylphosphine palladium (0), lithium chloride, and an aqueous solution of sodium carbonate afforded compound **49.4** which was converted to compound **49A** under acidic conditions. Treatment of **49.2** with pyrrolidine afforded compound **49.5**. Conversion of the ketone **49.5** to the enol triflate derivative **49.6** was achieved using *N*-phenylbis(trifluoromethanesulphonimide) **1.4** as triflating reagent. Suzuki type coupling of the enol triflate derivative **49.6** with 4-(*N,N-*diethylaminocarbonyl)phenyl boronic acid **1.6** in ethylene glycol dimethyl ether in the presence of tetrakis triphenylphosphine palladium (0), lithium chloride, and an aqueous solution of sodium carbonate afforded compound **49.7** which was converted to compound **49B** under acidic conditions. Conversion of the enol triflate **1.5d** to the corresponding boron derivative **49.8** was achieved using 4,4,5,5-tetramethyl-2-(4,4,5,5-tetramethyl-1,3,2-dioxaborolan-2-yl)-1,3,2-dioxaborolane **1.14** and dichloro[1,1'-bis(diphenylphosphino)ferrocene]palladium(II) dichloromethane adduct {[Pd(dppf)C1₂•CH₂C1₂]}. Suzuki type coupling of the boron derivative **49.8** with the aryl iodide 35.8 in dioxane in the presence of dichloro[1,1'-bis(diphenylphosphino)ferrocene]palladium(II) dichloromethane adduct and an aqueous solution of potassium carbonate afforded compound **49.9**, which was converted to compound **49C** under acidic conditions. Hydrogenation of the unsaturated derivative **49C** in methanol in the presence of palladium, 10 wt.% (dry basis) on activated carbon provided the compound **49D** (racemic mixture).

The synthesis of compounds **50A-50D** is outlined in Scheme 50. Suzuki type coupling of the enol triflate derivative 2.5 with phenyl boronic acid **31.1g** in ethylene glycol dimethyl ether, in the presence of palladium, 10 wt.% (dry basis) on activated carbon, lithium chloride, and an aqueous solution of sodium carbonate afforded compound **50.1.** Conversion of the phenol **50.1** to the triflate derivative **50.2** was achieved using the triflating reagent *N-*phenylbis(trifluoromethanesulphonimide) **1.4.** Palladium catalyzed carbonylation of **50.2,** conducted in a mixture of dimethylsulfoxide/methanol using palladium (II) acetate, 1,1'-bis(diphenylphosphino)ferrocene (dppf) and carbon monoxide, provided the methyl ester **50.3** which was hydrolyzed under basic conditions to give the carboxylic acid derivatives **50.4.** Coupling of the carboxylic acid **50.4** with diethylamine (**1.12**) using TBTU as coupling agent afforded the tertiary amide **50.5.** Treatment of the Boc derivatives **50.3, 50.4, 50.5** and **50.1** with hydrochloric acid provided the final compounds **50A-50D,** respectively.

The synthesis of compounds **51A-51C** is outlined in Scheme 51. The 2'-hydroxyacetophenone **1.1a** was condensed with *tert-*butyl 2-methyl-4-oxapiperidine-1-carboxylate (**51.1**) (racemic mixture) in refluxing methanol in the presence of pyrrolidine to provide the racemic ketone **51.2.** Conversion of the ketone **51.2** to the enol triflate derivative **51.3** was achieved using *N*-phenylbis(trifluoromethanesulphonimide) **1.4** as triflating reagent. Suzuki type coupling of the enol triflate derivative **51.3** with 4-(*N,N-*diethylaminocarbonyl)phenyl boronic acid **1.6** in ethylene glycol dimethyl ether in the presence of tetrakis triphenylphosphine palladium (0), potassium bromide, and potassium phosphate afforded the Boc derivative **51.4** (product eluted as a single peak), which was converted to compound **51C** under acidic conditions. Chiral separation of the intermediate **51.4** provided resolved products **51.5** and **51.6**. The resolved products **51.5** and **51.6** were converted to compounds **51A** and **51B,** respectively under acidic conditions.

The synthesis of compounds **52A-52F** is outlined in Scheme 52. Treatment of compound **21C** with trifluoroacetic anhydride (**4.1)** in tetrahydrofuran in the presence of triethylamine provided the trifluoroacetamide derivative **52.1** which was converted to the sulfonyl chloride **52.2** using sulfur trioxide *N,N-*dimethylformamide complex (**4.3**) as sulfating agent. Condensation of hydrazine hydrate (**5.1**) with the sulfonyl chloride derivative **52.2** provided the sulfonyl hydrazide **52.3,** which was converted to the sulfone **52.4** by treatment with methyl iodide (**2.8c**) in the presence of sodium acetate. Deprotection of the trifluoroacetamide protecting group of **52.4** under basic conditions (potassium carbonate, methanol, water) provided the methyl sulfonyl analog (compound **52A**) [Note: compound **52A** (derived from compound **21C**) and **22E** (derived from compound **21B**) are enantiomeric with respect to one another]. Palladium catalyzed hydrogenation of compound **22E** affords compounds **52B** and **52C,** respectively. Palladium catalyzed hydrogenation of compounds **35B, 1Q,** and **1F** affords compounds **52D, 52E,** and **52F,** respectively.

The synthesis of compounds **53A-53F** is outlined in Scheme 53. Treatment of compounds **48.2** and **48.3** with hydrobromic acid provided the phenolic derivatives **53A** and **53B,** respectively. Suzuki type coupling of the enol triflate derivative **11.5** with 4-(carboxy)phenylboronic acid (**53.1**) in dioxane in the presence of tetrakis triphenylphosphine palladium (0), and an aqueous solution of potassium carbonate afforded the carboxylic acid derivative **53.2** which was converted to **53D** under acidic conditions. The carboxylic acid derivative **53D** was converted to its methyl ester analog **53C** under classical esterification conditions, i.e. in the presence of concentrated hydrochloric acid and methanol. Coupling of the carboxylic acid **53.2** with ammonium chloride (**3.4a**) in acetonitrile, in the presence of *N,N*-diisopropylethylamine (Hunig's base), using O-benzotriazol-1-yl-*N,N,N',N'-*tetramethyluronium tetrafluoroborate (TBTU) as coupling agent affords the primary aminocarbonyl derivative **53.3**. Coupling of the carboxylic acid **53.2** with ethylamine (**3.4c**) in acetonitrile, in the presence of *N,N*-diisopropylethylamine (Hunig's base), using *O-*benzotriazol-1-yl-*N,N,N',N'*-tetramethyluronium tetrafluoroborate (TBTU) as coupling agent afforded the secondary aminocarbonyl derivative **53.4**. Treatment of the derivative **53.4** with hydrochloric acid provided the final compounds **53F**. Treatment of the derivative **53.3** with hydrochloric acid provides the final compound **53E**.

The synthesis of compounds **54A** and **54B** is outlined in Scheme 54. Condensation of benzyl 4-oxoazepane-1-carboxylate (**54.1**) (obtained by treatment of azepan-4-one **21.3** with benzylchloroformate) with 2,2-dimethyl-1,3-dioxane-4,6-dione (Meldrum's acid; **38.1**) in the presence of pyridine and piperidine gave the derivative **54.2.** The compound **54.2** was subjected to conjugate addition by reaction with organo cuprate reagents derived from benzyl magnesium chloride (**28.3a**) and copper (I) iodide to yield, upon work-up, the sodium salt **54.3.** Hydrolysis and decarboxylation of the conjugate addition product **54.3** proceeded by heating a solution of **54.3** in *N,N*-dimethylformamide in the presence of water. Treatment of the corresponding carboxylic acid derivative **54.4** with oxalyl chloride followed by reaction of the resulting acyl chloride with aluminum chloride yielded the corresponding spiro piperidine derivative which was further protected as its *tert*-butyloxycarbonyl (Boc) derivative **54.5** by treatment with *tert*-butyloxycarbonyl anhydride (**4.7**). Conversion of the ketone **54.5** to the enol triflate derivative **54.6** was achieved using *N-*phenylbis(trifluoromethanesulphonimide) **1.4** as triflating reagent. Suzuki type coupling of the enol triflate derivatives **54.6** with 4-(*N,N-*diethylaminocarbonyl)phenyl boronic acid **1.6** in dioxane in the presence of tetrakis triphenylphosphine palladium (0) and an aqueous solution of potassium carbonate afforded compound **54.7,** which was converted to compound **54A** under acidic conditions. Suzuki type coupling of the enol triflate derivative **54.6** with 2-(*N,N-*diethylaminocarbonyl)-5-(4,4,5,5-tetramethyl-1,3,2-dioxoborolan-2-yl)pyridine **1.7** in dioxane in the presence of dichloro[1,1'-bis(diphenylphosphino)ferrocene]palladium(II) dichloromethane adduct and an aqueous solution of potassium carbonate afforded compound **54.8,** which was converted to compound **54B** under acidic conditions.

The synthesis of compounds **55A-C** is outlined in Scheme 55. The compound **38.2** was subjected to conjugate addition by reaction with organo cuprate reagents derived from 3,5-dimethoxybenzyl magnesium bromide (**55.1**) and copper (I) iodide to yield, upon work-up, the sodium salt **55.2**. Hydrolysis and decarboxylation of the conjugate addition product **55.2** proceeded by heating a solution of **55.2** in *N,N*-dimethylformamide in the presence of water. Treatment of the corresponding carboxylic acid derivative **55.3** with oxalyl chloride followed by reaction of the resulting acyl chloride with aluminum chloride yielded the corresponding spiro piperidine derivative, which was further protected as its CBz derivative **55.4** by treatment with benzylchloroformate. Conversion of the ketone **55.4** to the enol triflate derivative **55.5** was achieved using *N*-phenylbis(trifluoromethanesulphonimide) **1.4** as triflating reagent. Suzuki type coupling of the enol triflate derivatives **55.5** with 4-(*N,N-*diethylaminocarbonyl)phenyl boronic acid **1.6** in ethylene glycol dimethyl ether in the presence of tetrakis triphenylphosphine palladium (0), lithium chloride and an aqueous solution of sodium carbonate afforded compound **55.6** which was converted to either the compound **55C** by treatment with iodotrimethylsilane or the compound **55A** by treatment with boron tribromide. Treatment of **55A** with *tert*-butyloxycarbonyl anhydride (**4.7**) provided the corresponding Boc derivative **55.7.** Condensation of **55.7** with *N-*phenylbis(trifluoromethanesulphonimide) **1.4** provided the corresponding monotriflate derivative **55.8** which was converted to compound **55.9** by treatment with triethylsilane in the presence of 1,3-bis(diphenylphosphino)propane (dppp) and palladium (II) acetate. Acidic deprotection of the Boc-protecting group of **55.9** provided the compound **55B**.

The synthesis of compounds **56A -56D** is outlined in Scheme 56. Boron tribromide-mediated demethylation of **28.8b** provided the phenolic derivative **56A,** which was converted to **56B** under hydrogenation conditions. Similarly, boron tribromide-mediated demethylation of **40.3** provided the phenolic derivative **56C,** which was converted to **56D** under hydrogenation conditions.

The synthesis of compounds **57A-57D** is outlined in Scheme 57. Treatment of compound **31J** with trifluoroacetic anhydride (**4.1**) in tetrahydrofuran in the presence of triethylamine provided the trifluoroacetamide derivative **57.1** which was converted to the sulfonyl chloride **57.2** using sulfur trioxide *N,N-*dimethylformamide complex (**4.3**) as sulfonating agent. Condensation of **57.2** with methylamine (**3.4b**) and dimethylamine (**3.4j**) affords the corresponding sulfonamide derivatives **57.3** which are converted to compounds **57A, 57B** under basic conditions. Condensation of **57.2** with ammonium hydroxide yields the compound **57C.** Condensation of hydrazine hydrate (**5.1**) with the sulfonyl chloride derivative **57.2** provided the sulfonyl hydrazide **57.4,** which was converted to the sulfone **57.5** by treatment with methyl iodide (**2.8c**) in the presence of sodium acetate. Deprotection of the trifluoroacetamide protecting group of **57.5** under basic conditions (potassium carbonate, methanol, water) provided the compound **57D.**

The synthesis of compounds **58A-58D** is outlined in Scheme 58. Suzuki type coupling of the enol triflate derivative **2.5** with either 3-(1,3,2-dioxaborinan-2-yl)pyridine (**3.6a**) or benzo[b]thiophen-2-ylboronic acid (**31.1n**) in dioxane in the presence of dichloro[1,1'-bis(diphenylphosphino)ferrocene]palladium(II) dichloromethane adduct (abbreviated as [Pd(dppf)C1₂•CH₂C1₂]) and an aqueous solution of potassium carbonate afforded compounds **58.1**. The derivatives **58.1a** and **58.1b** were then converted to the final products **58A** and **58B,** resepectively, under acidic conditions. Suzuki type coupling of the enol triflate derivative **2.5** with phenyl boronic acid (**31.1g**) in ethylene glycol dimethyl ether in the presence of tetrakis triphenylphosphine palladium (0) and an aqueous solution of potassium carbonate afforded compound **58.2**. Removal of the silyl protecting group of **58.2** using a solution of tetrabutylammonium fluoride (TBAF) in tetrahydrofuran gave the phenolic derivatives **58.3**. Preparation of each of the ether derivatives **58.5** from the phenol **58.3** was achieved by alkylation reaction using the appropriate alkyl iodide or alky chloride (**2.8c, 58.4**) reagent. Treatment of the Boc derivatives **58.5** with hydrochloric acid provided the final compounds **58C-D**.

The synthesis of compounds **59A-59L** is outlined in Scheme 59. The alkyl bromide derivatives used as starting materials for the synthesis of compounds **59A-59L** were either commercially available (**59.2a, 59.2b**) or prepared from the corresponding alcohols (preparation of 59.2c and **59.2d**). Treatment of the amines **59.2** with ethyl 2,2,2-trifluoroacetate (**59.3**) in methanol in the presence of triethylamine provided the trifluoroacetamide derivatives **59.4.** Alkylation of 5-(4-bromophenyl)-2H-tetrazole (**59**.6) (obtained by treatment of 4-bromobenzonitrile (**59.5**) with sodium azide and ammonium chloride in *N,N*-dimethylformamide) with the alkyl bromide derivatives **59.4,** provided the derivatives **59.7**. Suzuki type coupling of the boronate derivative **32.1** with the aryl bromide derivatives **59.7** in dioxane in the presence of tetrakis triphenylphosphine palladium (0) and an aqueous solution of potassium carbonate afforded the compounds **59.8** which were converted to the derivatives **59.9** under basic conditions. The Boc protecting group of **59.9** was removed using hydrochloric acid to generate the compounds **59A-59D.** Treatment of the amines **59.9** with acetic anhydride in dichloromethane in the presence of triethylamine provided the acetamides **59.10**, which were converted to the final products **59E-59H** under acidic conditions. Treatment of the amines **59.9** with methane sulfonyl chloride in dichloromethane in the presence of triethylamine provided the sulfonamides **59.11**, which were converted to the final products **59I-59L** under acidic conditions.

In some instances, the compounds of the invention have the potential for chirality but were prepared in racemic form. As one skilled in the art would readily recognize, the racemic mixtures of intermediates or final products initially prepared as their racemates may be partially or completely resolved into any, some, or all of the enantiomers contained in the racemate as described herein, for example in the separation of intermediates leading to **39F** and **39G**. As such, racemic mixtures, mixtures enriched in or or more stereoisomers, and pure enantiomers are considered to be within the ambit of the present invention.

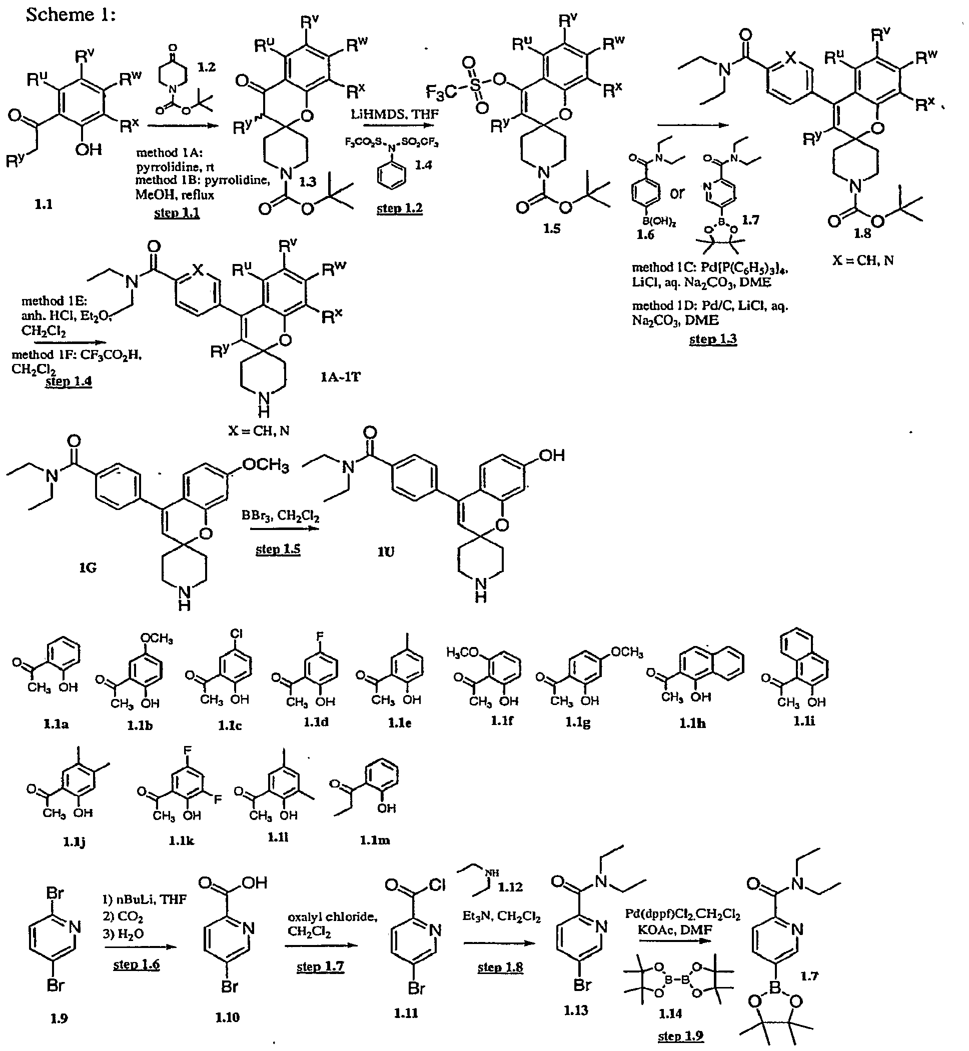

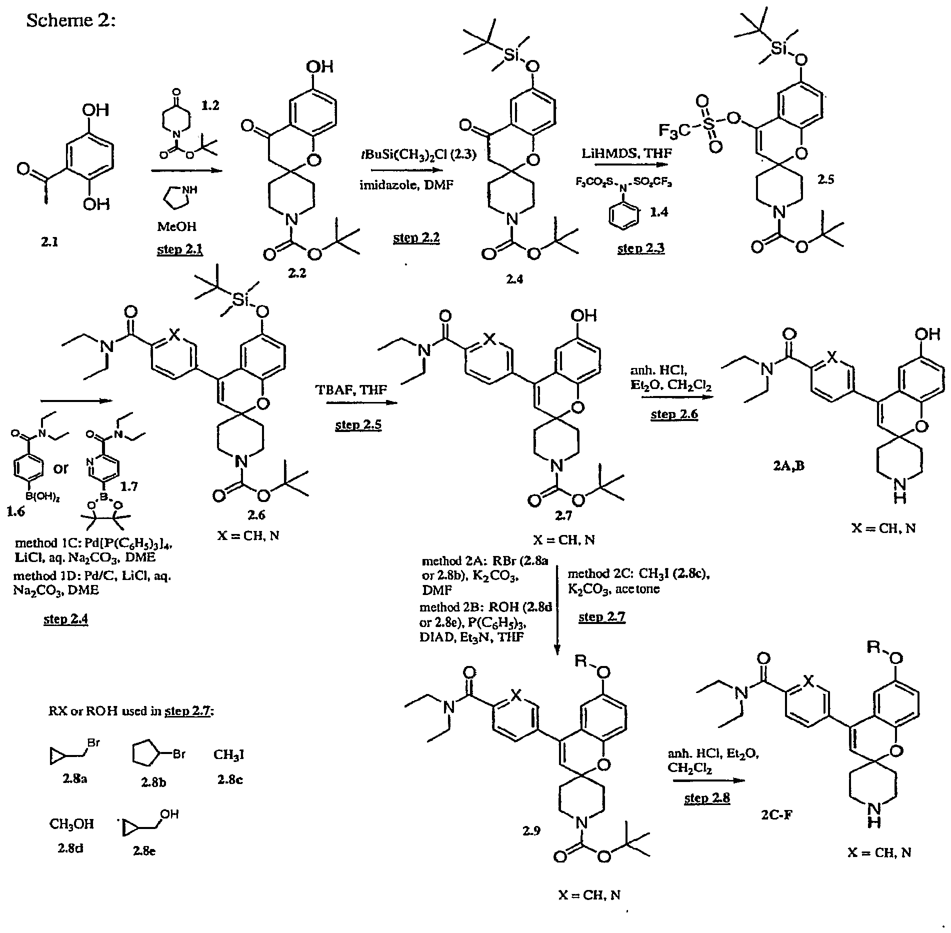

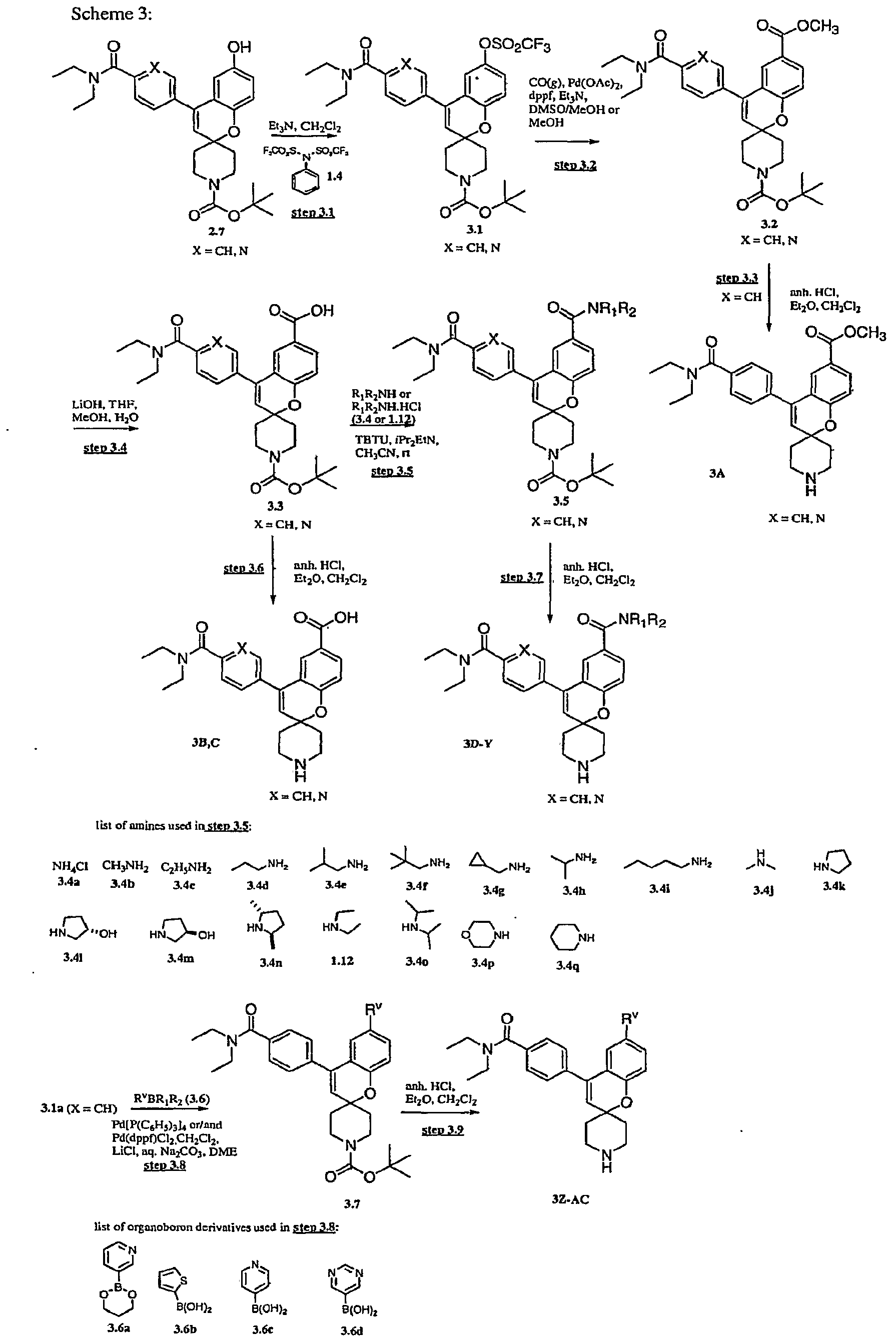

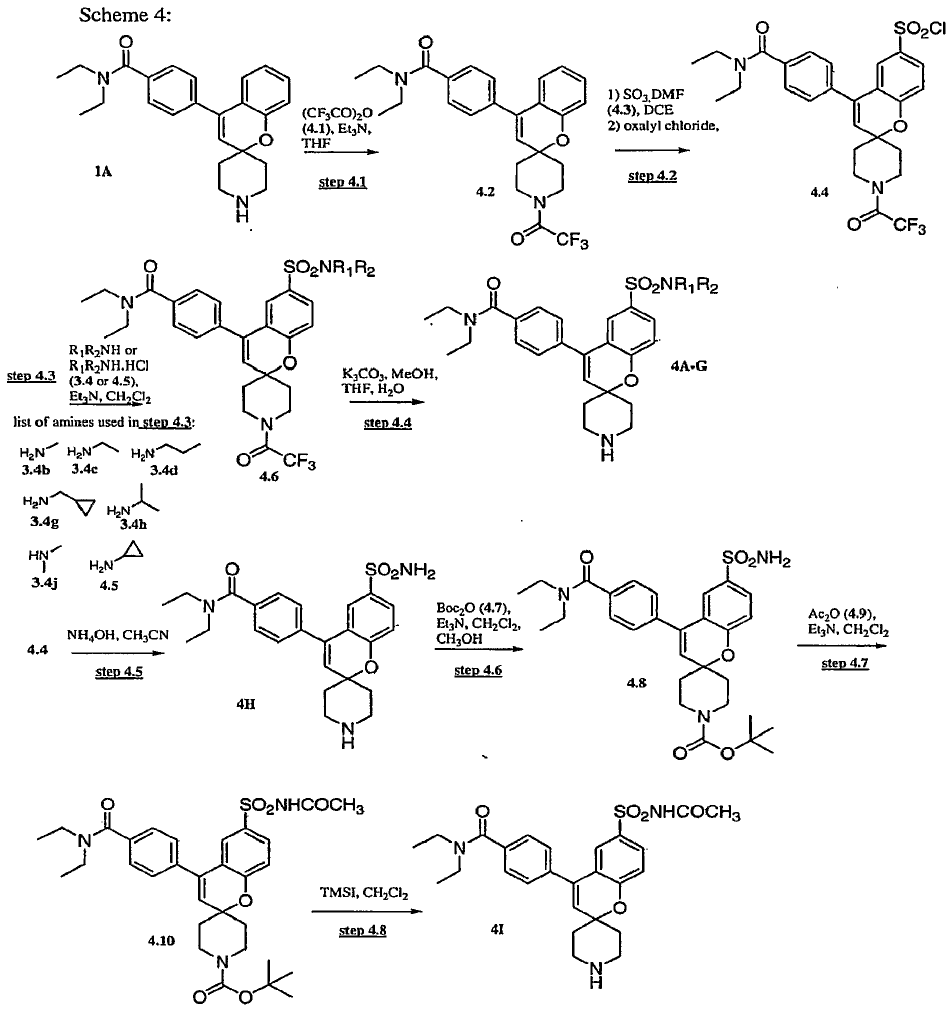

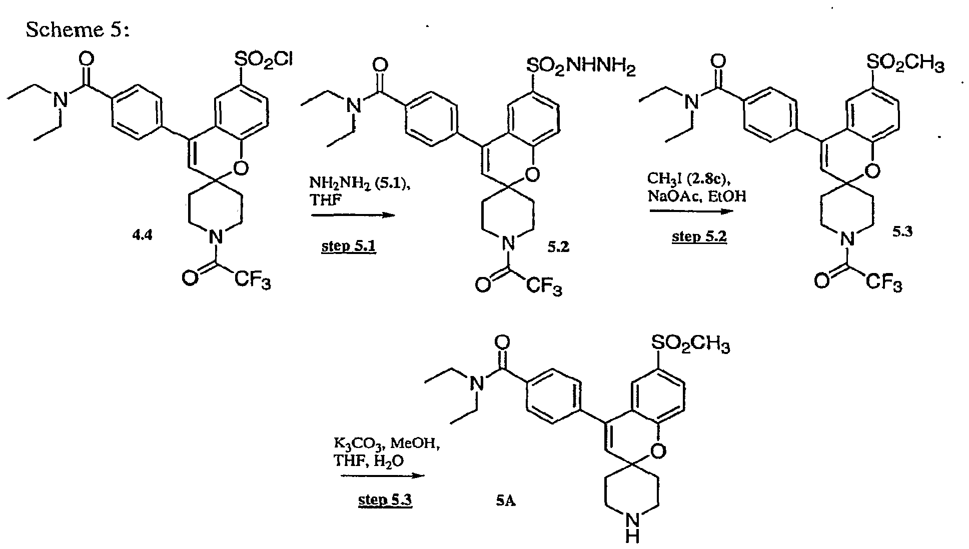

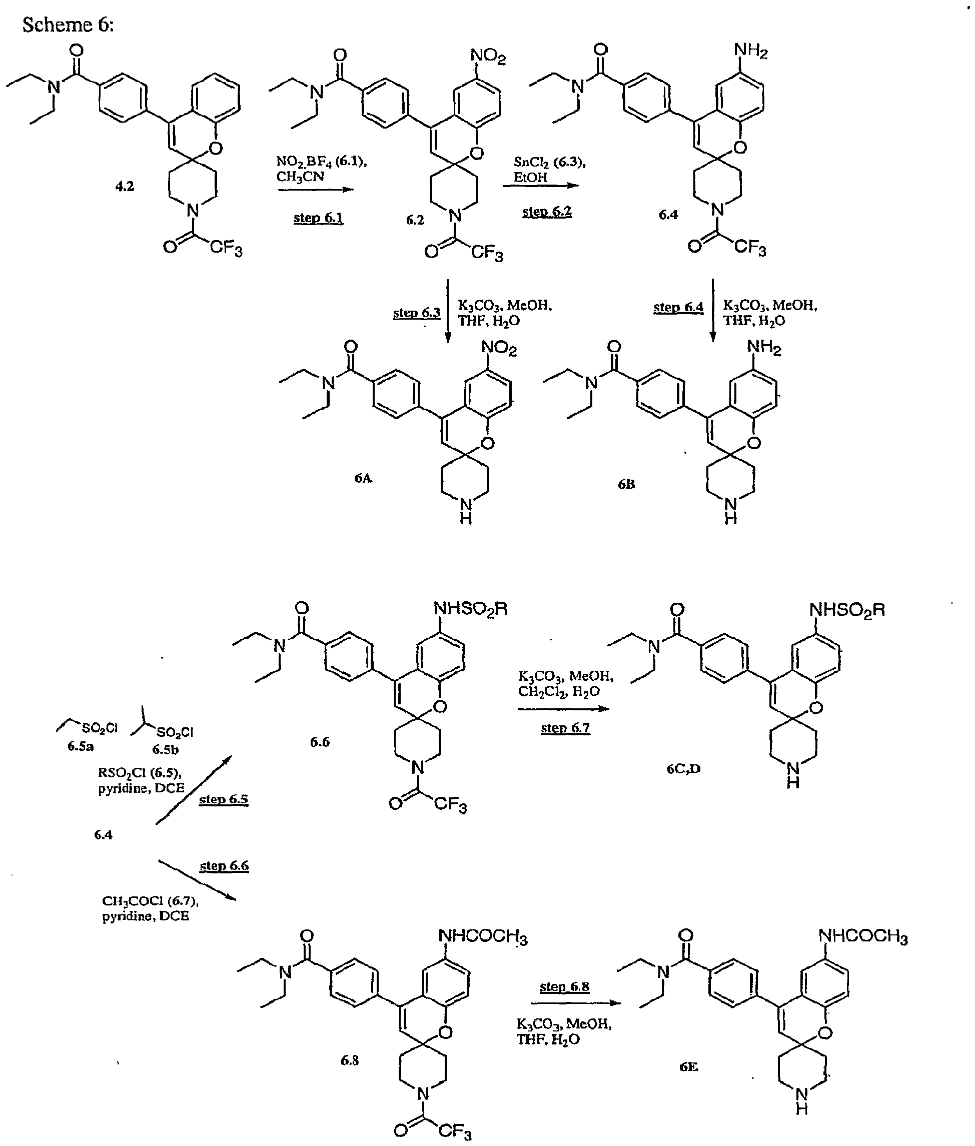

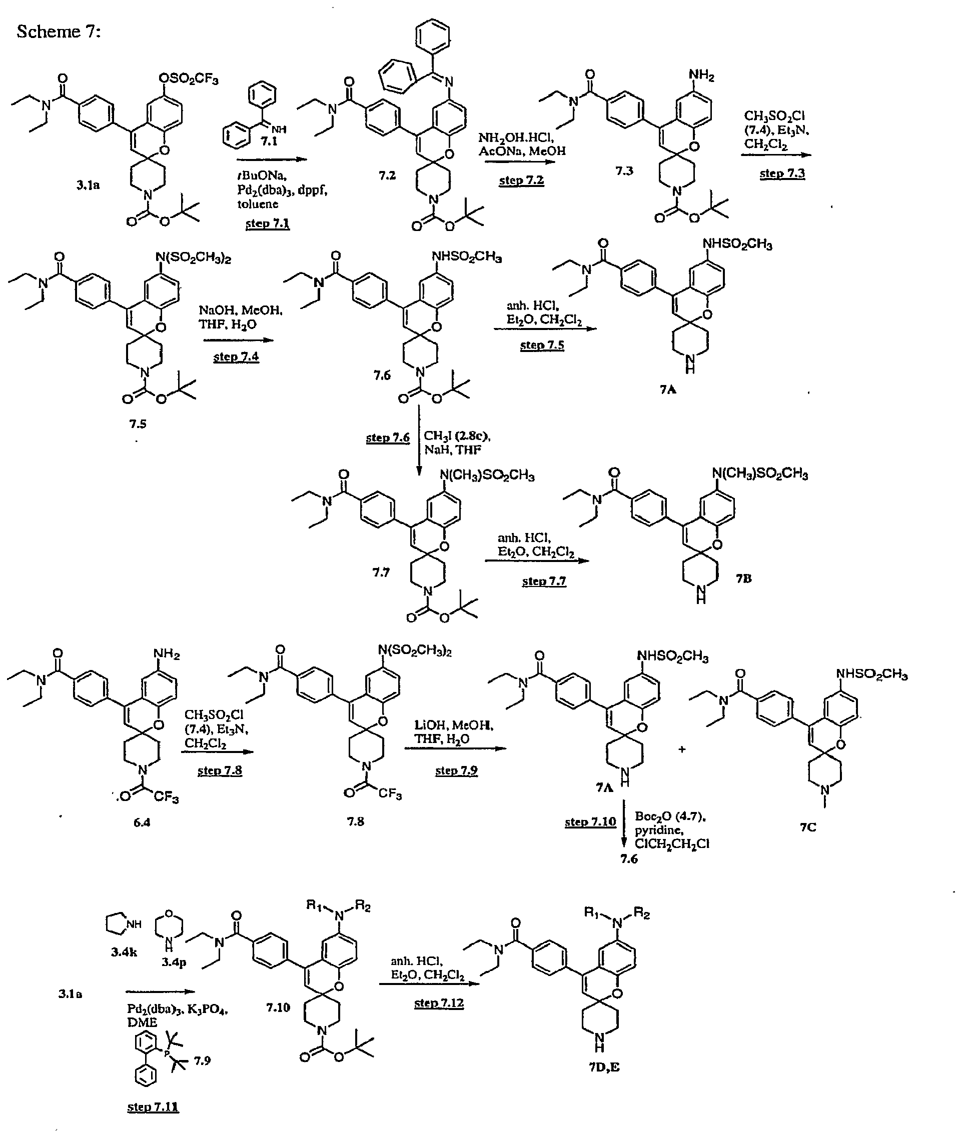

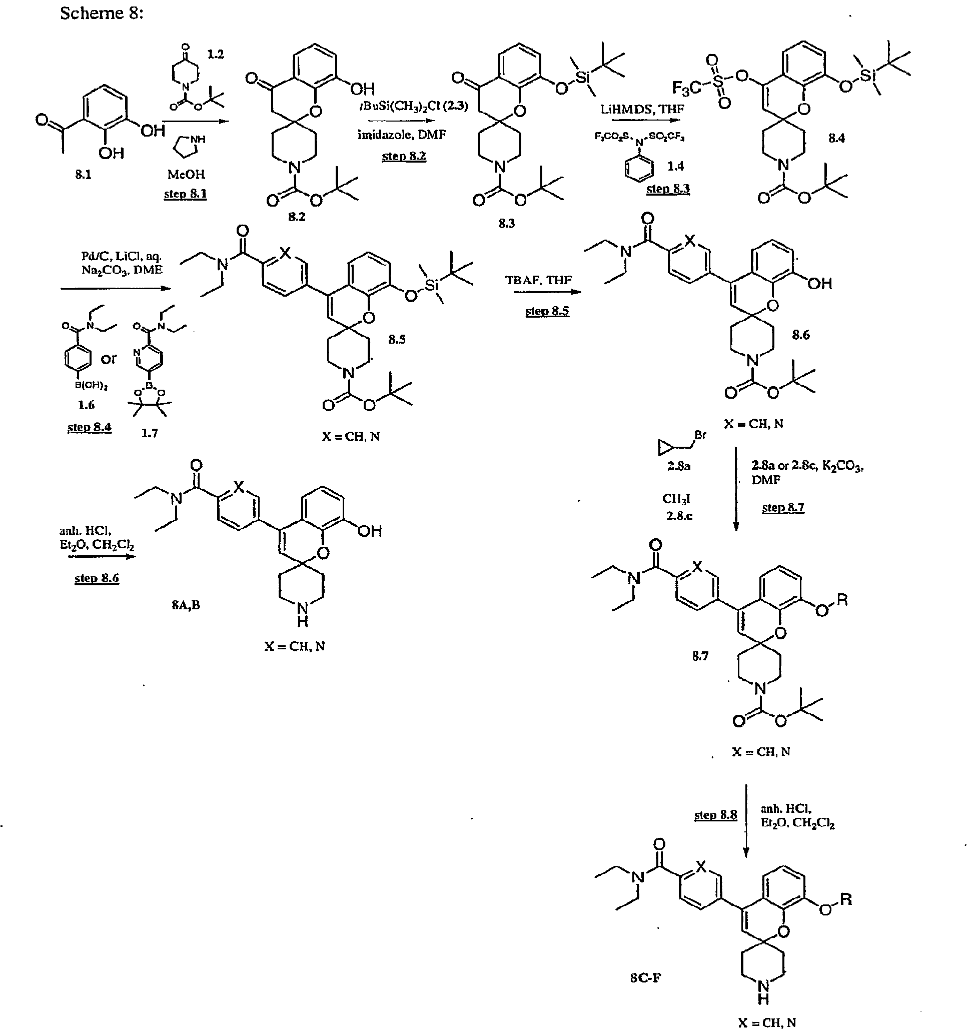

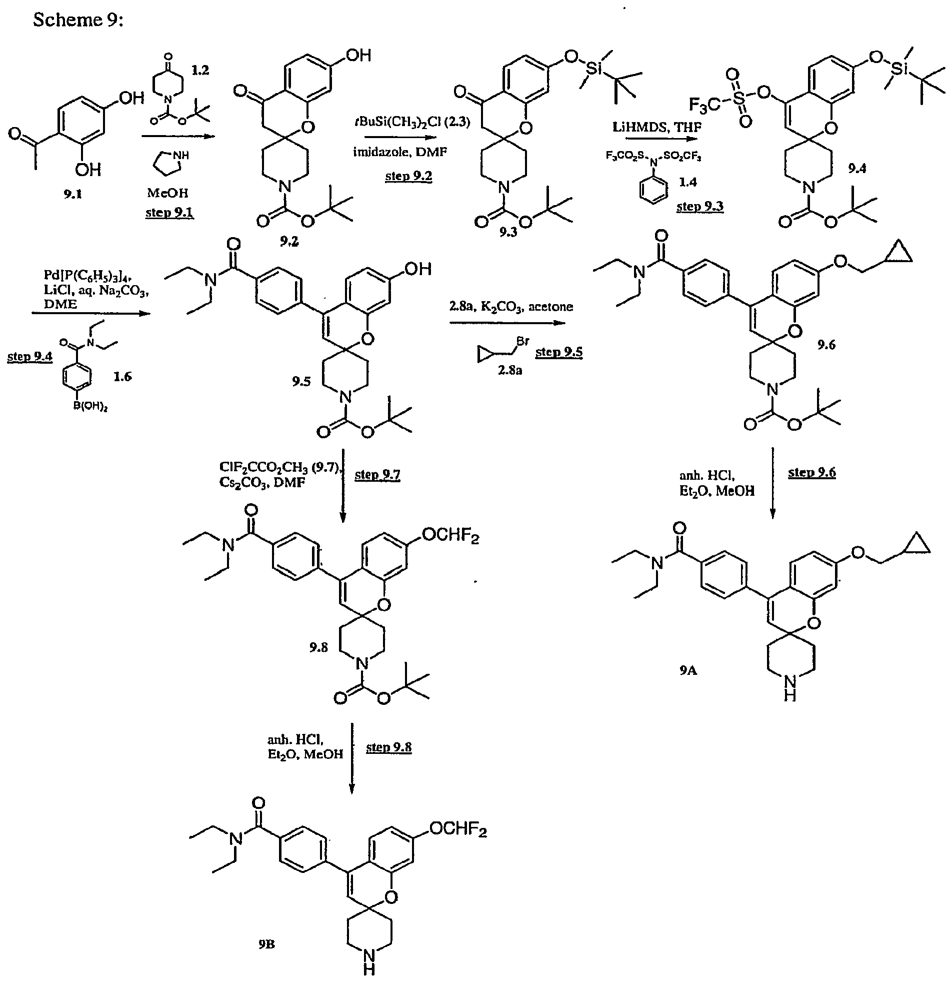

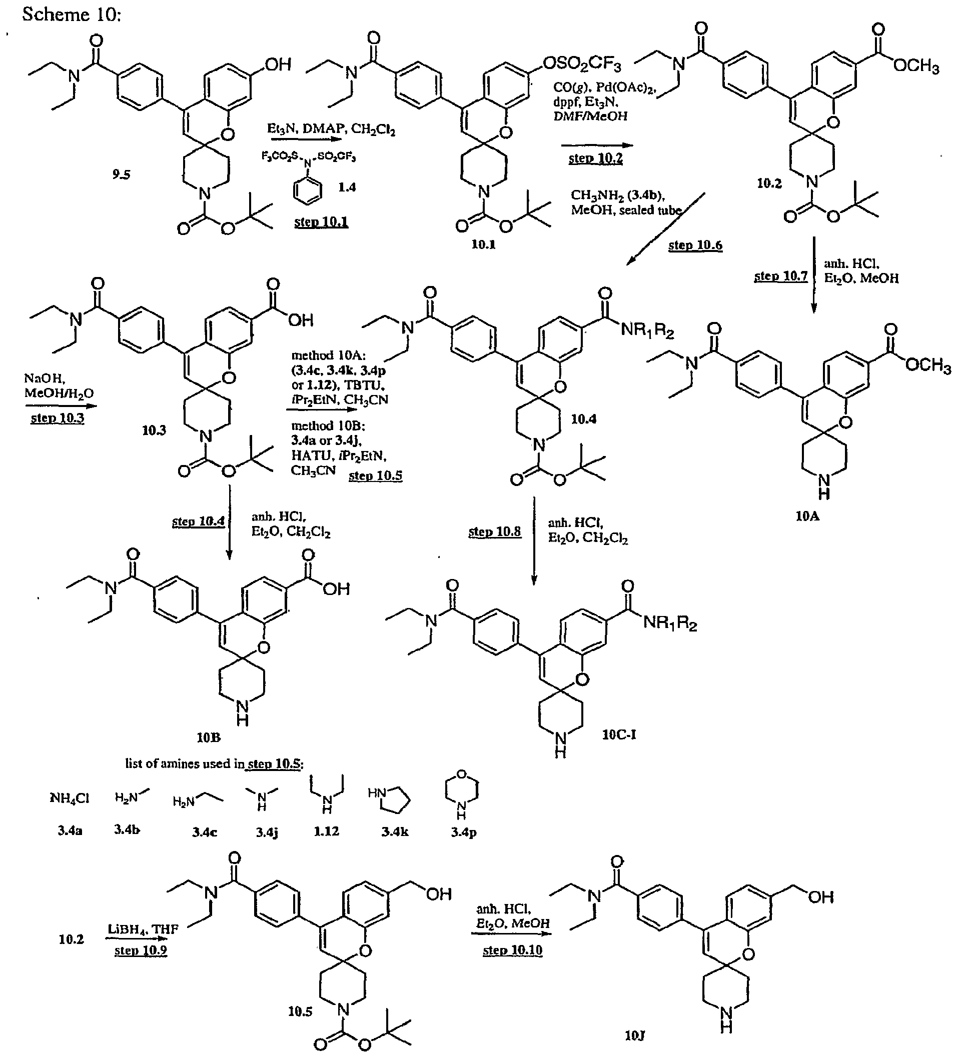

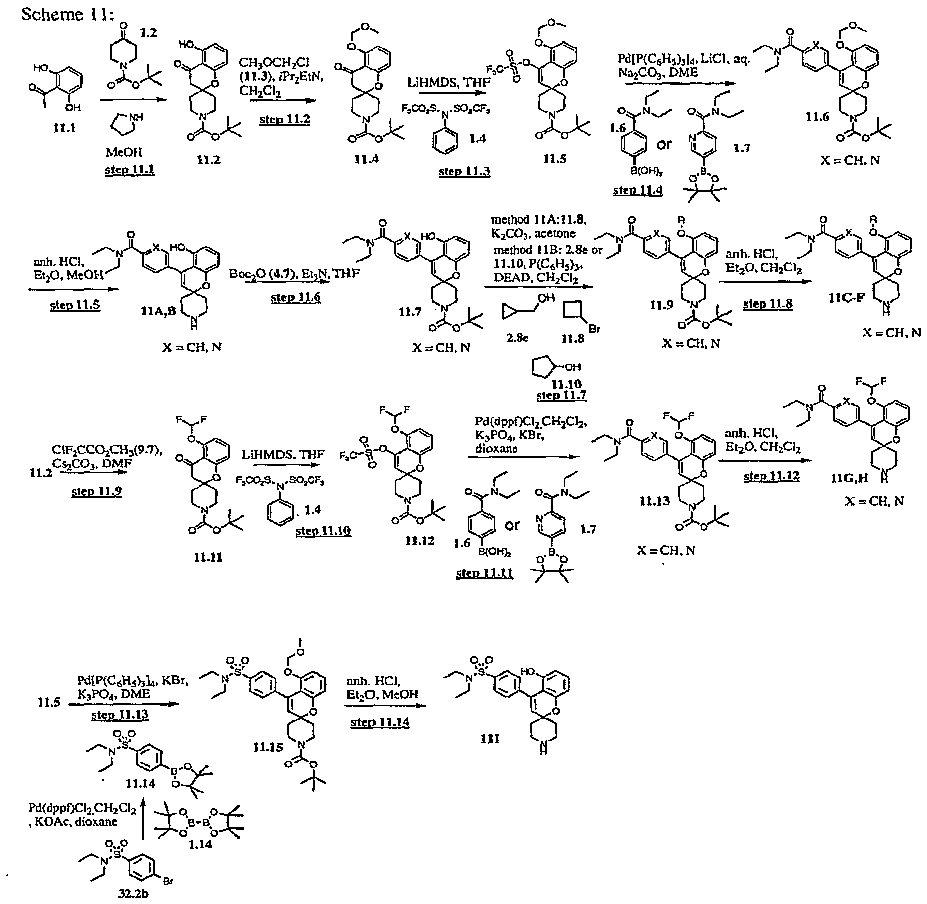

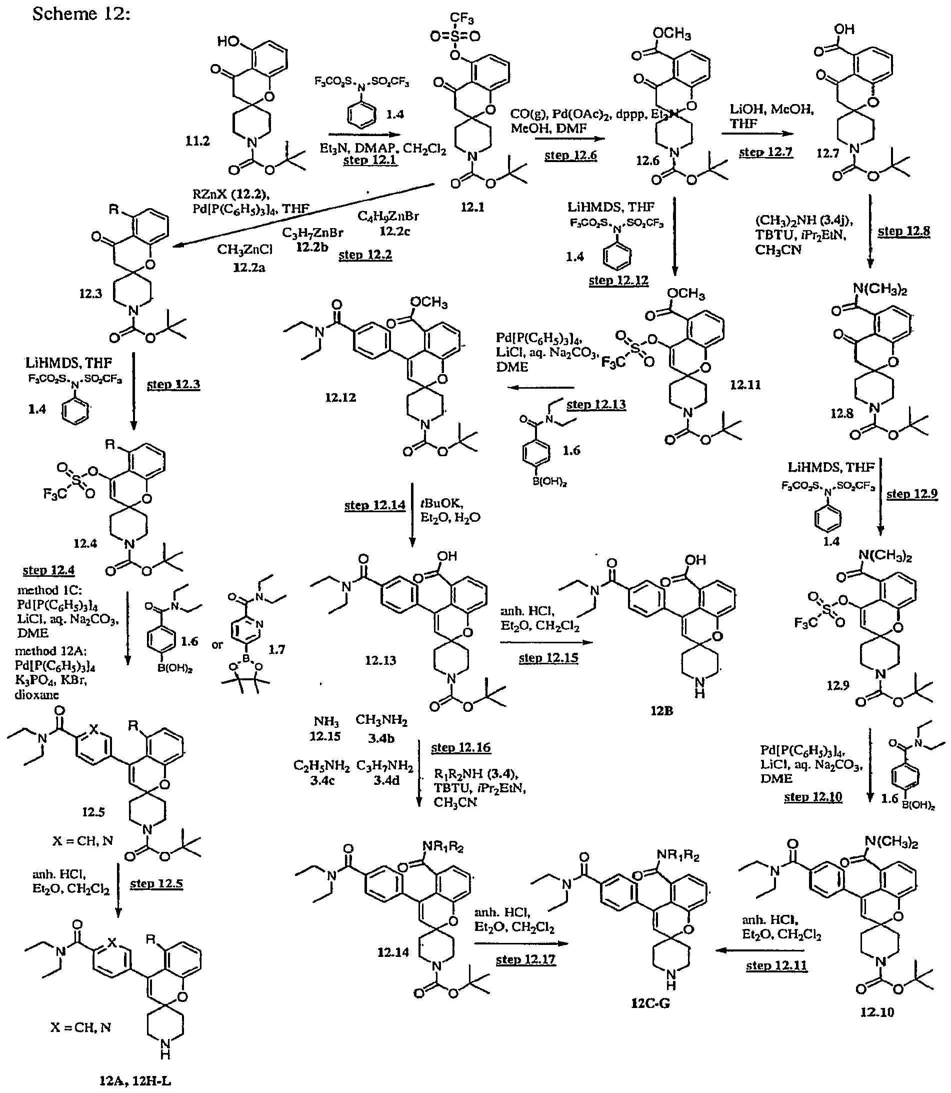

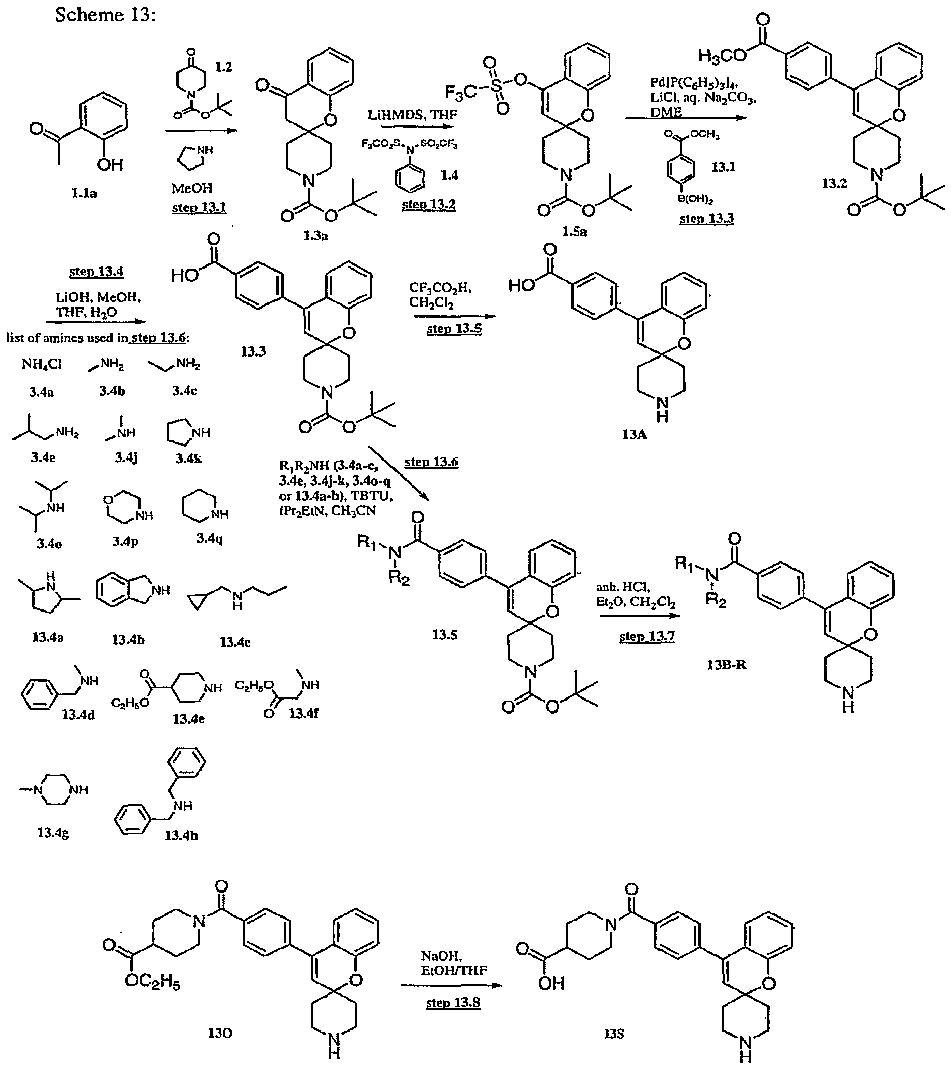

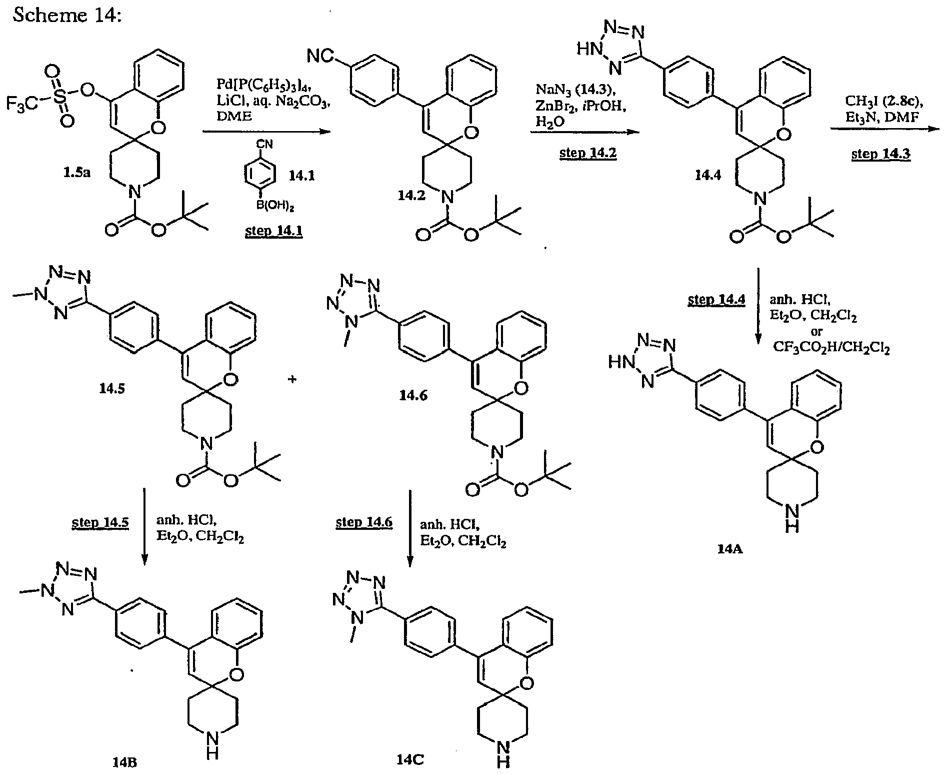

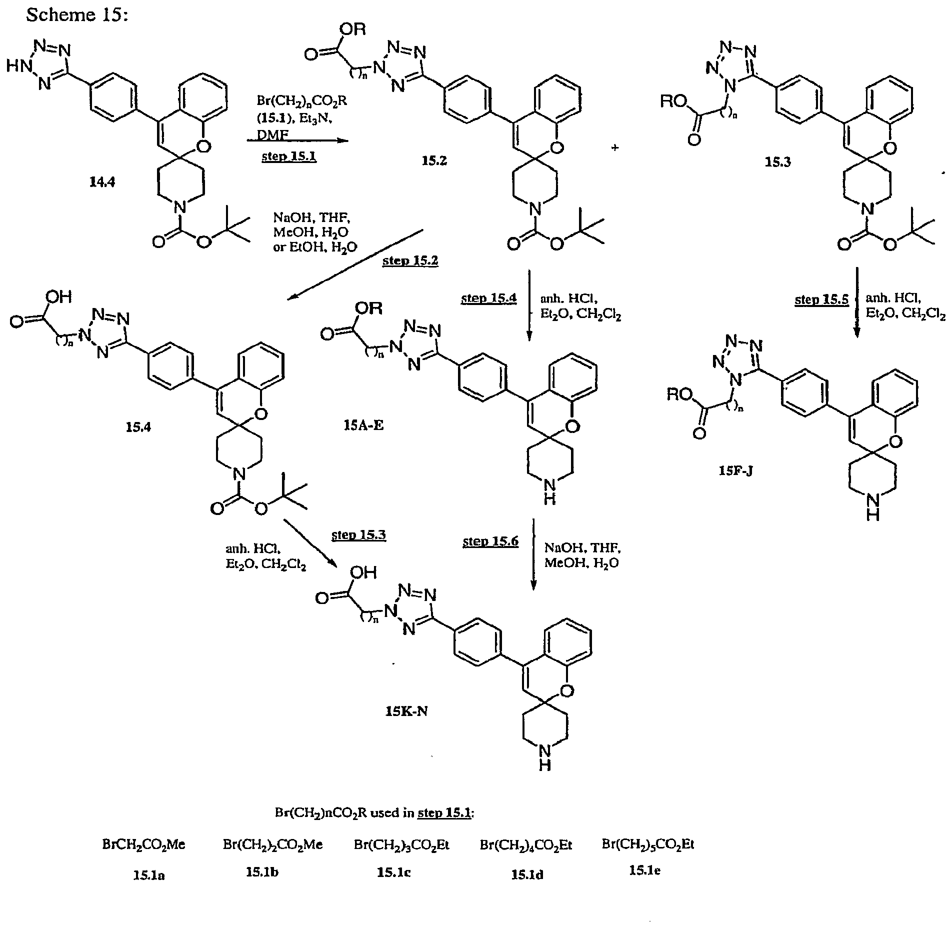

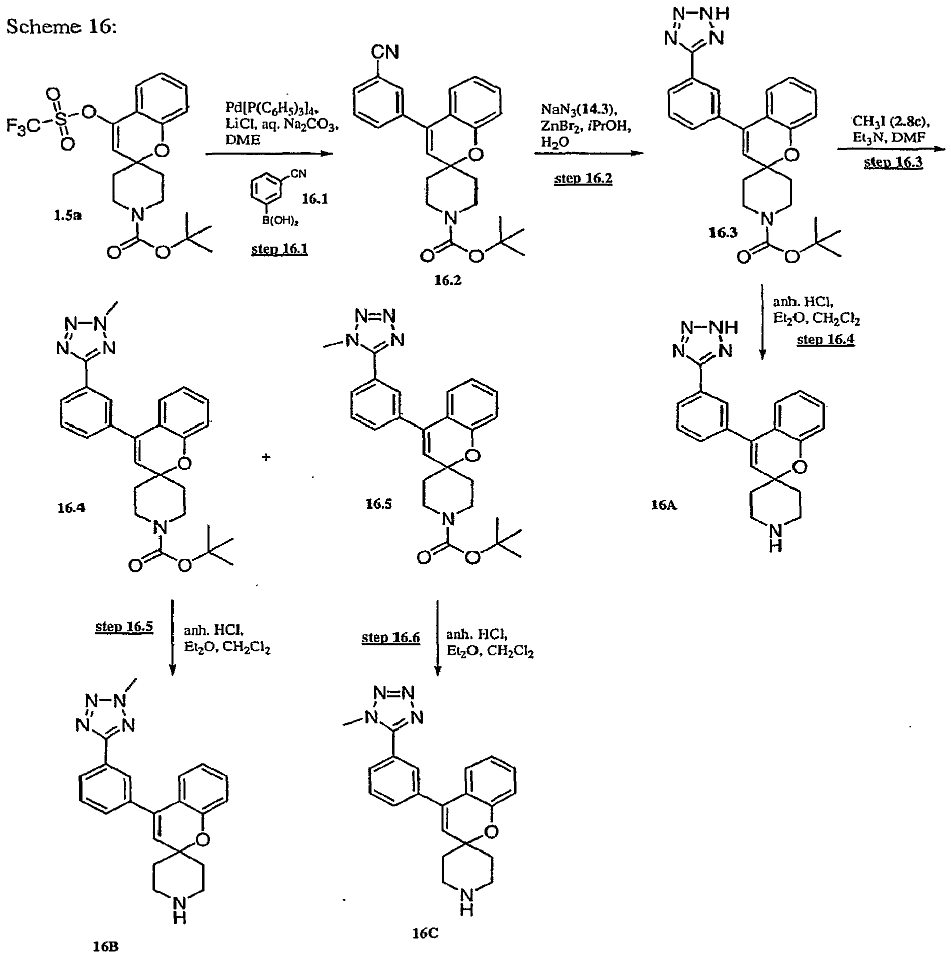

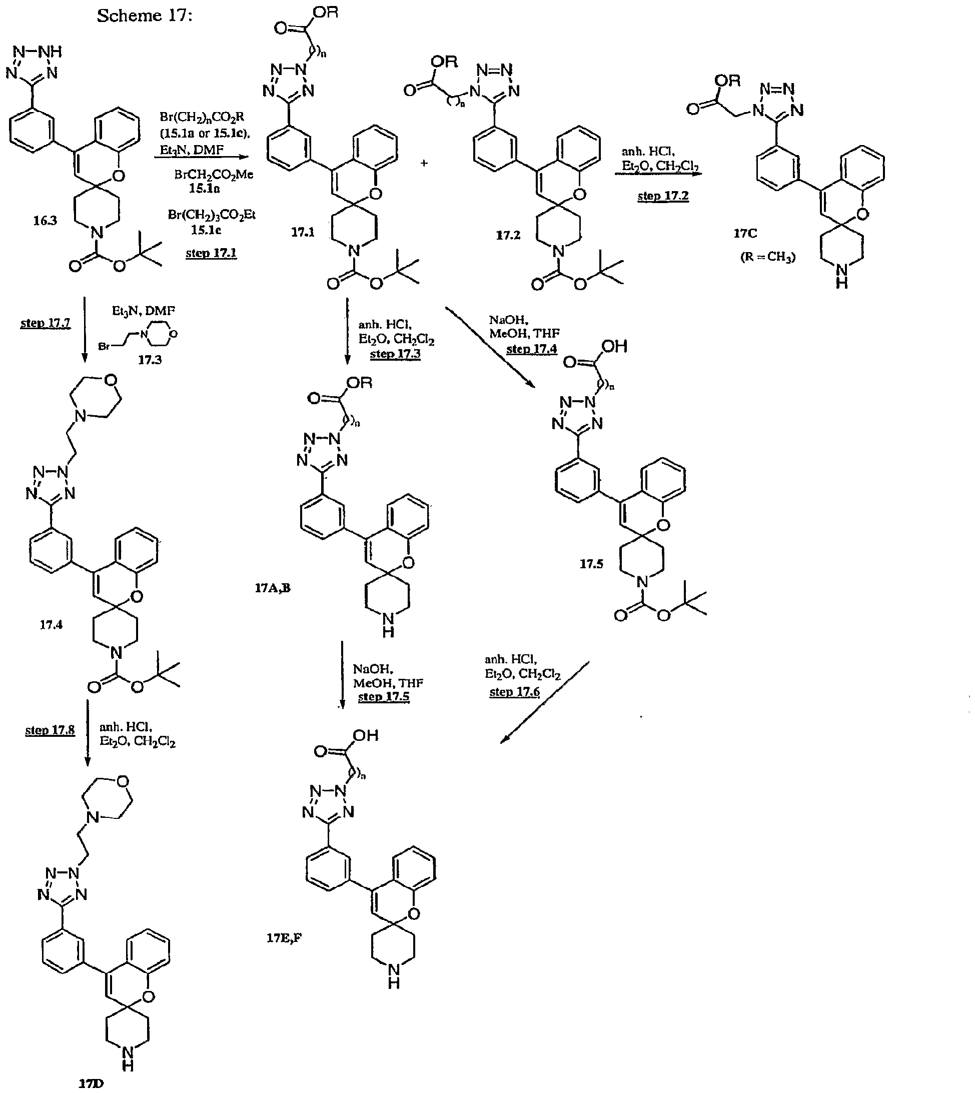

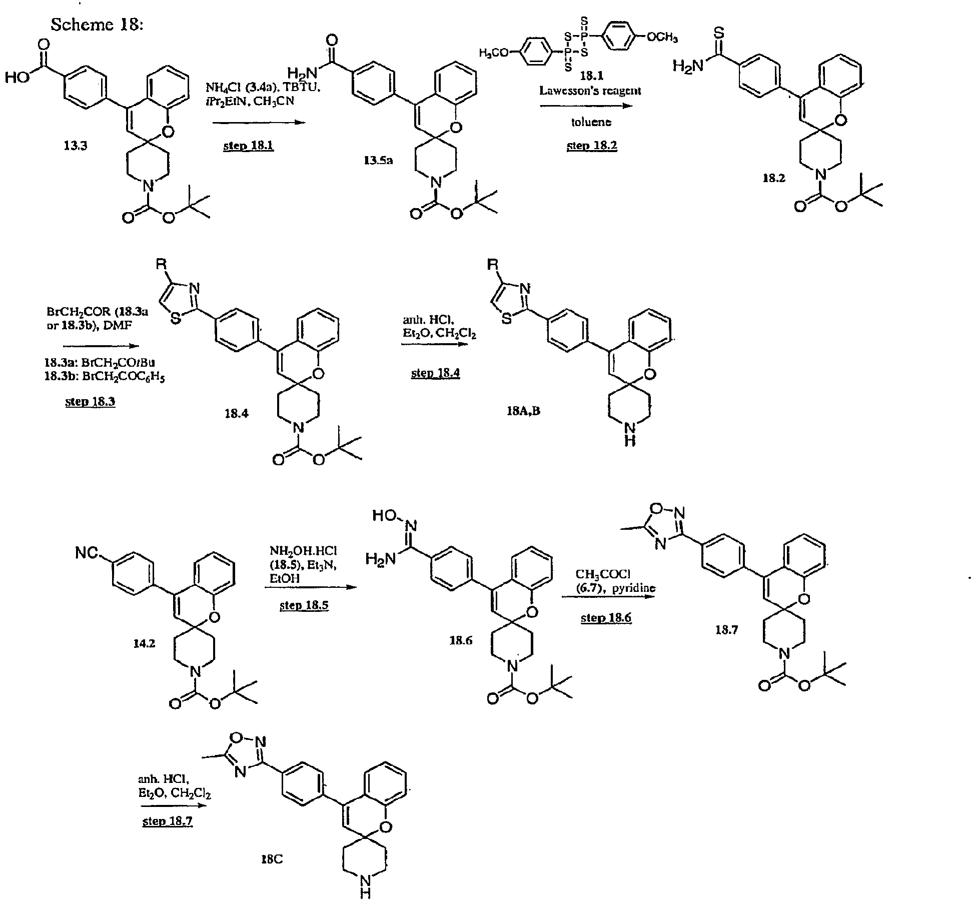

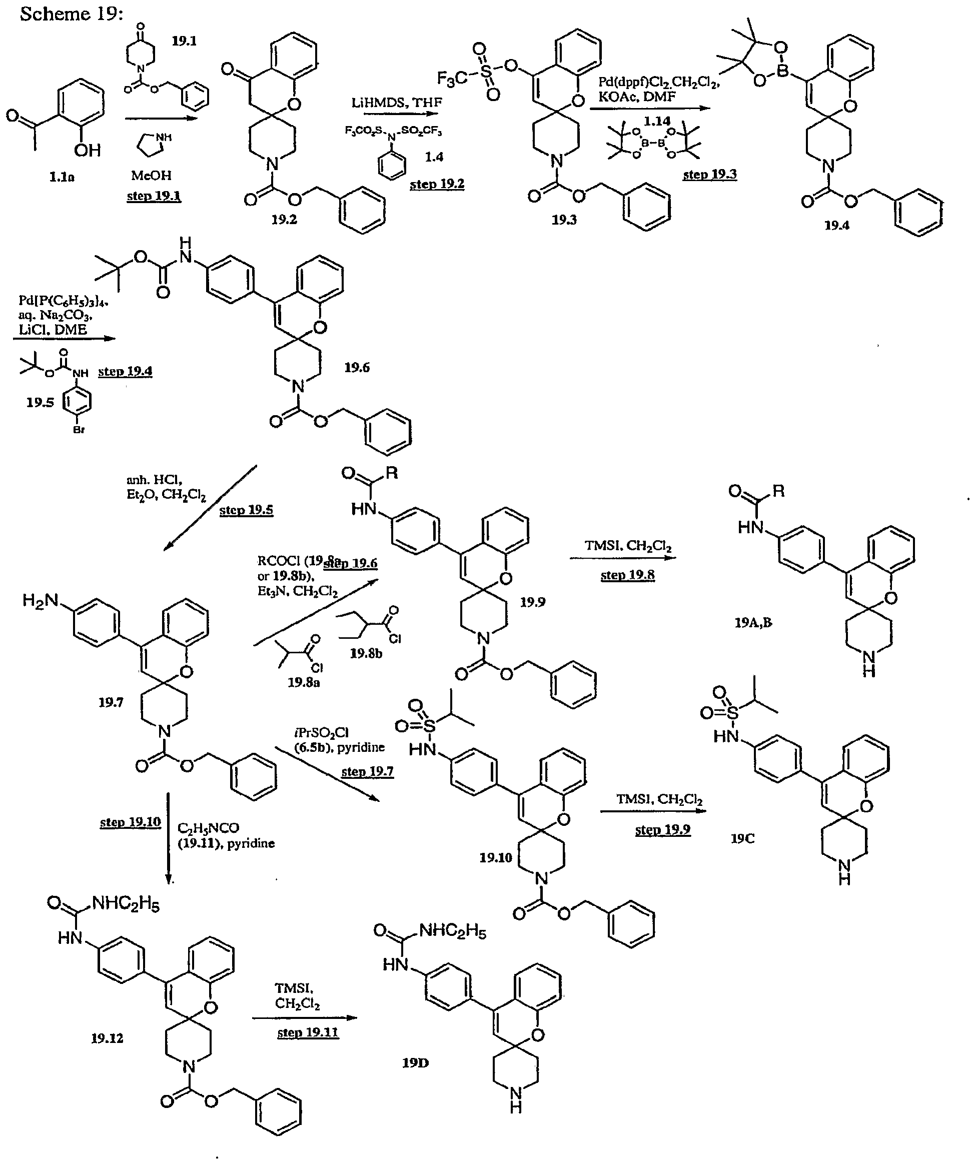

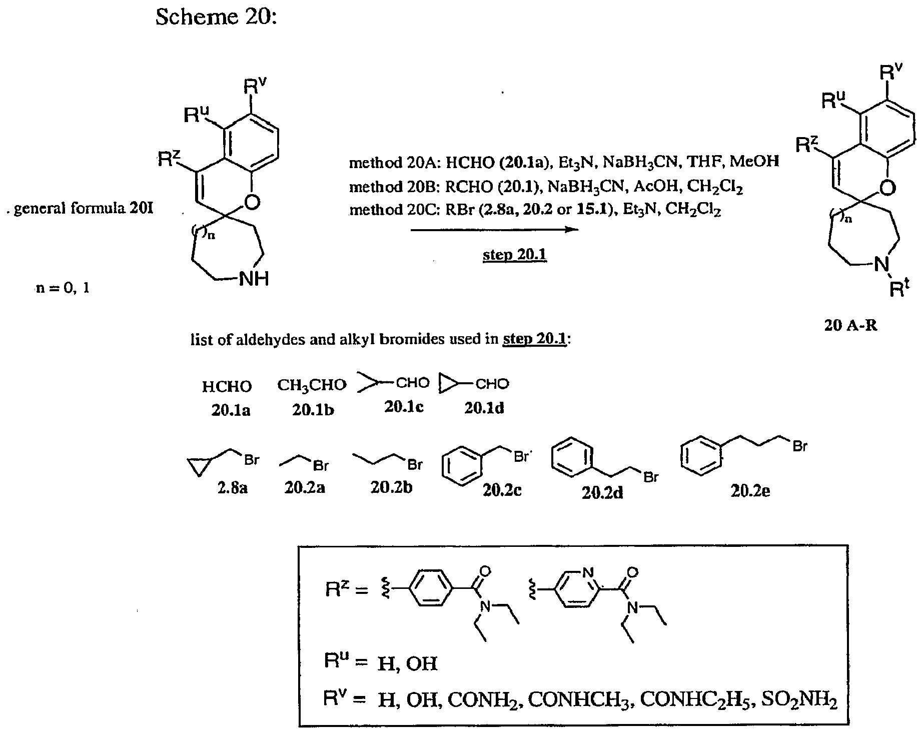

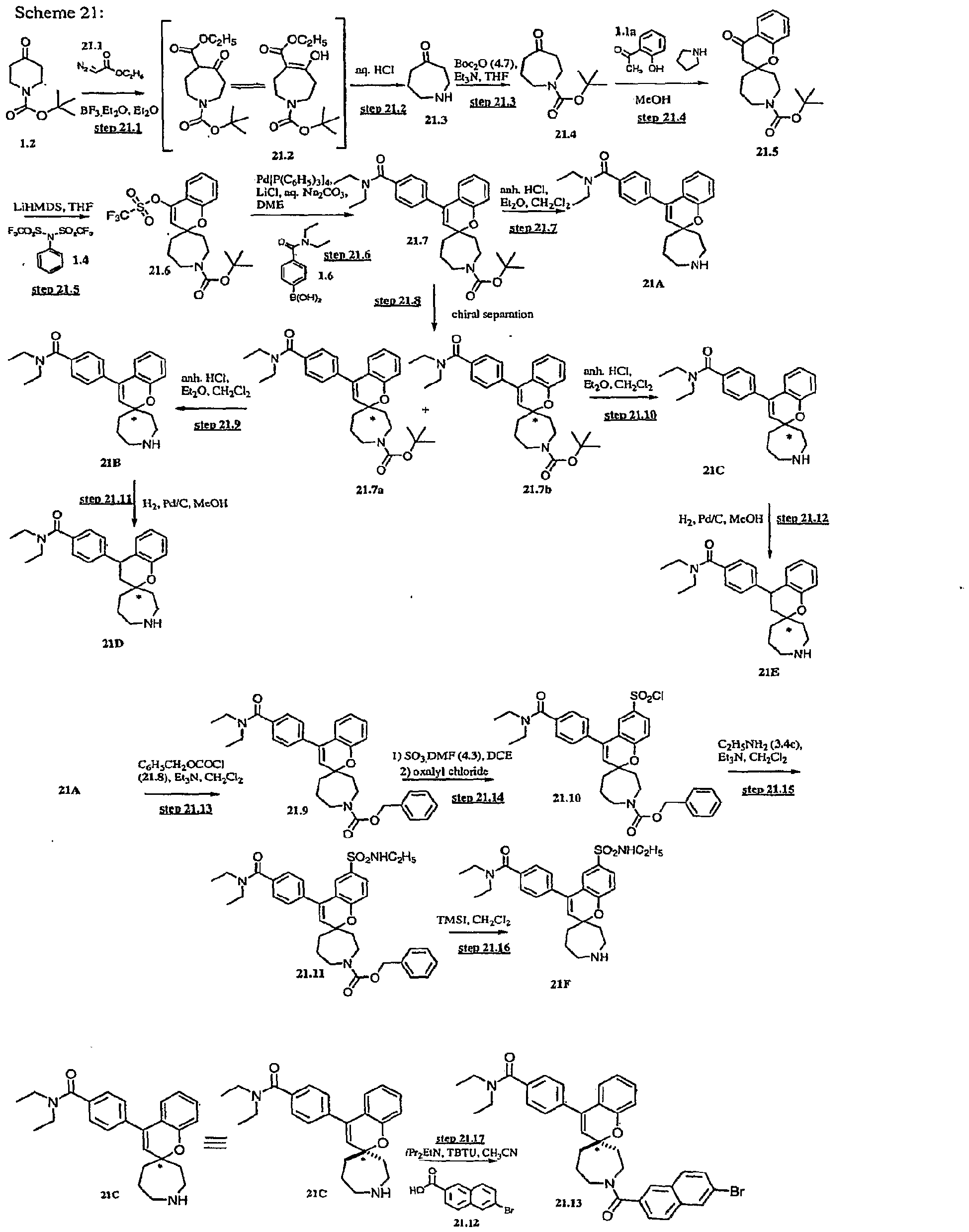

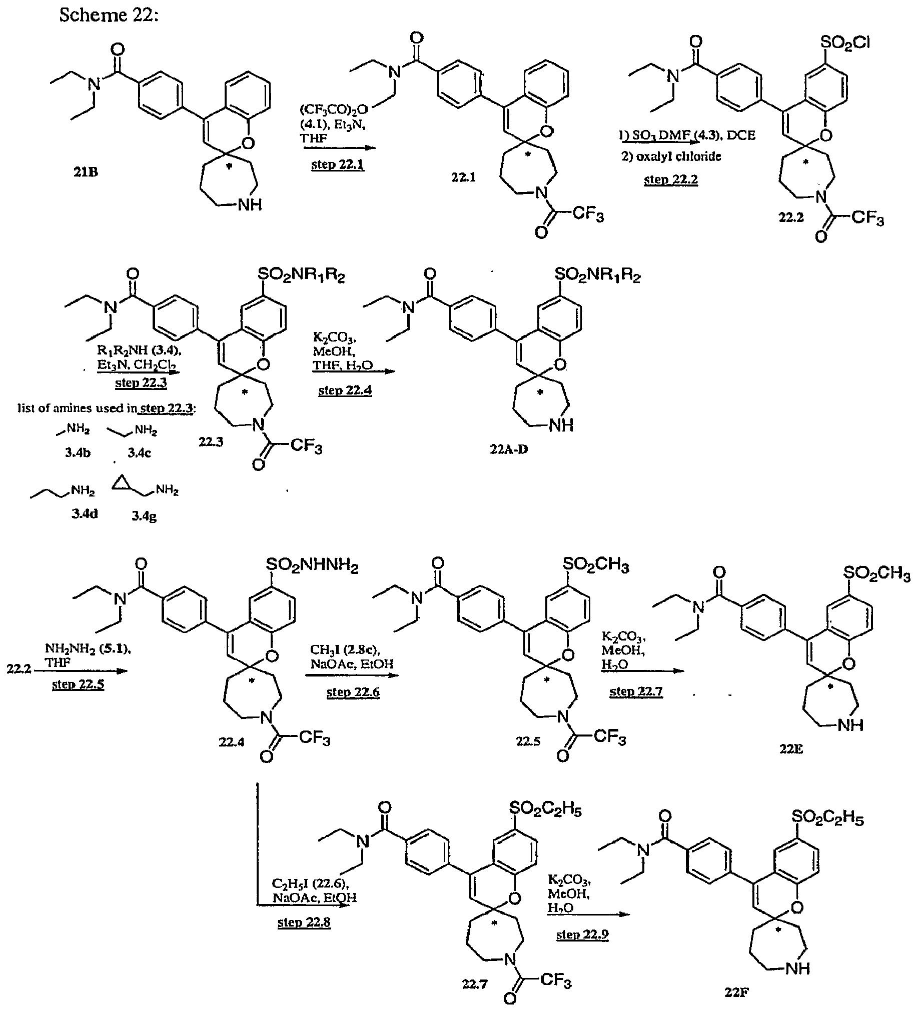

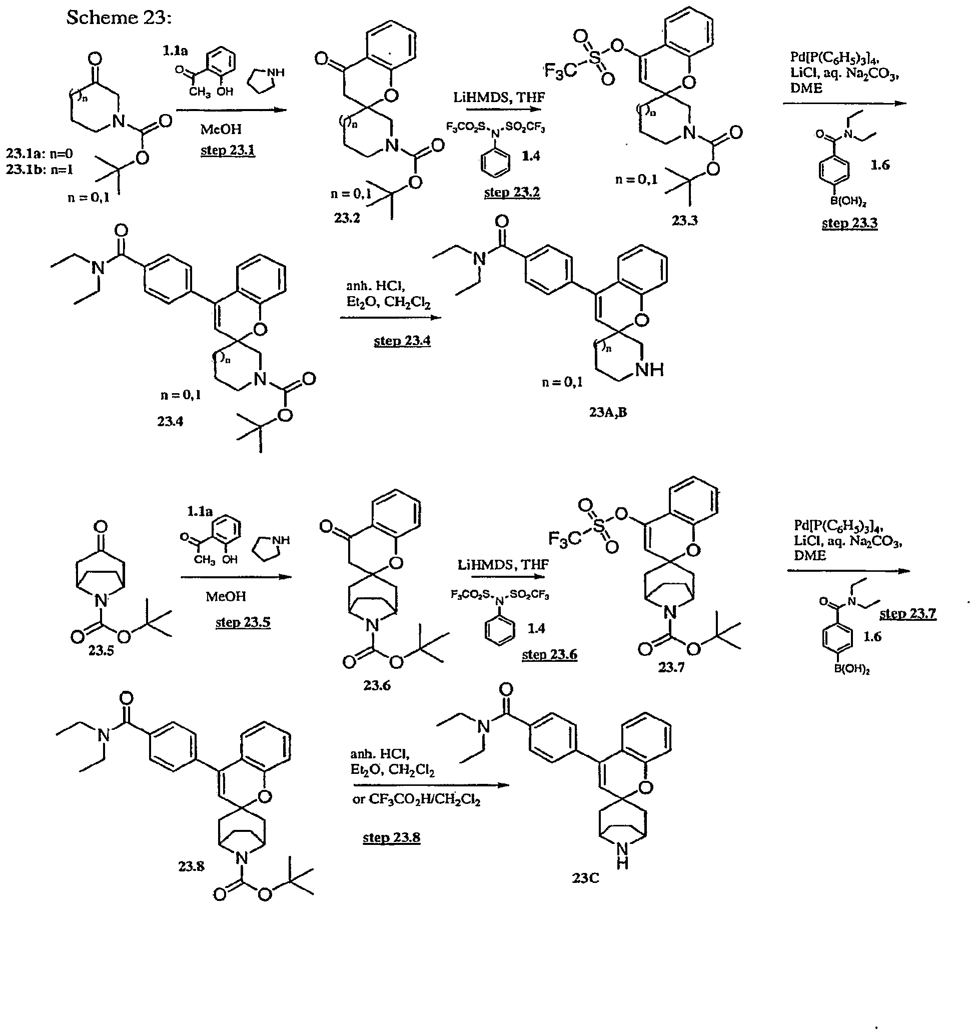

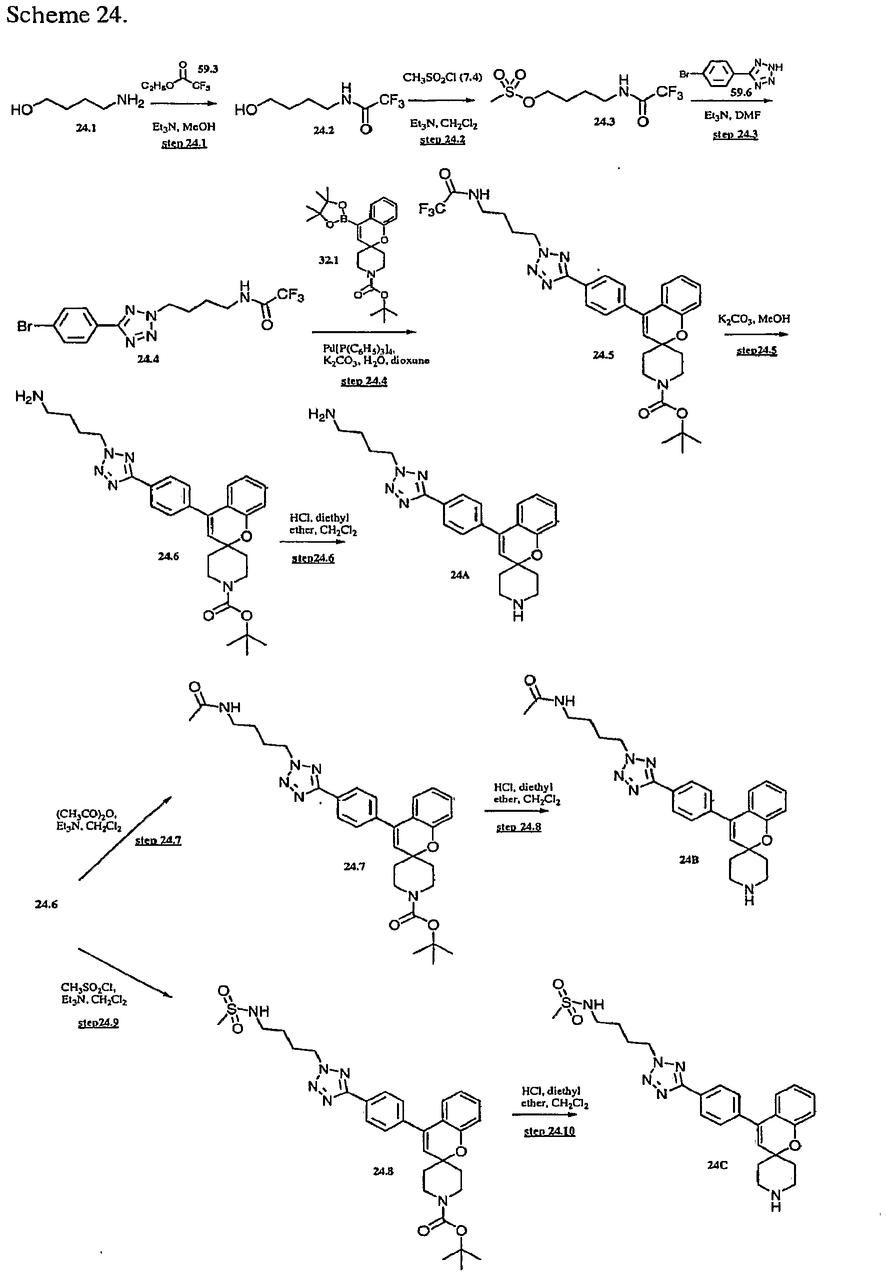

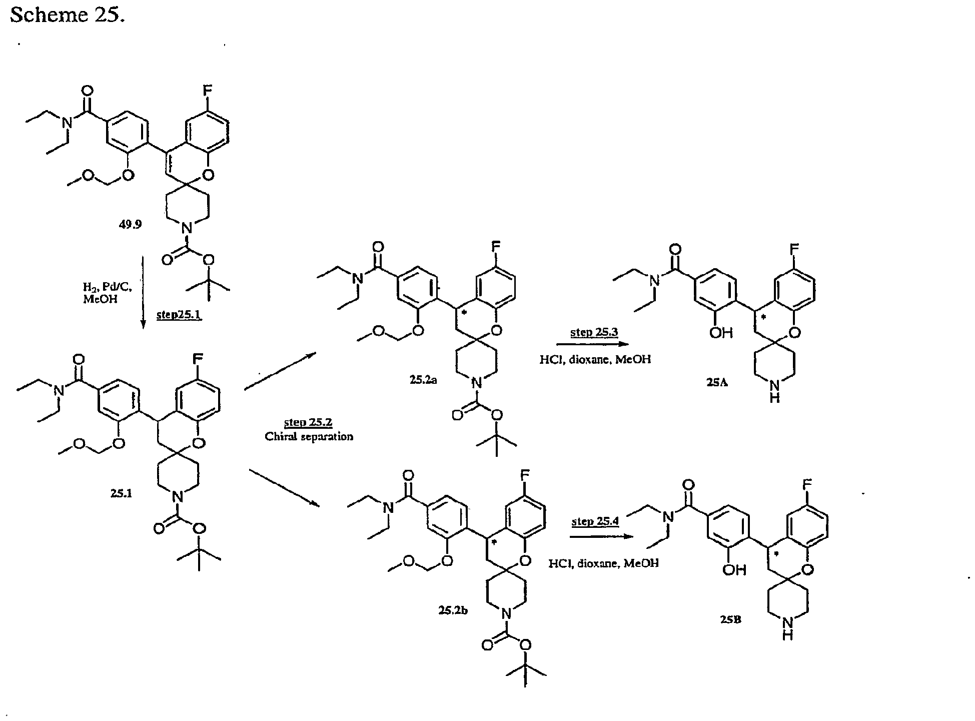

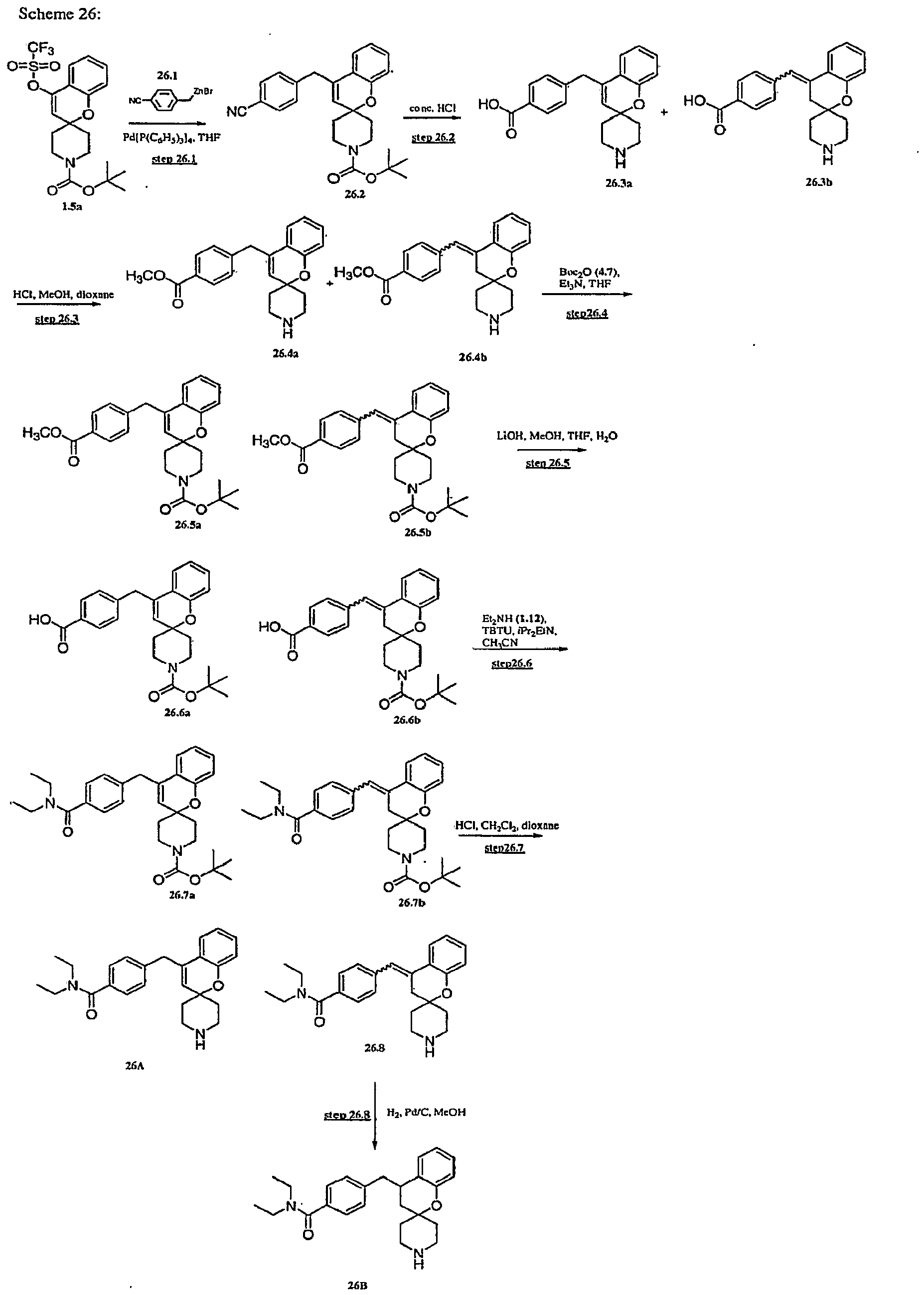

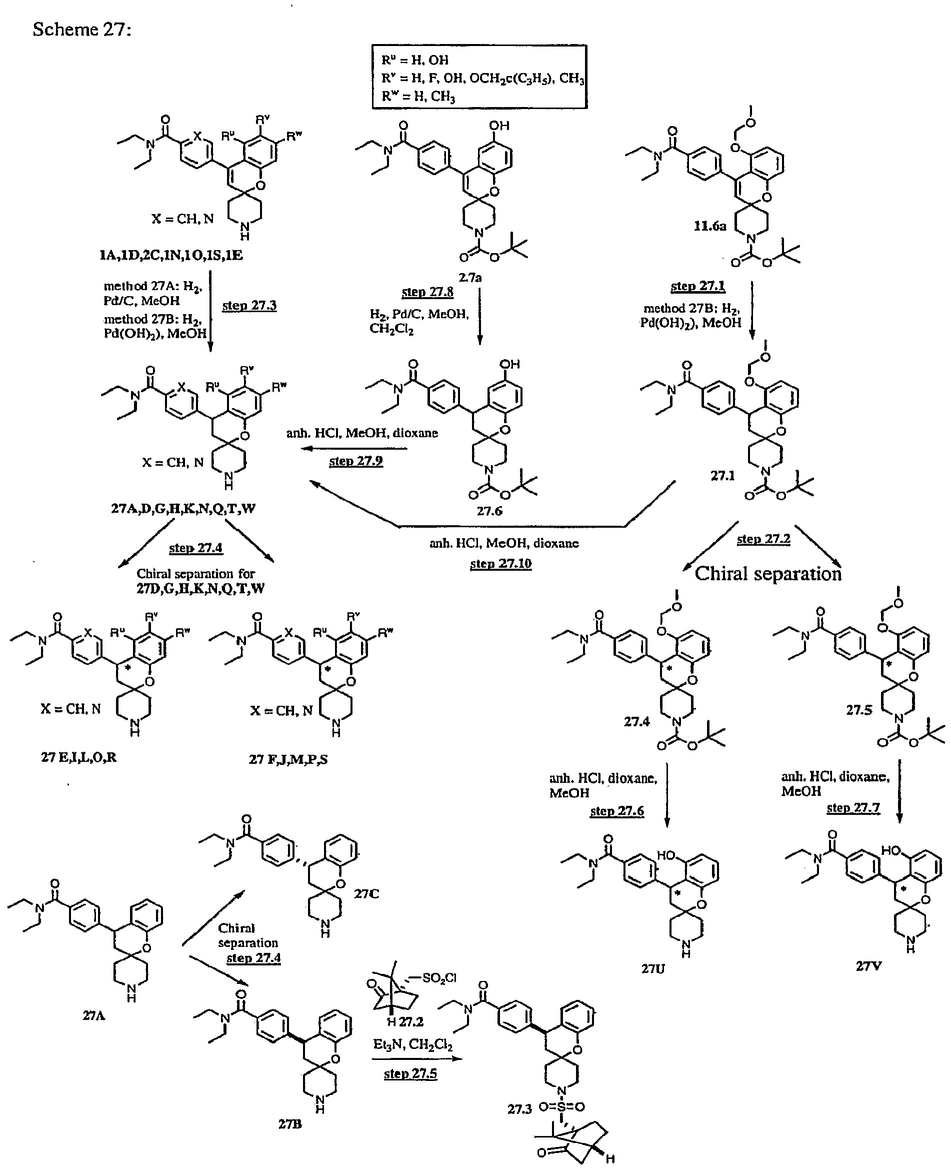

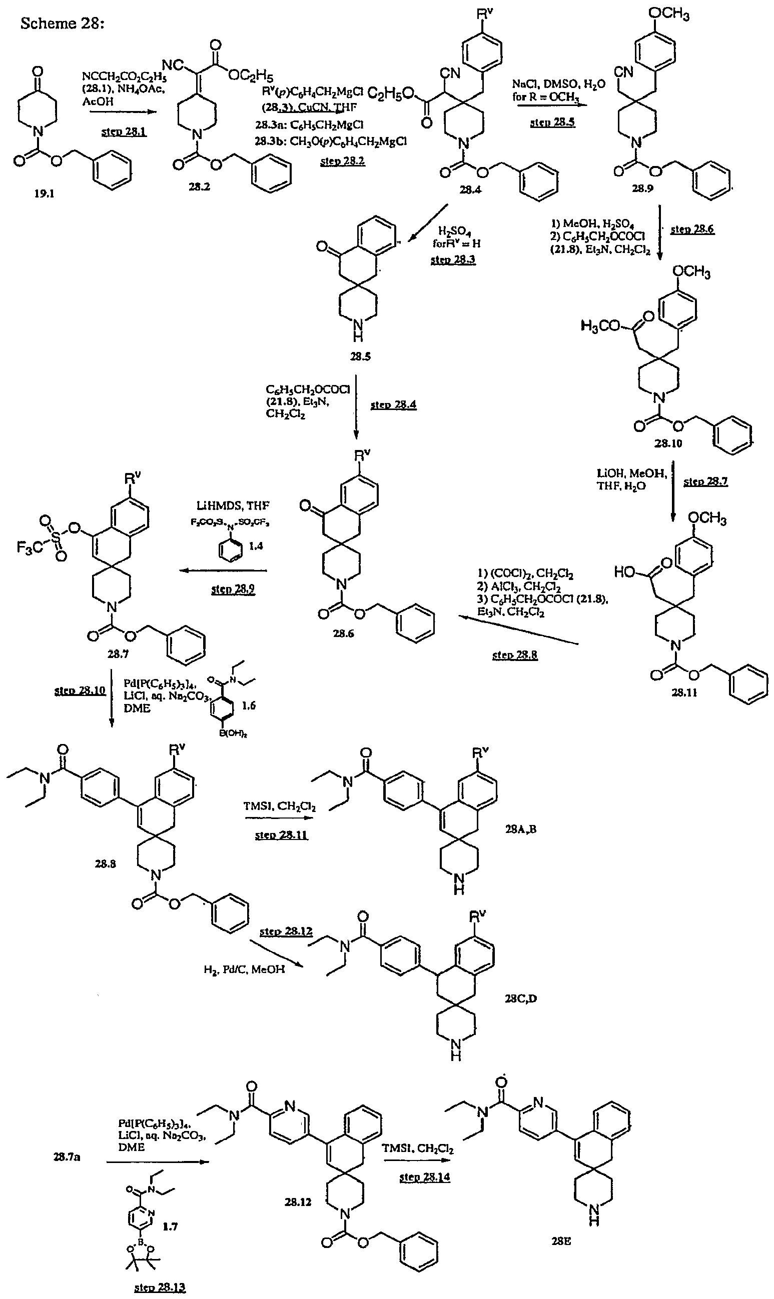

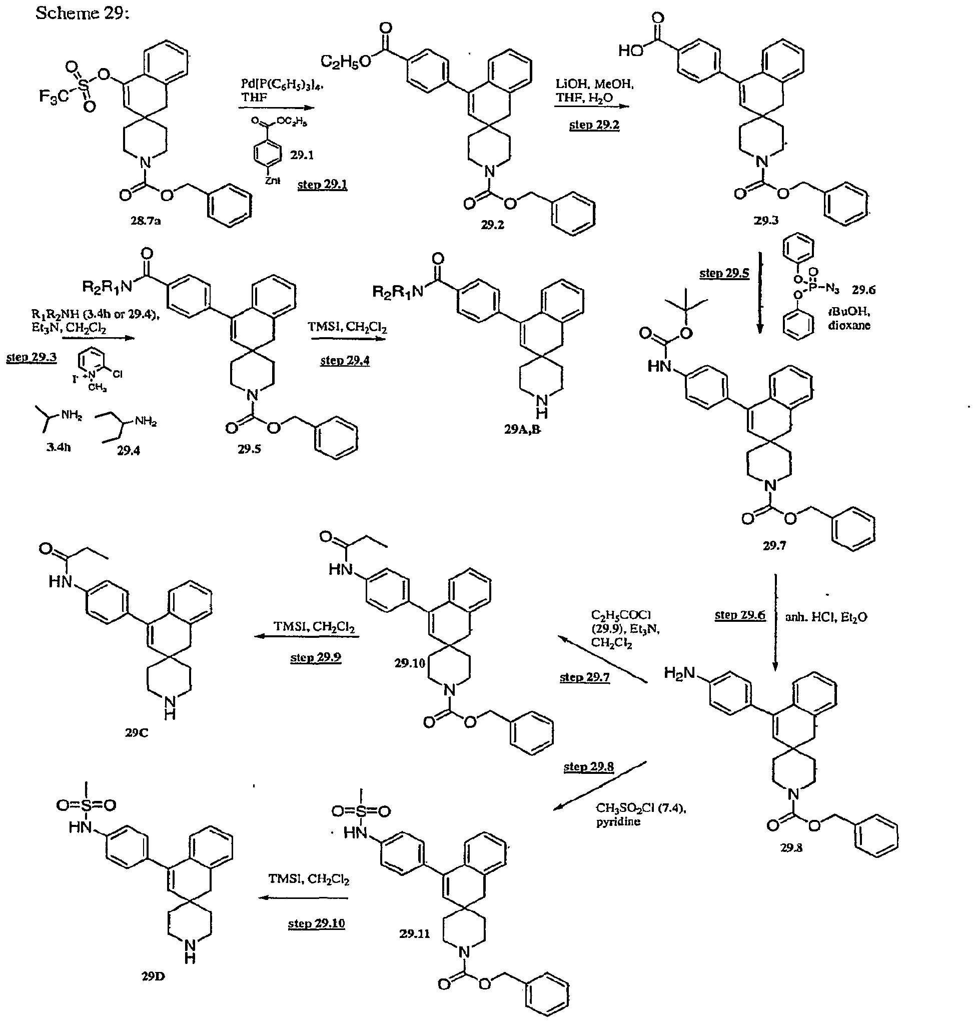

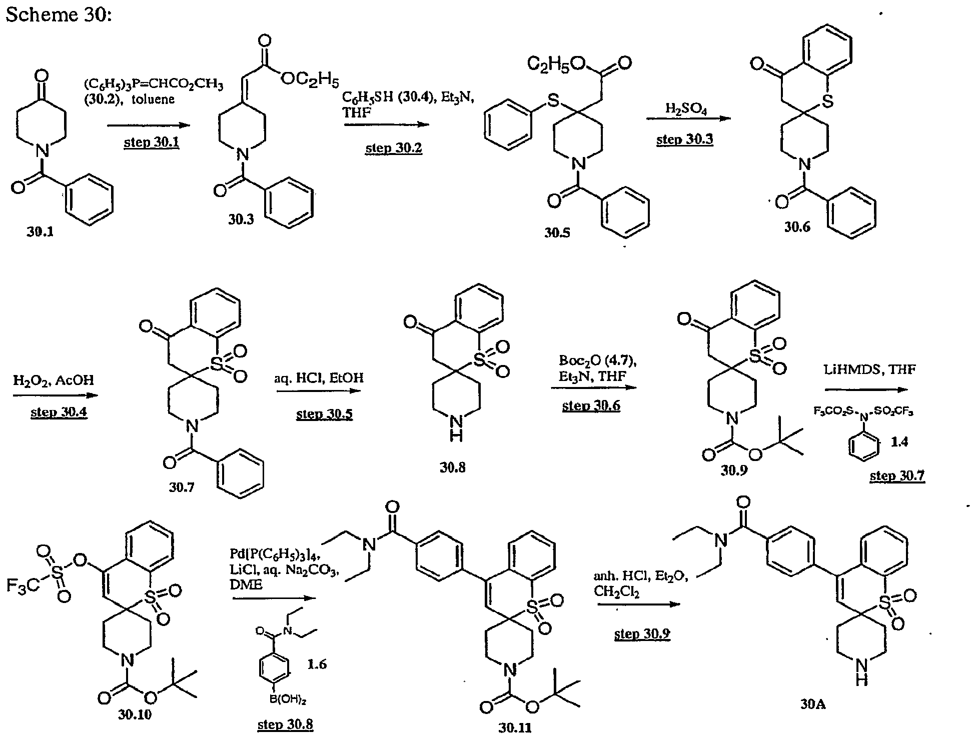

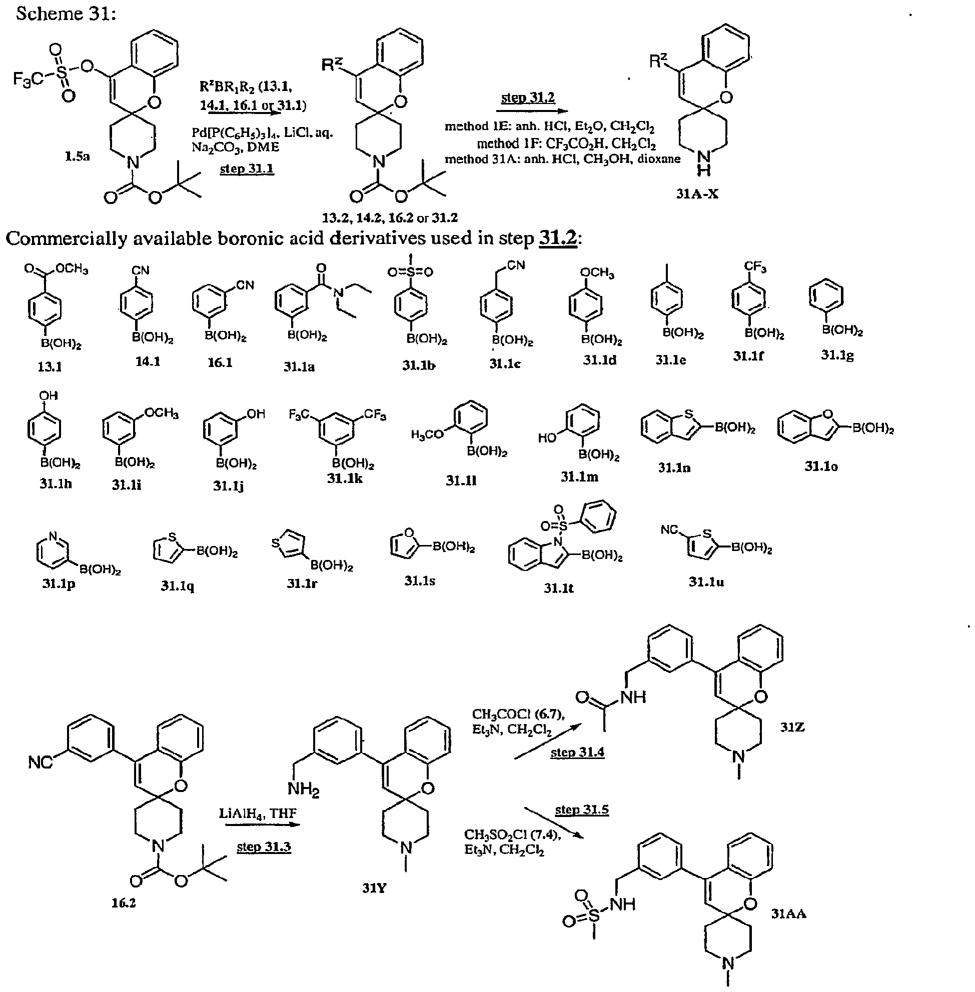

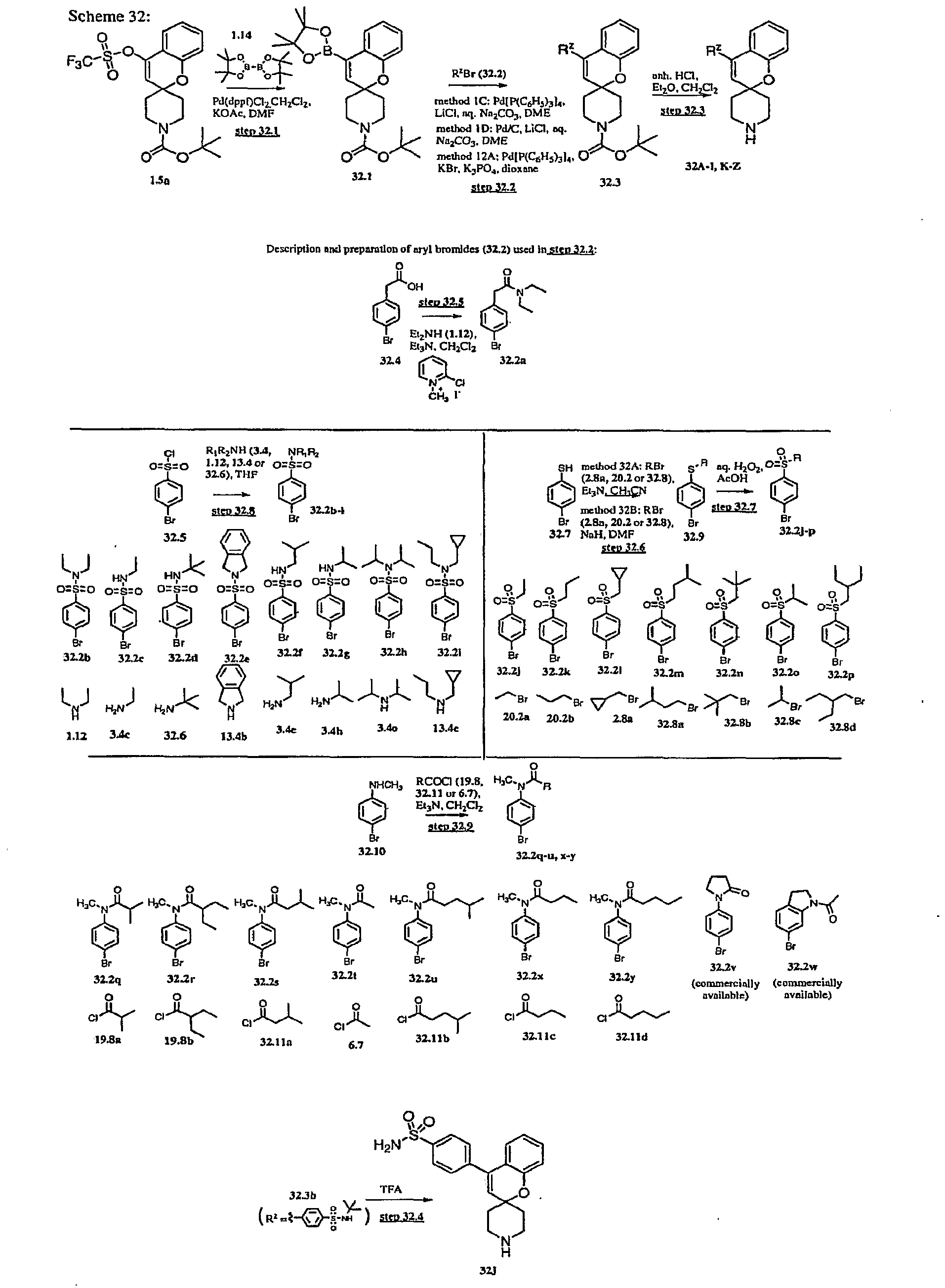

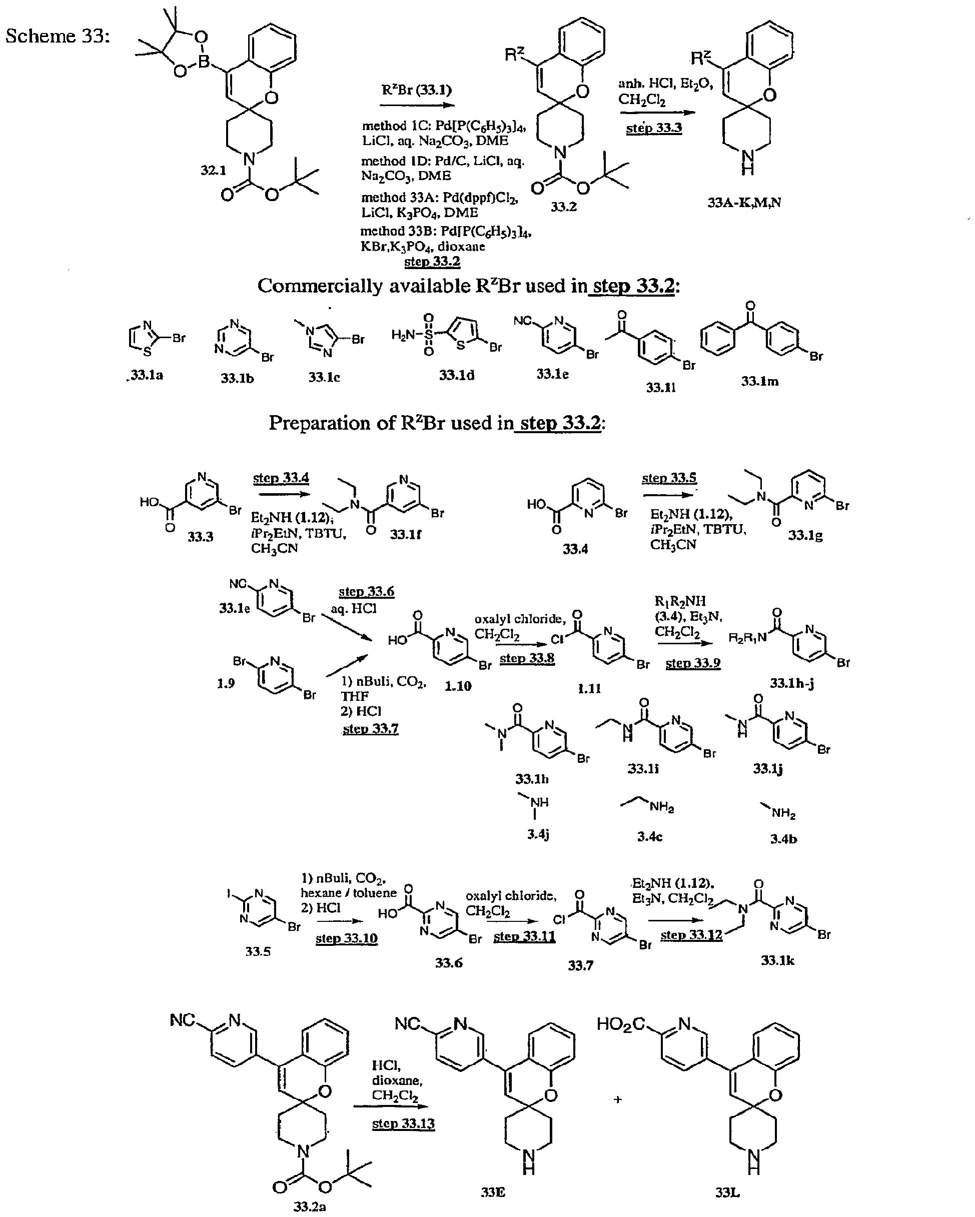

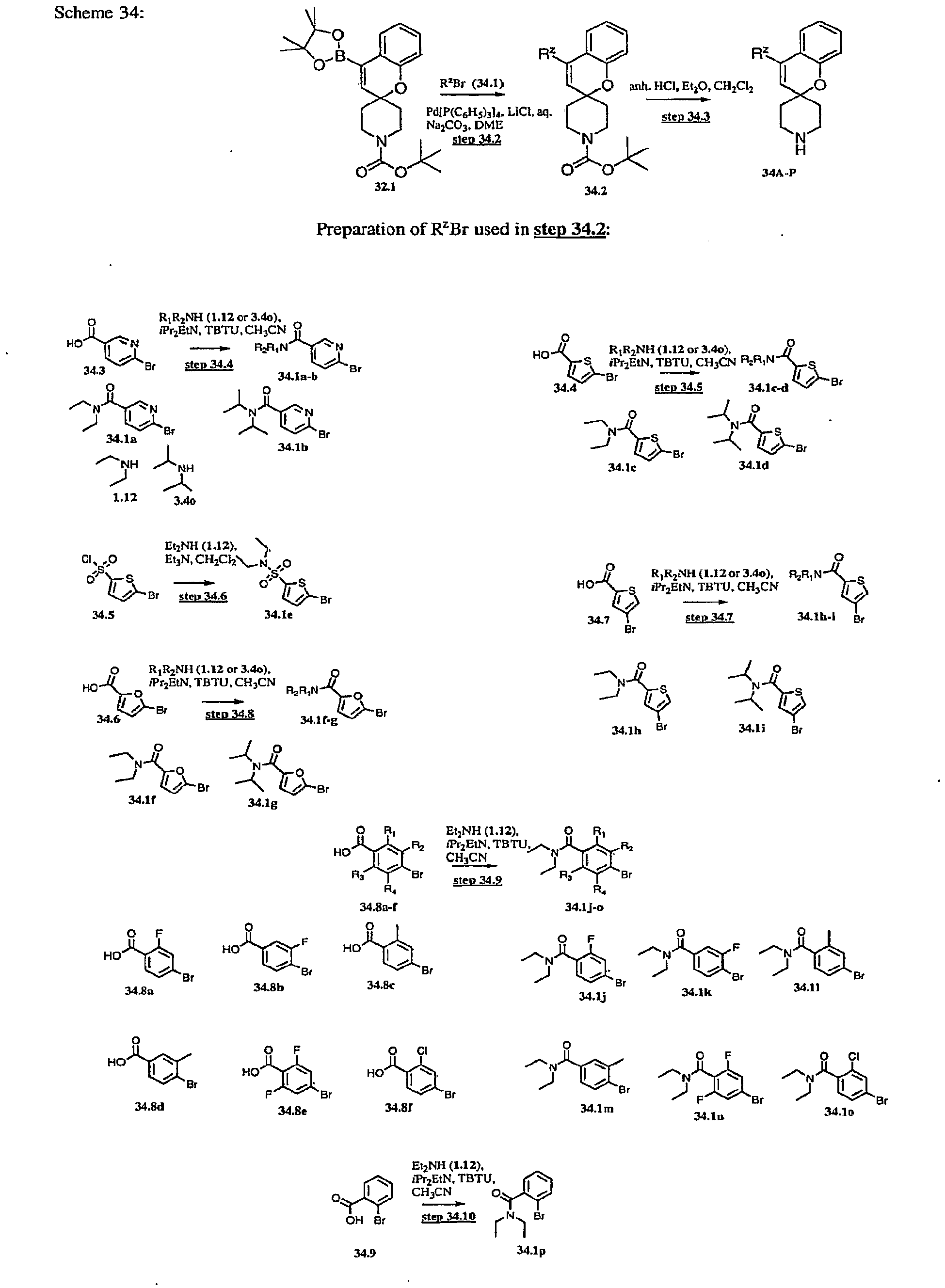

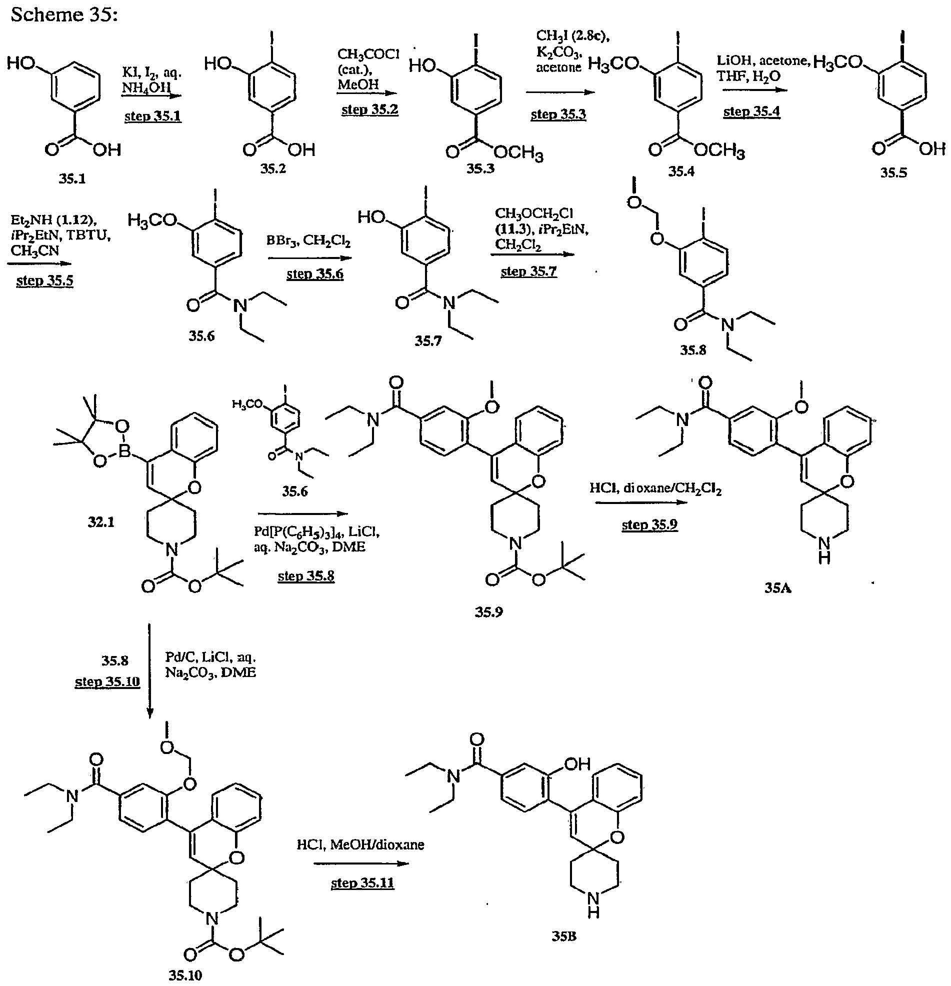

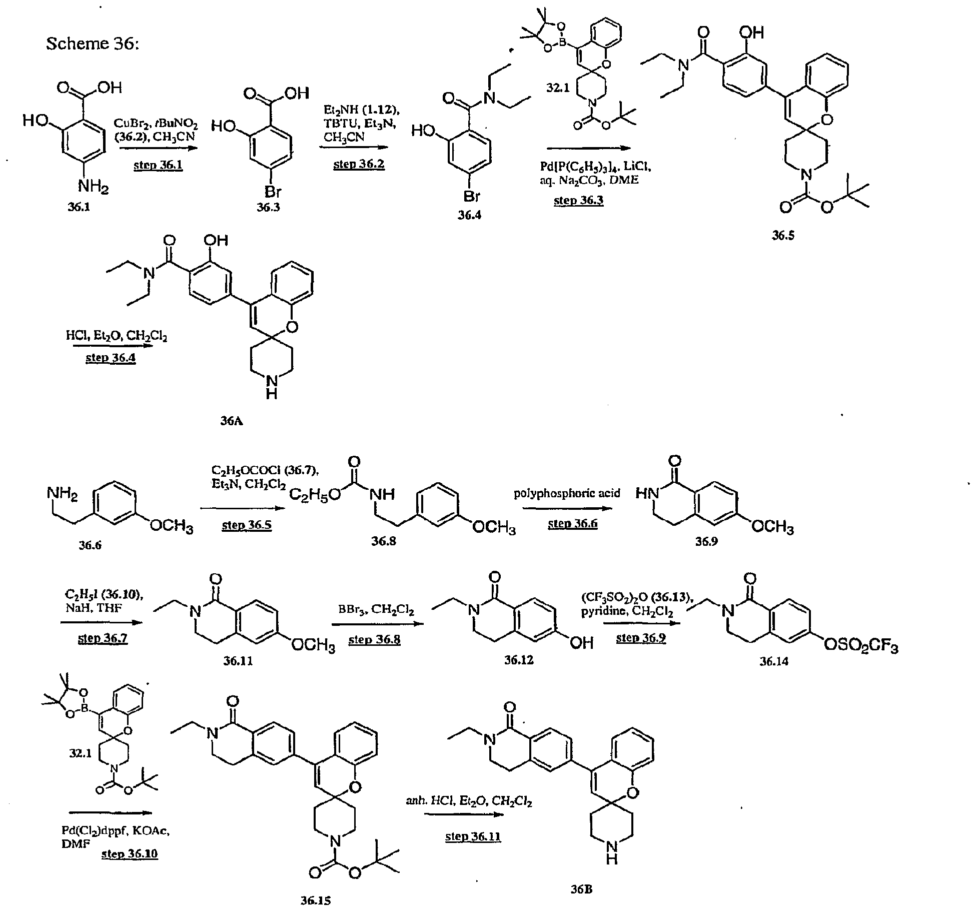

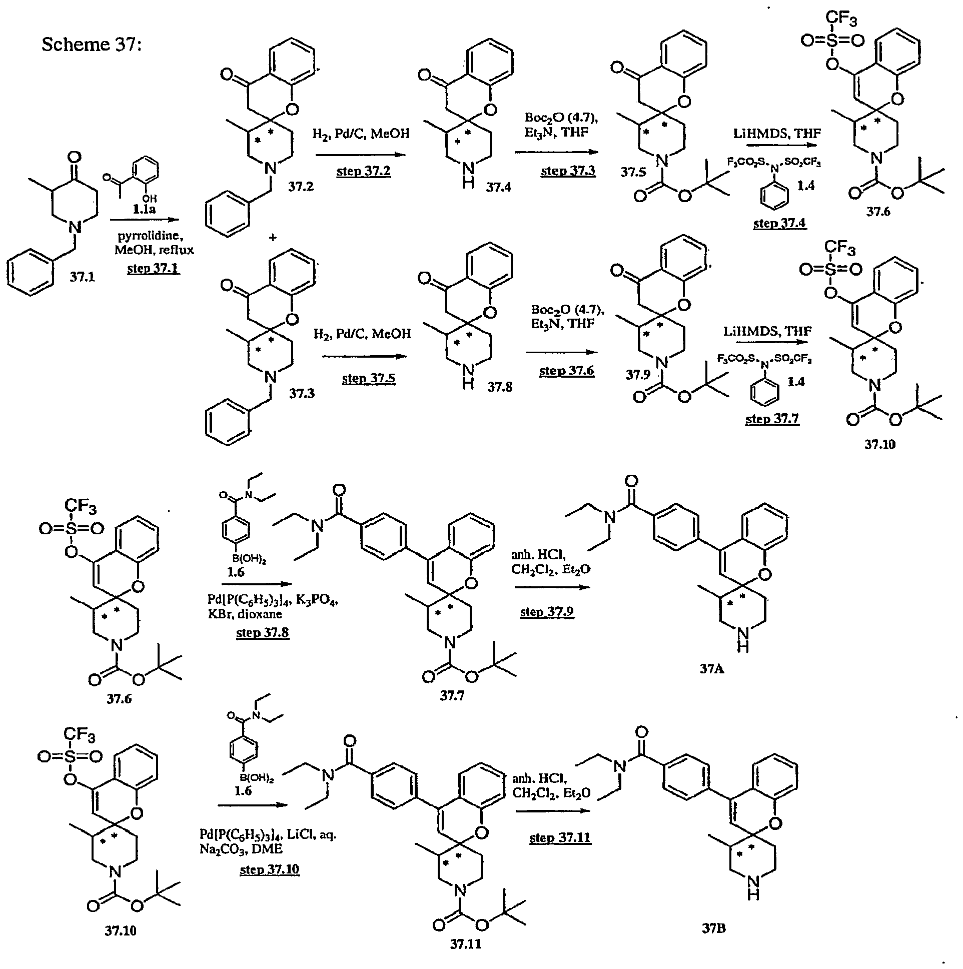

**37A** and **37B** are diastereomeric with respect to one another, but each is a racemic mixture of its two possible enantiomers. Their absolute stereochemistry has not been conclusively established.

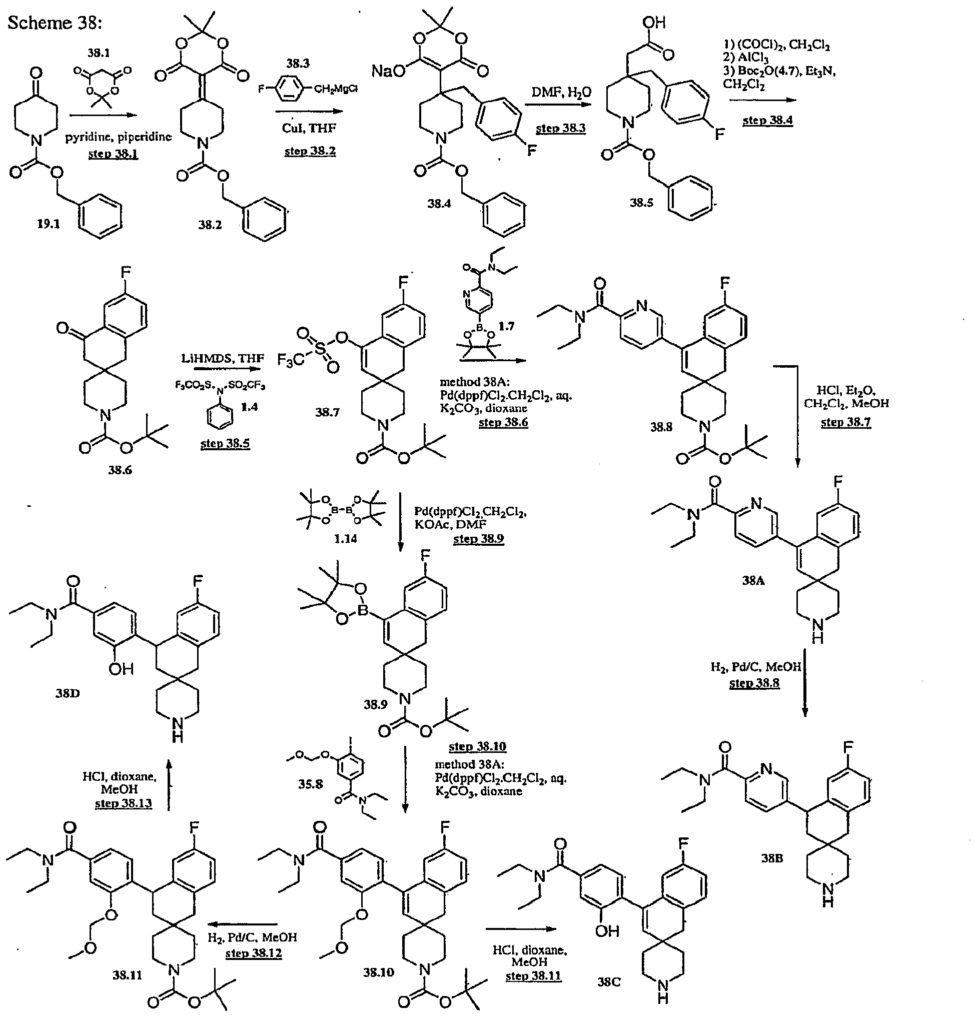

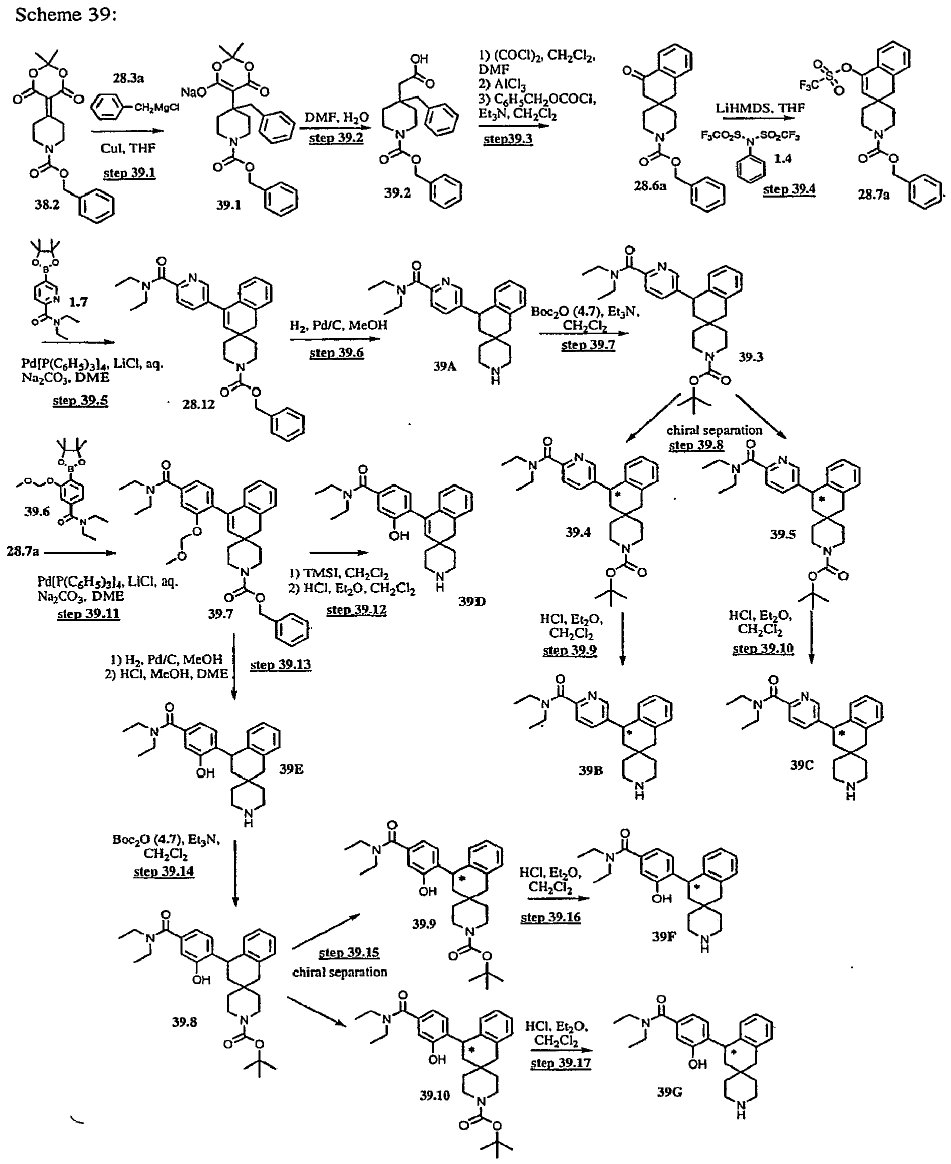

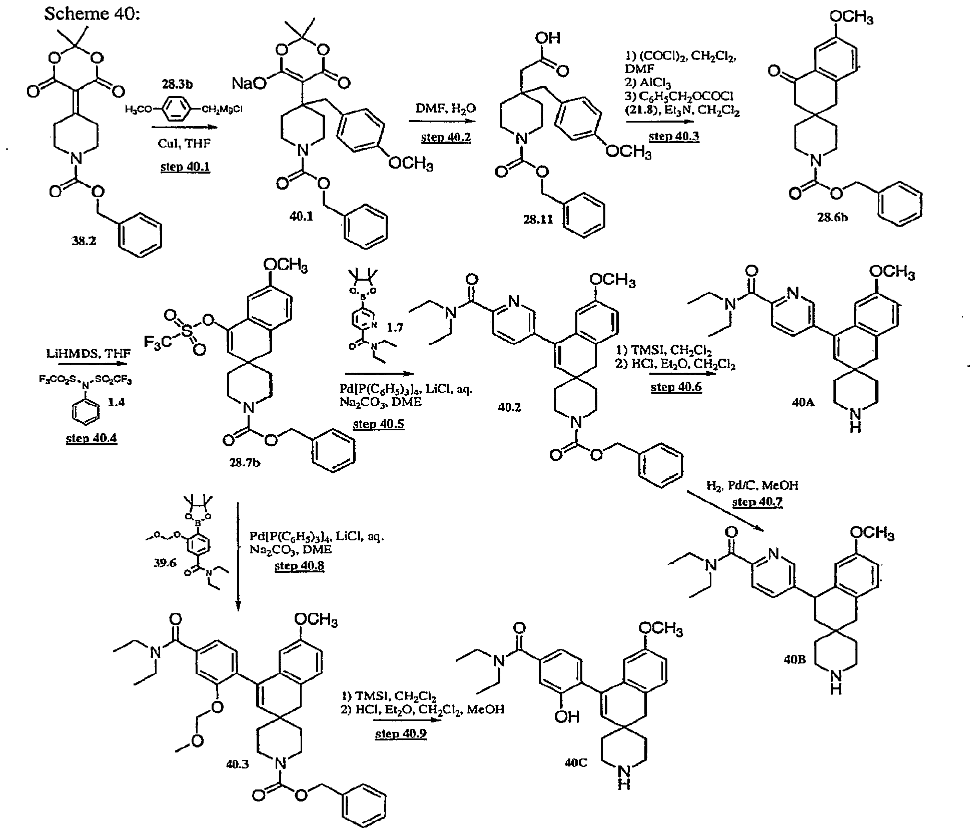

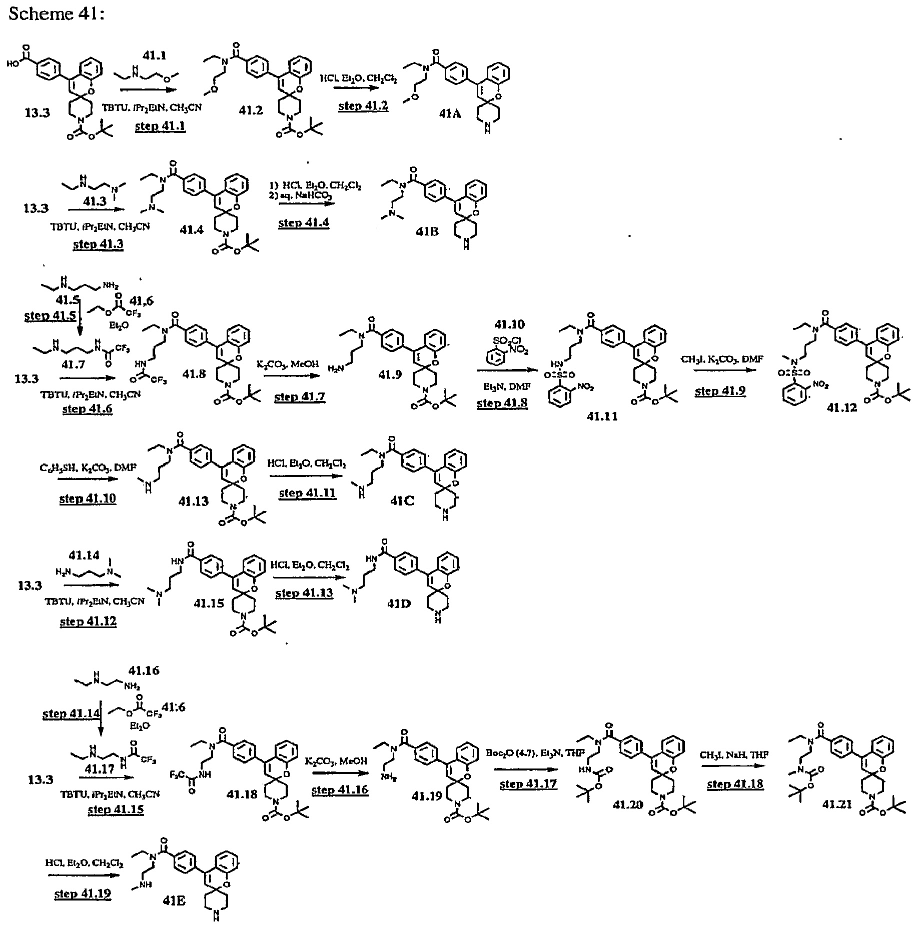

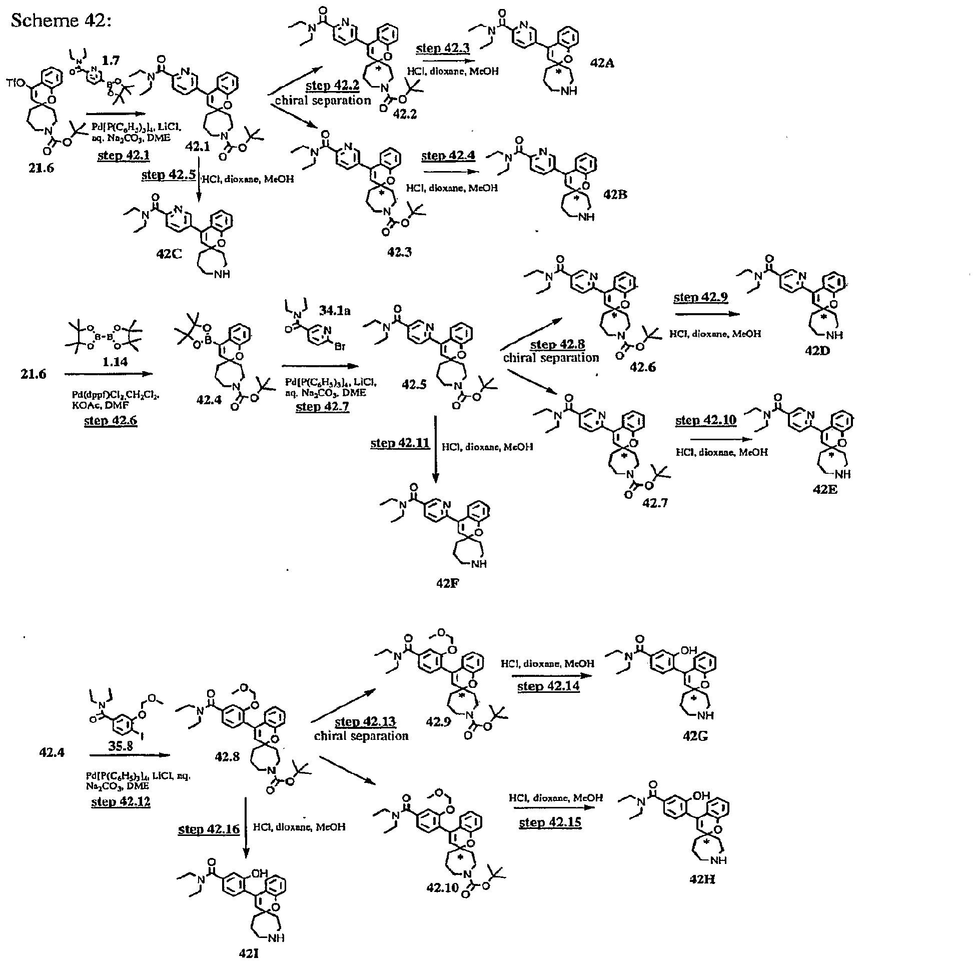

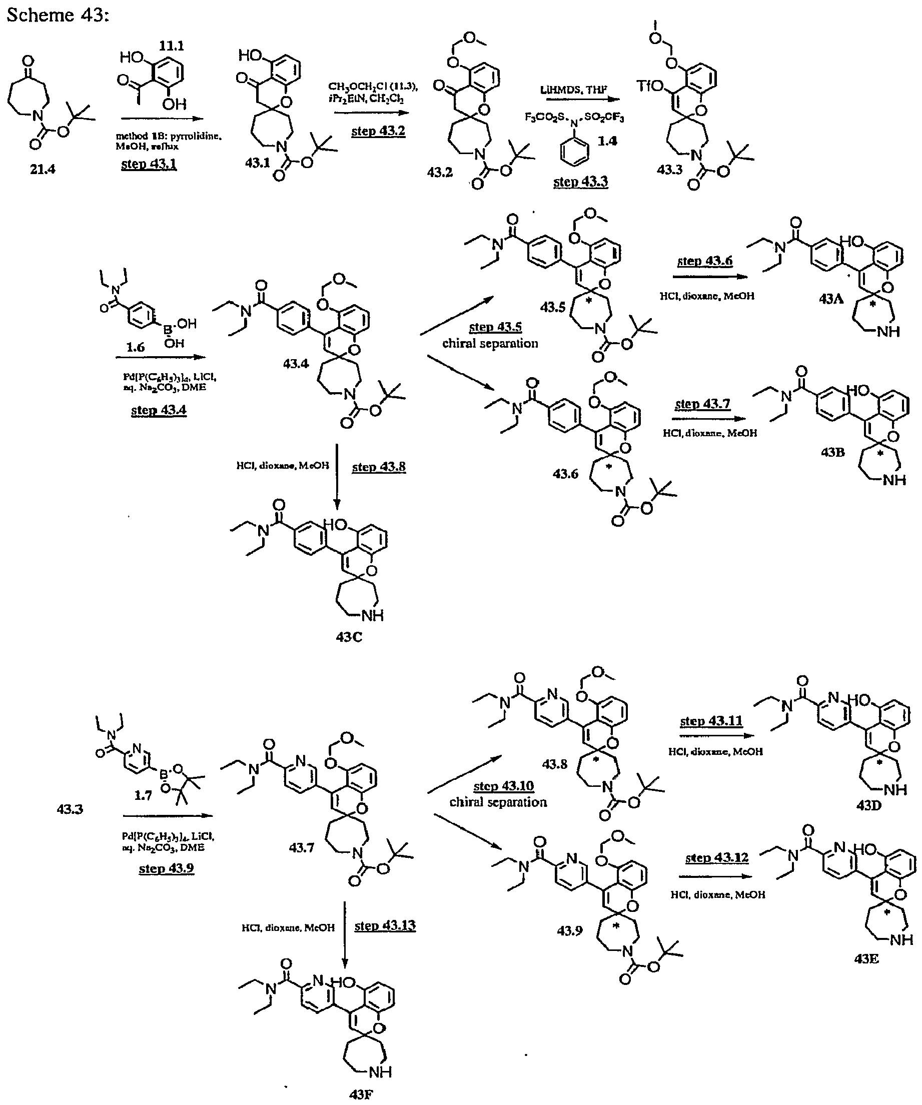

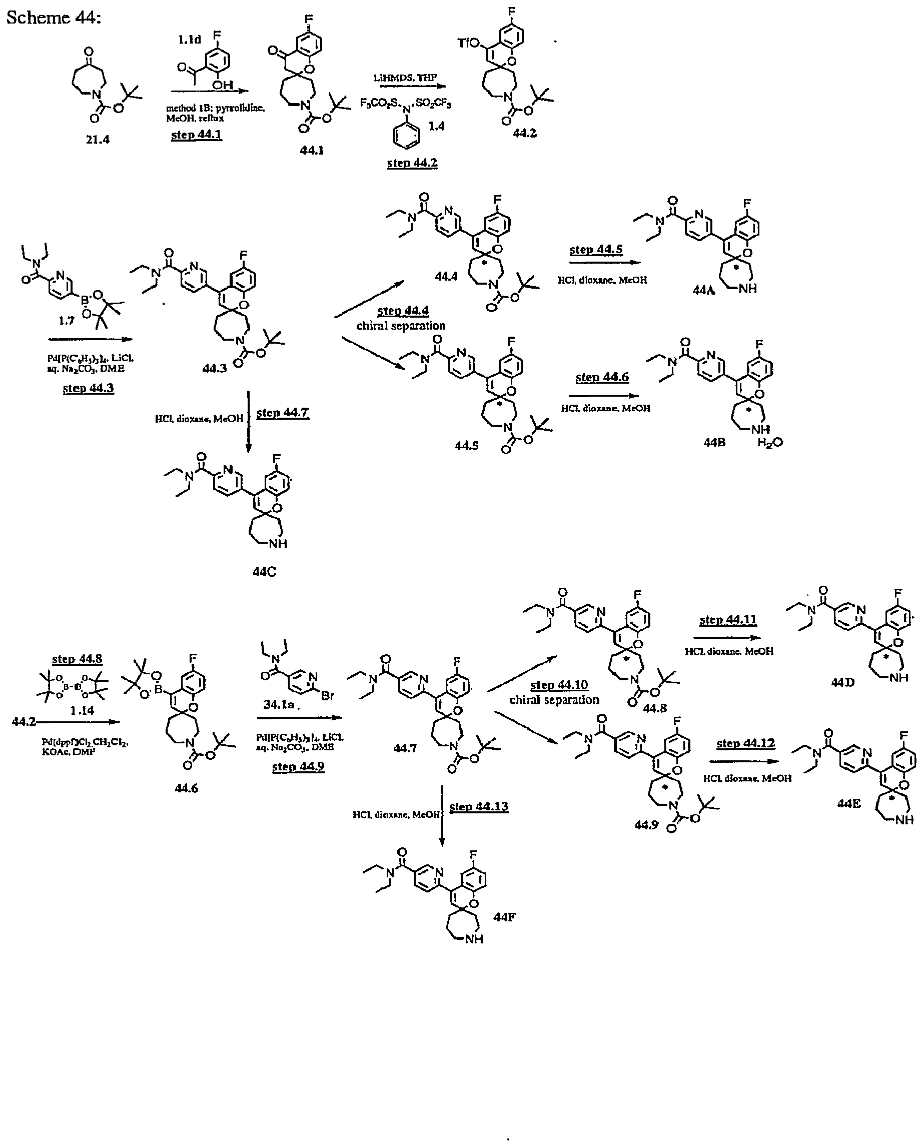

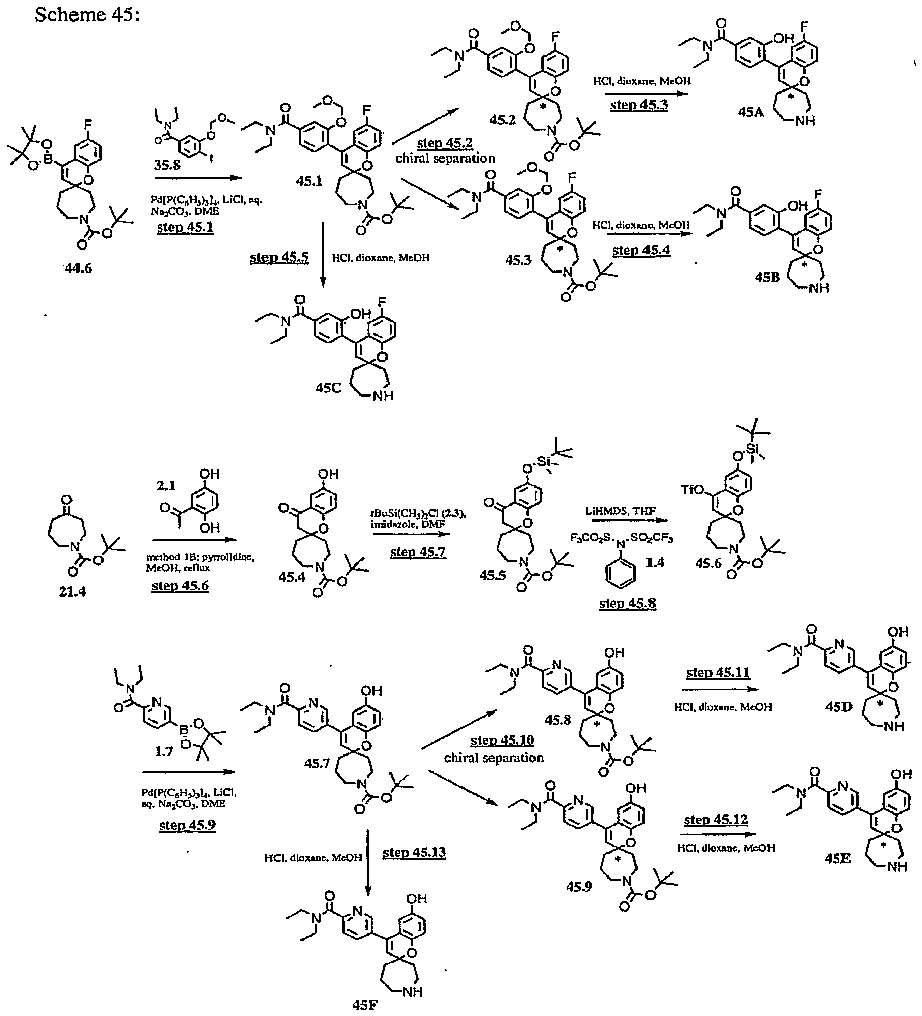

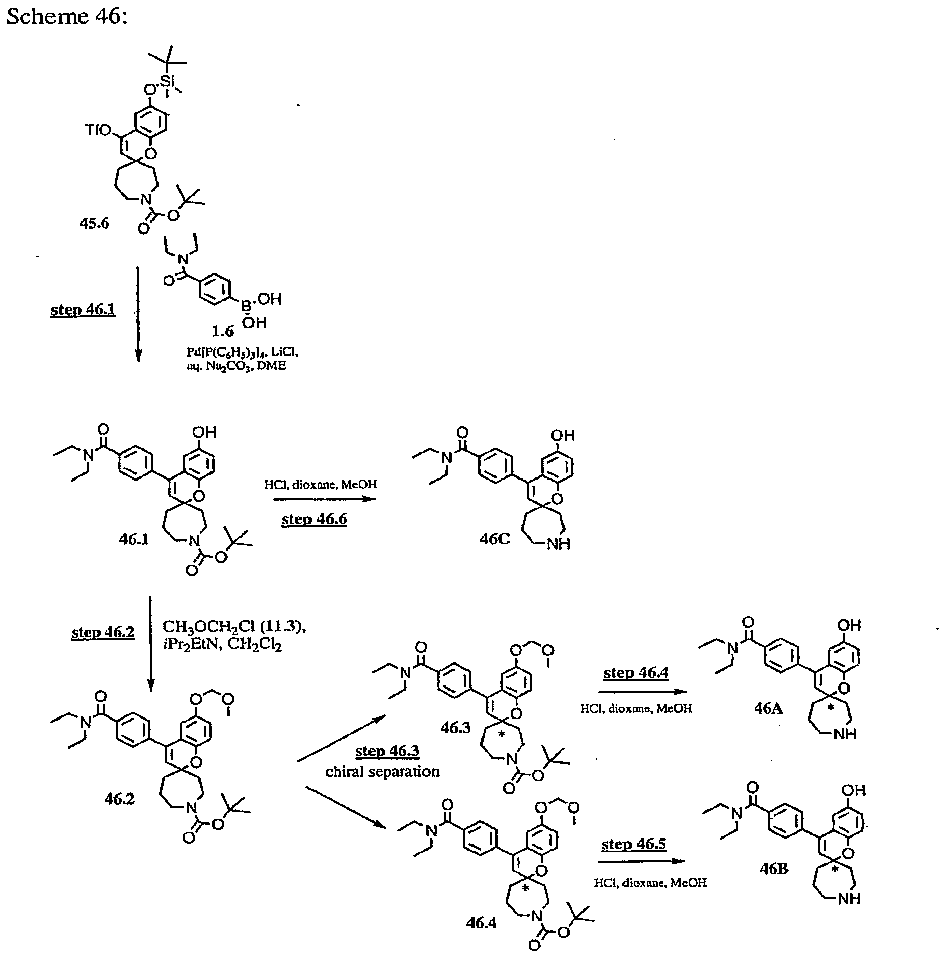

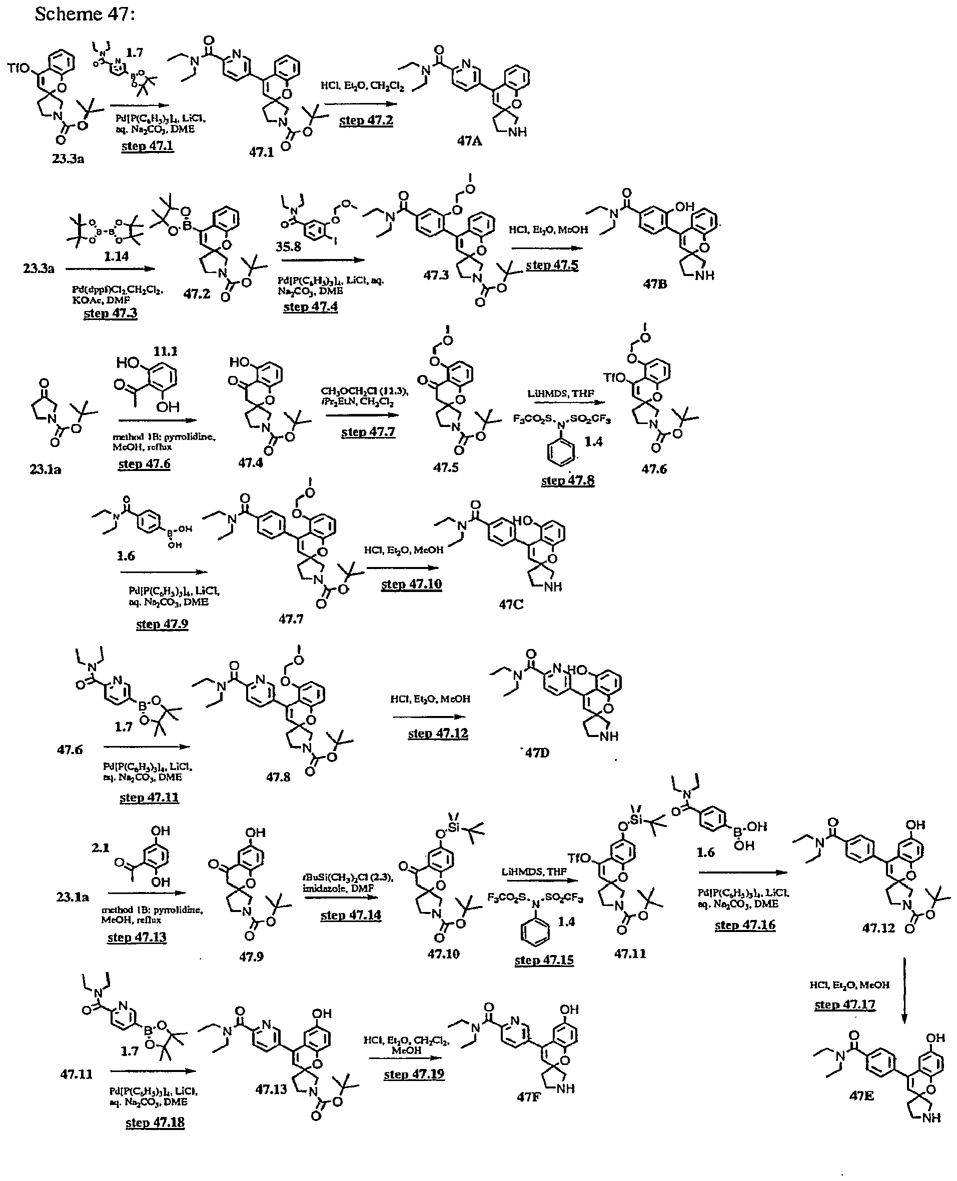

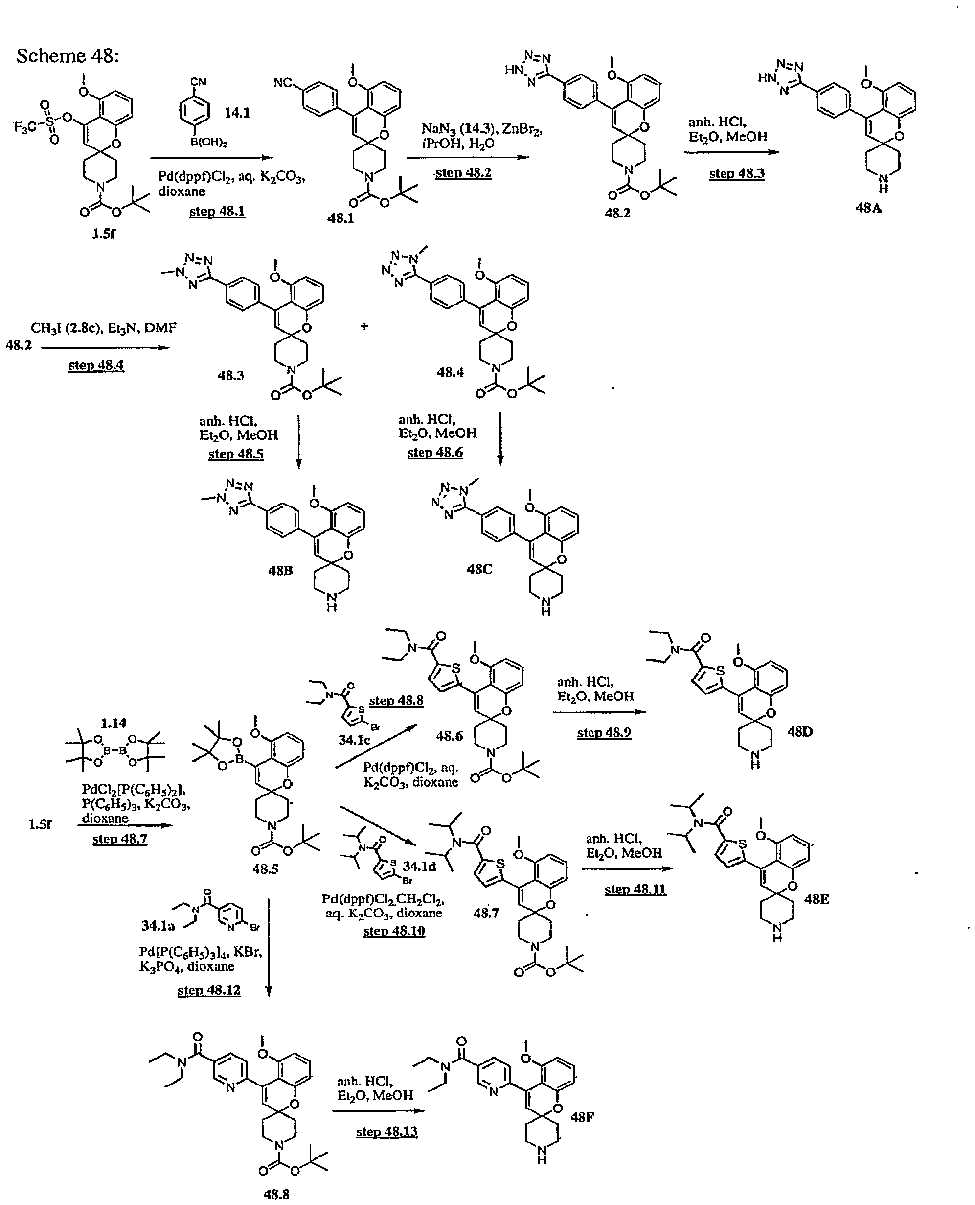

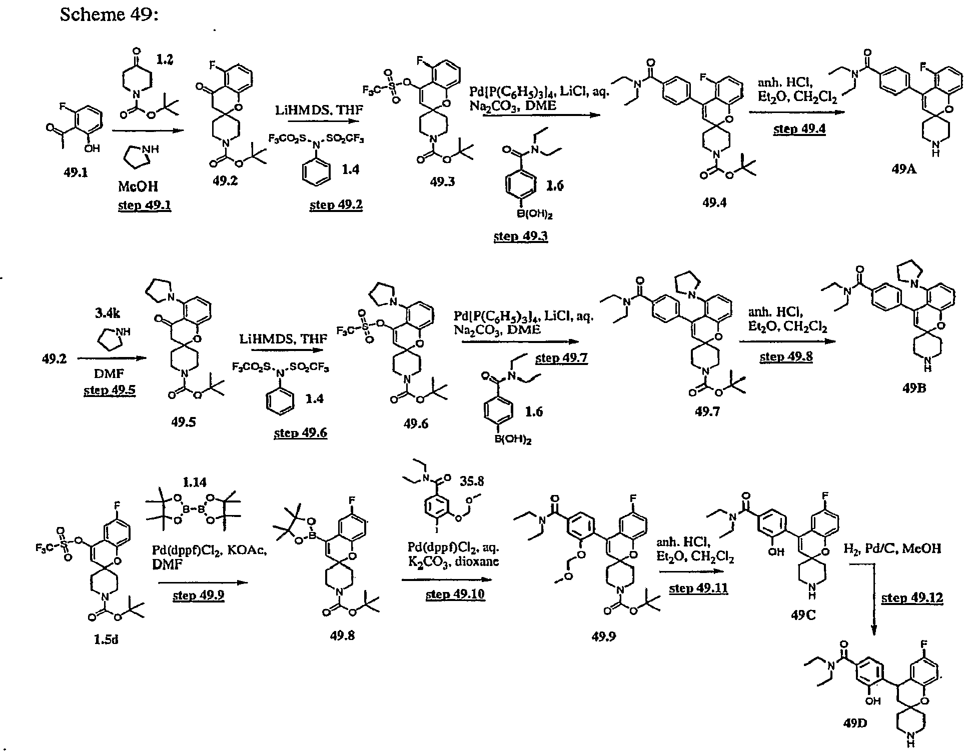

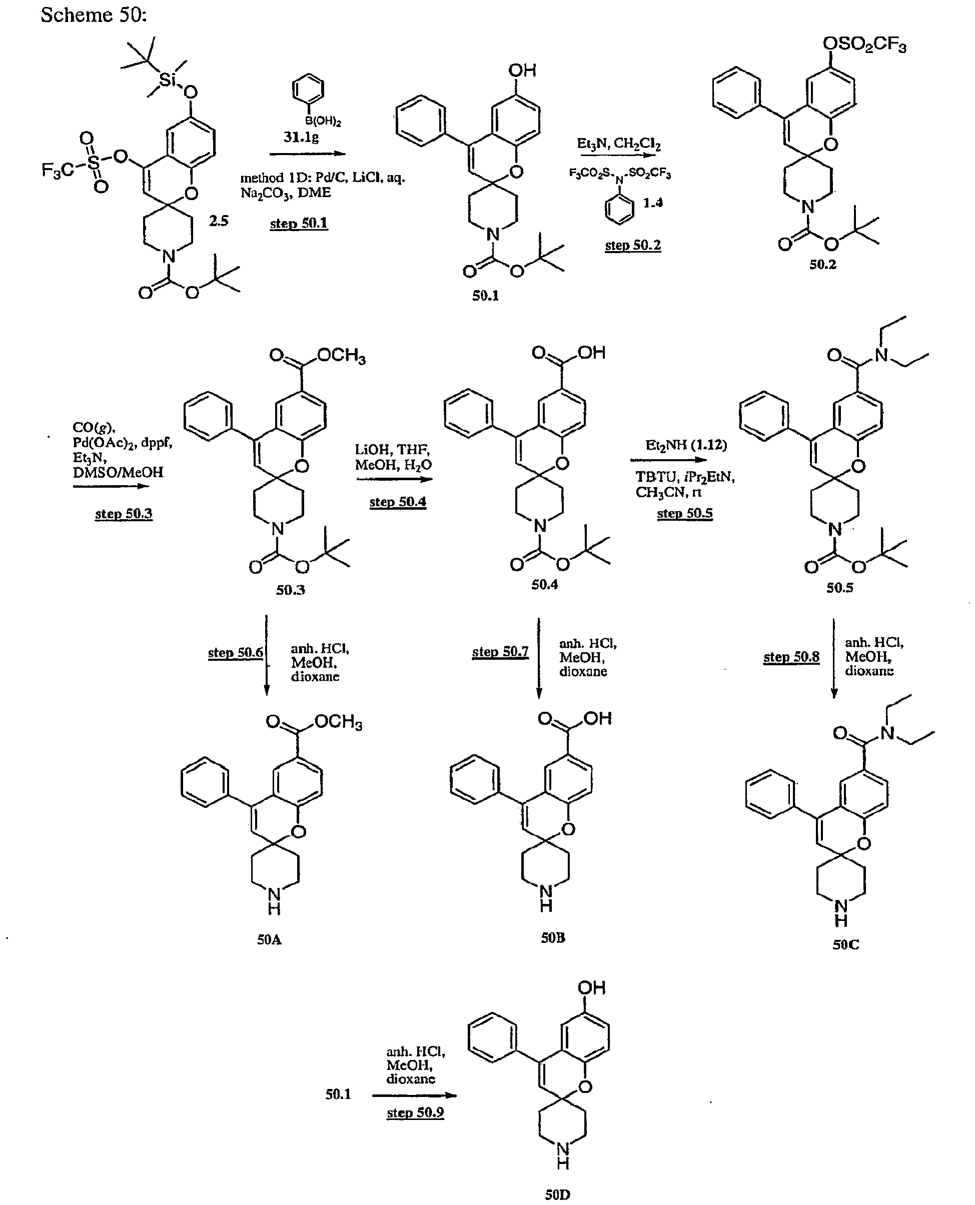

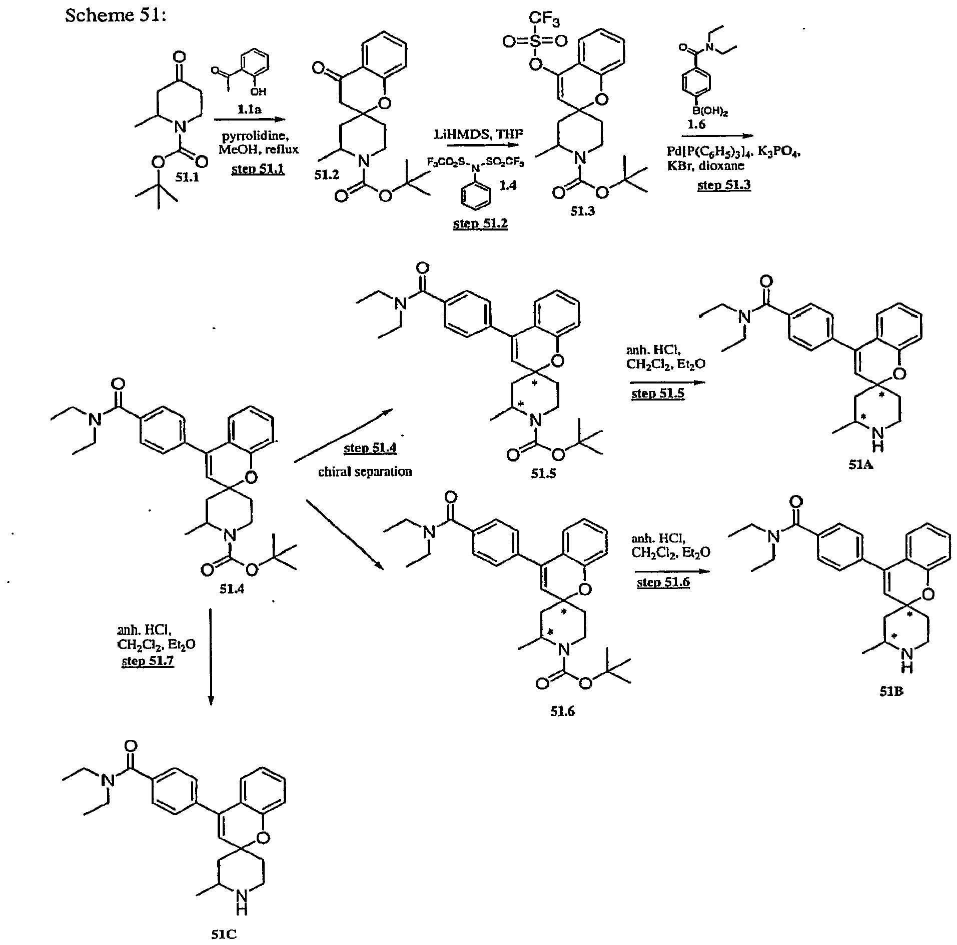

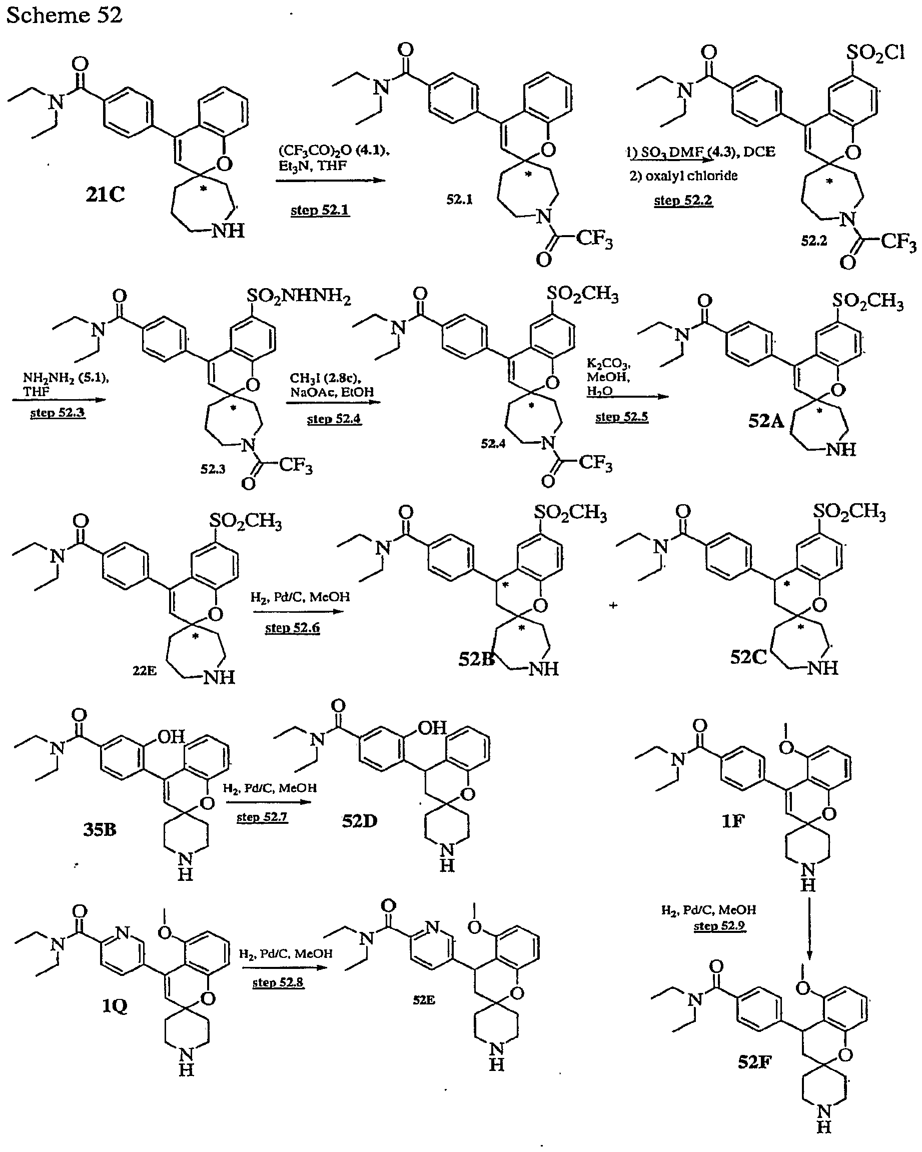

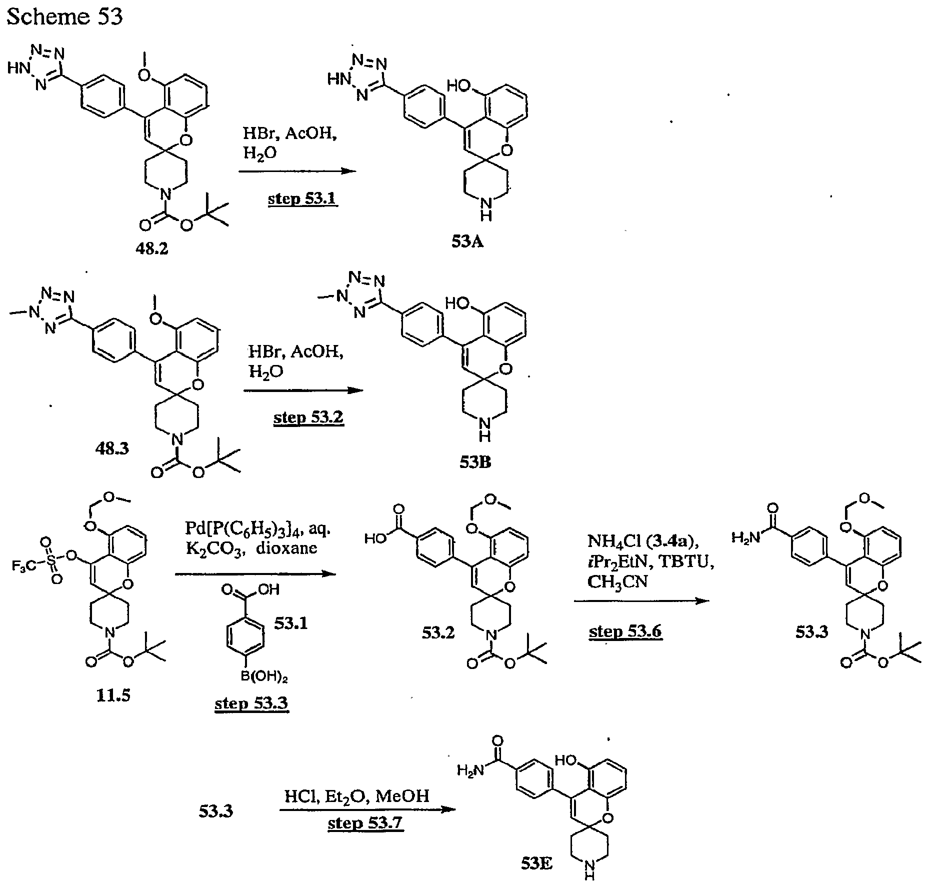

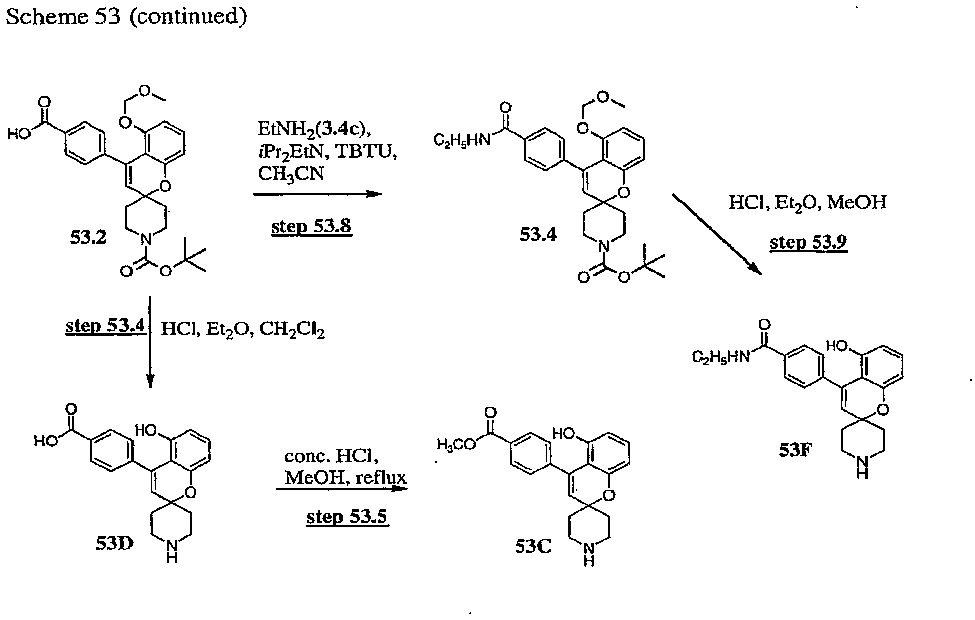

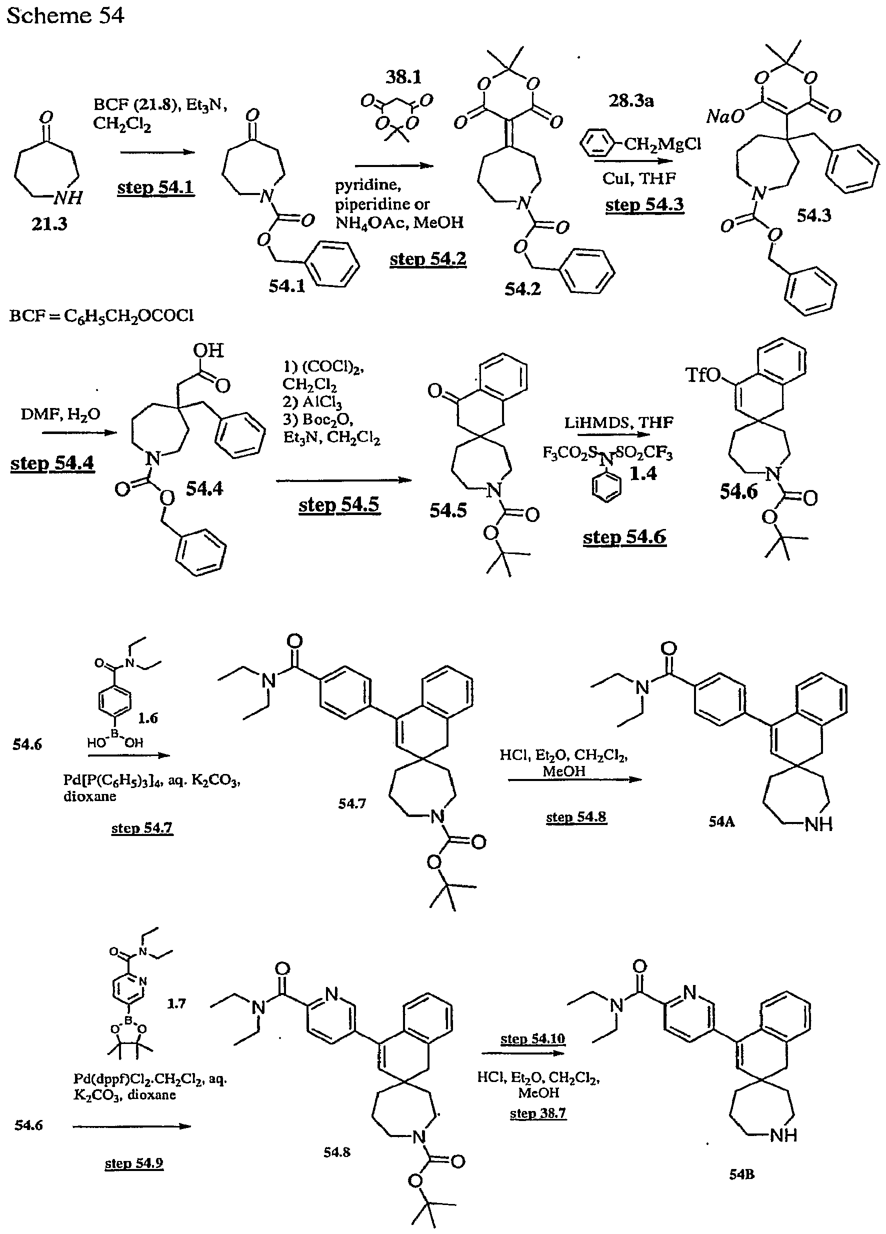

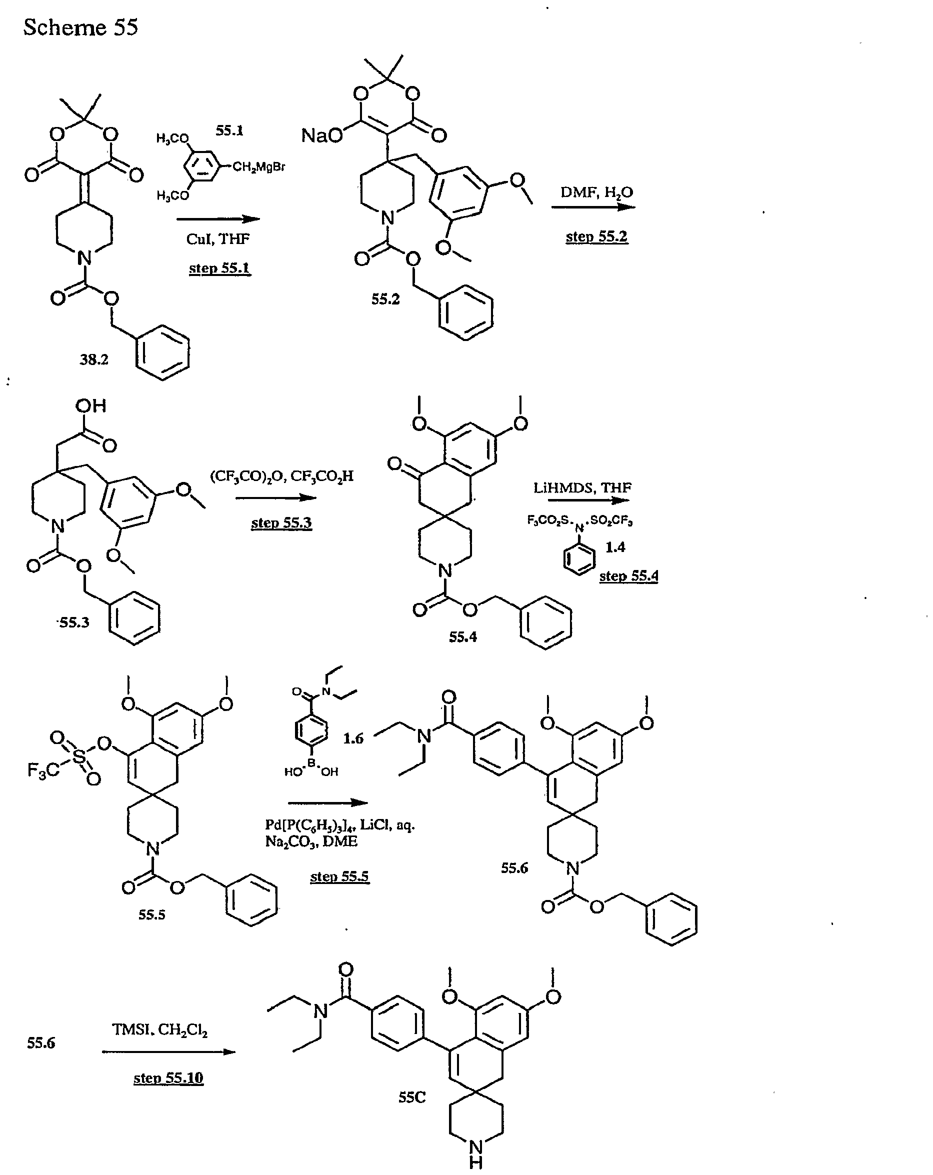

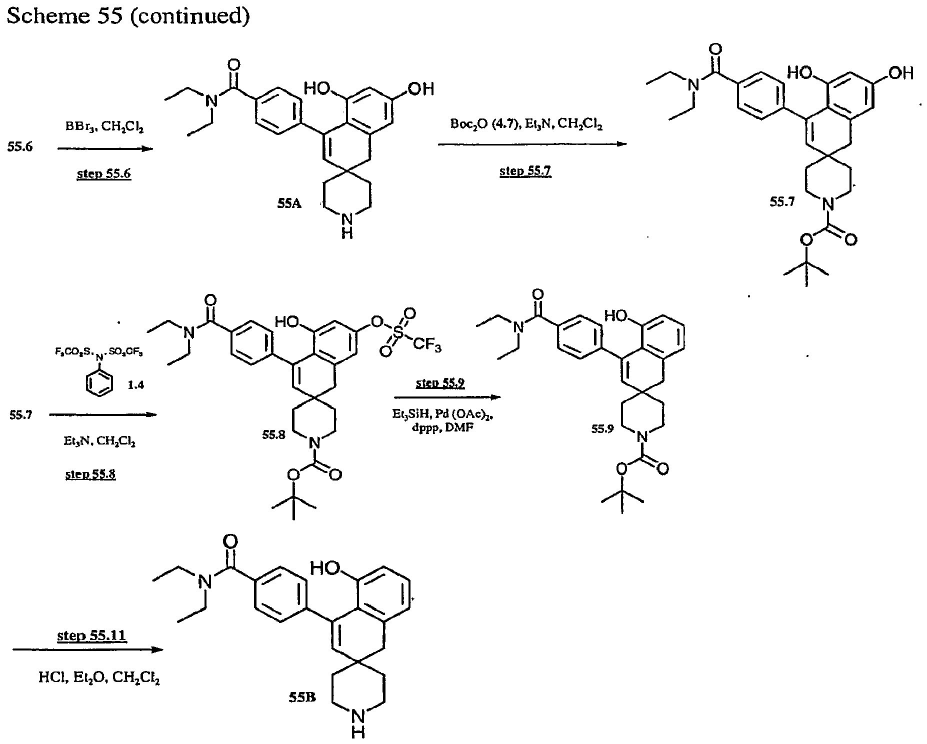

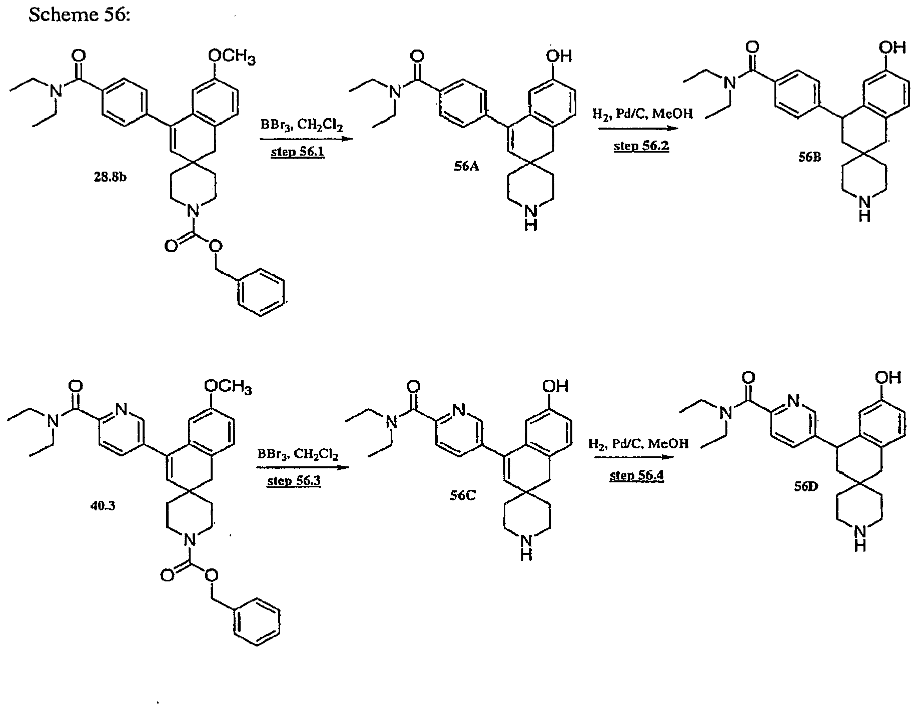

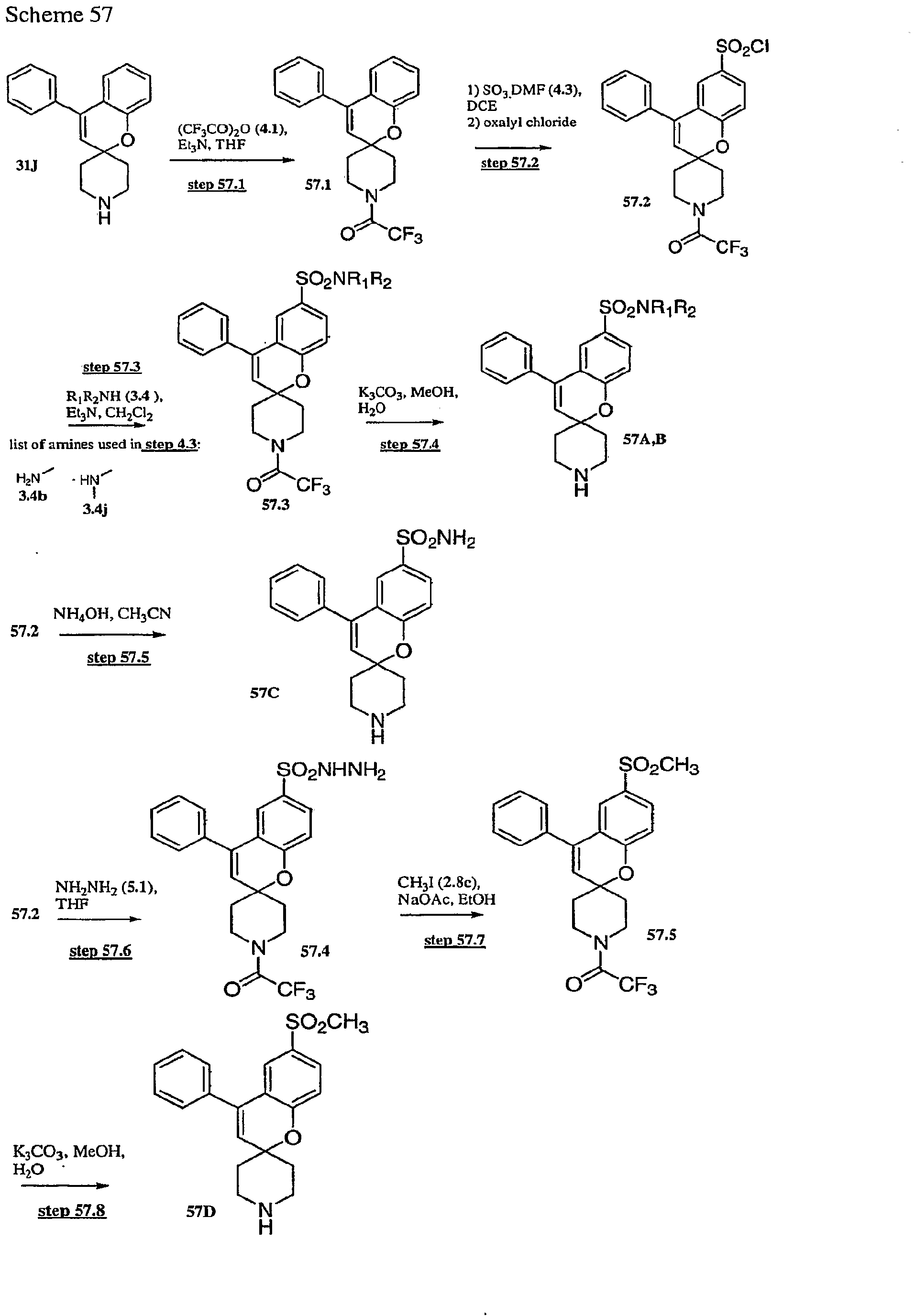

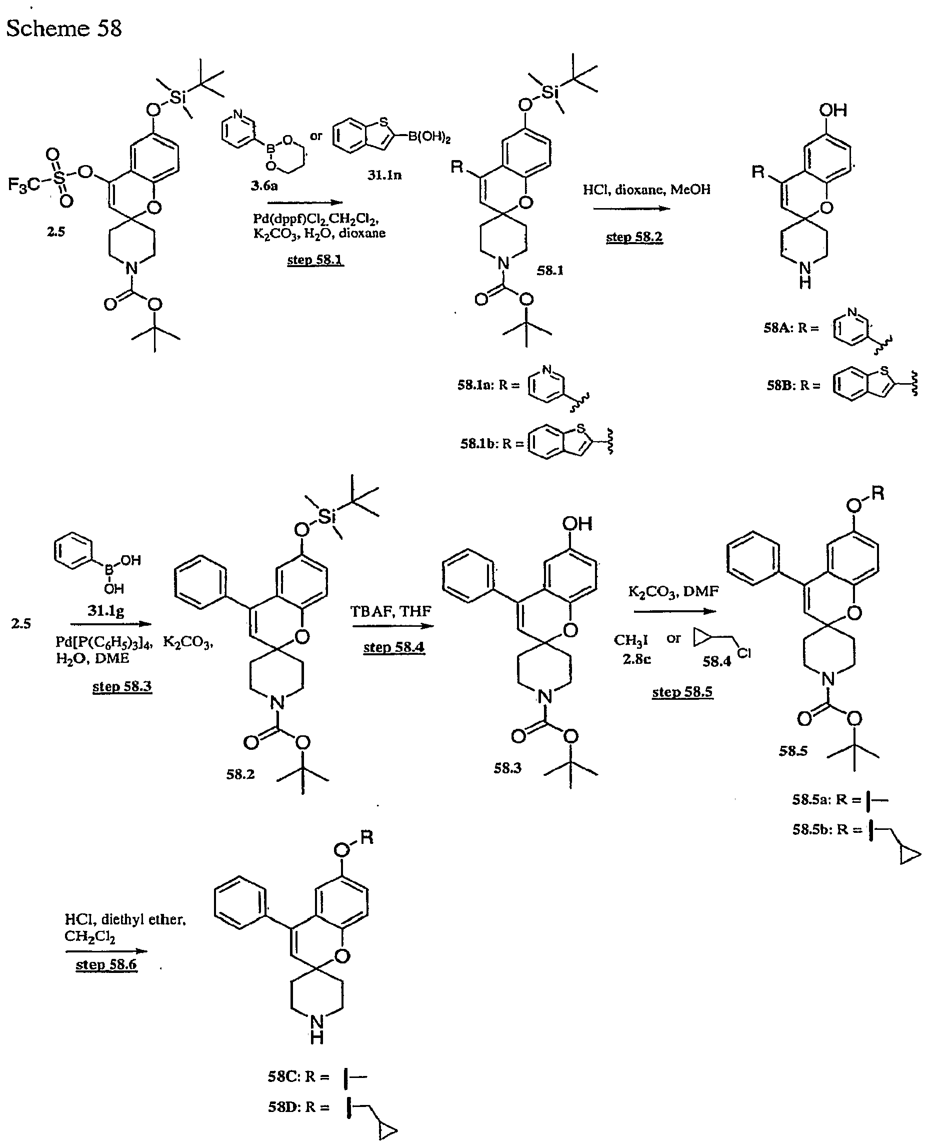

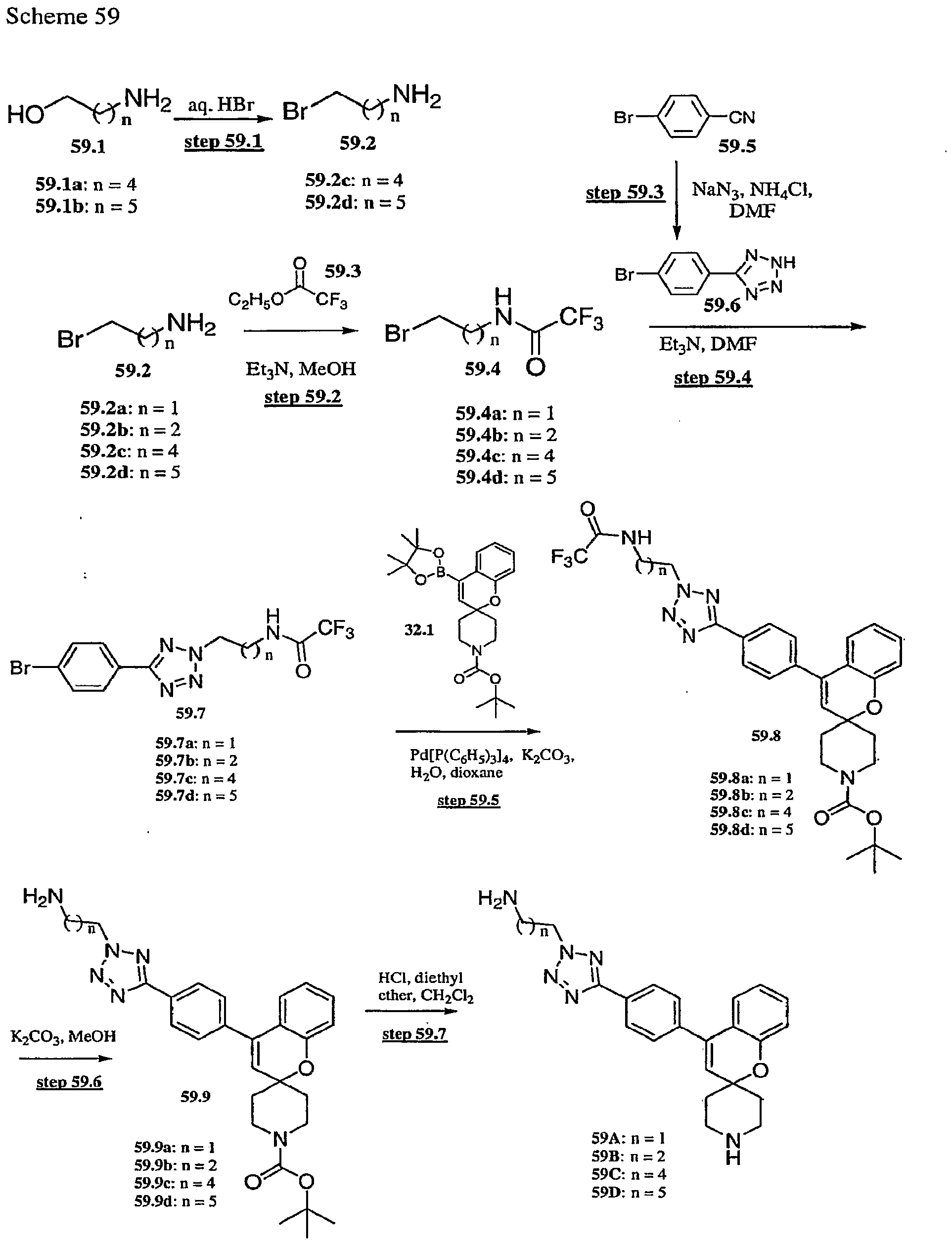

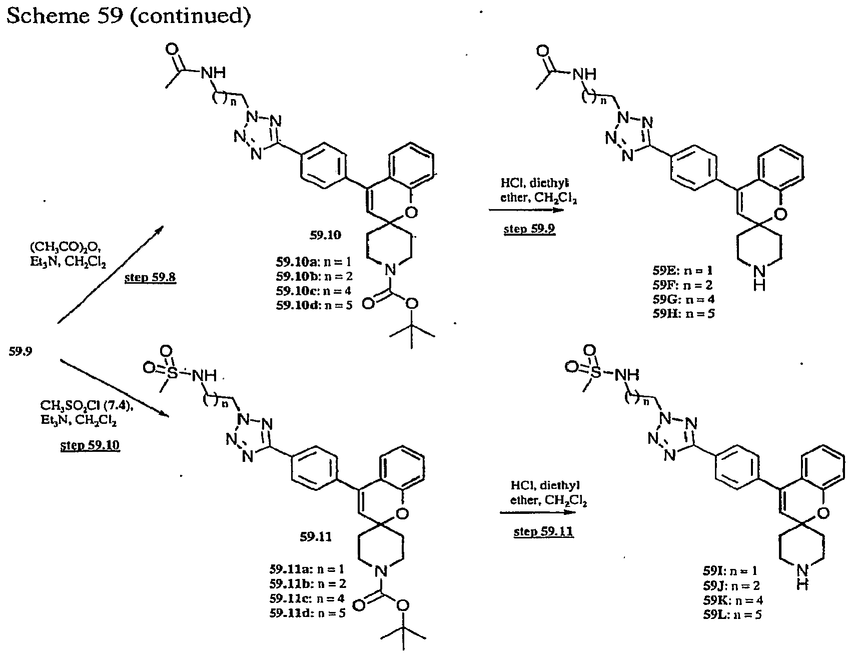

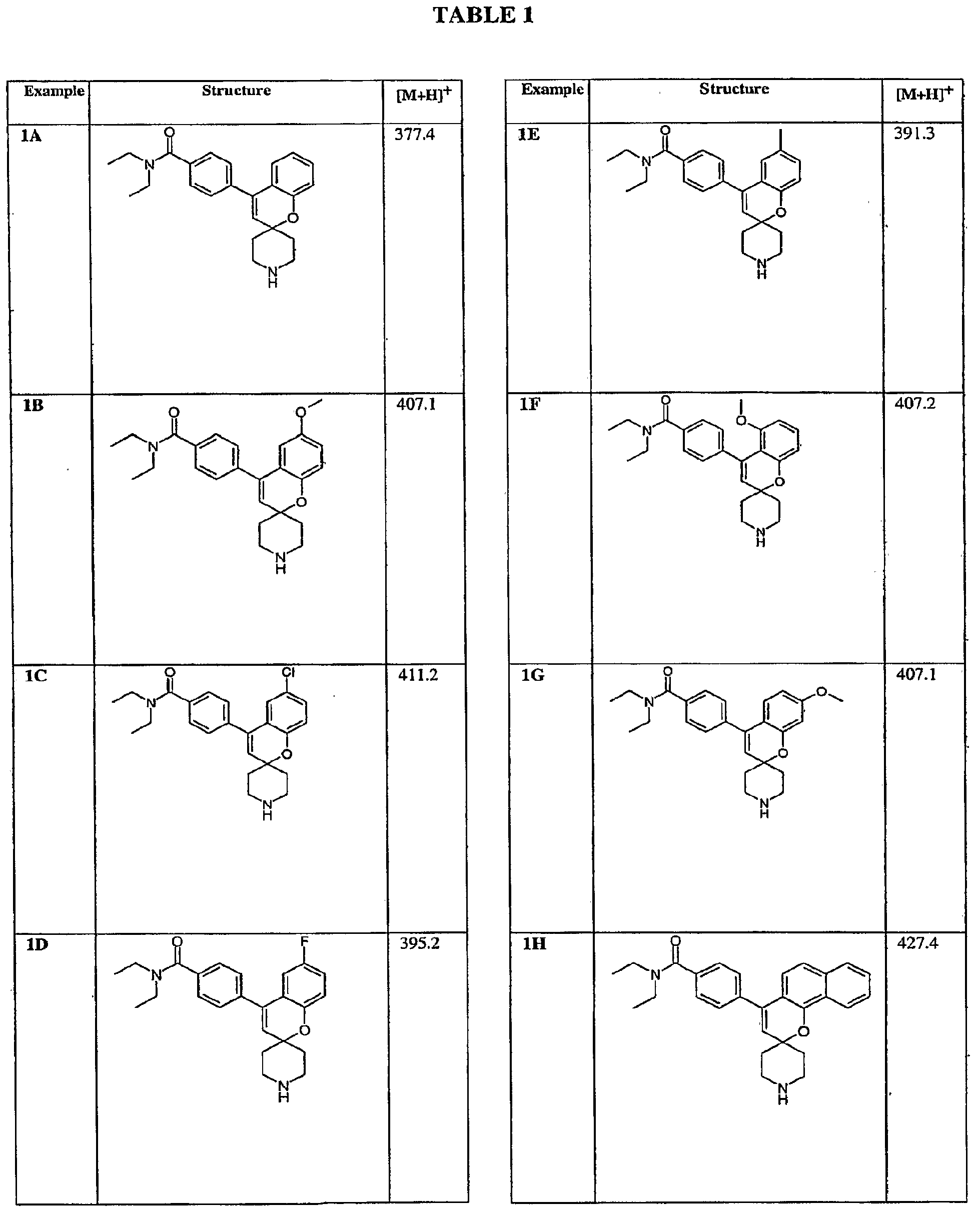

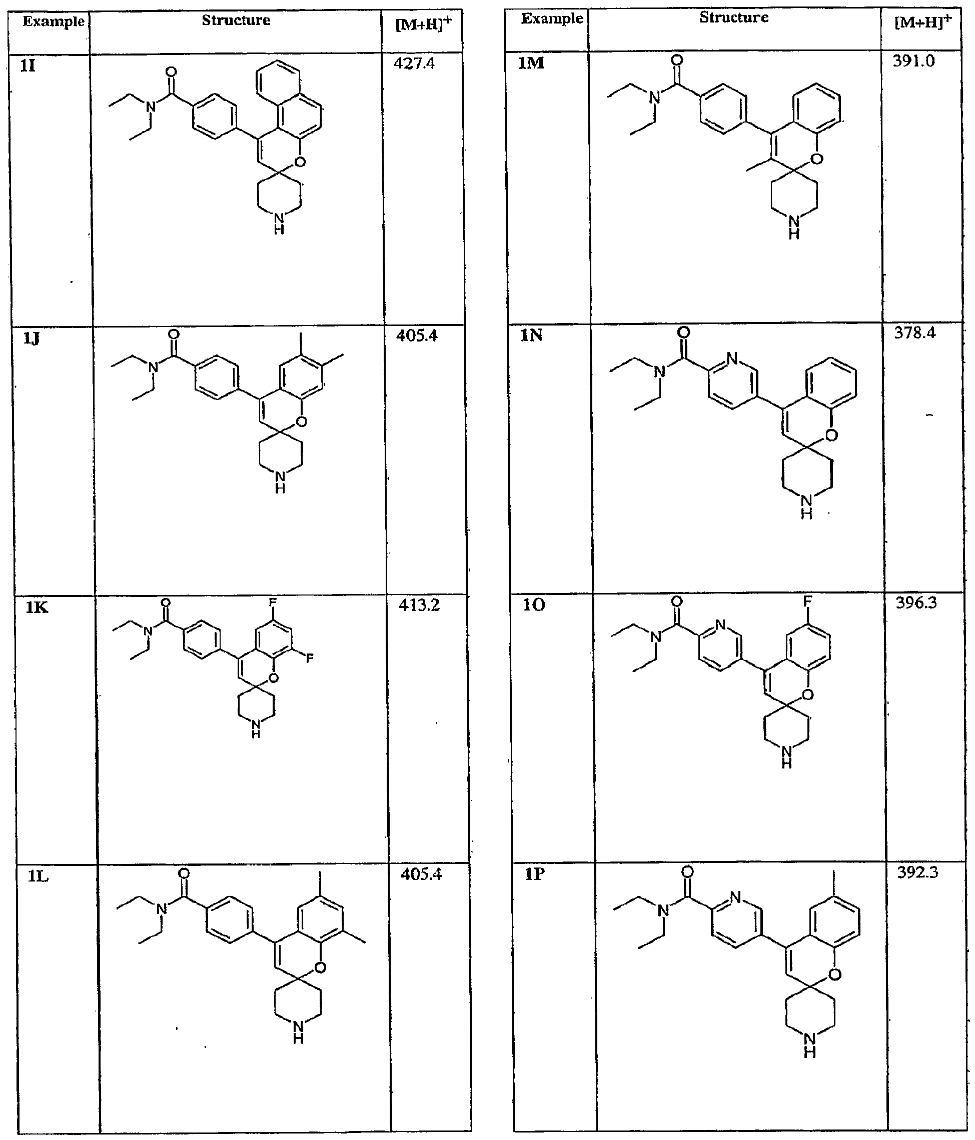

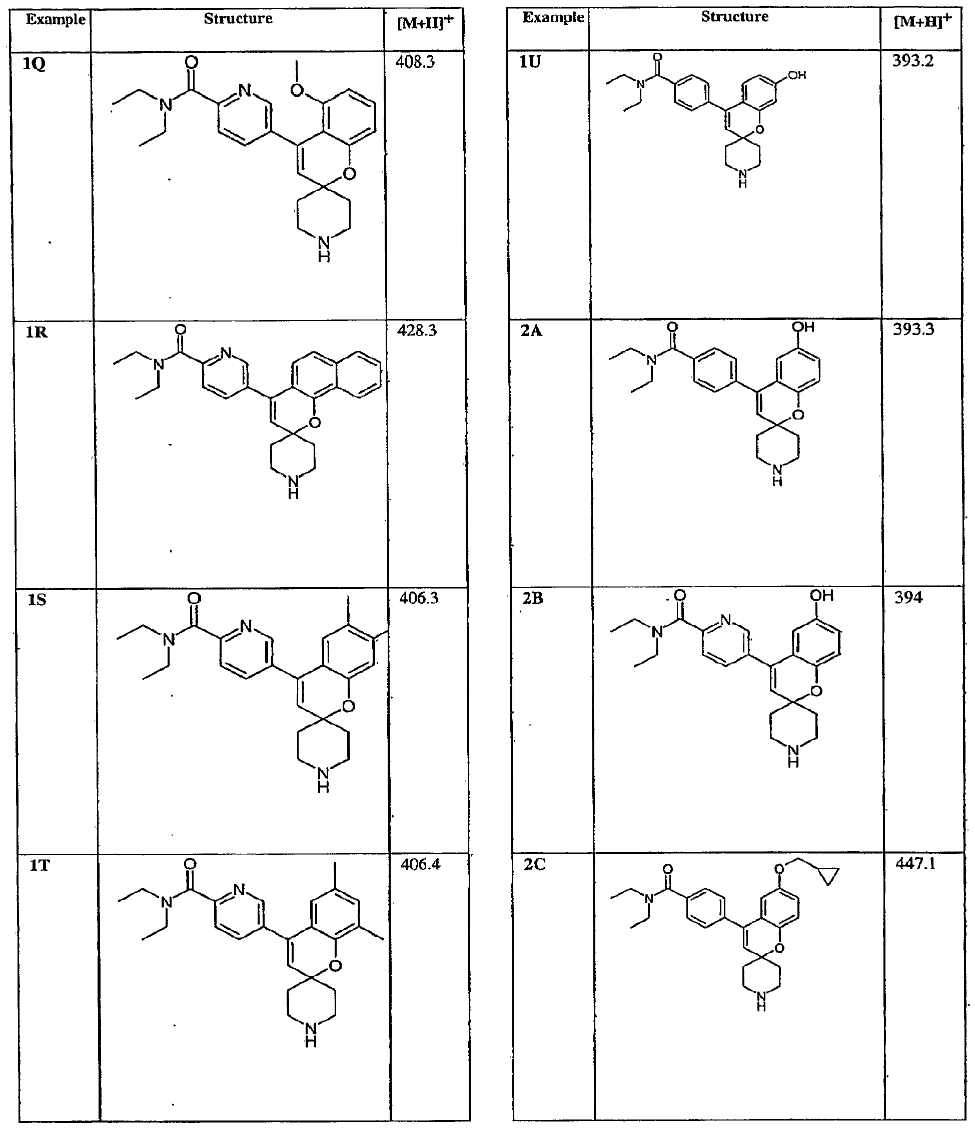

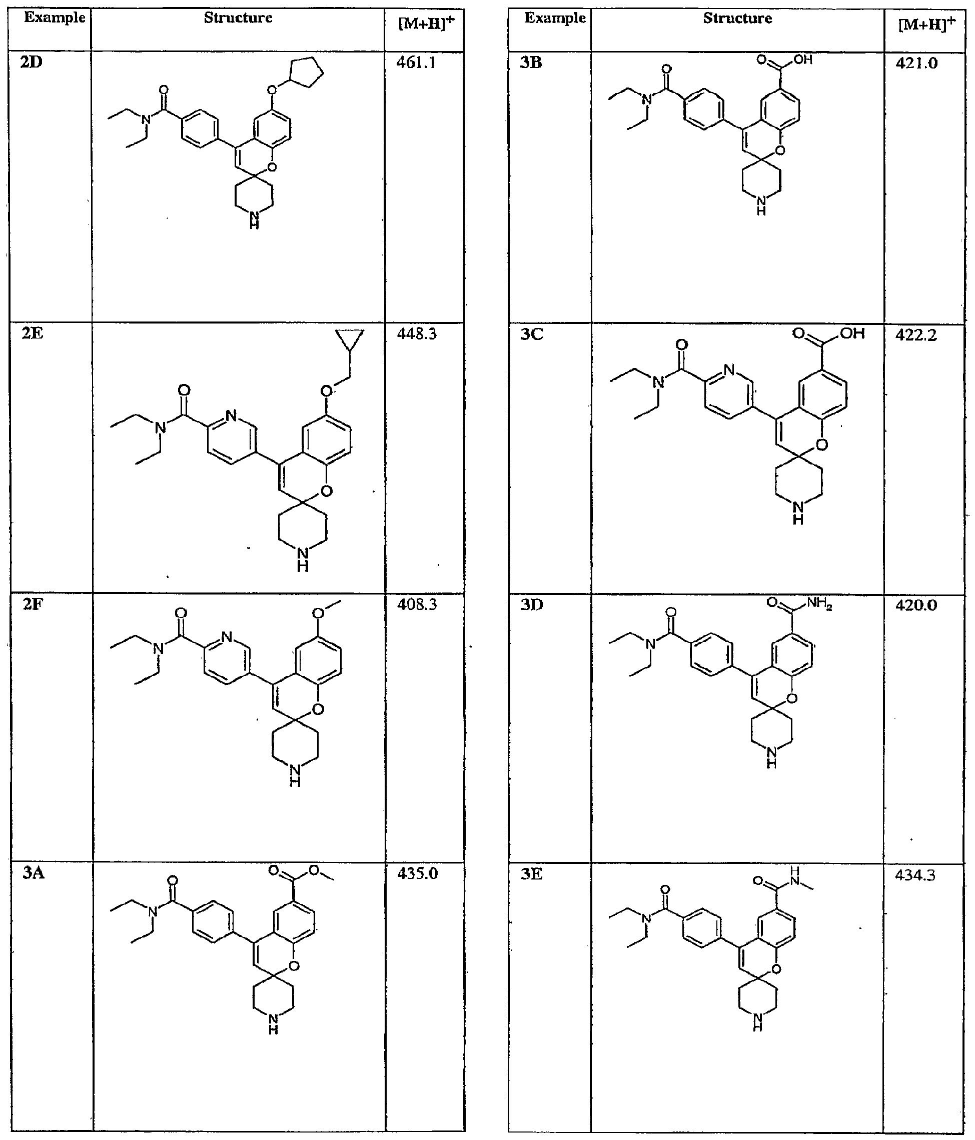

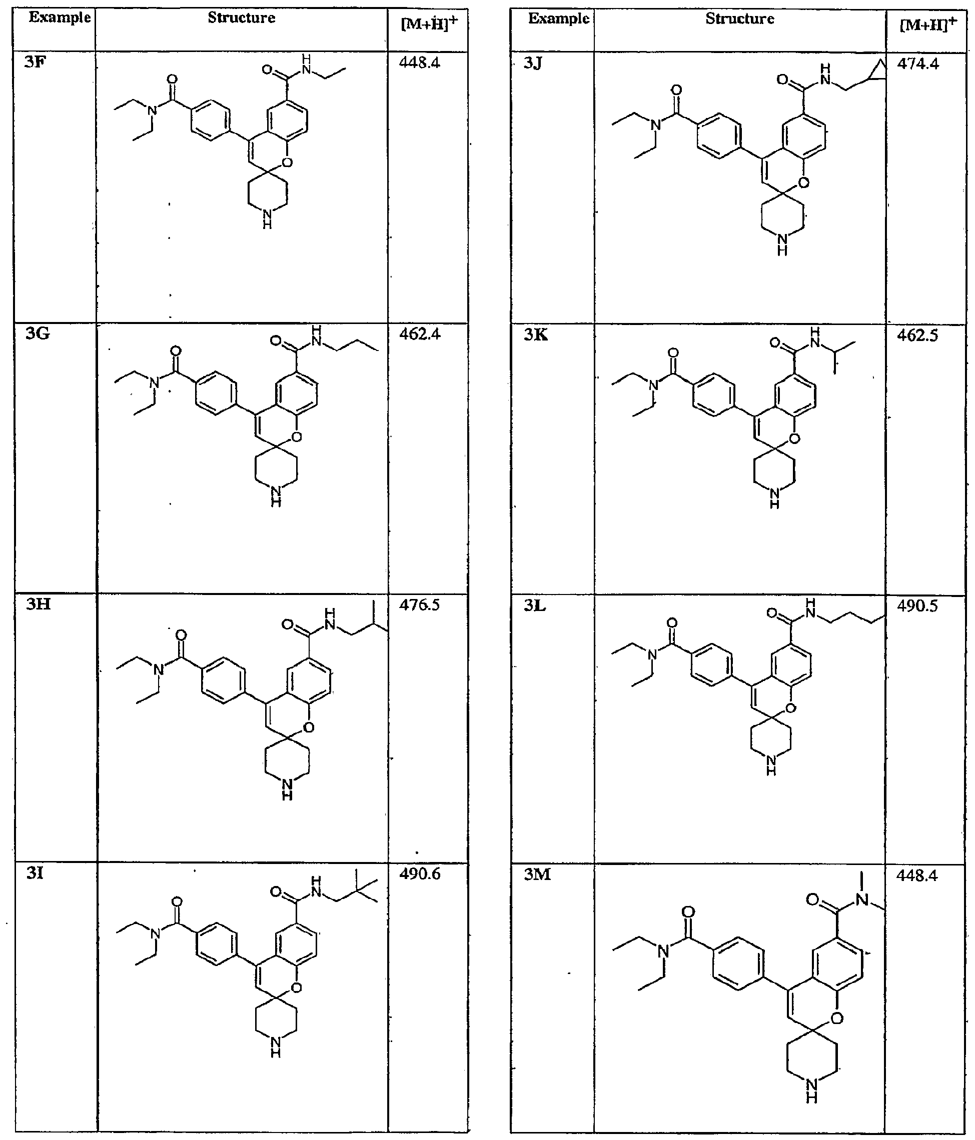

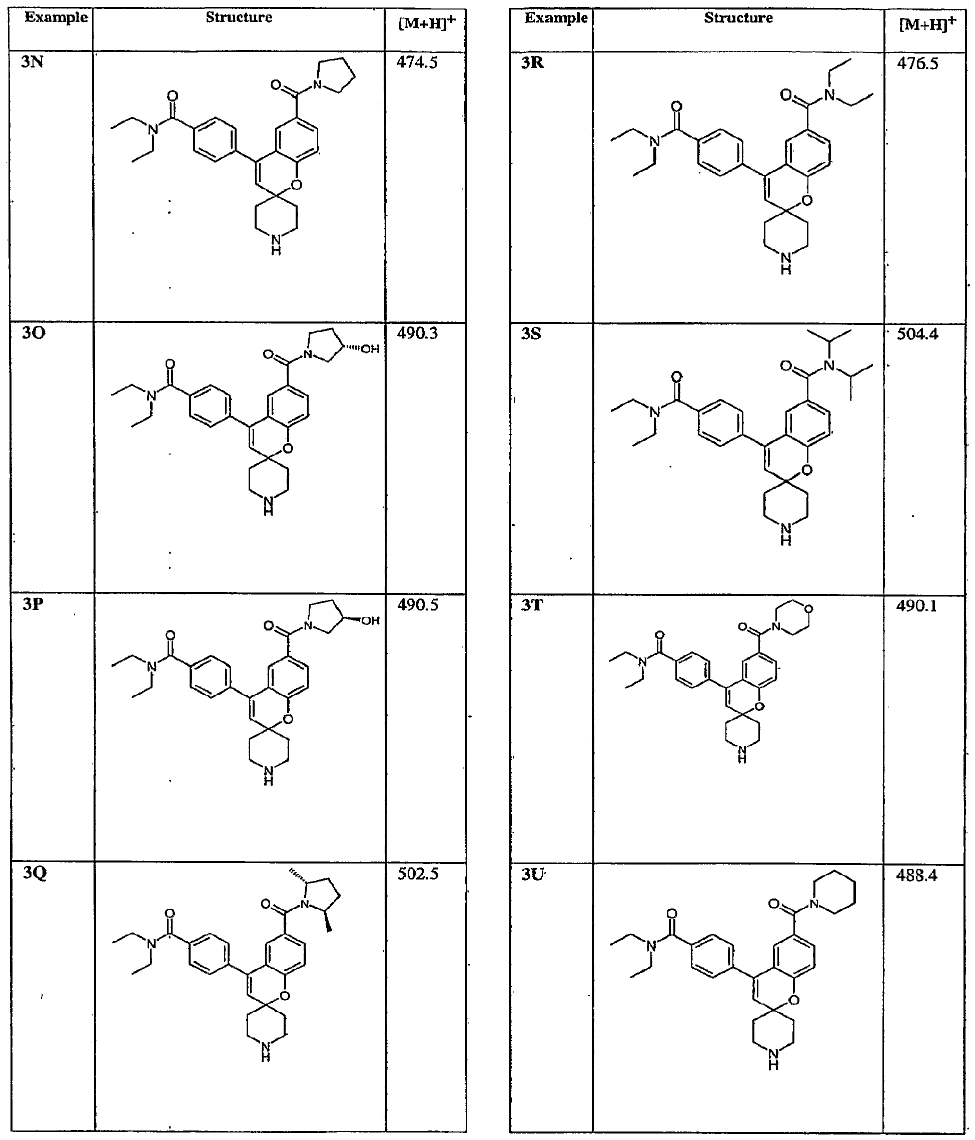

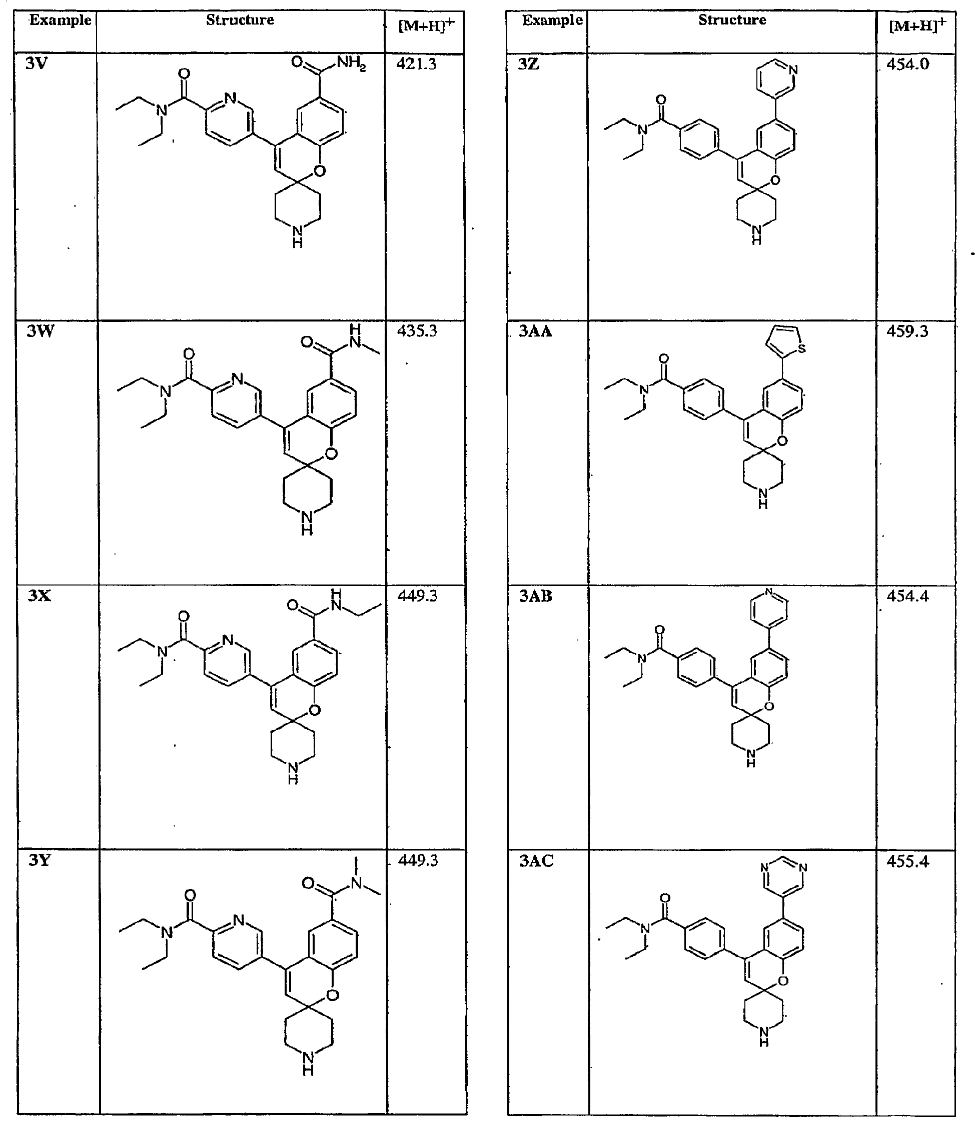

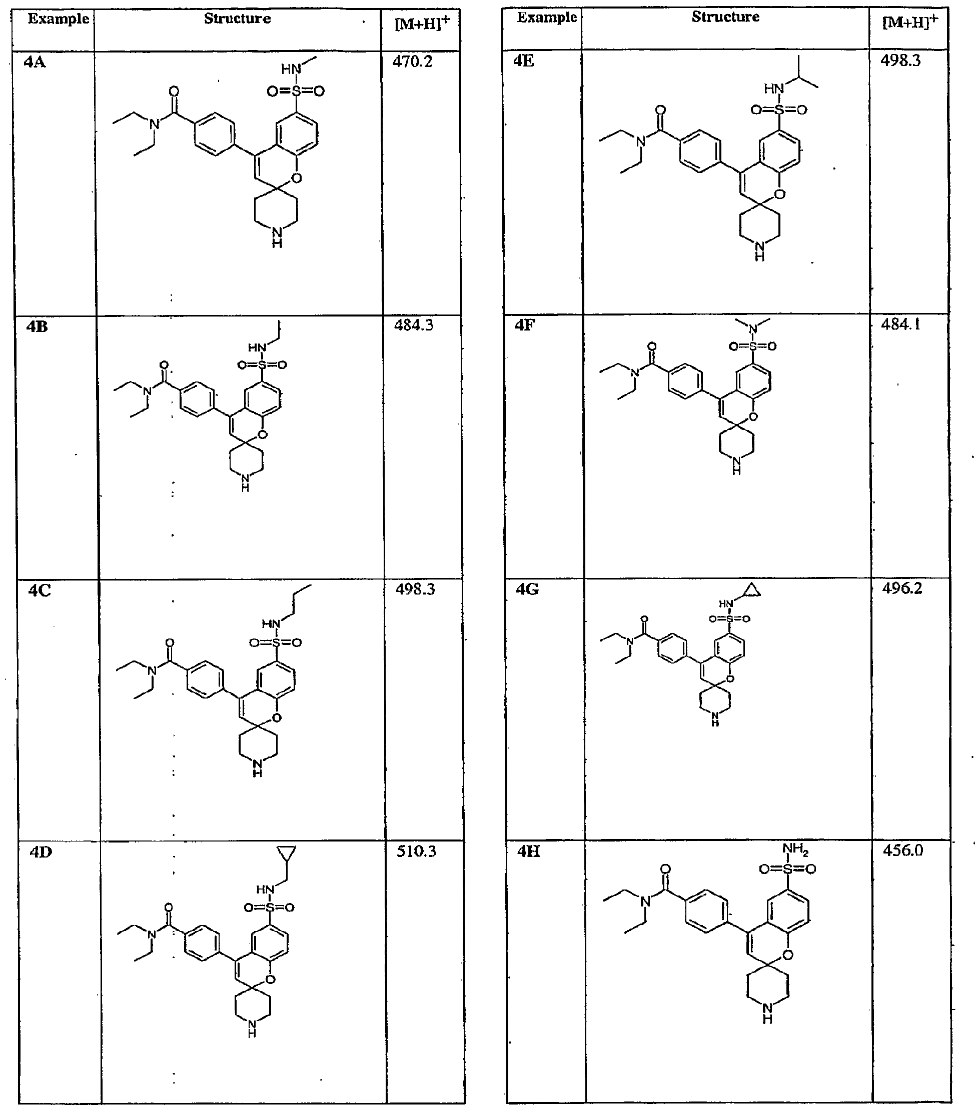

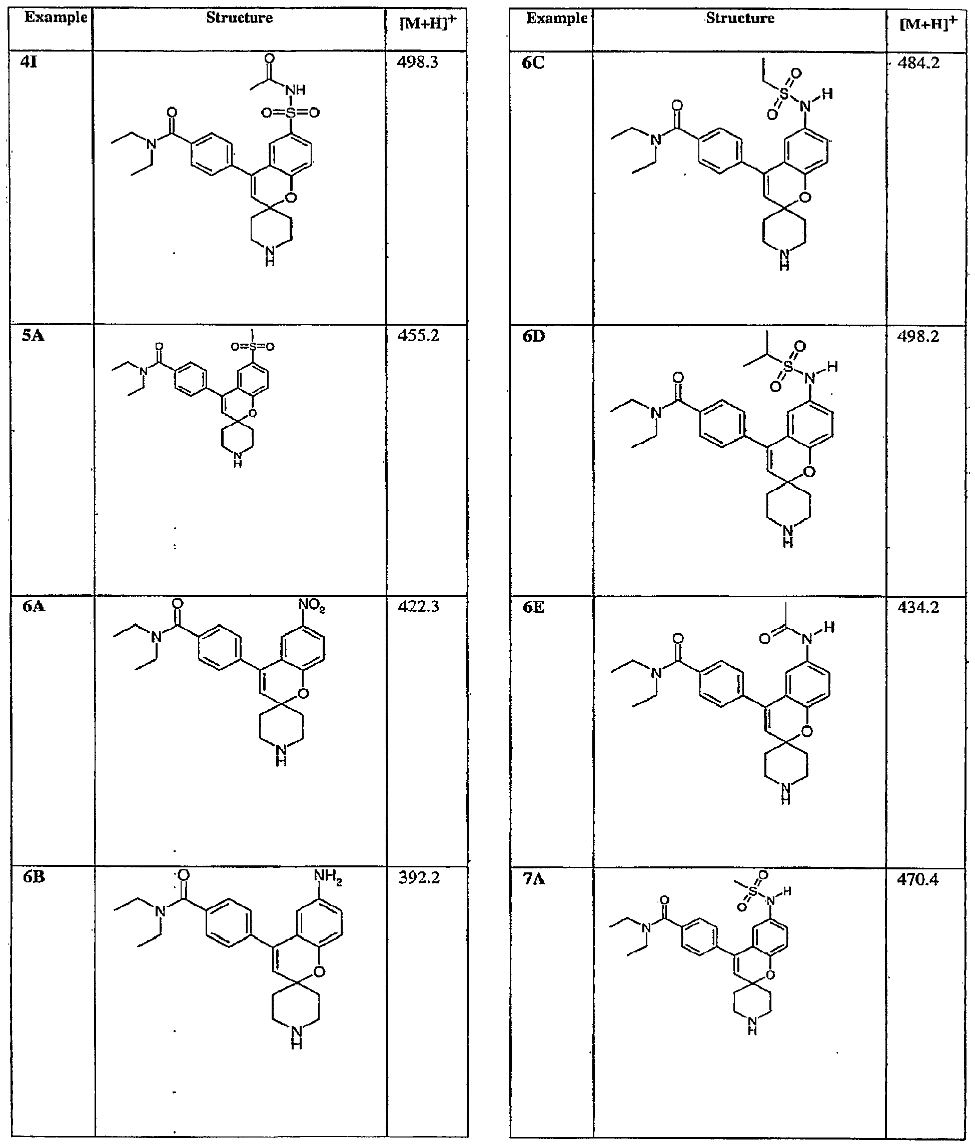

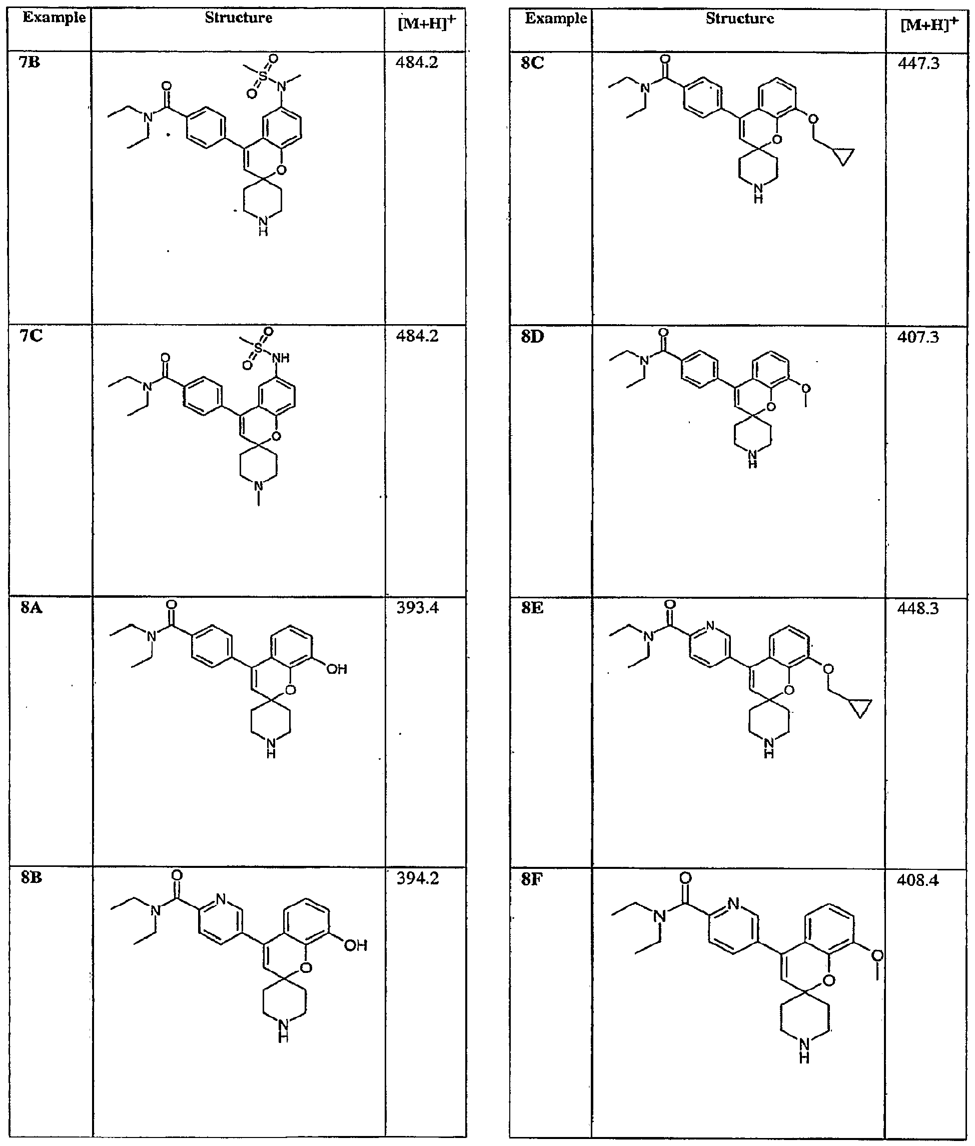

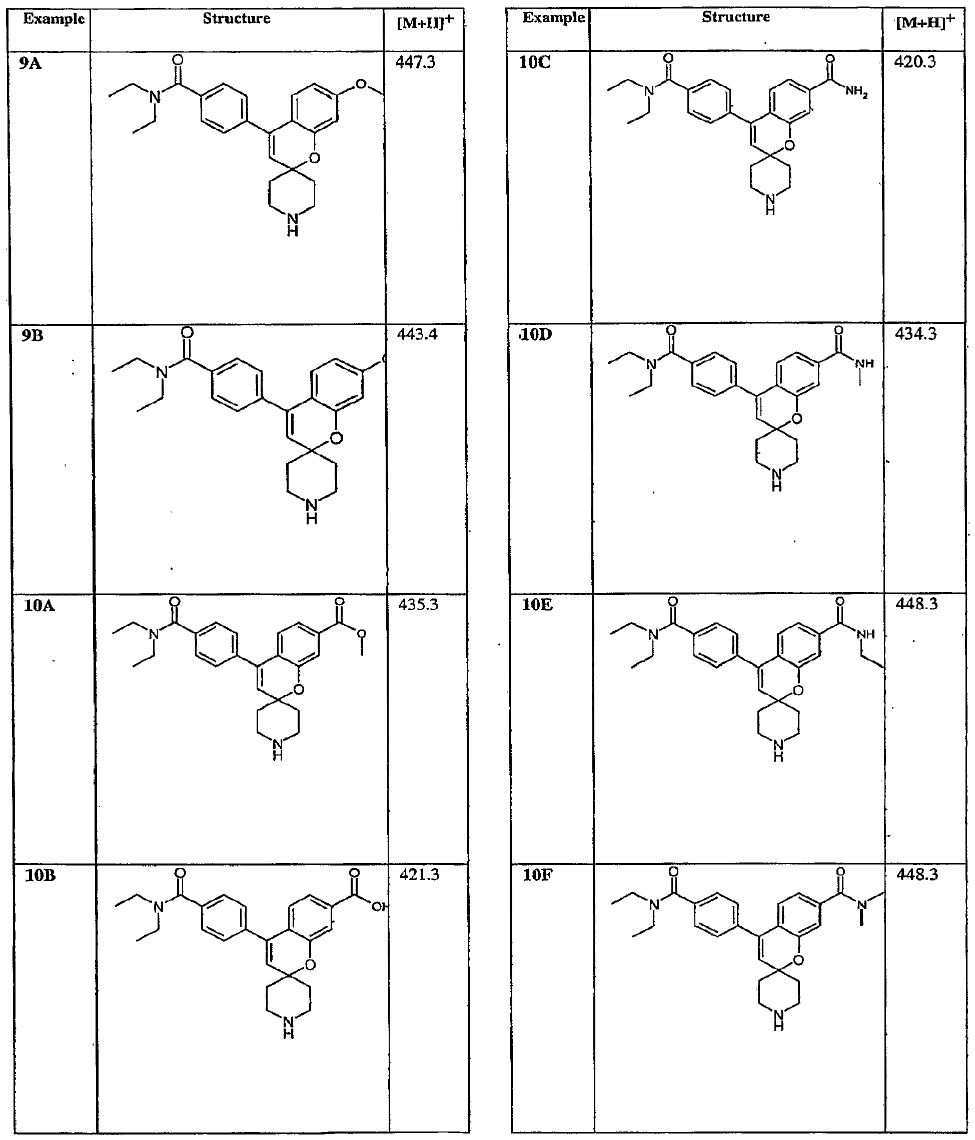

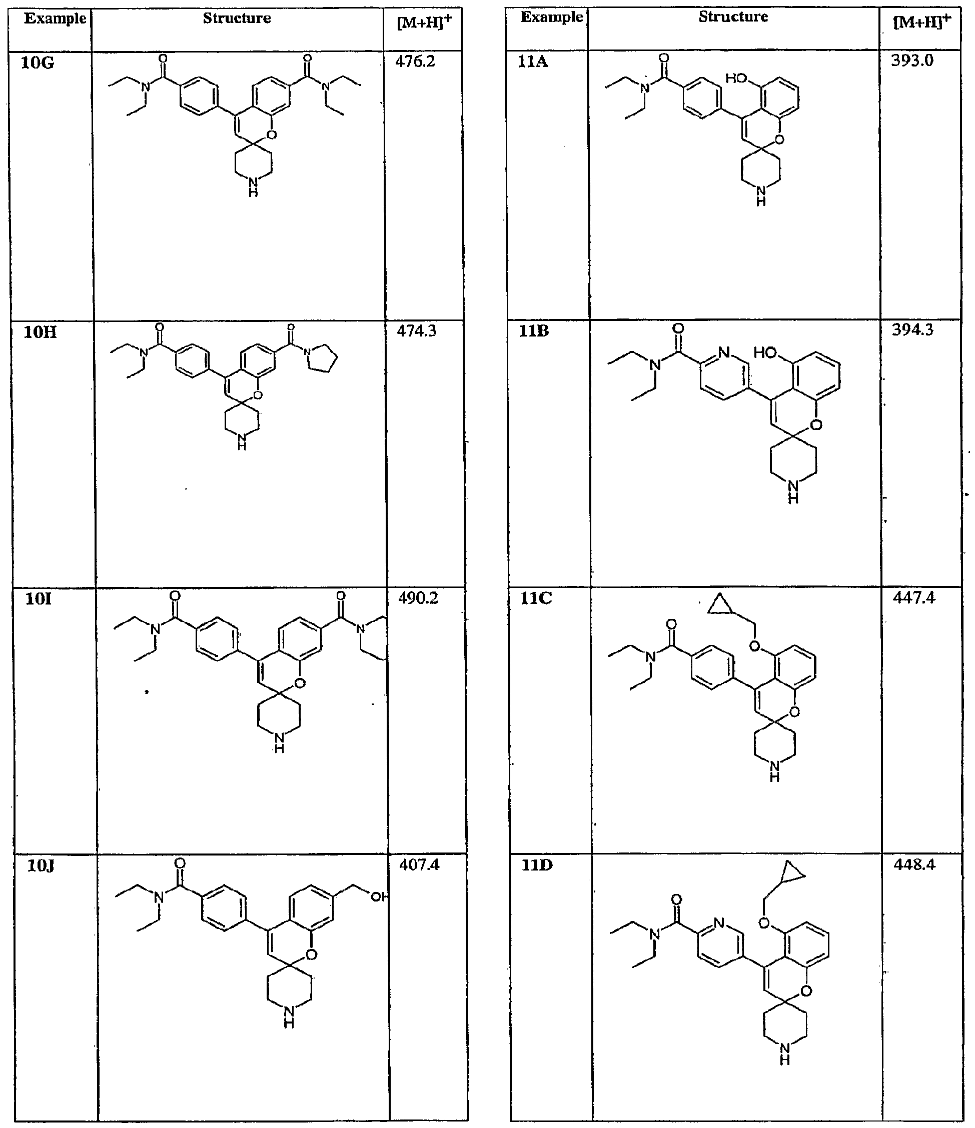

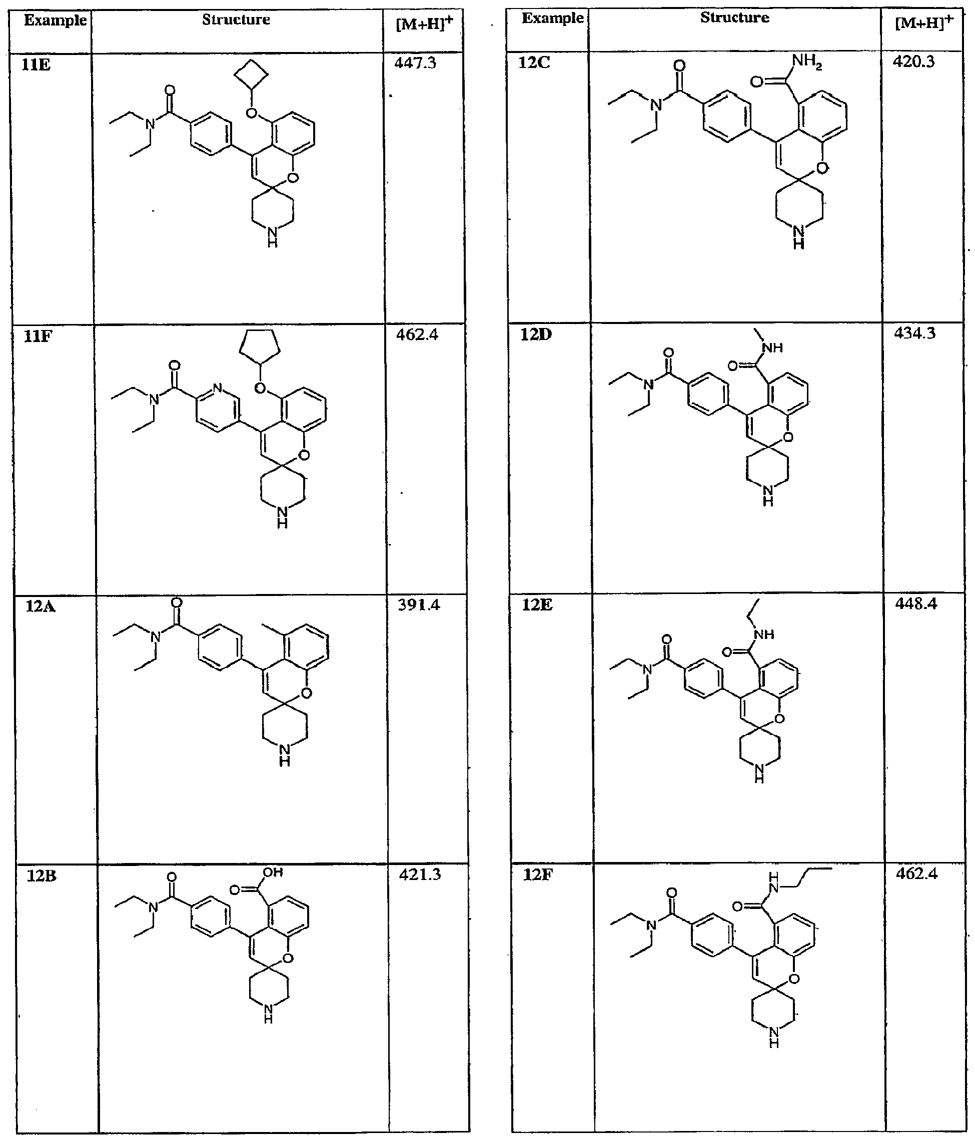

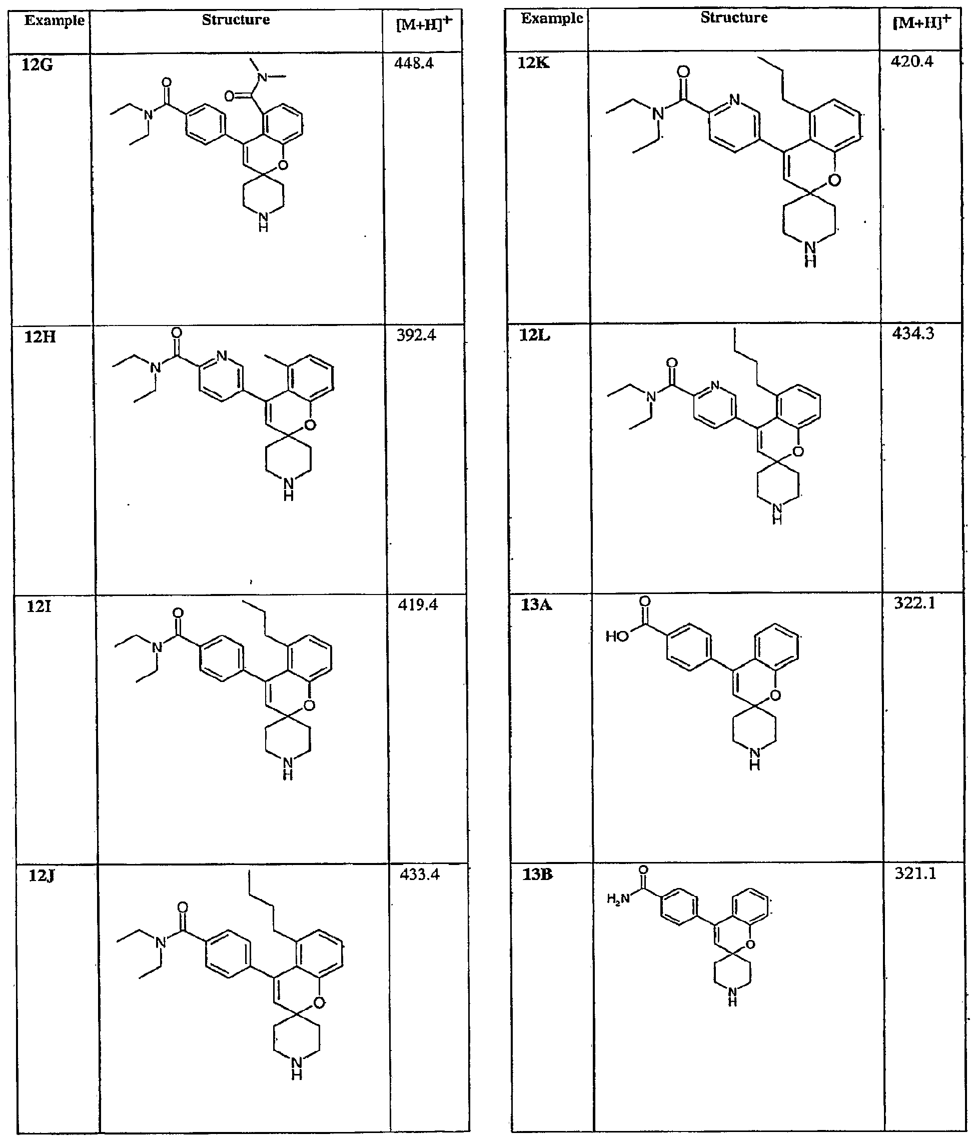

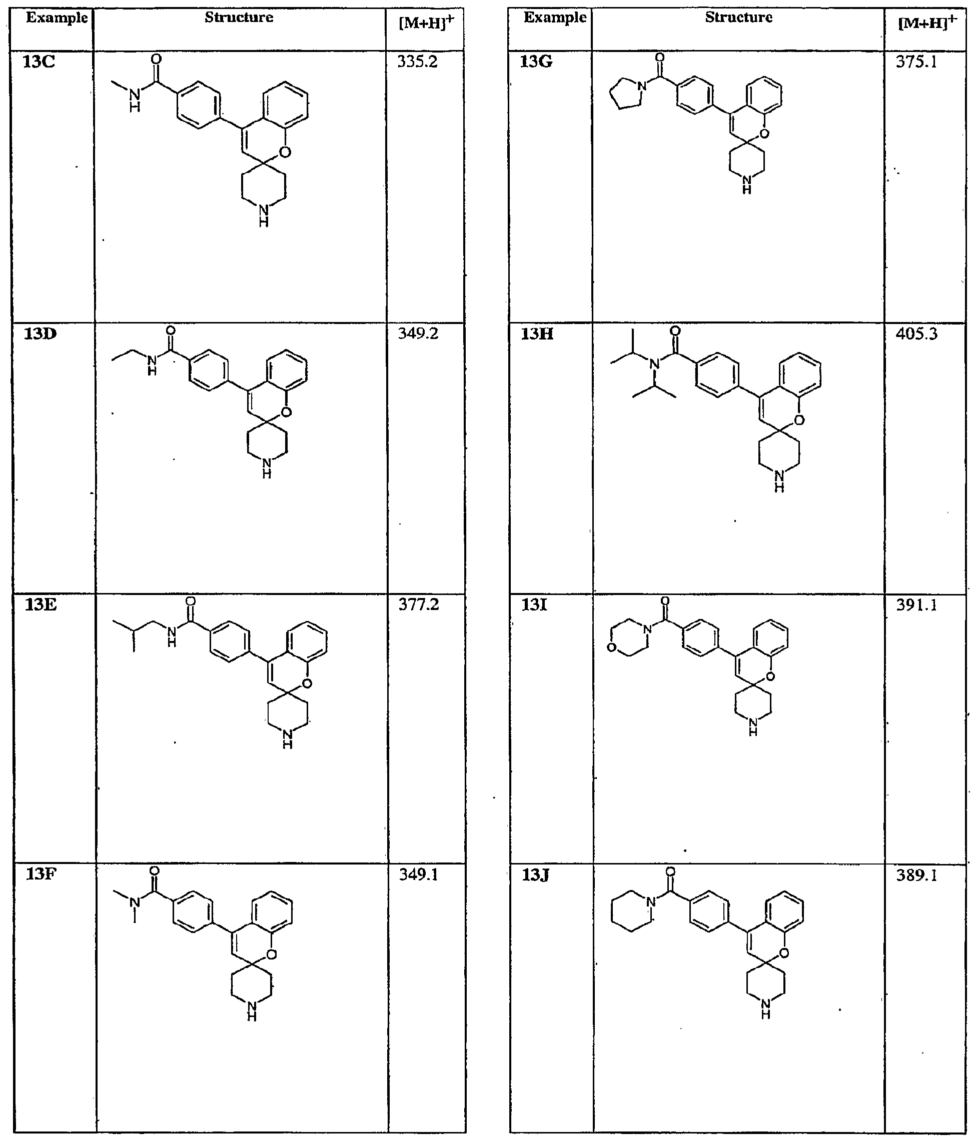

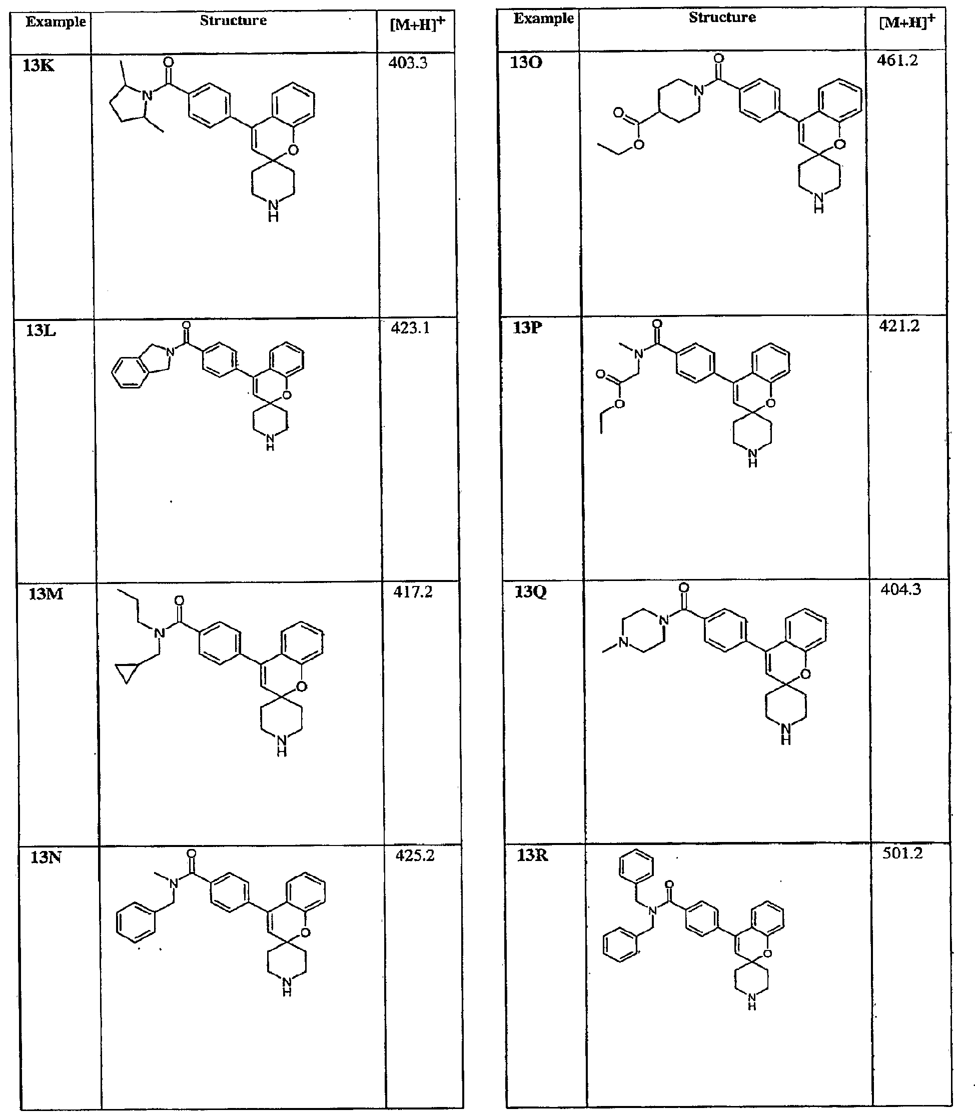

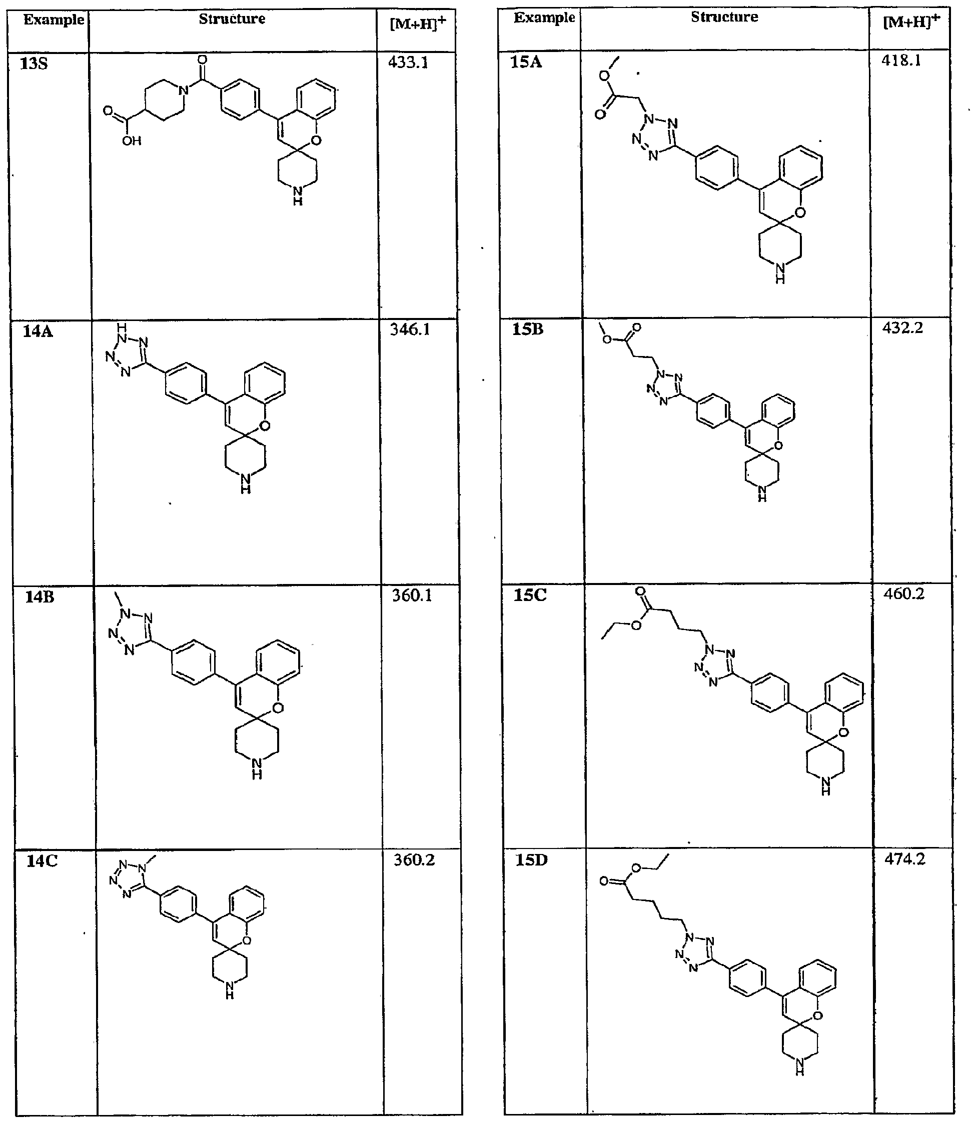

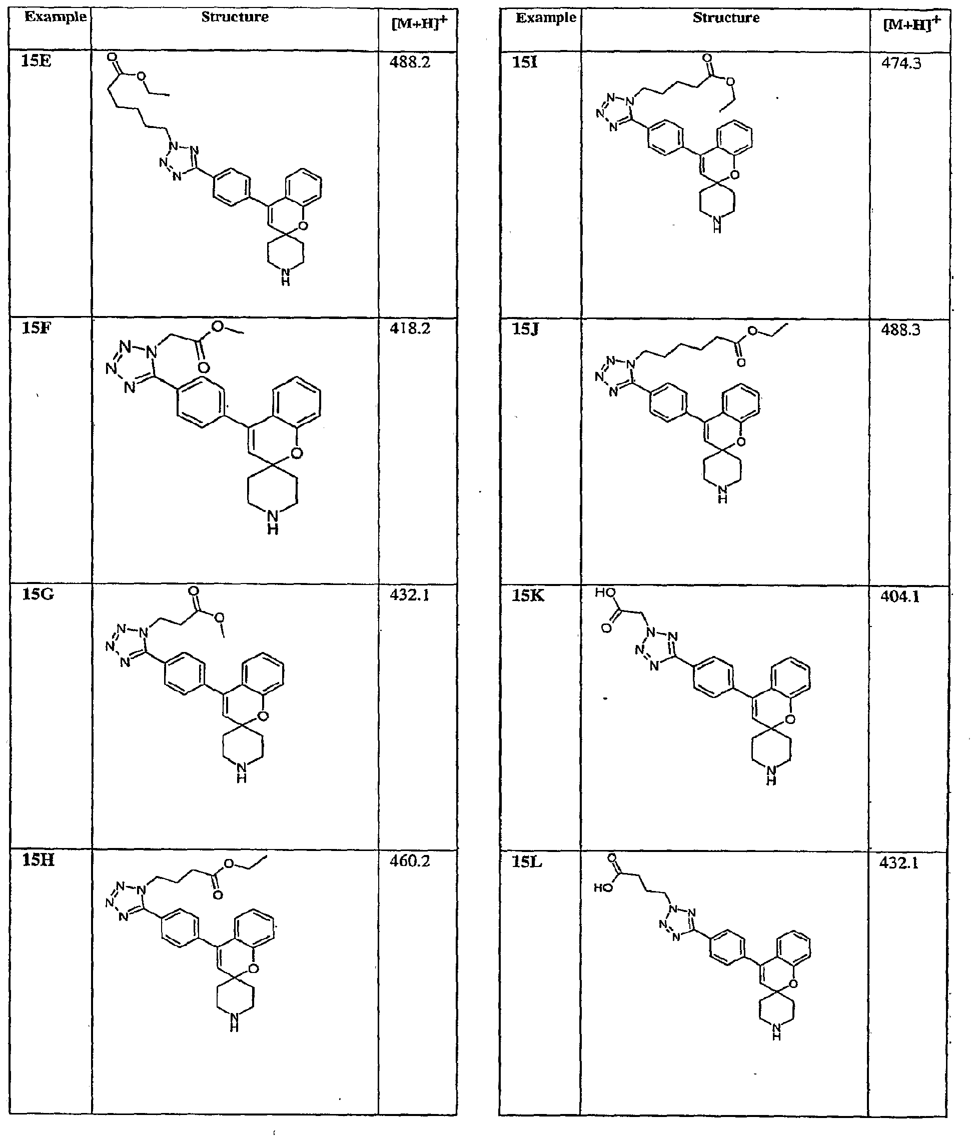

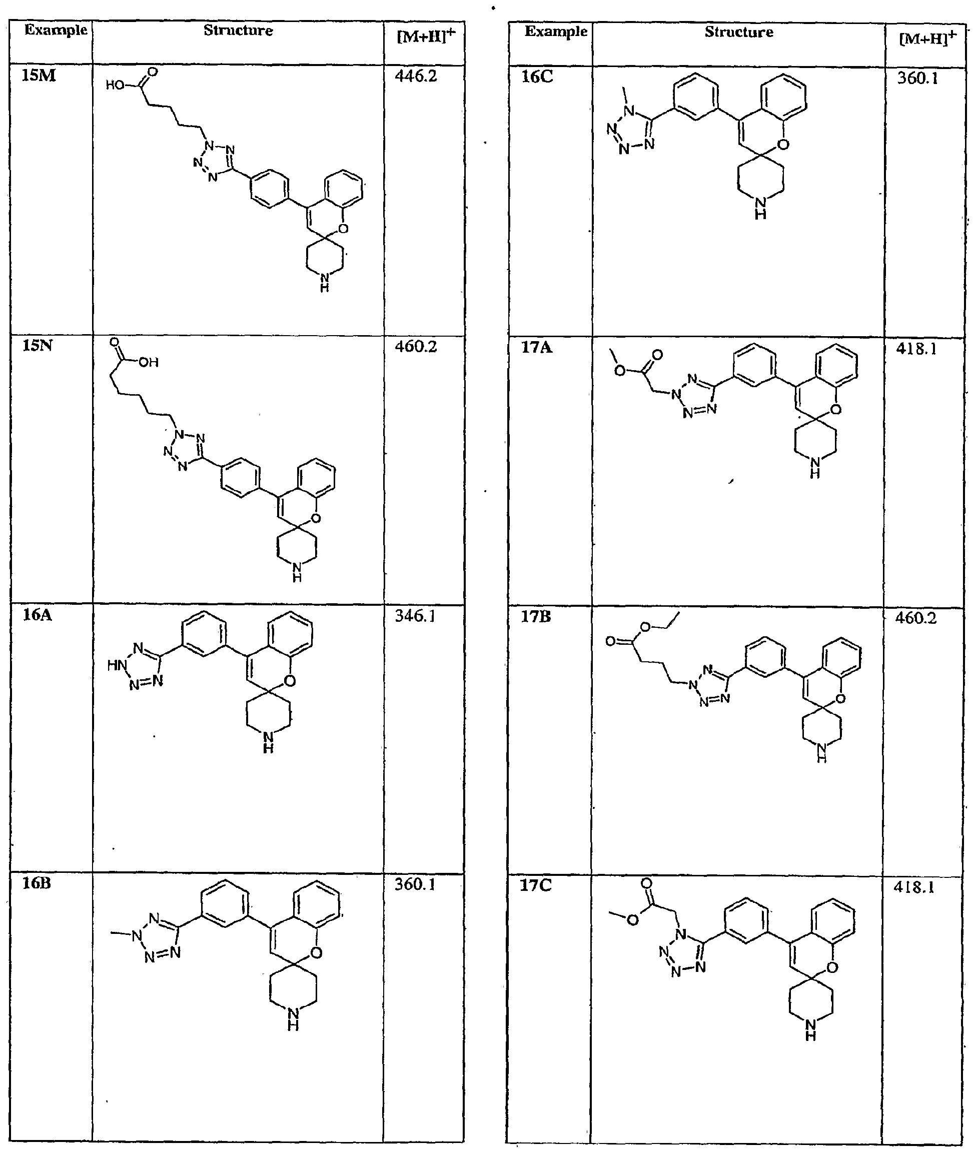

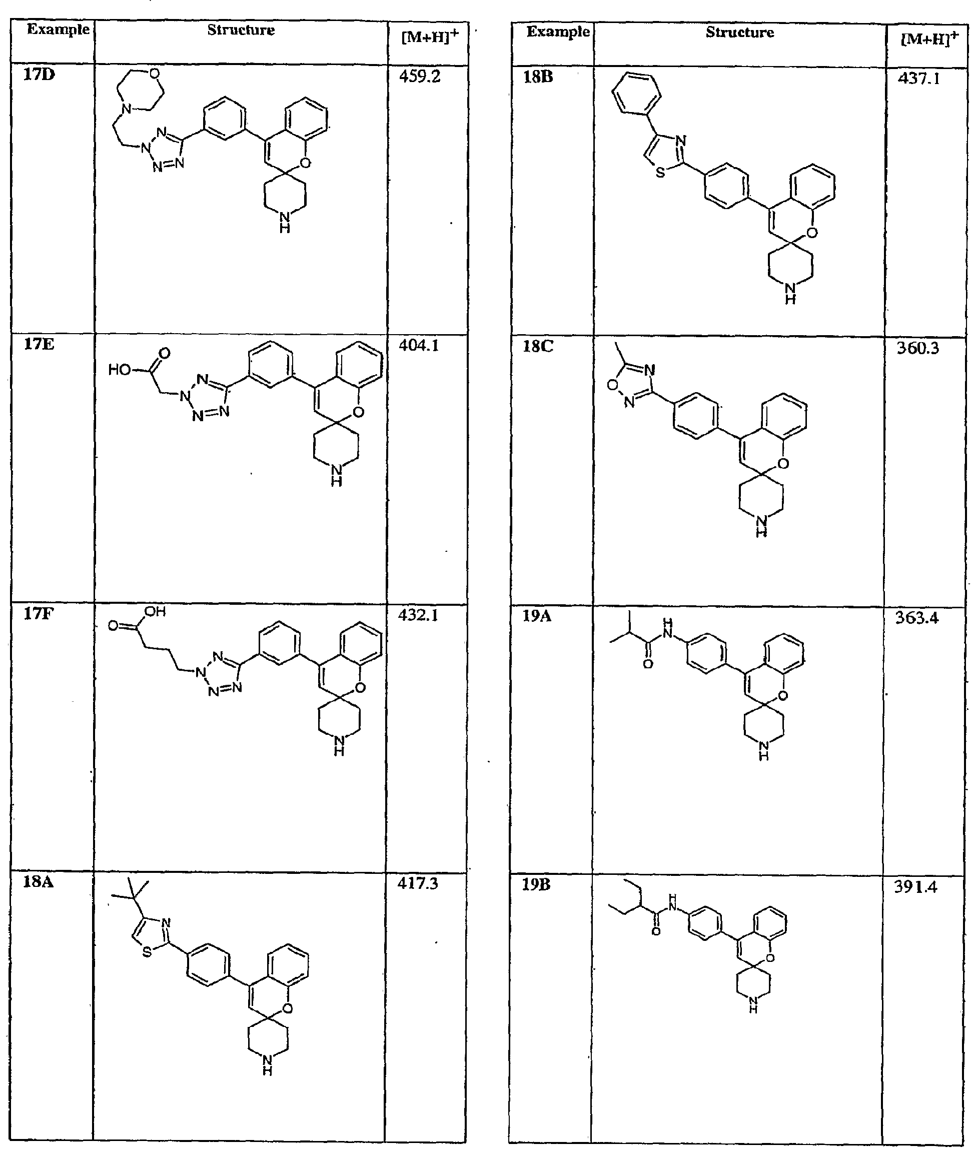

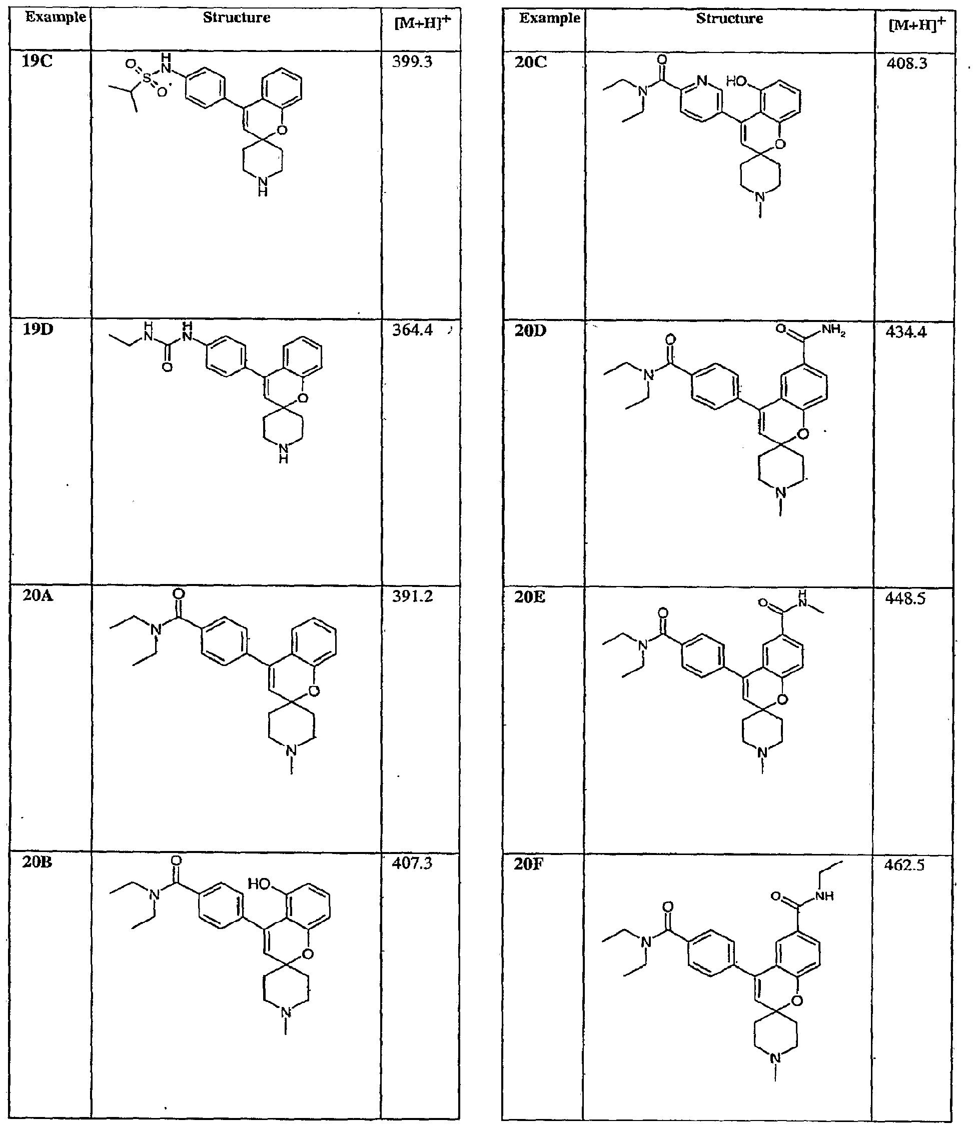

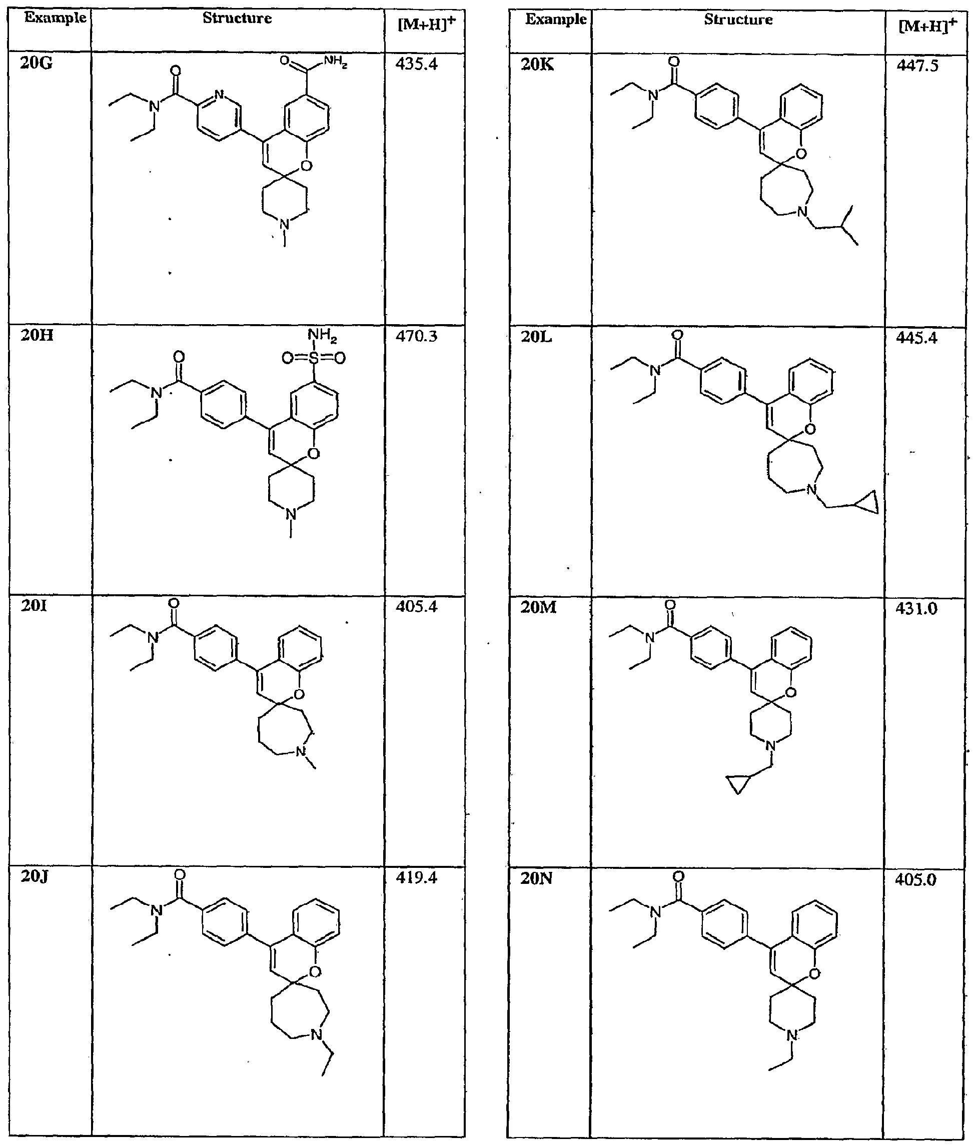

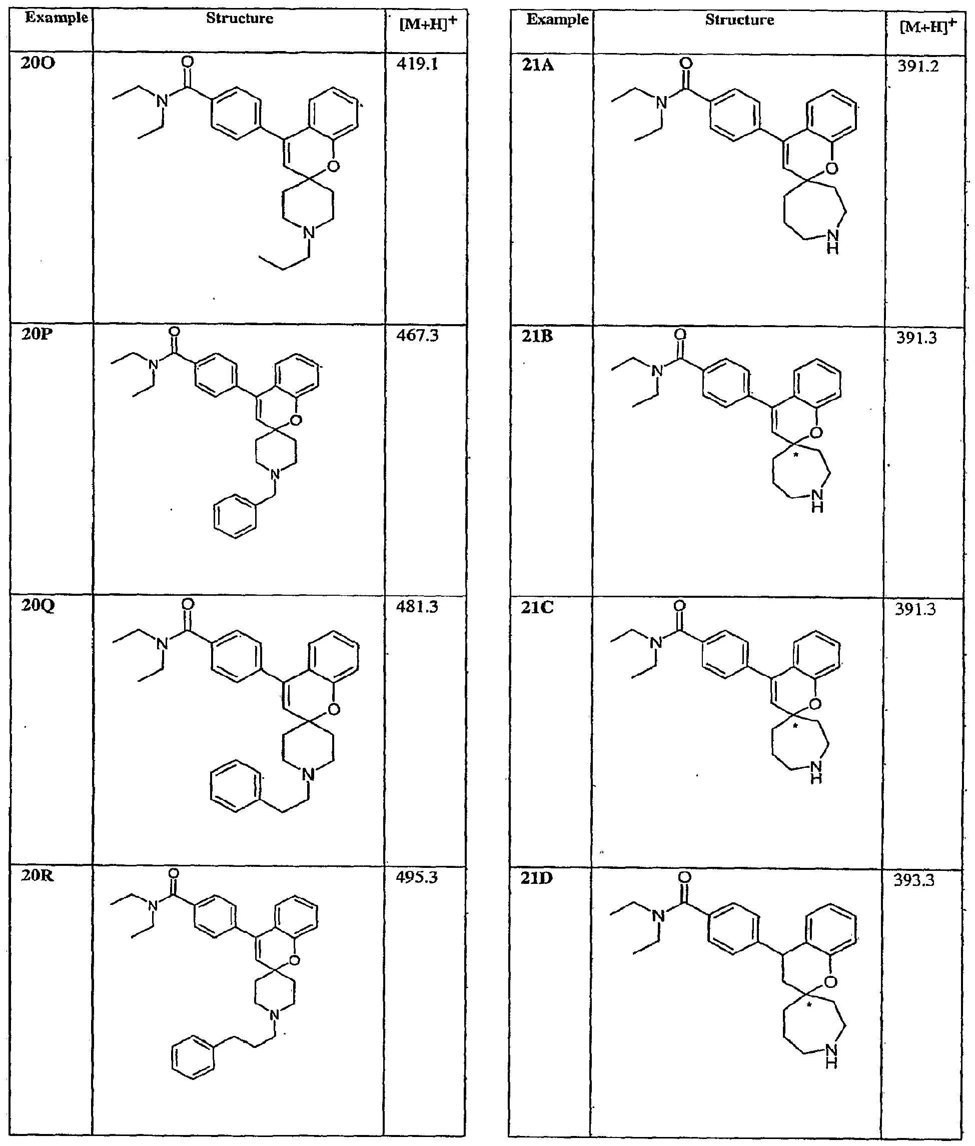

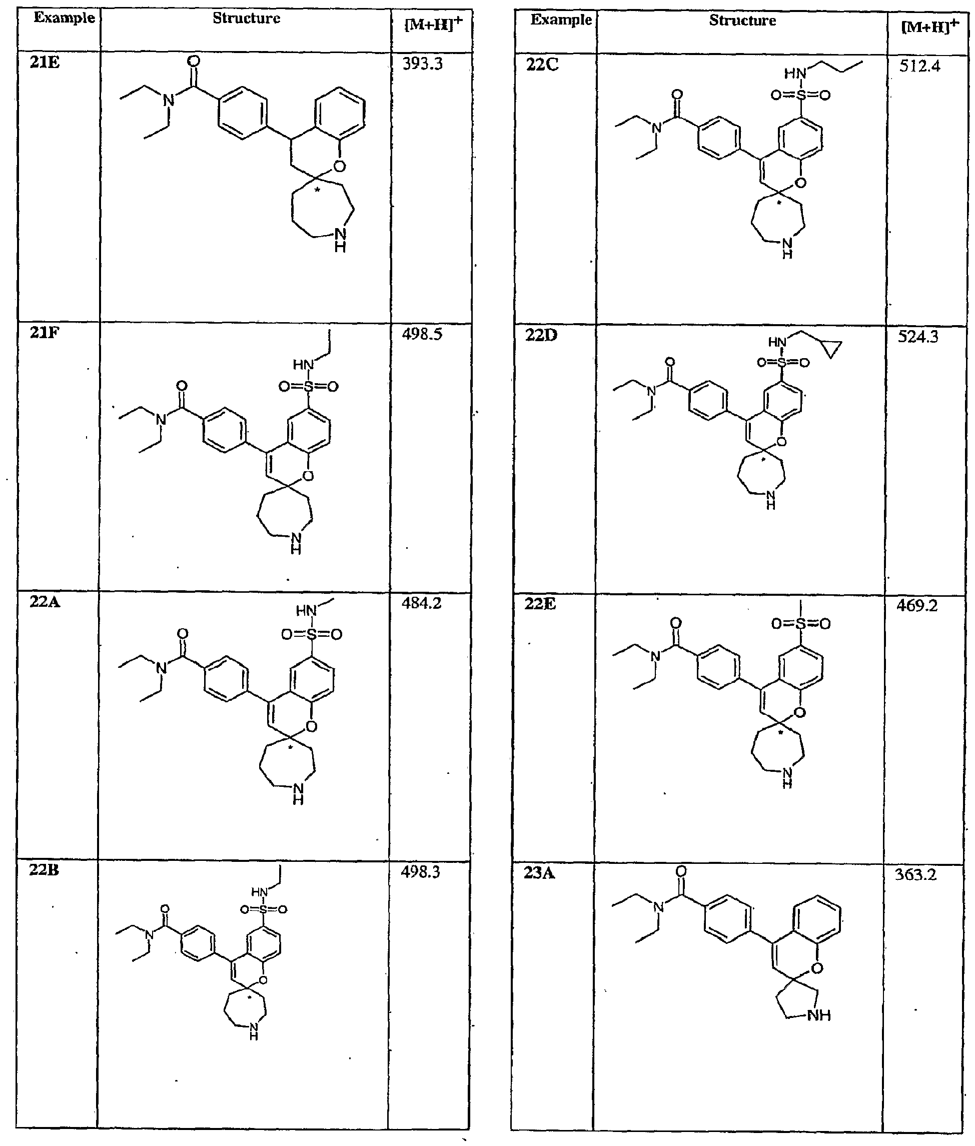

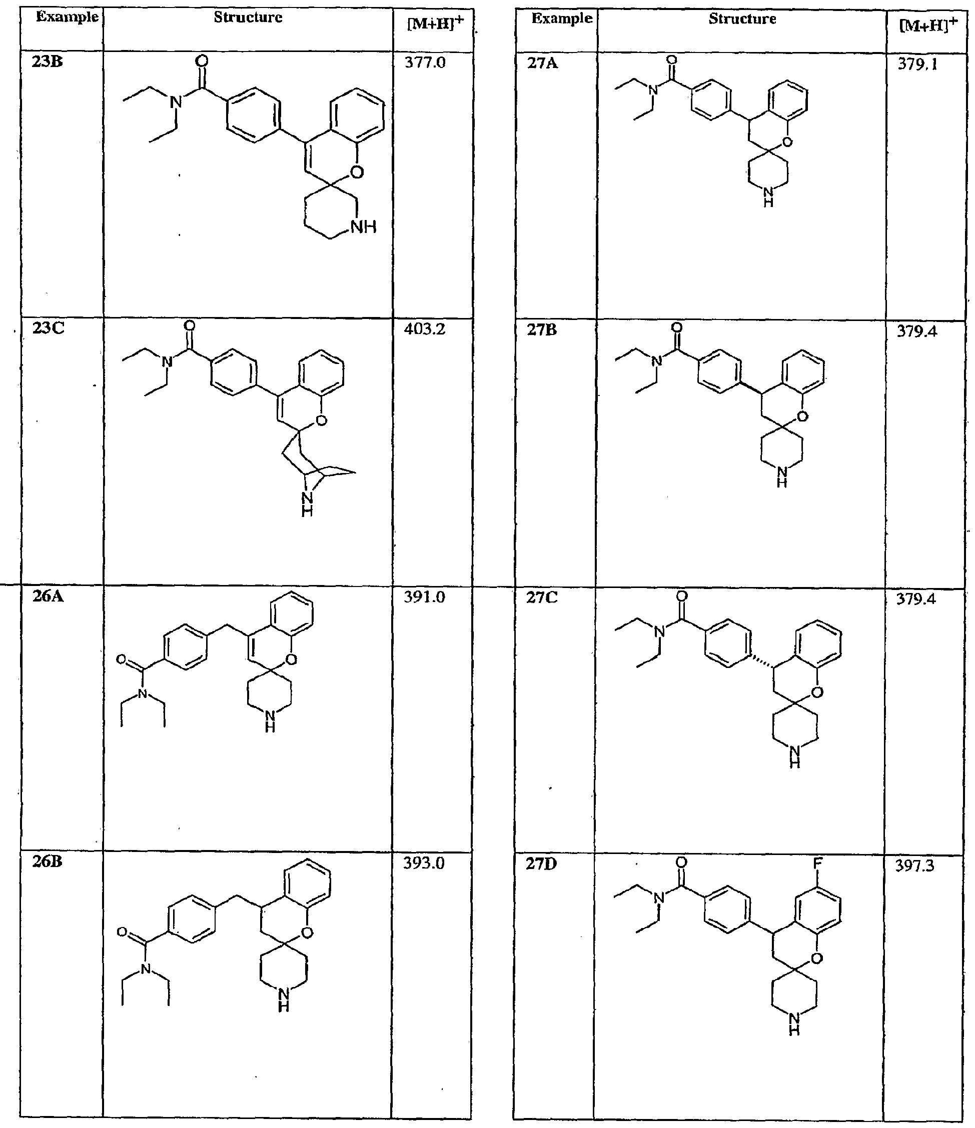

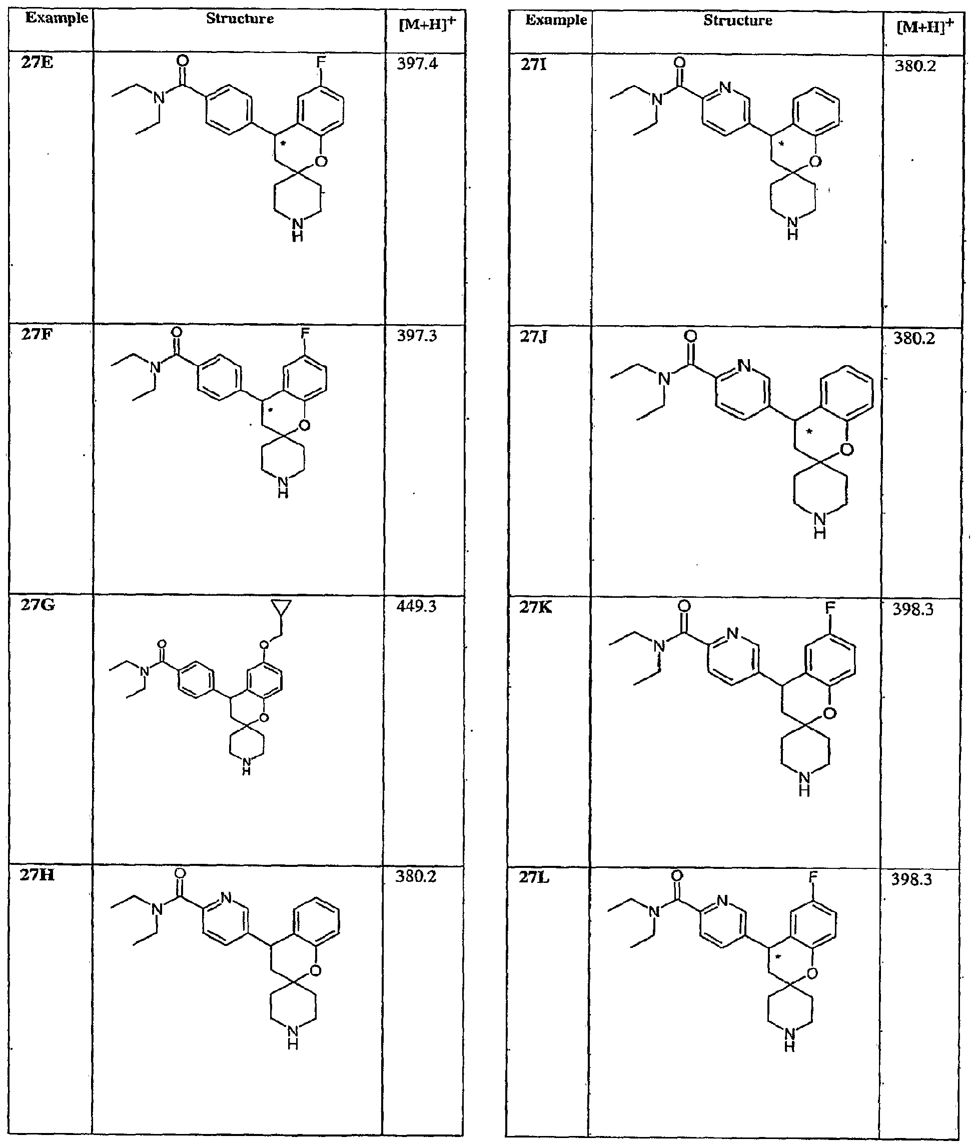

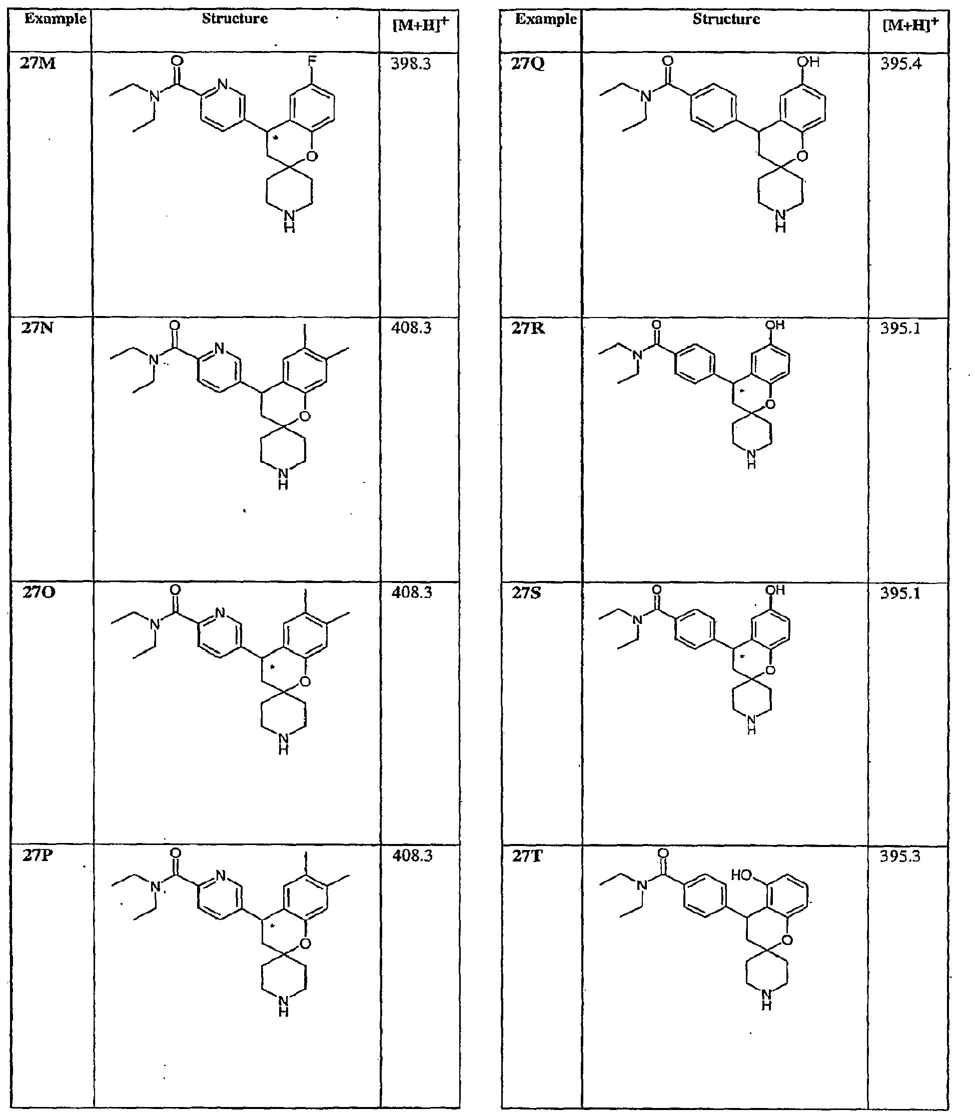

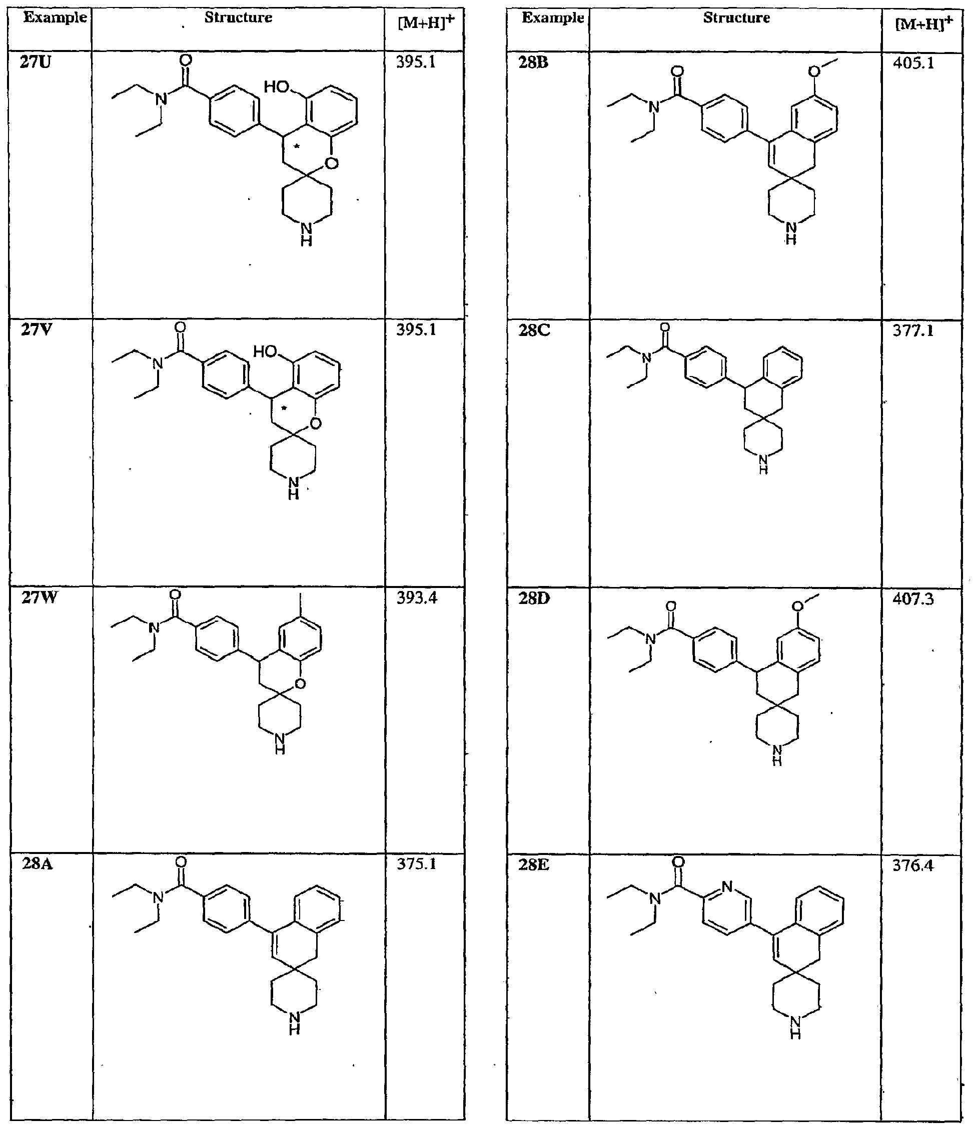

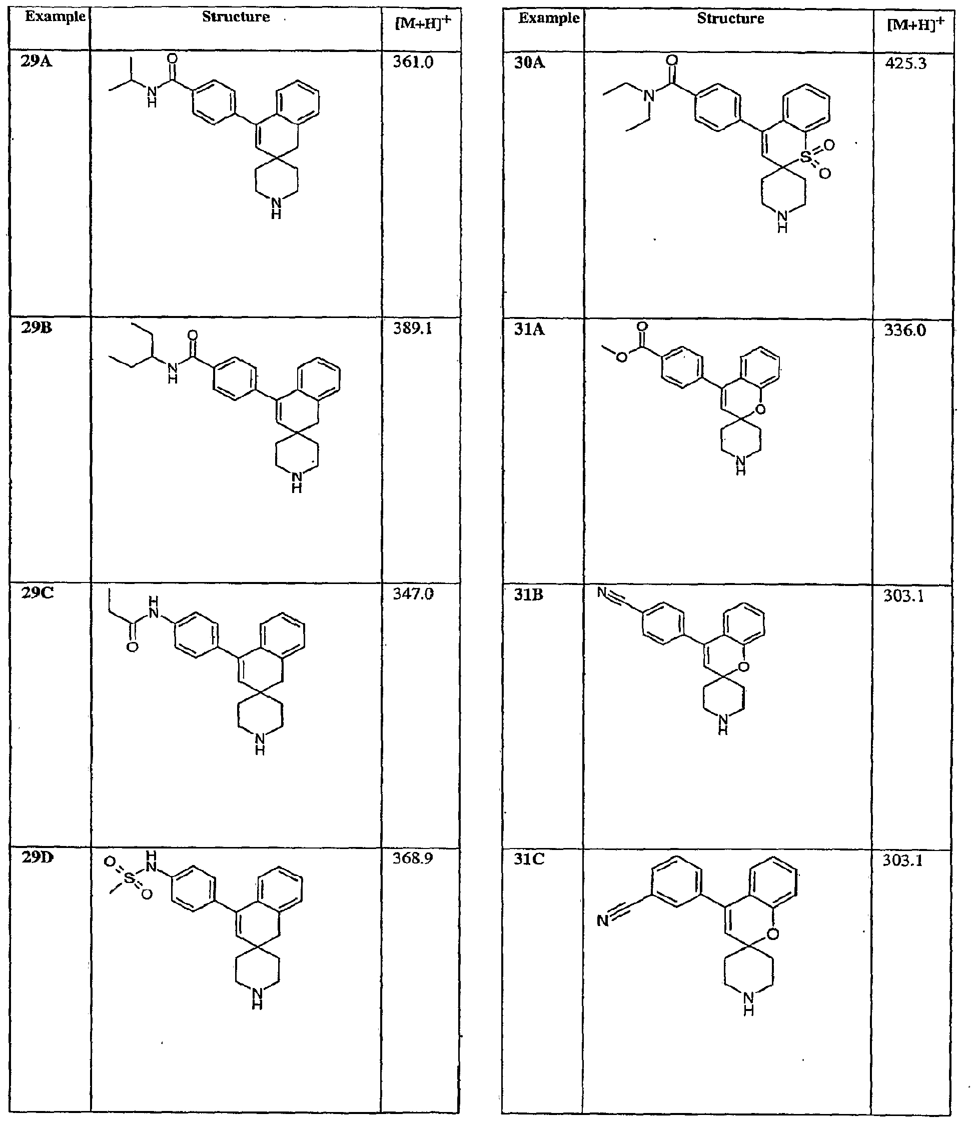

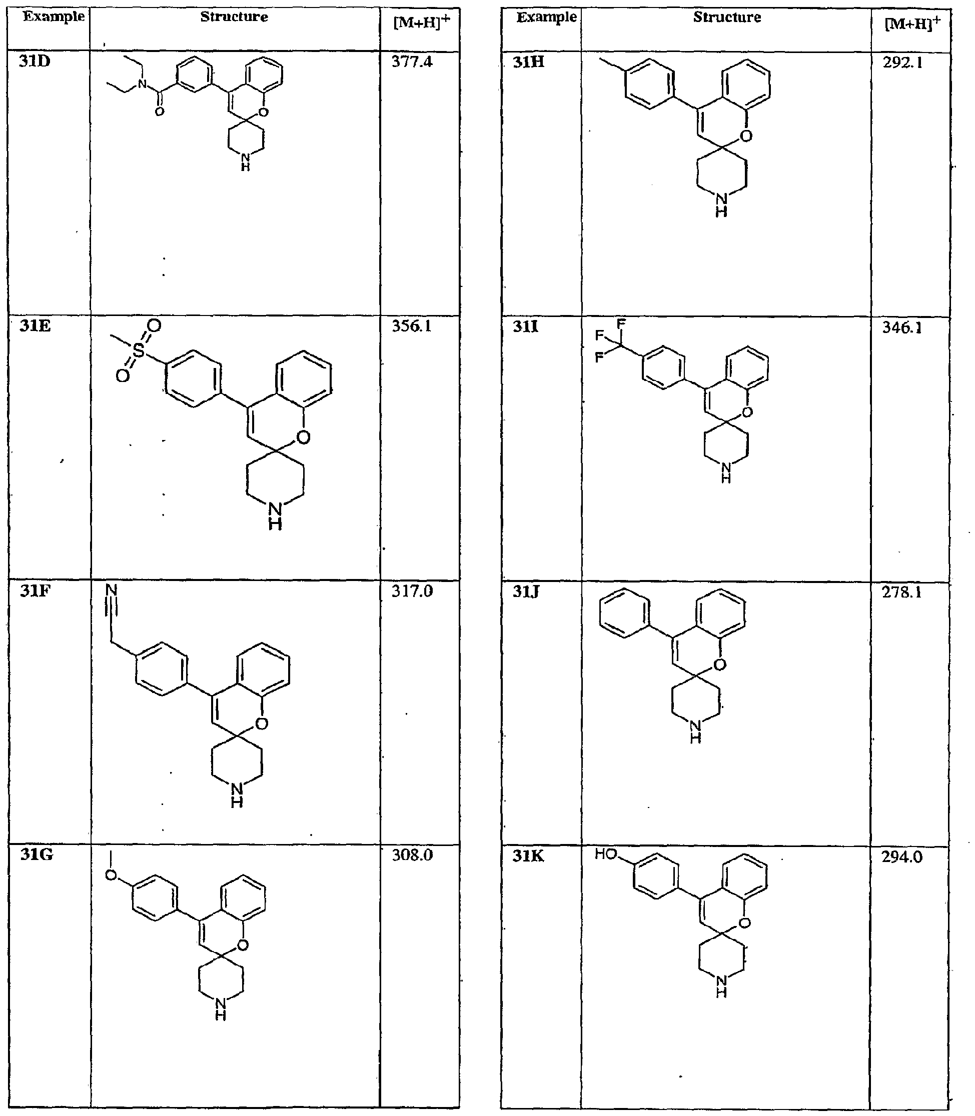

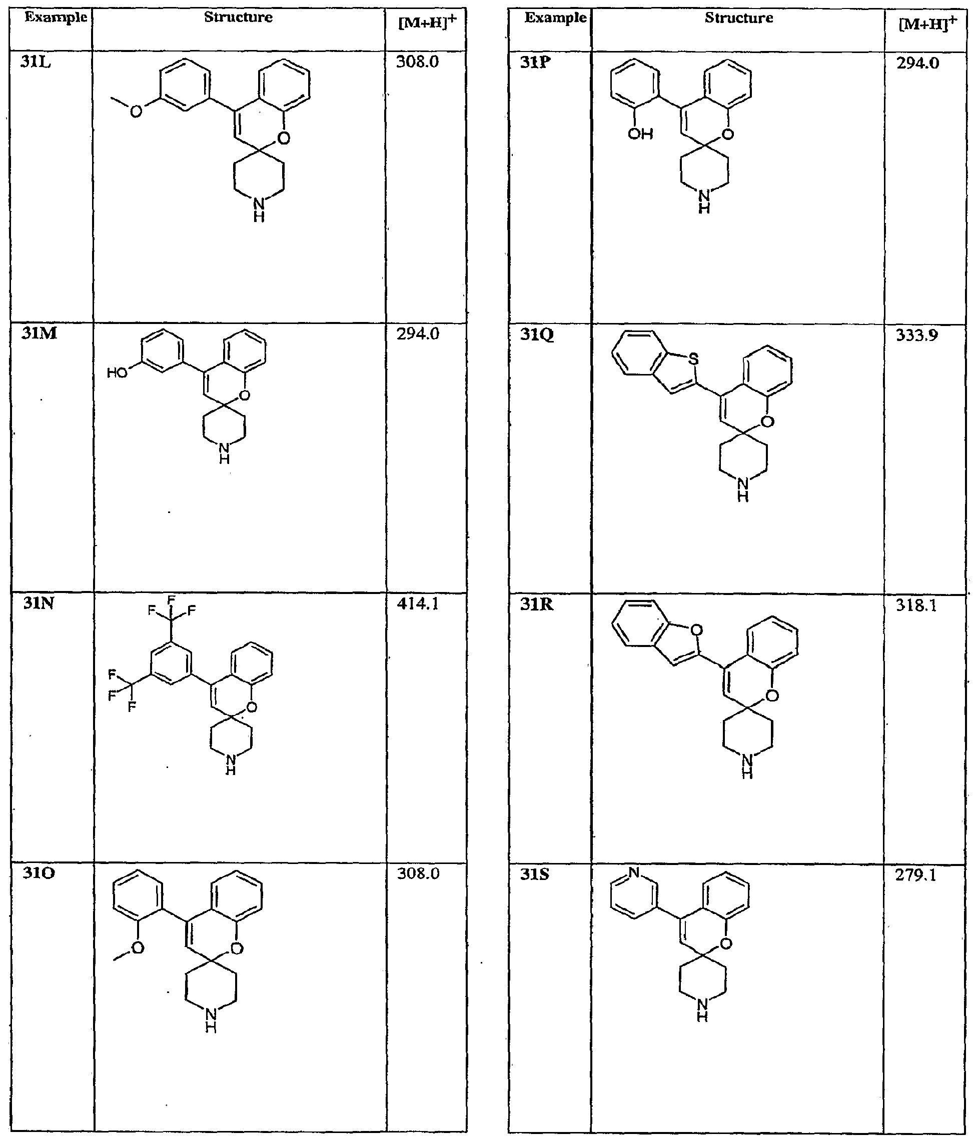

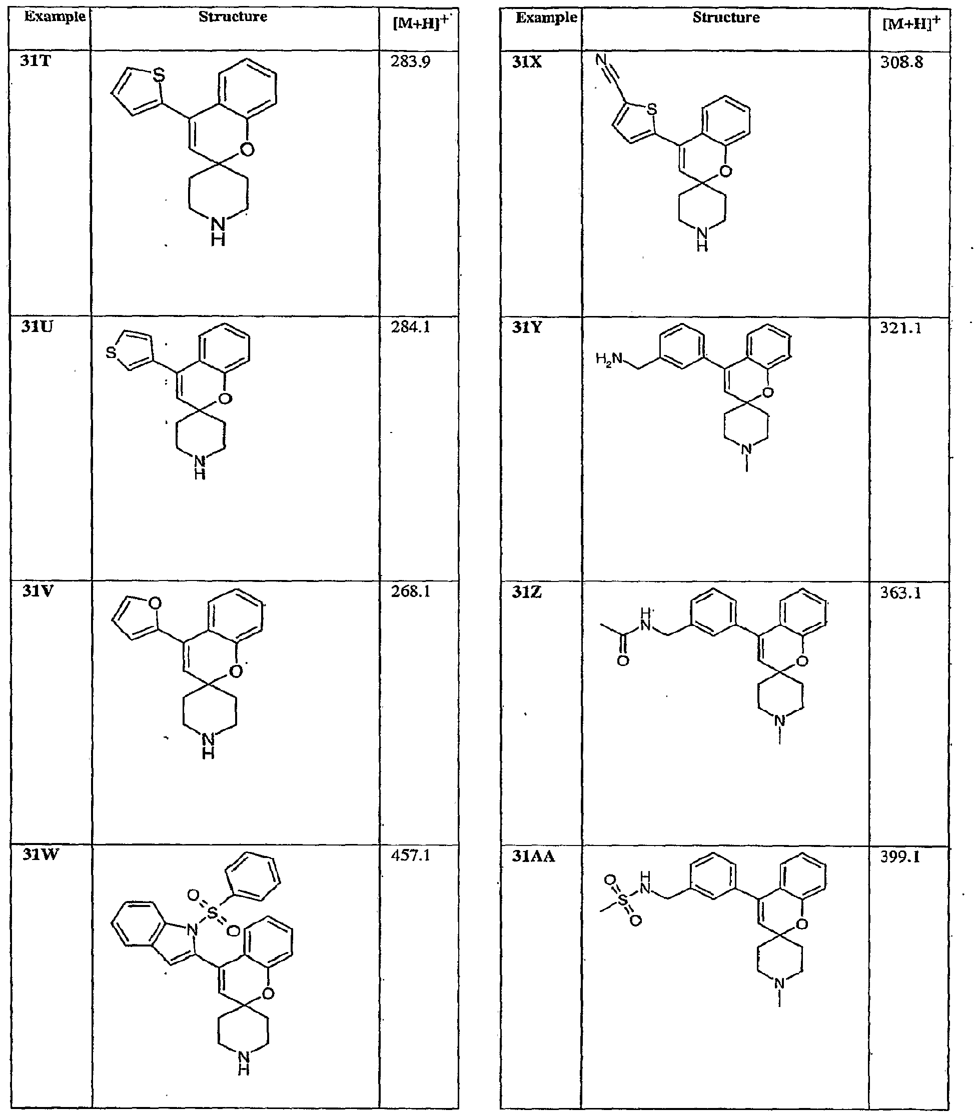

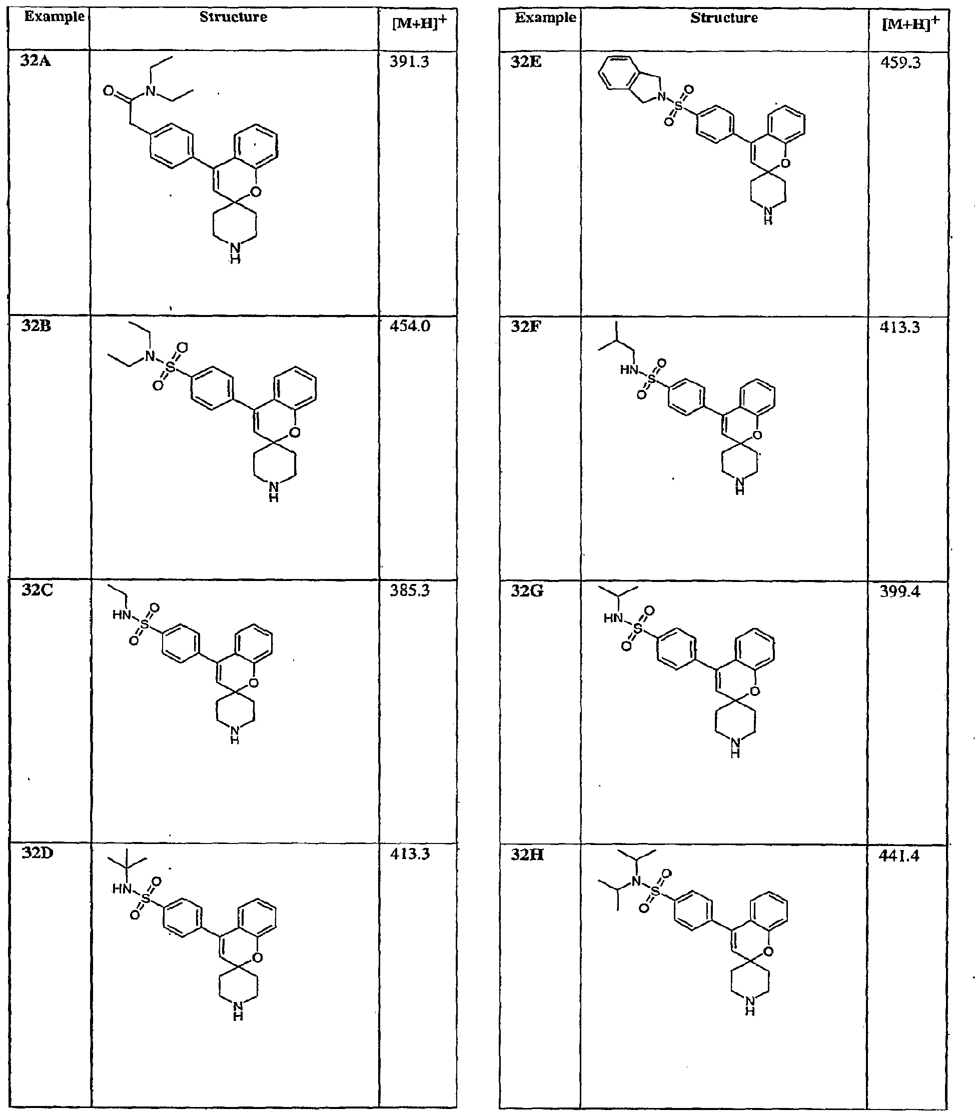

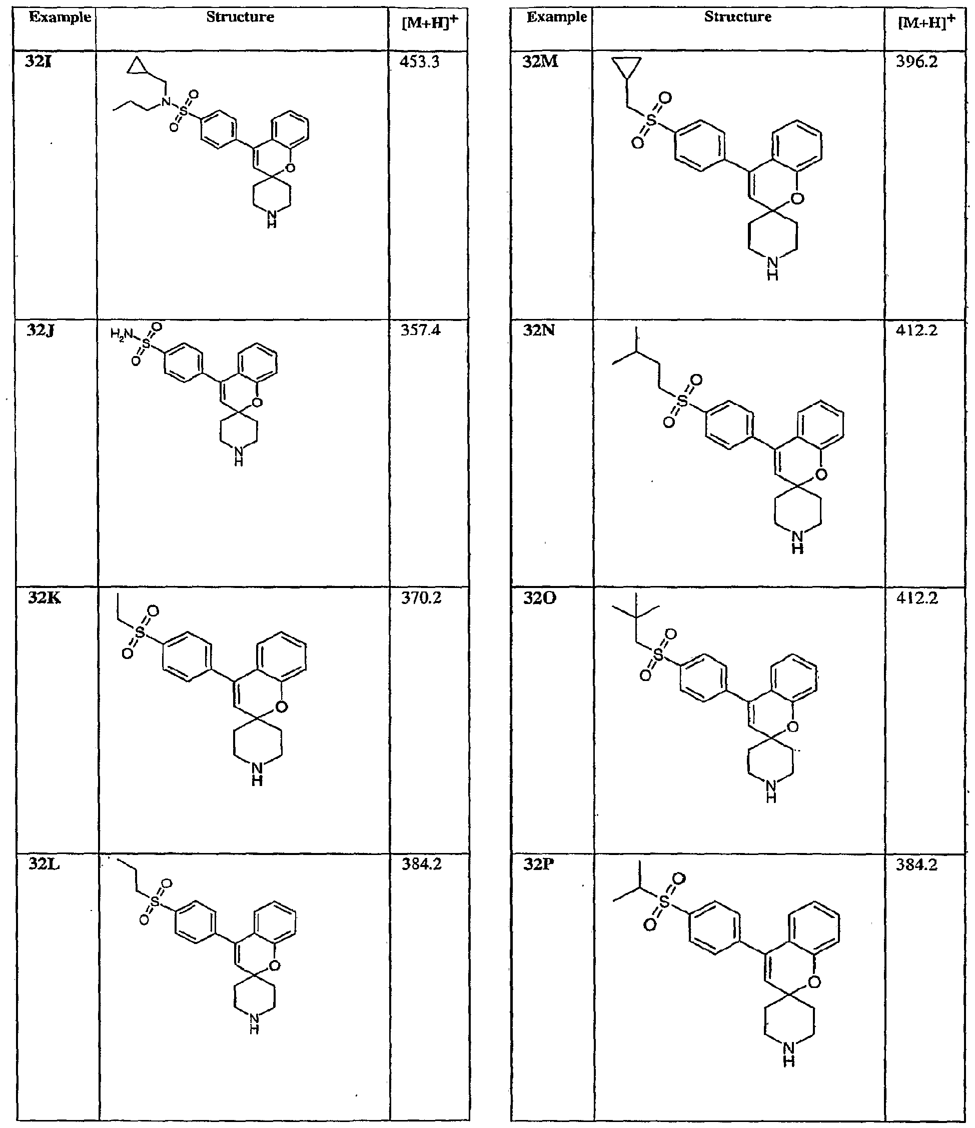

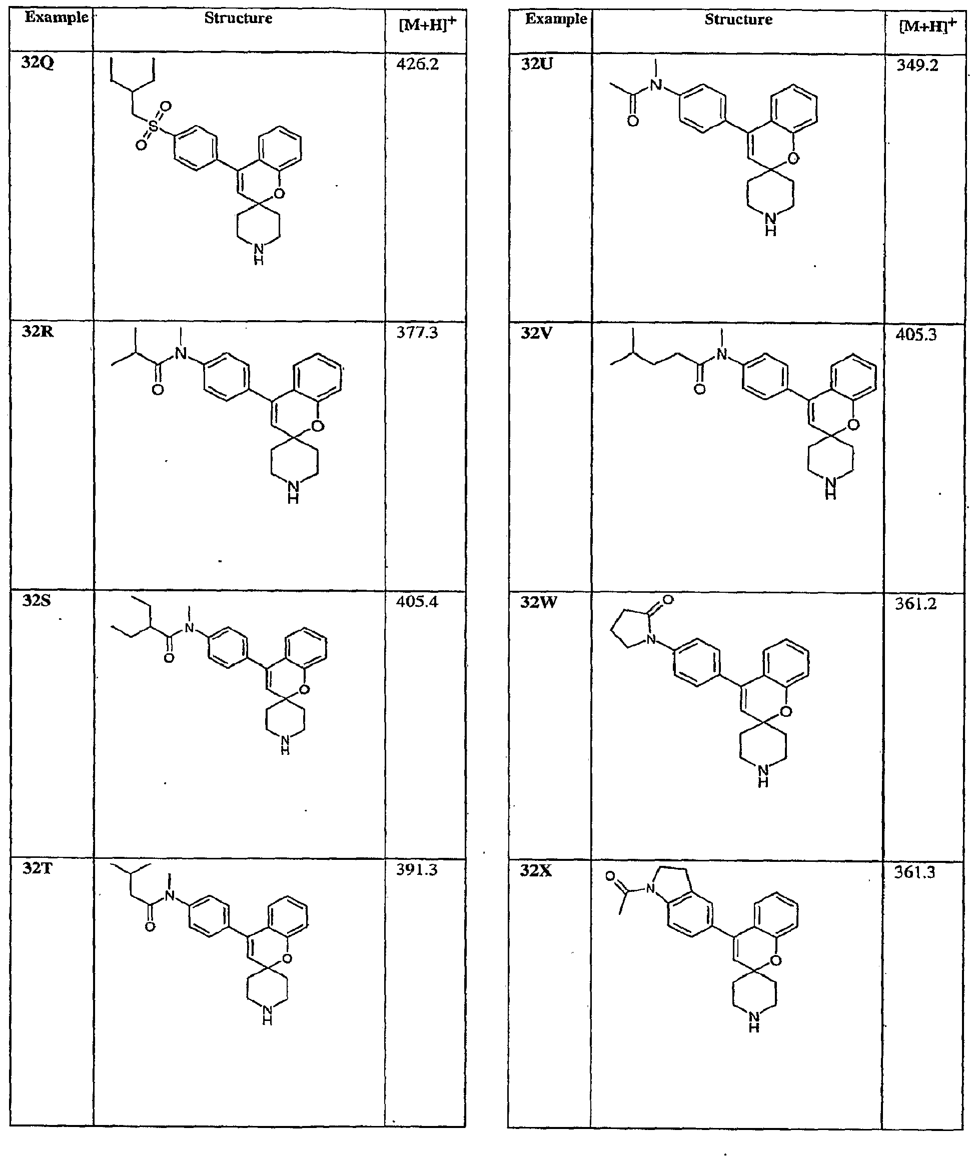

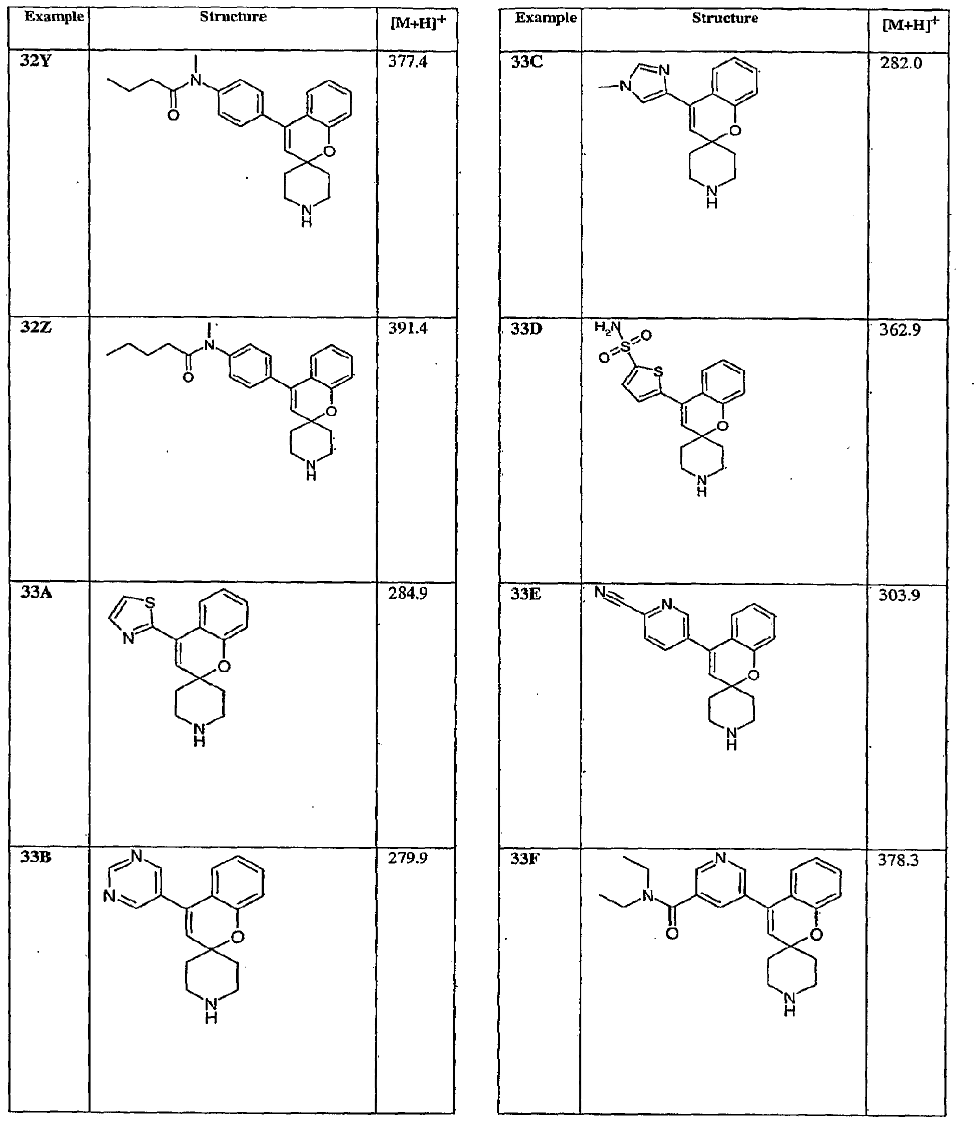

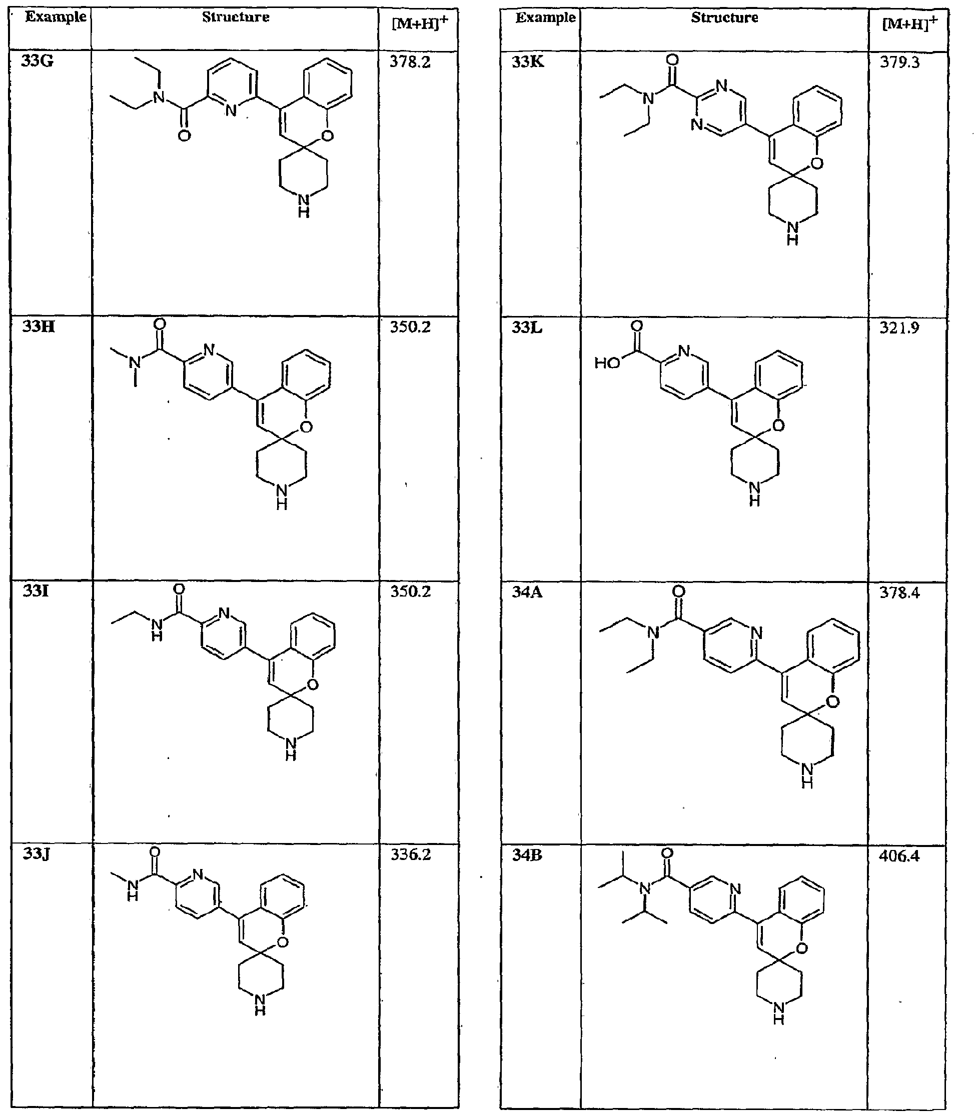

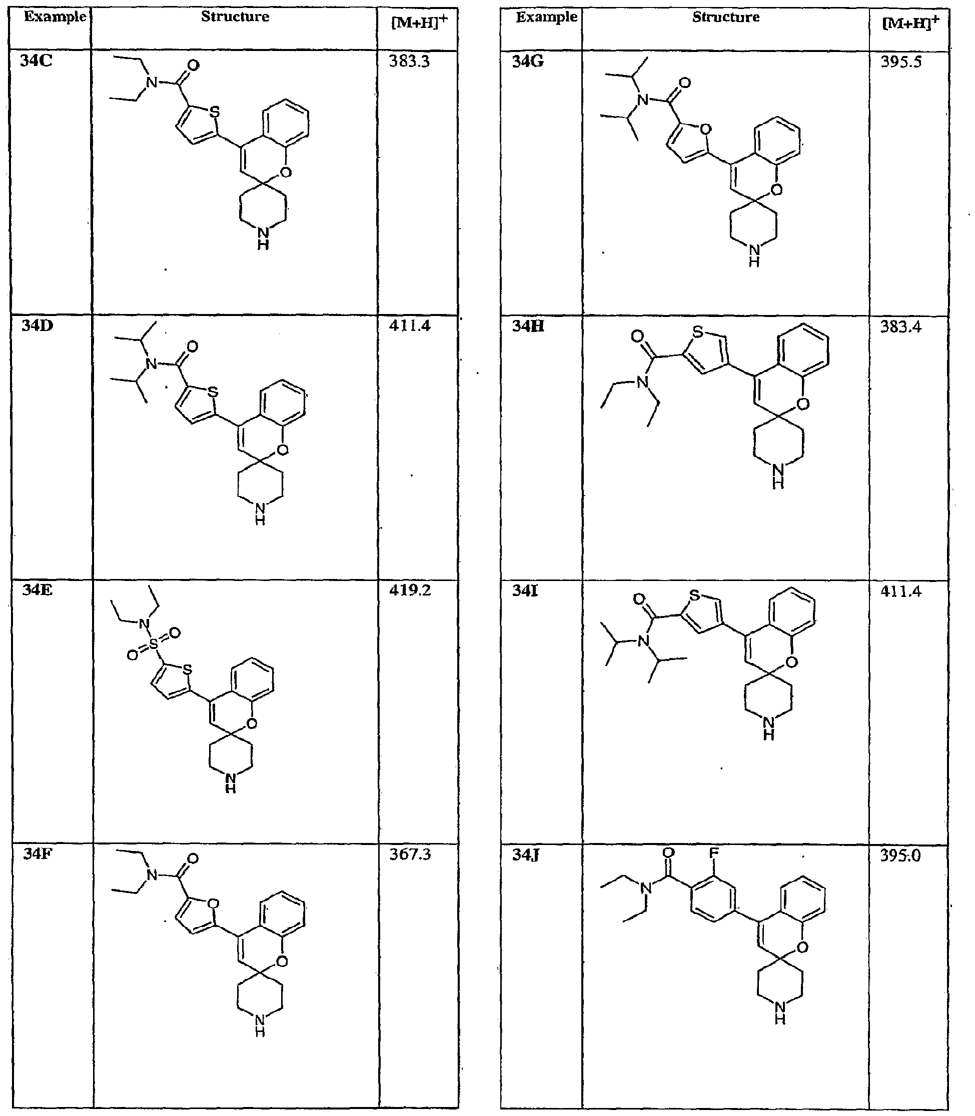

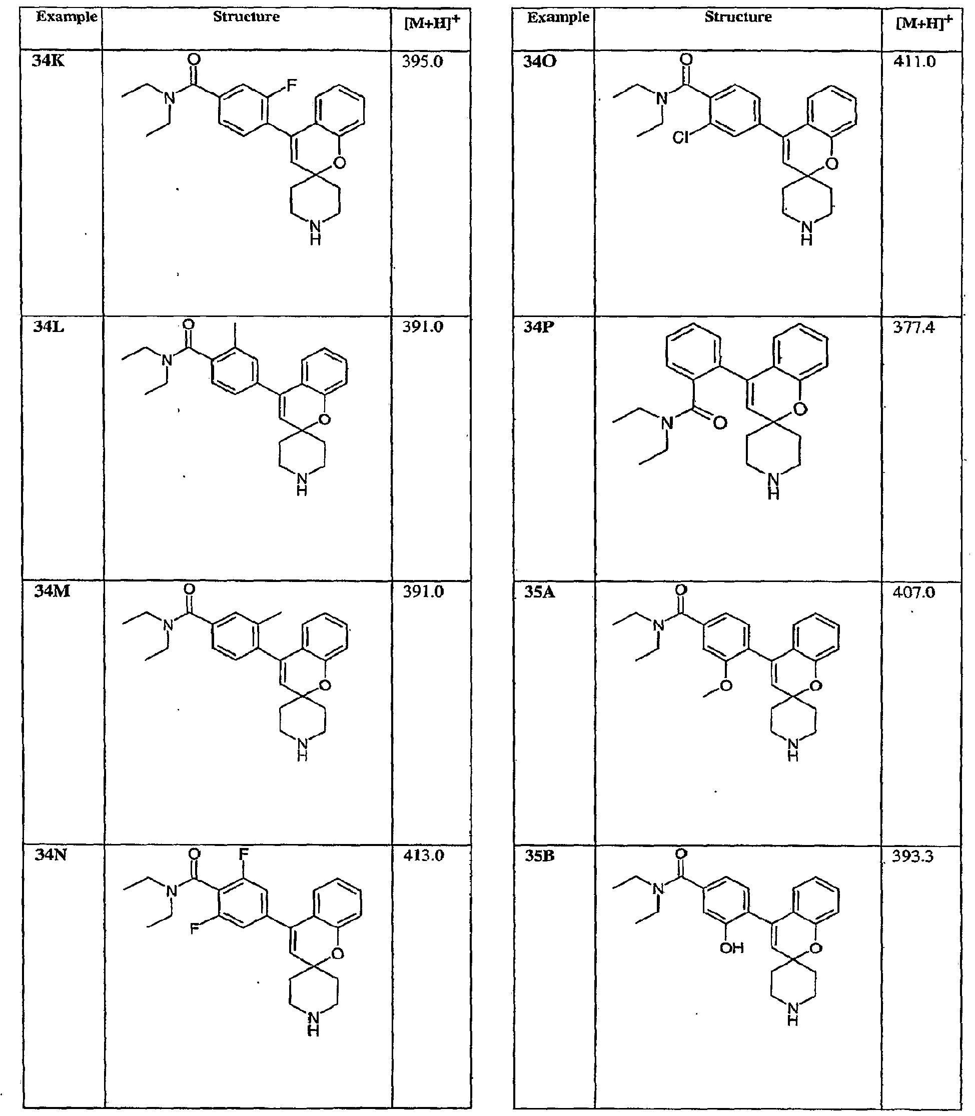

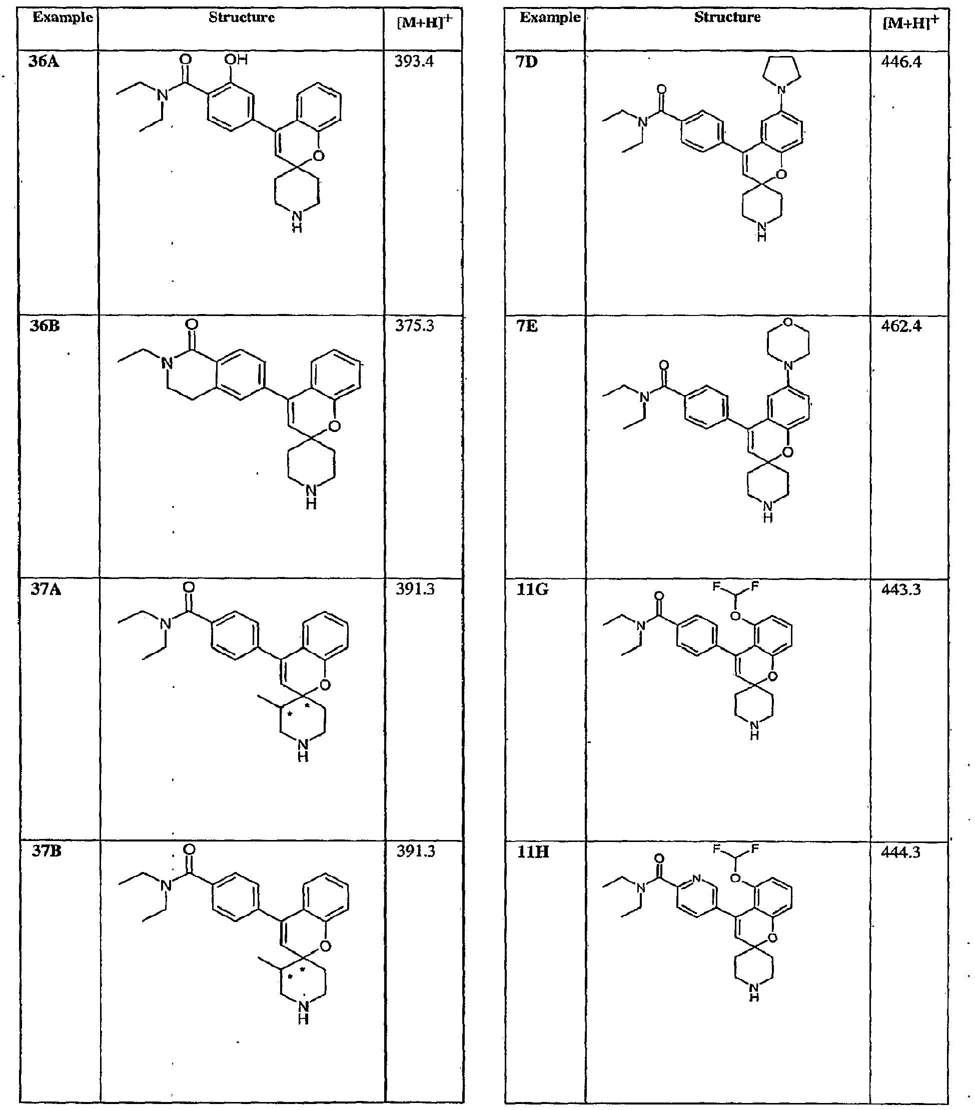

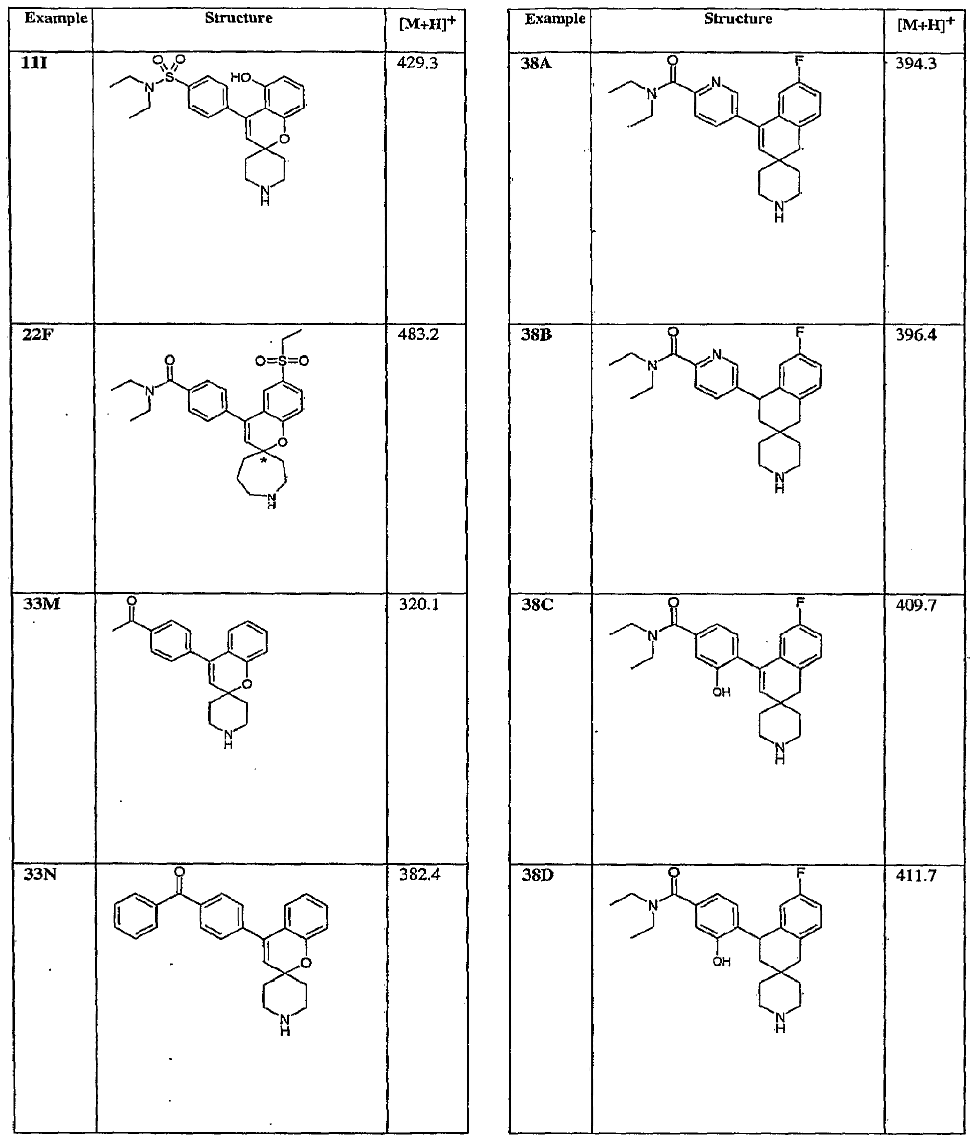

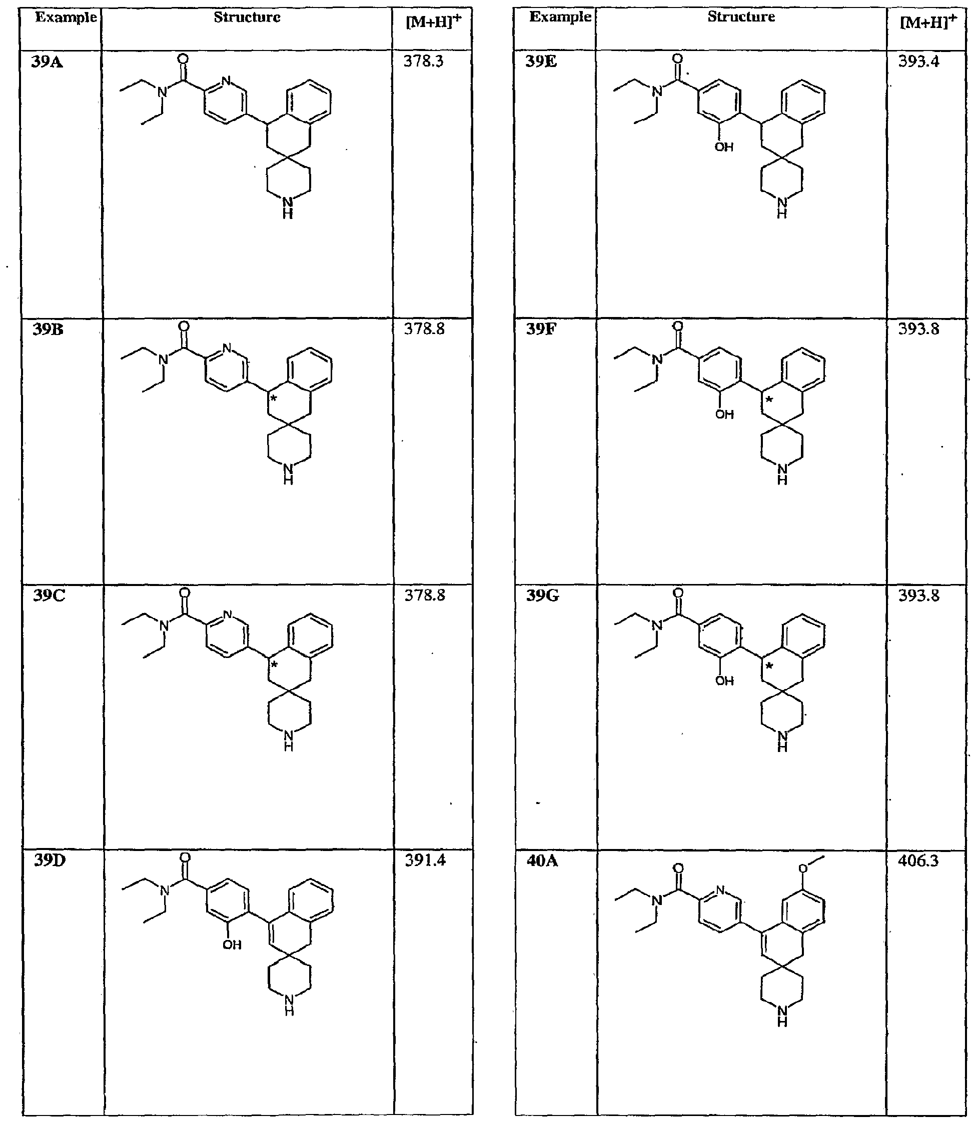

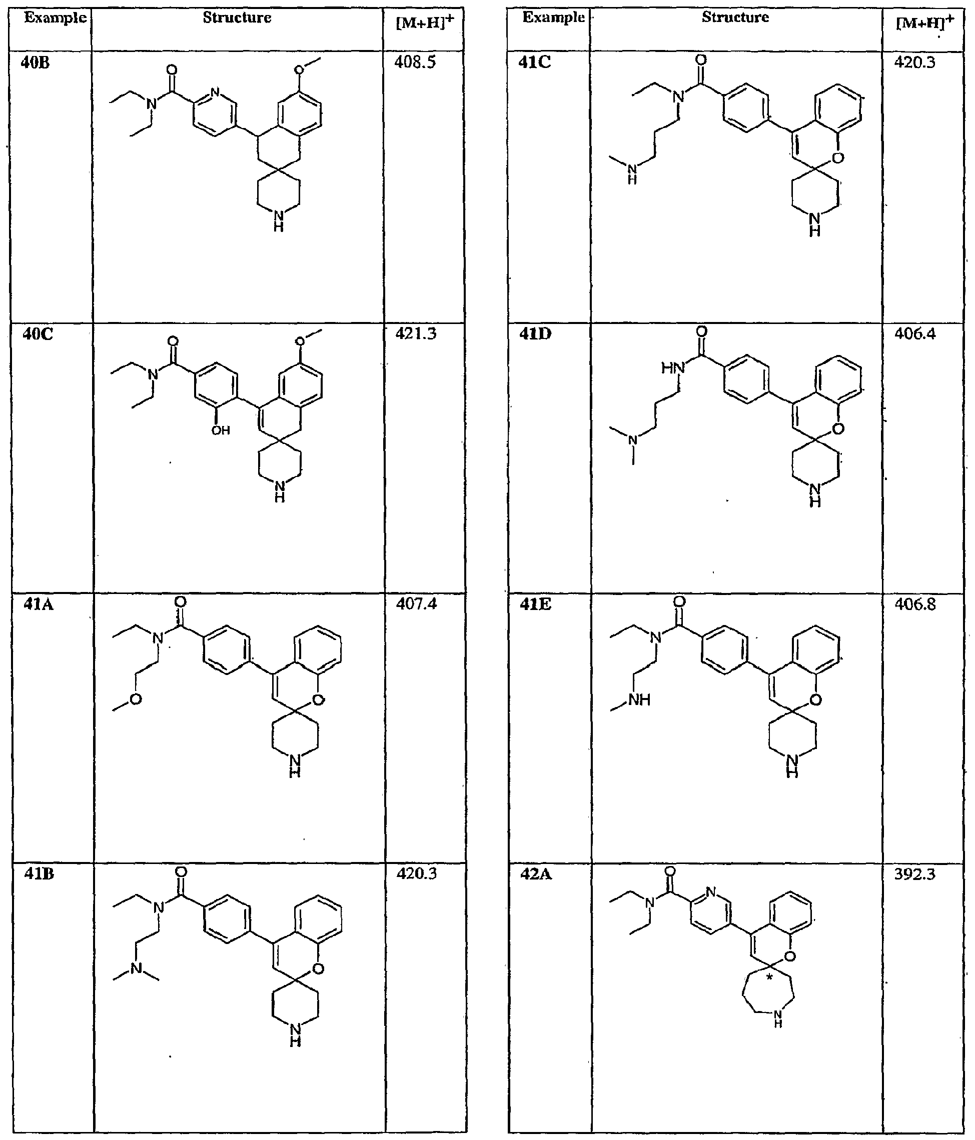

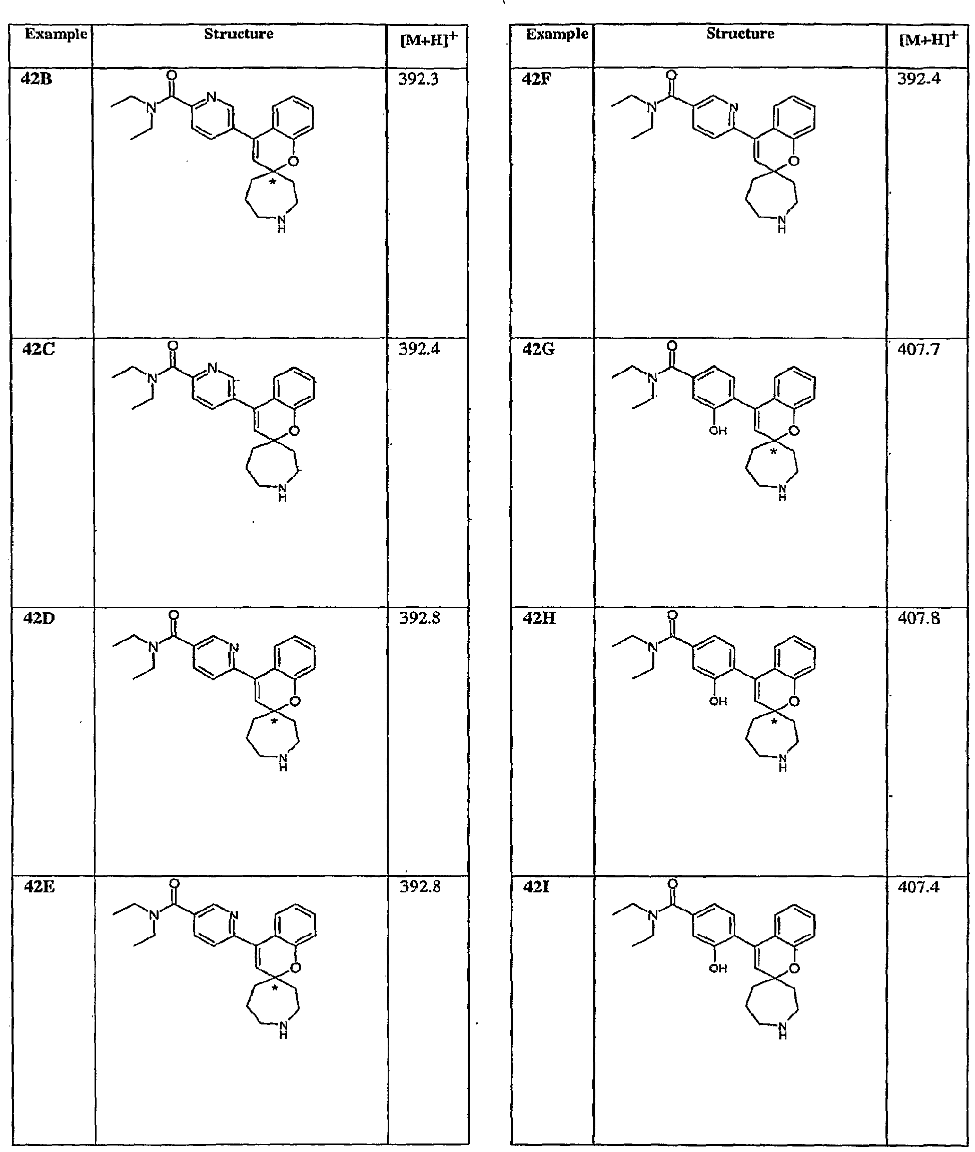

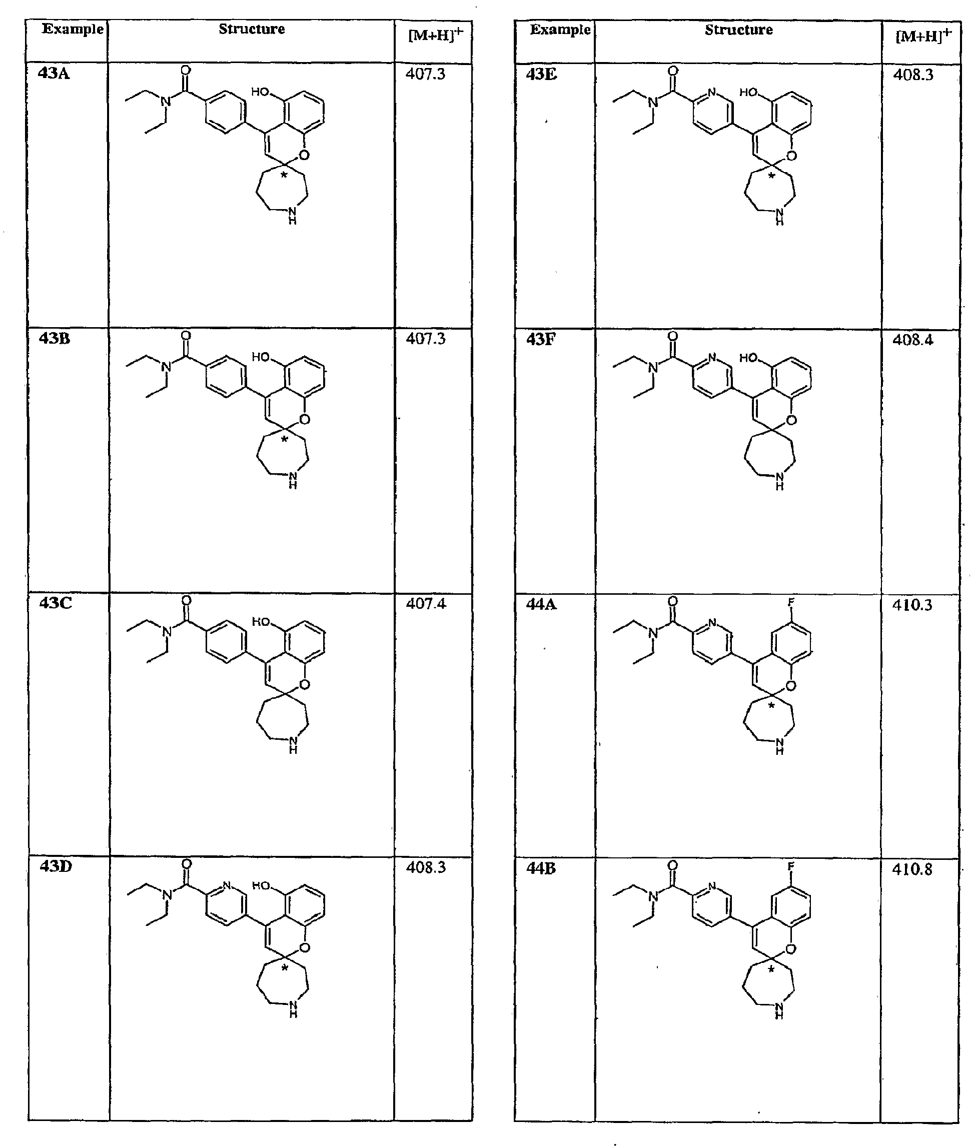

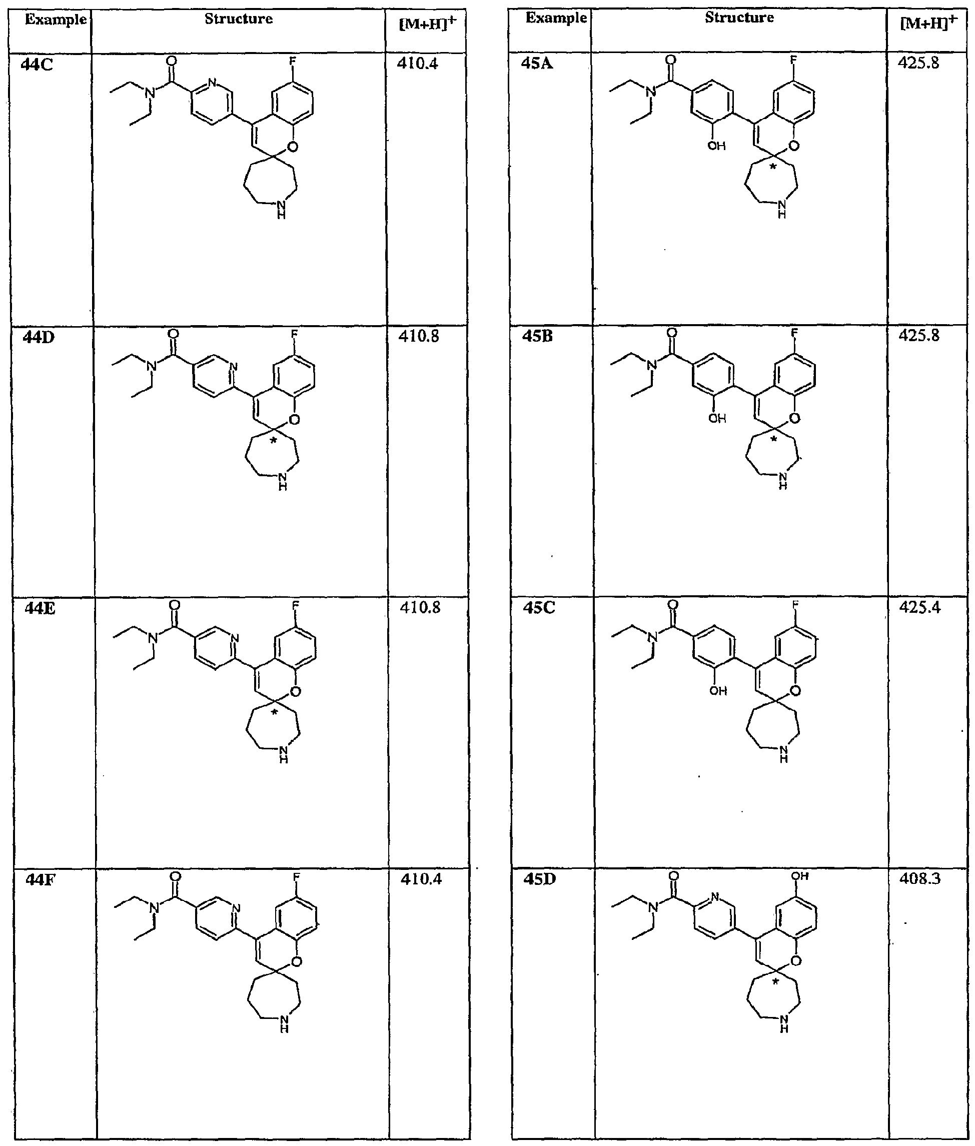

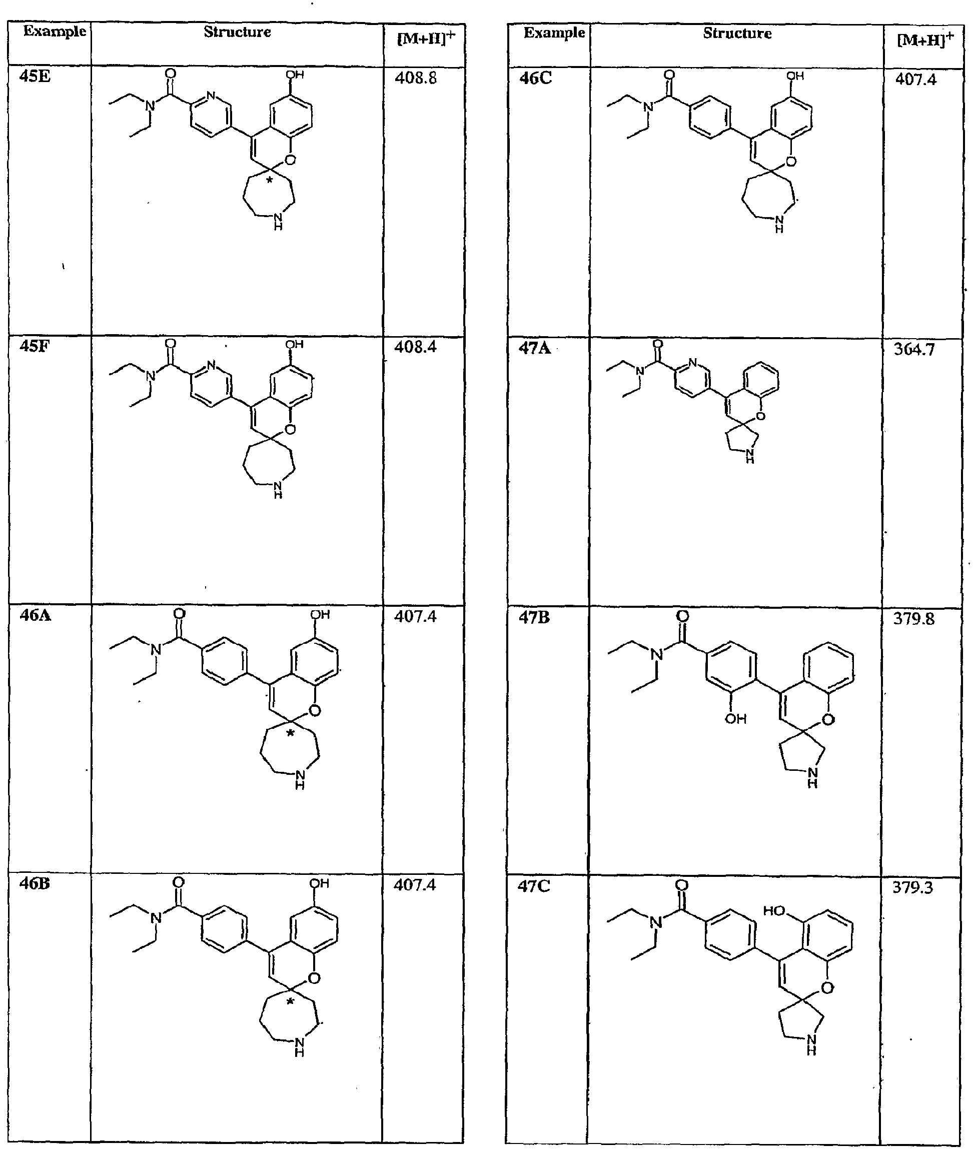

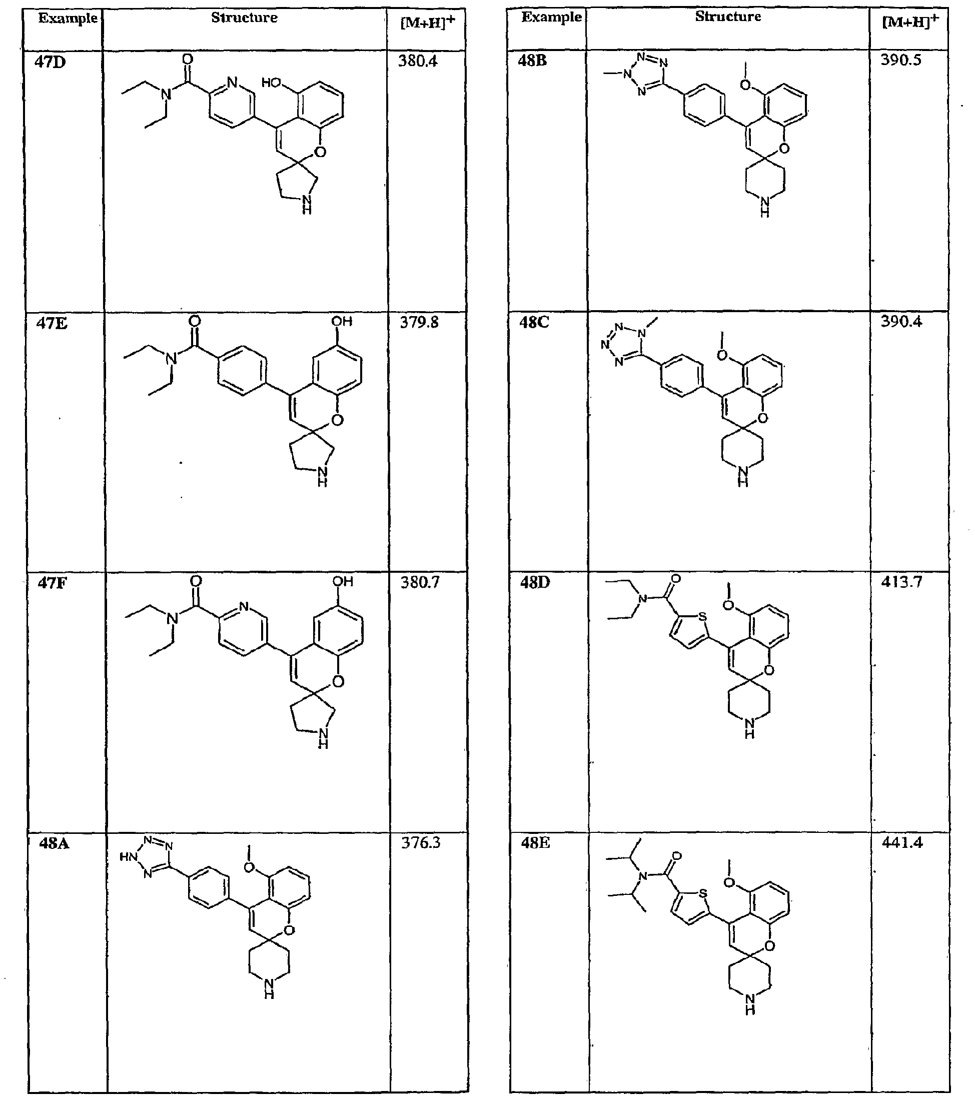

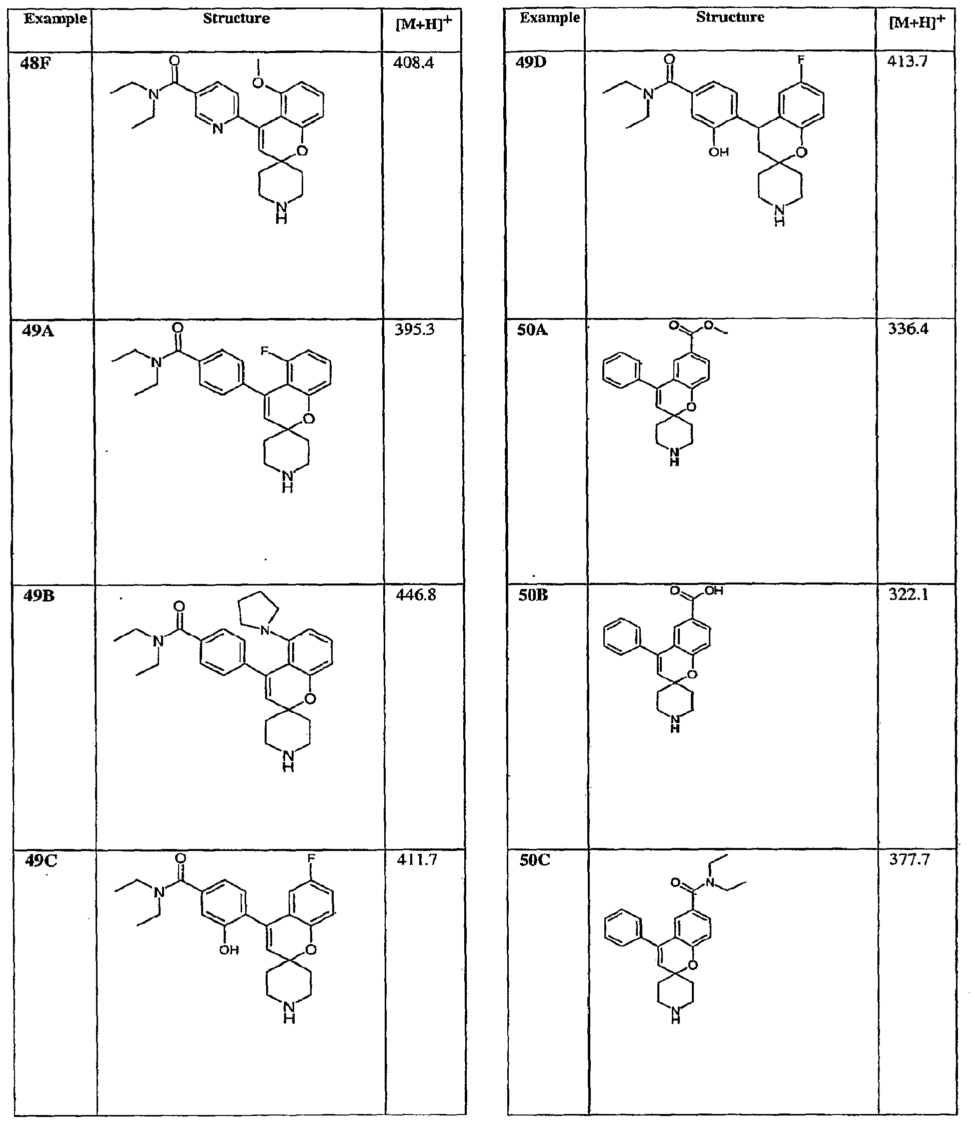

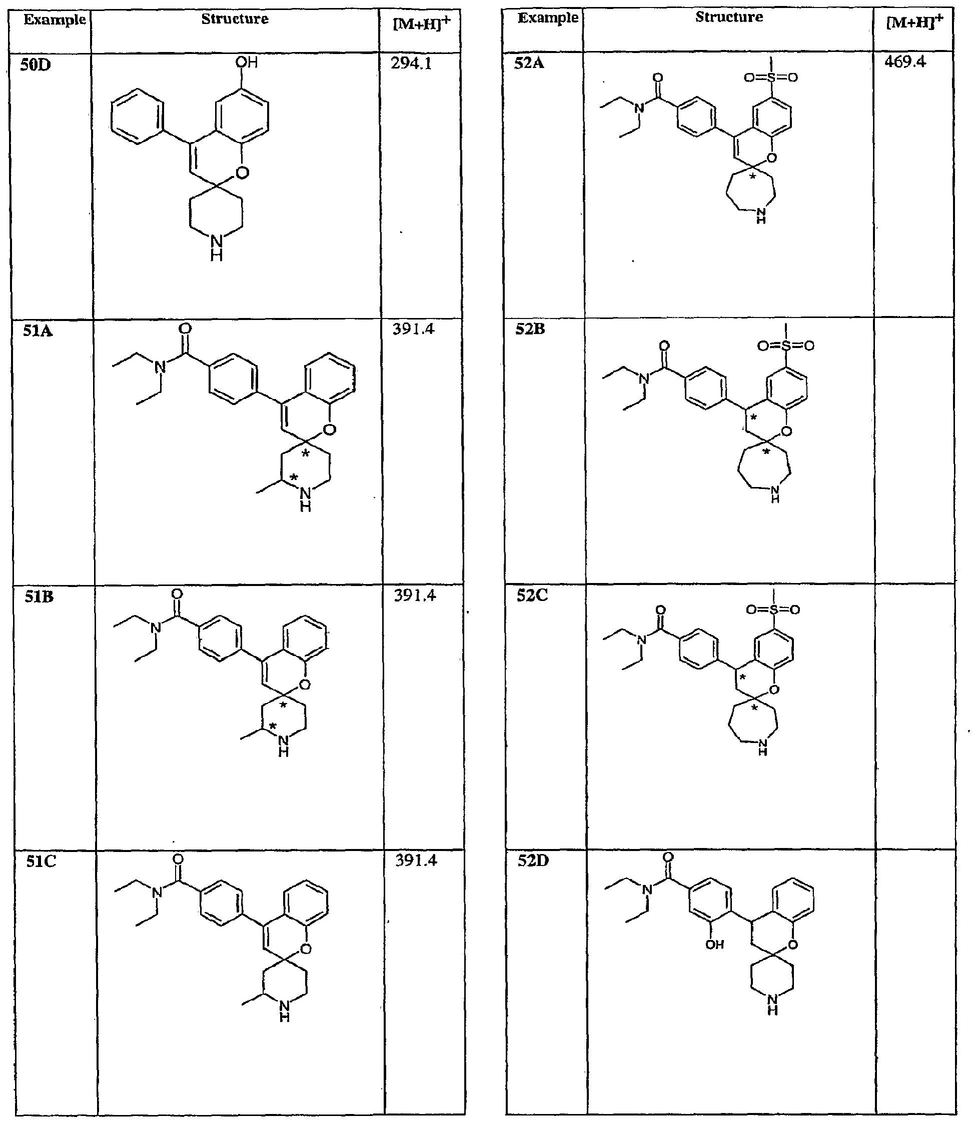

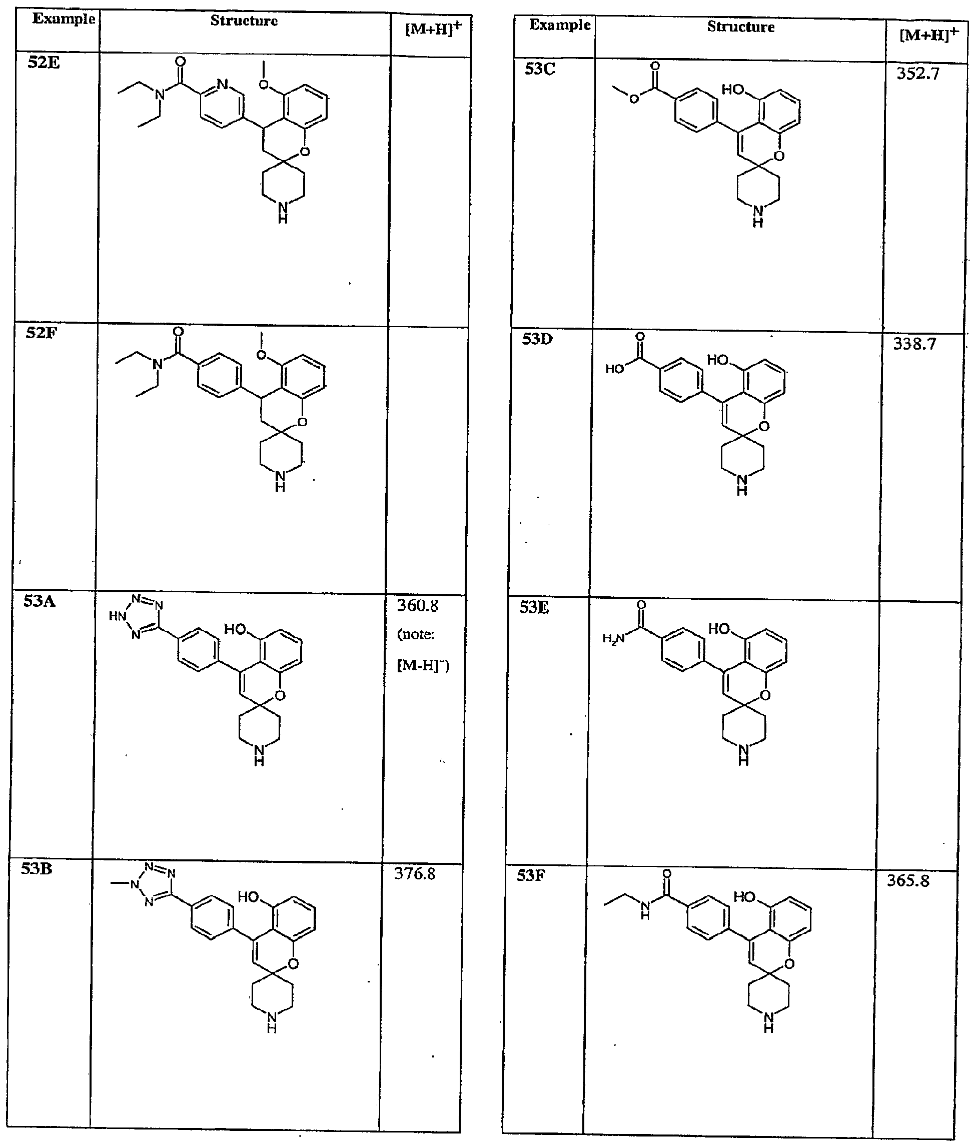

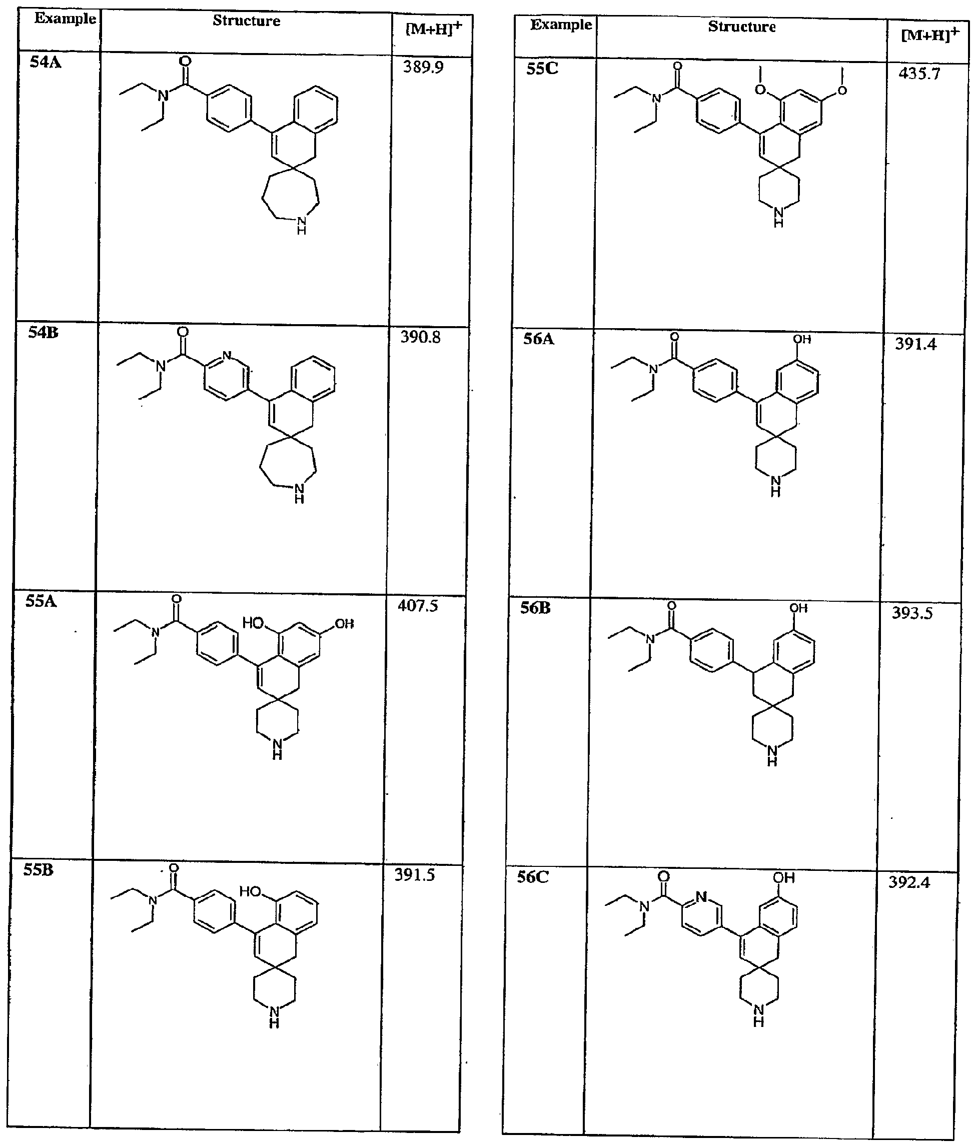

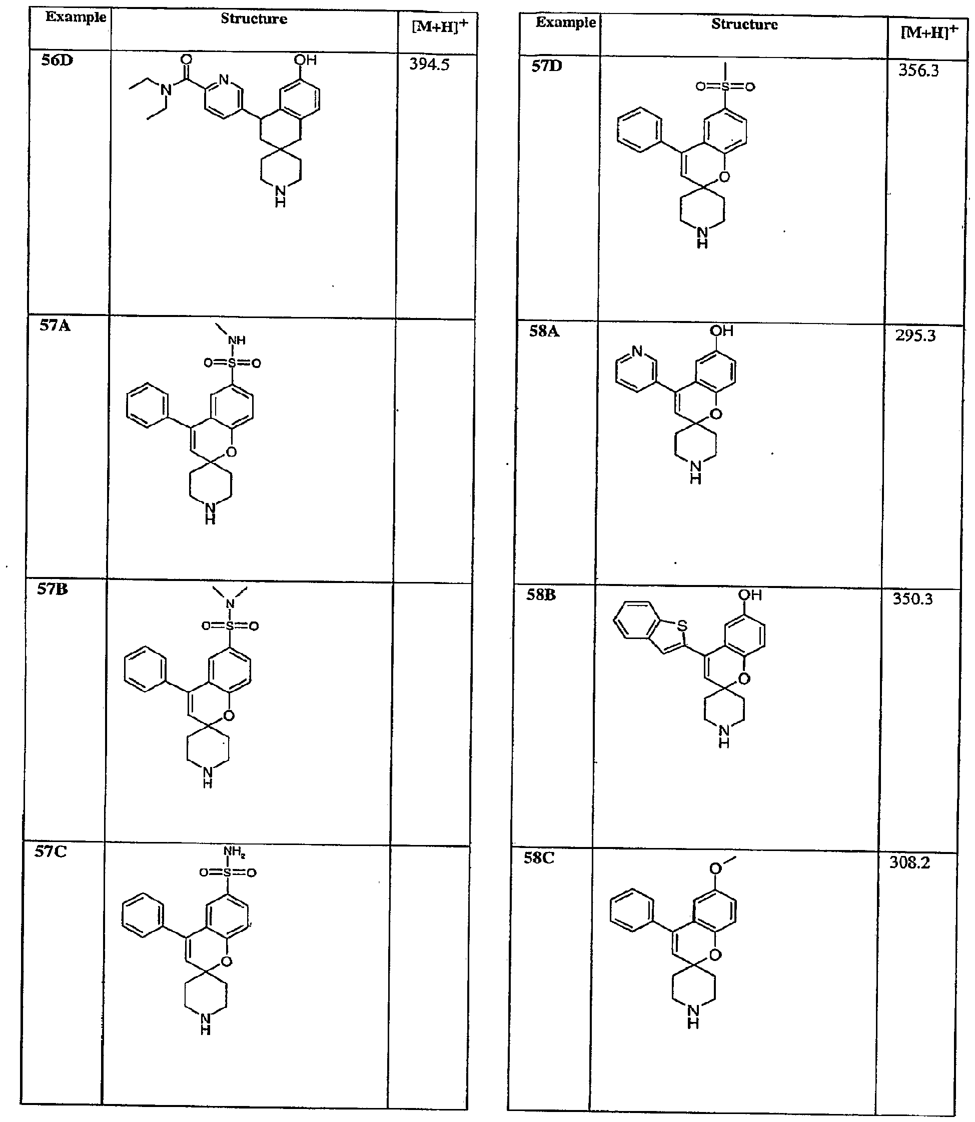

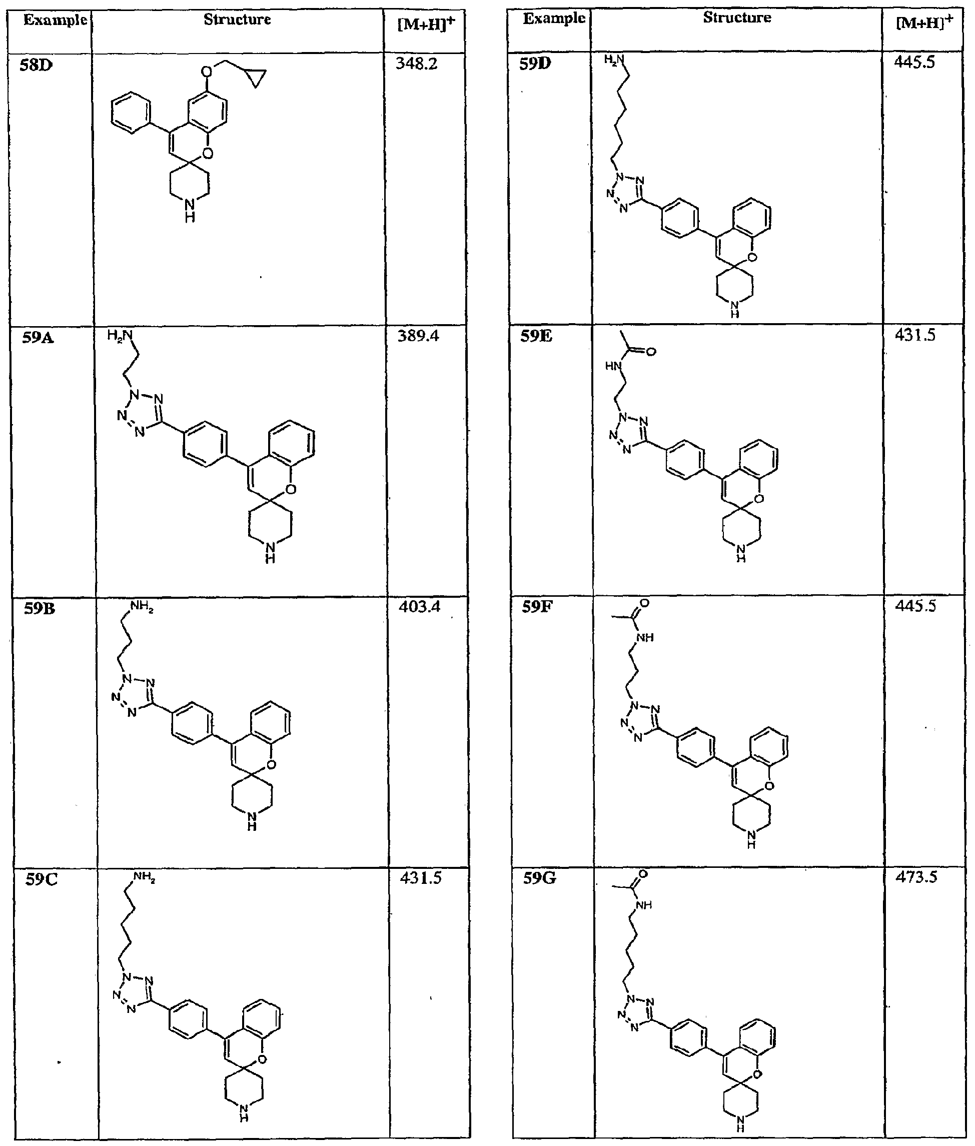

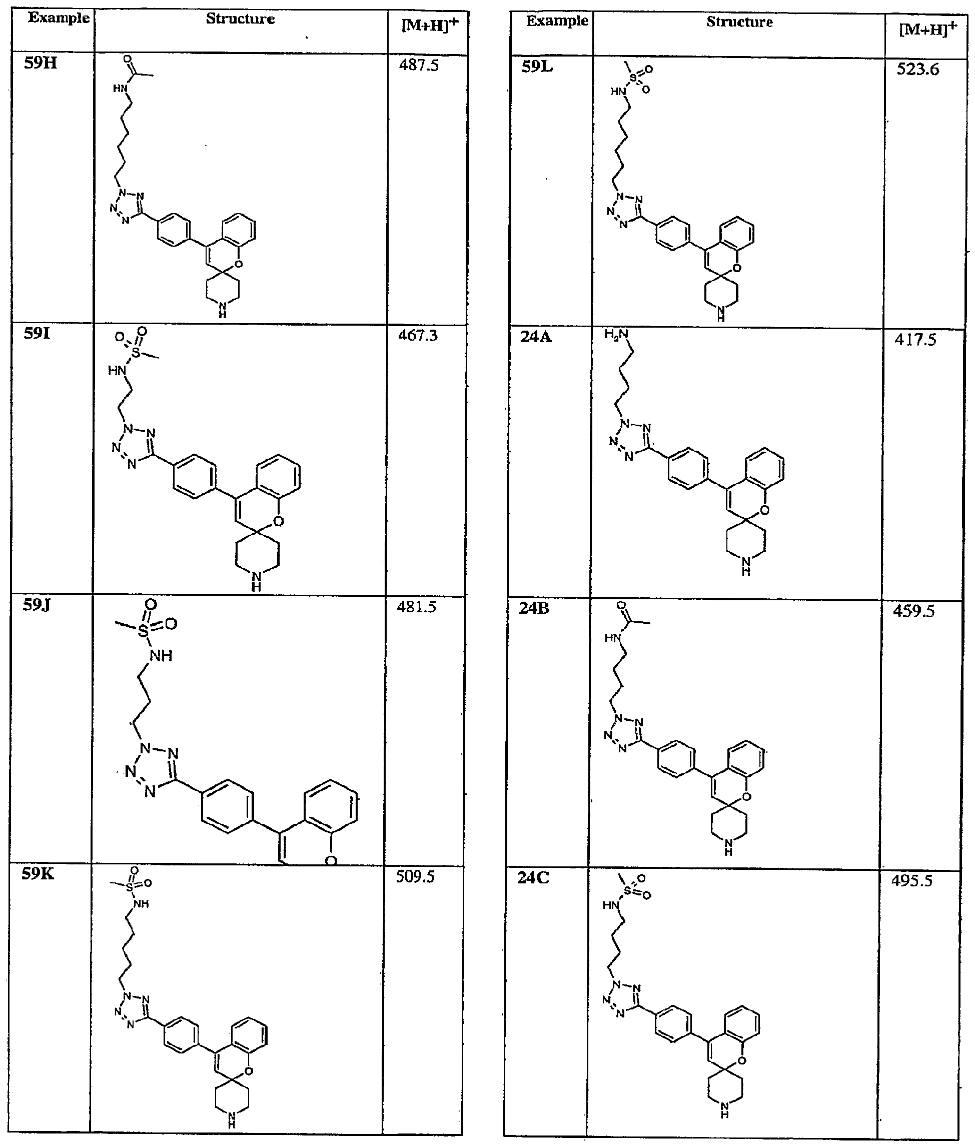

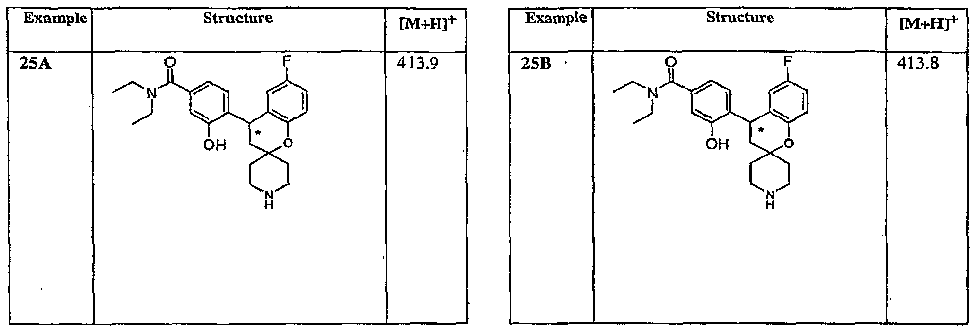

**21B** and **21C** are enantiomeric with respect to one another, and the absolute stereochemistry of **21C** was established by X-ray crystallography of derivative 21.13; hence, the absolute stereochemistry of 21B was established by inference.
**21D** and **21E** are diastereomeric with respect to one another, but their absolute stereochemistry has not been conclusively established.
**24B** and **24C** are geometric isomers with respect to one another (wherein the hydroxyl is either equatorial or axial), but the conformation of each has not been conclusively established.
**24D** and **24E** are geometric isomers with respect to one another (wherein the hydroxyl is either equatorial or axial), but the conformation of each has not been conclusively established.
**24F** and **24G** are geometric isomers with respect to one another (wherein the hydroxyl is either equatorial or axial), but the conformation of each has not been conclusively established.
**25A** and **25B** are enantiomeric with respect to one another, but their absolute stereochemistry has not been conclusively established.
**27B** and **27C** are enantiomeric with respect to one another, and their absolute stereochemistry was conclusively established using X-ray crystallography.
**27E** and **27F** are enantiomeric with respect to one another, but their absolute stereochemistry has not been conclusively, established.
**27I** and **27J** are enantiomeric with respect to one another, but their absolute stereochemistry has not been conclusively established.
**27L** and **27M** are enantiomeric with respect to one another, but their absolute stereochemistry has not been conclusively established.
**270** and **27P** are enantiomeric with respect to one another, but their absolute stereochemistry has not been conclusively established.
**27R** and **27S** are enantiomeric with respect to one another, but their absolute stereochemistry has not been conclusively established.
**27U** and **27V** are enantiomeric with respect to one another, but their absolute stereochemistry has not been conclusively established.
**37A** and **37B** are diastereomeric with respect to one another, but each is a racemic mixture of its two possible enantiomers. Their absolute stereochemistry has not been conclusively established.
**39B** and **39C** are enantiomeric with respect to one another, but their absolute stereochemistry has not been conclusively established.
**39F** and **39G** are enantiomeric with respect to one another, but their absolute stereochemistry has not been conclusively established.
**42A** and **42B** are enantiomeric with respect to one another, but their absolute stereochemistry has not been conclusively established.
**42D** and **42E** are enantiomeric with respect to one another, but their absolute stereochemistry has not been conclusively established.
**42G** and **42H** are enantiomeric with respect to one another, but their absolute stereochemistry has not been conclusively established.
**43A** and **43B** are enantiomeric with respect to one another, but their absolute stereochemistry has not been conclusively established.
**43D** and **43E** are enantiomeric with respect to one another, but their absolute stereochemistry has not been conclusively established.
**44A** and **44B** are enantiomeric with respect to one another, but their absolute stereochemistry has not been conclusively established.
**44D** and **44E** are enantiomeric with respect to one another, but their absolute stereochemistry has not been conclusively established.
**45A** and **45B** are enantiomeric with respect to one another, but their absolute stereochemistry has not been conclusively established.
**45D** and **45E** are enantiomeric with respect to one another, but their absolute stereochemistry has not been conclusively established.
**46A** and **46B** are enantiomeric with respect to one another, but their absolute stereochemistry has not been conclusively established.
**51A** and **51B** are resolvable by chiral column chromatography as individual peaks with respect to one another, but their absolute stereochemistry has not been conclusively established.

### Effect of Compounds in Object Recognition Task

### Methods

**Galantamine ((4aS,6R,8aS)-4a,5,9,10,11,12-hexahydro-3-methoxy-11-methyl-6-H-benzofuro[3a,3,2ef]** [2]benzazepin-6-ol) is a reversible, competitive acetylcholinesterase inhibitor. It is used to treat the symptoms of Alzheimer's Disease.

### Summary of data

Spending more time on the novel than on the familiar object indicates memory for the latter. The data are summarized in Table 2. Vehicle-treated rats spent a mean of 63% of time on the novel object. The means for all of the doses of test articles were numerically higher with values of: 70 and 78% for galantamine, 71 and 75% for a first representative compound of formula IV, and 73 and 81% for a second representative compound of formula IV. The increases for galantamine (1.7 mg/kg) and the two representative compounds of formula IV (30 mg/kg) were significantly different from vehicle-treated rats.

**TABLE 2**

| **Test Compound** | **Dose** | **% Time Novel Object** |
|---|---|---|
| | **(mg/kg p.o)** | **(Mean ± SEM)** |
| **Vehicle** | | |
| | | 63 ± 4 |
| **First Representative Compound of Formula IV** | | |
| | 3 | 71 ± 8 |
| | 30 | 75 ± 7 |
| **Second Representative Compound of Formula IV** | | |
| | 3 | 75 ± 5 |
| | 30 | 81 ± 5 |
| **Galantamine (Comparative)** | | |
| | 0.3 | 70 ± 6 |
| | 1.7 | 78 ± 5 |

| | | |
|---|---|---|
| Values significantly different (*p* < 0.05) from vehicle-treated rats | | |

**Reference**
Ennaceur A and Delacour J (1988) A new one-trial test for neurobiological studies of memory in rats. 1: Behavioral data. Behav Brain Res 31: 47-59.

The disclosures of each patent, patent application and publication cited or described in this document are hereby incorporated herein by reference, in their entirety.

Those skilled in the art will appreciate that numerous changes and modifications can be made to the preferred embodiments of the invention and that such changes and modifications can be made without departing from the spirit of the invention. It is, therefore, intended that the appended claims cover all such equivalent variations as fall within the true spirit and scope of the invention.

### Aspects of the Invention

1. A method for enhancing cognitive function, comprising the step of:
   administering to a patient in need thereof an effective amount of a compound of formula IV: 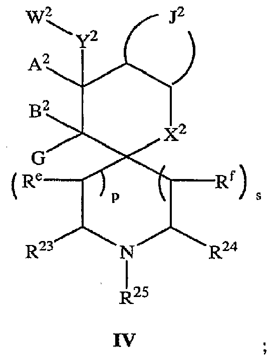 wherein:
      Y² is a single bond or -[C(R^{c})(R^{d})]ₖ-
      each R^{c}, R^{d}, R^{e}, and R^{f} is independently H or alkyl;
      W² is aryl, alkaryl, heterocycloalkylaryl, heteroaryl, alkylheteroaryl, heteroarylaryl, or alkylheteroarylaryl;
      R²³ and R²⁴ are each independently H or alkyl;
      R²⁵ is H, alkyl, -(CH₂)-alkenyl, -(CH₂)-alkynyl, cycloalkyl, alkylcycloalkyl, aralkyl, or heteroarylalkyl;
      each k is independently 1, 2, or 3;
      p is 0, 1, 2 or 3;
      s is 0, 1, 2 or 3, provided that the sum of p and s is ≤4;
      A² and B² are each independently H or alkyl, or together form a double bond;
      G is H or alkyl;
      X² is -CH₂-, -O-, or -S- -SO₂-; and
      J² forms a 6- to 10-membered aryl when taken together with the carbon atoms to which it is attached;
      or a stereoisomer, prodrug, pharmaceutically acceptable salt, hydrate, solvate, acid salt hydrate, or N-oxide thereof.
2. A method according to aspect 1, wherein Y² is a single bond.
3. A method according to aspect 1, wherein X² is -CH₂-, or -O-.
4. A method according to aspect 1, wherein X² is -CH₂-.
5. A method according to aspect 1, wherein X² is -O-.
6. A method according to aspect 1, wherein W² is aryl, alkaryl, heteroaryl, alkylheteroaryl, heteroarylaryl, or alkylheteroarylaryl.
7. A method according to aspect 1, wherein said aryl, alkaryl, heteroaryl, alkylheteroaryl, heteroarylaryl, or alkylheteroarylaryl in W² is optionally substituted with at least one of alkyl, aryl, hydroxyl, carboxyl, *N,N*-dialkylaminocarbonyl, -S(=O)₂-N(alkyl)₂, -N(H)S(=O)₂-alkyl, and -N(alkyl)C(=O)-alkyl.
8. A method according to aspect 1, wherein W² is: 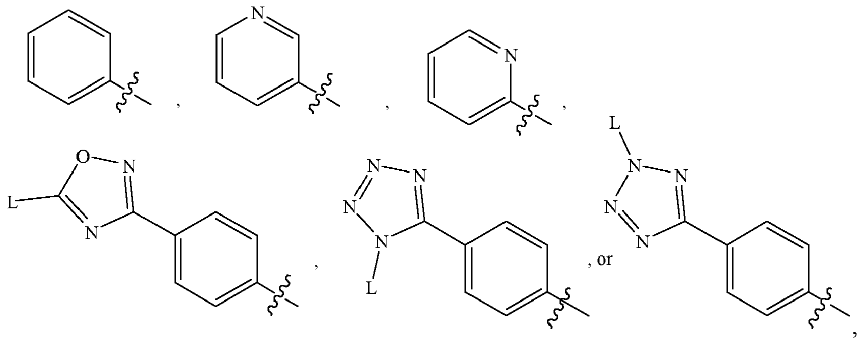 wherein W² is optionally substituted with at least one of alkyl, aryl, hydroxyl, carboxyl, *N,N*-dialkylaminocarbonyl, -S(=O)₂-N(alkyl)₂, -N(H)S(=O)₂-alkyl, and -N(alkyl)C(=O)-alkyl; and L is H or alkyl.
9. A method according to aspect 1, wherein W² is: 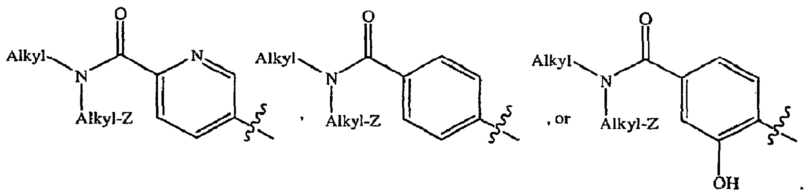
10. A method according to aspect 1, wherein W² is: 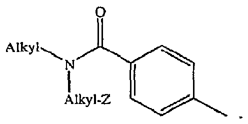
11. A method according to aspect 1, wherein R²³ and R²⁴ are each H.
12. A method according to aspect 1, wherein R²⁵ is H or alkyl.
13. A method according to aspect 1, wherein R^{c}, R^{d}, R^{e} and R^{f} are each H.
14. A method according to aspect 1, wherein p is 1 or 2.
15. A method according to aspect 1, wherein p is 1.
16. A method according to aspect 1, wherein s is 1.
17. A method according to aspect 1, wherein J² forms a 6-membered aryl ring when taken together with the carbon atoms to which it is attached.
18. A method according to aspect 1, wherein G is H.
19. A method according to aspect 1, wherein A² and B² are each H.
20. A method according to aspect 1, wherein A² and B² taken together form a double bond between the carbon atoms to which they are attached.
21. A method according to aspect 1, wherein said compound of formula IV is a compound of formula VI: 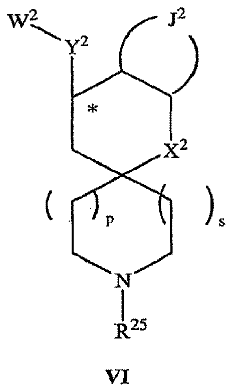 or a stereoisomer, prodrug, pharmaceutically acceptable salt, hydrate, solvate, acid salt hydrate, or N-oxide thereof;
   wherein:
   p is 0, 1, or 2;
   s is 0, 1, or 2; and
   X² is 0 or CH₂.
22. A method according to aspect 1, wherein said compound of formula IV is a compound of formula VII: 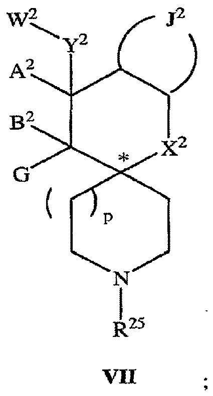 or a stereoisomer, prodrug, pharmaceutically acceptable salt, hydrate, solvate, acid salt hydrate, or N-oxide thereof;
   wherein:
   p is 0 or 2; and
   X² is 0 or CH₂.
23. A method according to aspect 1, wherein said compound of formula IV is a compound of formula VIII: 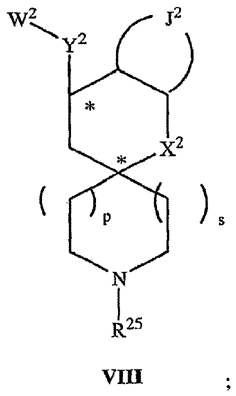 or a stereoisomer, prodrug, pharmaceutically acceptable salt, hydrate, solvate, acid salt hydrate, or N-oxide thereof;
   wherein:
   p is 0 or 2; and
   X² is 0 or CH₂.
24. A method according to aspect 1, wherein said compound of formula IV is a compound of formula IX: 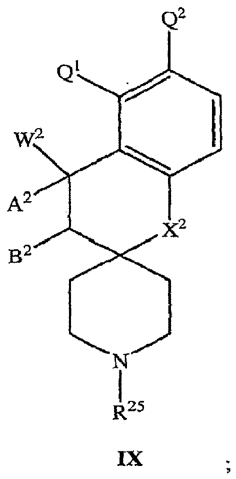 or a stereoisomer, prodrug, pharmaceutically acceptable salt, hydrate, solvate, acid salt hydrate, or N-oxide thereof;
   wherein:
   X² is 0 or CH₂;
   Q¹ and Q² is H, alkyl, alkoxy, OCF₂H, OCF₃, hydroxy, chloro, fluoro, SO₂-alkyl, SO₂NR^{g}R^{h} where at least one of Q¹ or Q² is H;
   R²⁵ is H or methyl;
   A² and B² are H or taken together to form a double bond;
   W² is phenyl or pyridinyl ring, each substituted with 1 to 3 substitutions selected from the group consisting of alkyl, alkoxy, carboxy, -COO-alkyl, -CONR^{g}R^{h}, SO₂NR^{g}R^{h}, NRⁱSO₂R^{j};
   R^{g} and R^{h} are H or alkyl, or taken together with the nitrogen to which they are attached form a 4 to 7 membered heterocyclalkyl; and
   Rⁱ and R^{j} are H or alkyl.
25. A method according to aspect 1, wherein said compound of formula IV is a compound of formula X: 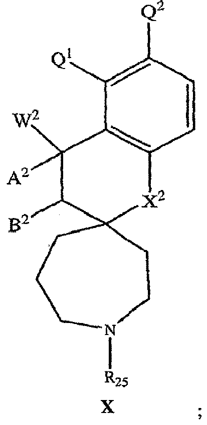 or a stereoisomer, prodrug, pharmaceutically acceptable salt, hydrate, solvate, acid salt hydrate, or N-oxide thereof;
   wherein:
   X² is O or CH₂;
   Q¹ and Q² is H, alkyl, alkoxy, OCF₂H, OCF₃, hydroxy, chloro, fluoro, SO₂-alkyl, SO₂NR^{g}R^{h} where at least one of Q¹ or Q² is H;
   R²⁵ is H or methyl;
   A² and B² are H or taken together to form a double bond;
   W² is phenyl or pyridinyl ring, each substituted with 1 to 3 substitutions selected from the group consisting of alkyl, alkoxy, carboxy, -COO-alkyl, -CONR^{g}R^{h}, SO₂NR^{g}R^{h}, NRⁱSO₂R^{j};
   R^{g} and R^{h} are H or alkyl, or taken together with the nitrogen to which they are attached form a 4 to 7 membered heterocyclalkyl; and
   Rⁱ and R^{j} are H or alkyl.
26. A method according to aspect 1, wherein said compound of formula IV is:
   4-[(4-*N,N*-diethylaminocarbonyl)phenyl]-spiro[2H,1-benzopyran-2,4'-piperidine];
   4-[(2*-N,N-*diethylaminocarbonyl)pyrid-5-yl]-spiro[6-fluoro-2H,1-benzopyran-2,4'-piperidine];
   4-[(2-*N,N-*diethylaminocarbonyl)pyrid-5-yl]-spiro[5-methoxy-2H,1-benzopyran-2,4'-piperidine];
   4-[(4-*N,N-*diethylaminocarbonyl)phenyl]-spiro[5-hydroxy-2H,1-benzopyran-2,4'-piperidine];
   4-[(4-*N,N*-diethylaminocarbonyl)phenyl]-spiro[2H,1-benzopyran-2,4'-azepane];
   4-[(4-*N,N*-diethylaminocarbonyl)phenyl]-spiro[6-cyclopropylmethylaminosulfonyl-2H,1-benzopyran-2,4'-azepane];
   4-[(4-*N,N*-diethylaminocarbonyl)phenyl]-spiro[3,4-dihydro-2H,1-benzopyran-2,4'-piperidine];
   4-[(4-*N,N*-diethylaminocarbonyl)phenyl]-spiro[1,2-dihydronaphthalene-2,4'-piperidine];
   4-[(4*-N,N* diethylaminocarbonyl-2-hydroxy)phenyl]-spiro[2H,1-benzopyran-2,4'-piperidine];
   4-[(4-*N,N-*diethylaminocarbonyl-3-hydroxy)phenyl]-spiro[2H,1-benzopyran-2,4'-piperidine];
   4-[(4-*N,N*-diethylaminocarbonyl)phenyl]-3-methyl-spiro[2H,1-benzopyran-2,4'-piperidine];
   4-[(2-*N,N-*diethylaminocarbonyl)pyrid-5-yl]-spiro[2H,1-benzopyran-2,4'-piperidine];
   4-[(4-*N,N*-diethylaminocarbonyl)phenyl]-spiro[6-cyclopropylmethoxy-2H,1-benzopyran-2,4'-piperidine];
   4-[(2-*N,N*-diethylaminocarbonyl)pyrid-5-yl]-spiro[-6-cyclopropylmethoxy-2H,1-benzopyran*-2,4'-piperidine];*
   4-[(4*-N,N*-diethylaminocarbonyl)phenyl]-spiro[6-aminocarbonyl-2H,1-benzopyran-2,4'-piperidine];
   4-[(4-*N,N*-diethylaminocarbonyl)phenyl]-spiro[6-propylaminosulfonyl-2H,1-benzopyran-2,4'-azepane];
   4-[(4-*N,N*-diethylaminocarbonyl)phenyl]-spiro[6-methanesulfonyl-2H,1-benzopyran-2,4'-azepane];
   4-[(2-*N,N*-diethylaminocarbonyl)pyrid-5-yl]-spiro[3,4-dihydro-2H,1-benzopyran-2,4'-piperidine];
   4-[(2-*N,N-*diethylaminocarbonyl)pyrid-5-yl]-spiro[6-fluoro-3,4-dihydro-2H,1-benzopyran-2,4'-piperidine];
   4-[(5-*N,N*-diisopropylaminocarbonyl)pyrid-2-yl]-spiro[2H,1-benzopyran-2,4'-piperidine;
   4-[(4-*N,N*-diethylaminocarbonyl)phenyl]-spiro[6-ethylsulfonylamino-2H,1-benzopyran-2,4'-piperidine];
   4-[(*4-N,N-*diethylaminocarbonyl)phenyl]-spiro[6-methylsulfonylamino-2H,1-benzopyran-2,4'-piperidine];
   4-[(4-*N,N*-diethylaminocarbonyl)phenyl]-spiro[5-methyl-2H,1-benzopyran-2,4'-piperidine];
   4-[4-(2*H*-tetrazol-5-yl)phenyl]-spiro[2H,1-benzopyran-2,4'-piperidine];
   4-[4-(2-methyl-tetrazol-5-yl)phenyl]-spiro[2H,1-benzopyran-2,4'-piperidine];
   4-[3-(2-(3-carboxyprop-1-yl)-tetrazol-5-yl)phenyl]-spiro[2H,1-benzopyran-2,4'-piperidine];
   4-[4-(5-methyl-[1,2,4]oxadiazol-3-yl)phenyl]-spiro[2H,1-benzopyran-2,4'-piperidine];
   4-[(4-*N,N*-diethylaminocarbonyl)phenyl]-spiro[2H,1-benzopyran-2,4'-(1'-methylpiperidine)];
   4-[(4-*N,N-*diethylaminosulfonyl)phenyl]-spiro[2H,1-benzopyran-2,4'-piperidine]; and
   4-[(4-(*N*-methyl-*N*-(3-methylbutanoyl)-amino)phenyl]-spiro[2H,1-benzopyran-2,4'-piperidine];
   4*-[(4-*N,N-*diethylaminocarbonyl)phenyl]-spiro[3,4-dihydro-2H,1-benzopyran-2,4'-piperidine];
   4*-[(2-*N,N*-diethylaminocarbonyl)pyrid-5-yl]-spiro[3,4-dihydro-2H,1-benzopyran-2,4'-piperidine];
   4-[(4-*N,N-*diethylaminocarbonyl)phenyl]-spiro*[2H,1-benzopyran-2,4'-azepane];
   4-[(4-*N,N*-diethylaminocarbonyl)phenyl]-spiro* [6-cyclopropylmethylaminosulfonyl-2H,1-benzopyran-2,4'-azepane];
   4-[(4-*N,N*-diethylaminocarbonyl)phenyl]-spiro*[6-propylaminosulfonyl-2H,1-benzopyran-2,4'-azepane];
   4-[(4-*N,N*-diethylaminocarbonyl)phenyl]-spiro*[6-methanesulfonyl-2H,1-benzopyran-2,4'-azepane]; or
   a stereoisomer, partial stereoisomer, prodrug, pharmaceutically acceptable salt, hydrate, solvate, acid salt hydrate, or N-oxide thereof.
27. A method according to aspect 1, wherein said cognitive function is memory.
28. A method according to aspect 1, wherein said patient is suffering cognitive dysfunction.
29. A method according to 28, wherein said cognitive dysfunction is memory loss.
30. A method according to aspect 29, wherein said memory loss is age-associated, caused by electro-convulsive therapy, the result of brain damage, or a combination thereof.
31. A method according to aspect 30, wherein said brain damage is caused by a stroke, an anesthetic accident, head trauma, hypoglycemia, carbon monoxide poisoning, lithium intoxication, vitamin deficiency, or a combination thereof.
32. A method according to aspect 28, wherein said cognitive dysfunction is caused by a disease or condition selected from the group consisting of Alzheimer's Disease, mild cognitive impairment, age-related cognitive decline, vascular dementia, Parkinson's Disease dementia, amyotrophic lateral sclerosis, Huntington's Disease, stroke, traumatic brain injury, AIDS-associated dementia, schizophrenia, Lewy-body variant of Alzheimer's Disease with or without association with Parkinson's Disease, Creutzfeld-Jakob Disease, Korsakoff's Disorder, learning disabilities caused by degenerative disorders, learning disabilities caused by non-degenerative disorders, genetic conditions, cerebral senility, vascular dementia, electric shock induced amnesia, memory impairment associated with depression or anxiety, memory impairment associated with surgical procedures, Down's syndrome, and combinations thereof
33. A composition, comprising:
   a. a compound of formula IV;
   b. a second pharmaceutically active compound selected from the group consisting of cholinesterase inhibitor, NMDA receptor modulator, β amyloid modulator, β amyloid inhibitor, nicotinic cholinergic agent, nicotine receptor partial agonist, estrogenic agent, selective estrogen receptor modulator, muscarinic agonist, neurotransmitter precursor, neurotransmitter reuptake inhibitor, Coenzyme Q10, *Ginkgo biloba,* phosphatidylserine, vitamin E, and combinations thereof; and
   c. a pharmaceutically acceptable carrier.

## Claims

1. A compound of formula IV: 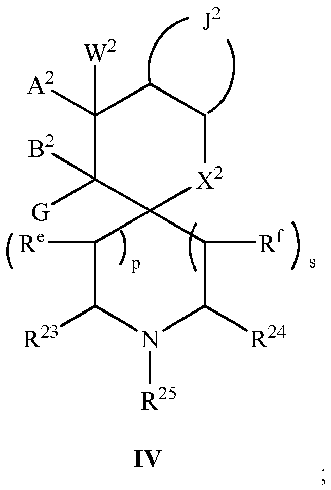 wherein:
each R^{e}, and R^{f} is independently H or alkyl;
W² is aryl, alkaryl, heterocycloalkylaryl, heteroaryl, alkylheteroaryl, heteroarylaryl, or alkylheteroarylaryl;
R²³ and R²⁴ are each independently H or alkyl;
R²⁵ is H, alkyl, -(CH₂)-alkenyl, -(CH₂)-alkynyl, cycloalkyl, alkylcycloalkyl, aralkyl, or heteroarylalkyl;
p is 0, 1, 2 or 3;
s is 0, 1, 2 or 3, provided that the sum of p and s is ≤4;
A² and B² are each independently H or alkyl, or together form a double bond;
G is H or alkyl;
X² is -O-; and
J² forms a 6- to 10-membered aryl when taken together with the carbon atoms to which it is attached;
or a stereoisomer, prodrug, pharmaceutically acceptable salt, hydrate, solvate, acid salt hydrate, or N-oxide thereof;
for use in enhancing cognitive function.

2. A compound according to claim 1, wherein said compound of formula IV is a compound of formula XXVIII: 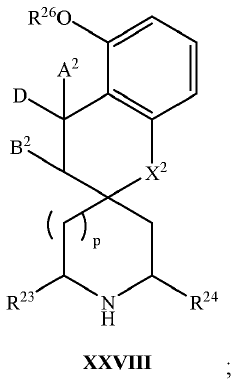 wherein:
D is: 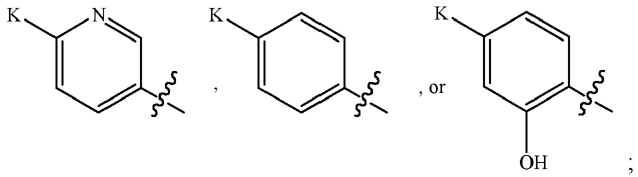
K is carboxy (-COOH), -C(=O)-O-alkyl, -S(=O)₂-N(alkyl)(alkyl), heteroaryl, alkylheteroaryl, aminocarbonyl (-C(=O)-NH₂), or N-alkylaminocarbonyl (-C(=O)-NH(alkyl));
R²³, R²⁴, and R²⁶ are each independently H or alkyl;
p is 1 or 2;
A² and B² are each H, or together form a double bond; and
X² is or -O-;
or a stereoisomer, prodrug, pharmaceutically acceptable salt, hydrate, solvate, acid salt hydrate, or N-oxide thereof;
for use in enhancing cognitive function.

3. A compound according to Claim 1, wherein W² is aryl, alkaryl, heteroaryl, alkylheteroaryl, heteroarylaryl, or alkylheteroarylaryl, optionally substituted with at least one of alkyl, aryl, hydroxyl, carboxyl, *N,N*-dialkylaminocarbonyl, -S(=O)₂-N(alkyl)₂, -N(H)S(=O)₂-alkyl, and -N(alkyl)C(=O)-alkyl.

4. A compound according to claim 1, wherein W² is: 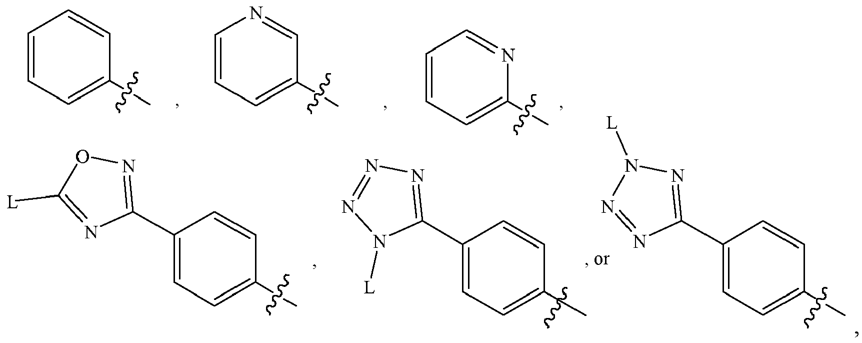 and
wherein W² is optionally substituted with at least one of alkyl, aryl, hydroxyl, carboxyl, *N,N*-dialkylaminocarbonyl, -S(=O)₂-N(alkyl)₂, -N(H)S(=O)₂-alkyl, and -N(alkyl)C(=O)-alkyl; and L is H or alkyl.

5. A compound according to claim 1, wherein W² is:

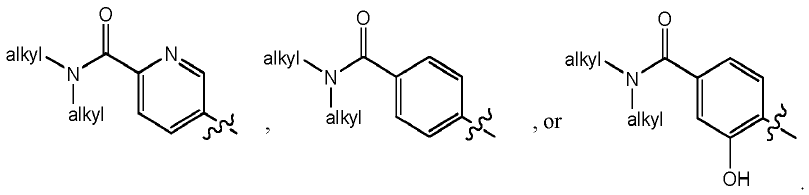

6. A compound according to claim 1, wherein W² is:

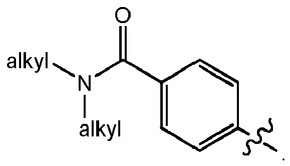

7. A compound according to claim 1, wherein:
i) R²³ and R²⁴ are each H; or
ii) R²⁵ is H or alkyl; or
iii) R^{e} and R^{f} are each H.

8. A compound according to claim 1, wherein J² forms a 6-membered aryl ring when taken together with the carbon atoms to which it is attached.

9. A compound according to claim 1, wherein said compound of formula IV is a compound of formula IX: 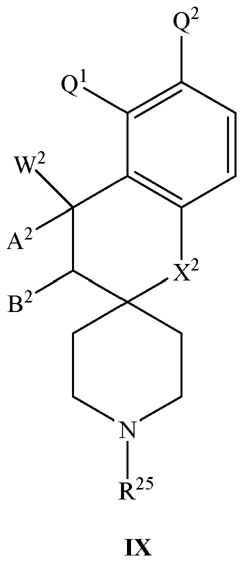 or a stereoisomer, prodrug, pharmaceutically acceptable salt, hydrate, solvate, acid salt hydrate, or N-oxide thereof;
wherein:
Q¹ and Q² is H, alkyl, alkoxy, OCF₂H, OCF₃, hydroxy, chloro, fluoro, SO₂-alkyl, SO₂NR^{g}R^{h}, where at least one of Q¹ or Q² is H;
R²⁵ is H or methyl;
A² and B² are H or taken together to form a double bond;
W² is phenyl or pyridinyl ring, each substituted with 1 to 3 substitutions selected from the group consisting of alkyl, alkoxy, carboxy, -COO-alkyl, -CONR^{g}R^{h}, SO₂NR^{g}R^{h}, NRⁱSO₂R^{j};
R^{g} and R^{h} are H or alkyl, or taken together with the nitrogen to which they are attached form a 4 to 7 membered heterocyclalkyl; and
Rⁱ and R^{j} are H or alkyl.

10. A compound according to claim 1, wherein said compound of formula IV is:

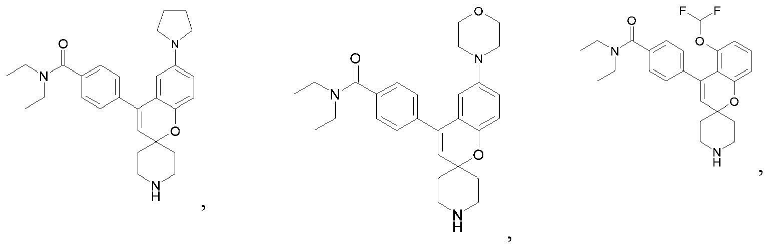

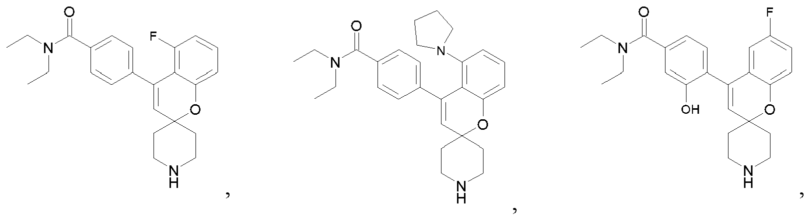

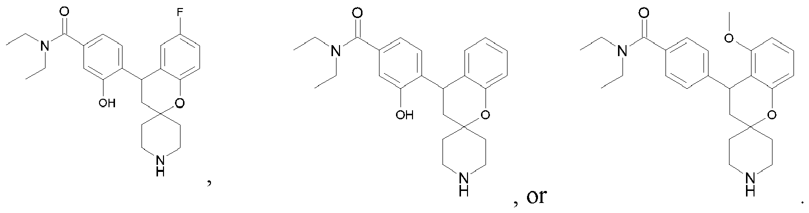

11. A compound according to claim 1, wherein said compound of formula IV is:

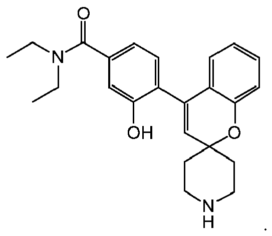

12. A compound according to claim 1, wherein said compound of formula IV is:

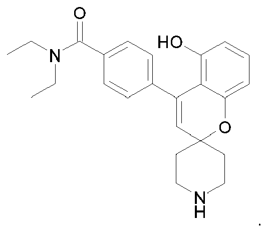

13. A compound according to Claim 2, wherein D is:

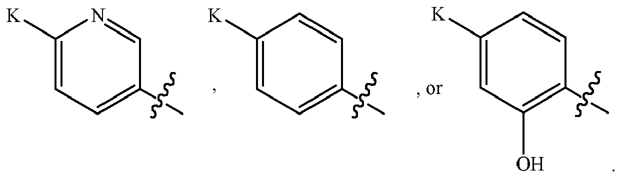

14. A compound according to Claim 2, wherein D is:

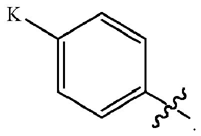

15. A compound according to Claim 2, wherein K is alkyltetrazolyl, and the alkyl group of said alkyltetrazolyl is C₁-C₃alkyl, preferably said C₁-C₃alkyl is methyl.

16. A compound according to any one of Claims 1 and 2, wherein A² and B² taken together form a double bond between the carbon atoms to which they are attached.

17. A compound according to Claim 2, wherein said compound of formula XXVIII is:
i) 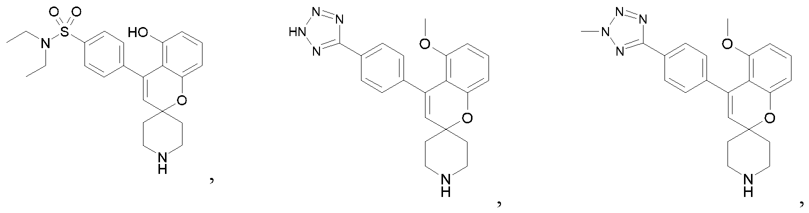 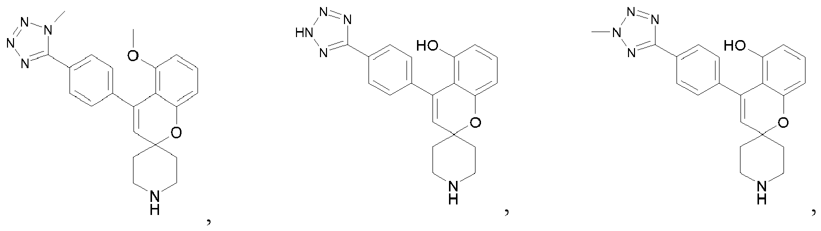 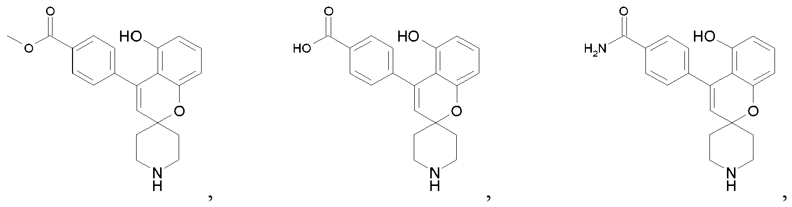 or

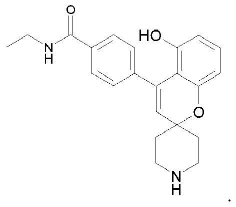

18. A compound according to Claim 2, wherein said compound of formula XXVIII is:

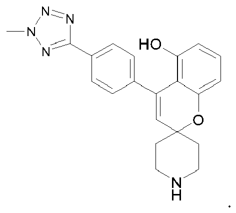

19. A compound according to any one of claims 1 to 18,
i) wherein said cognitive function is memory;
ii) wherein said patient is suffering cognitive dysfunction, such as memory loss, wherein said memory loss is preferably age-associated, caused by electro-convulsive therapy, as the result of brain damage, or a combination thereof;
b) and, for example, wherein said brain damage is caused by a stroke, an anesthetic accident, head trauma, hypoglycemia, carbon monoxide poisoning, lithium intoxication, vitamin deficiency, or a combination thereof.

20. A compound according to claim 19, part ii), wherein said cognitive dysfunction is caused by a disease or condition selected from the group consisting of Alzheimer's Disease, mild cognitive impairment, age-related cognitive decline, vascular dementia, Parkinson's Disease dementia, amyotrophic lateral sclerosis, Huntington's Disease, stroke, traumatic brain injury, AIDS-associated dementia, schizophrenia, Lewy-body variant of Alzheimer's Disease with or without association with Parkinson's Disease, Creutzfeld-Jakob Disease, Korsakoff's Disorder, learning disabilities caused by degenerative disorders, learning disabilities caused by non-degenerative disorders, genetic conditions, cerebral senility, vascular dementia, electric shock induced amnesia, memory impairment associated with depression or anxiety, memory impairment associated with surgical procedures, Down's syndrome, and combinations thereof.

21. A composition, comprising:
a. a compound according to any one of claims 1 to 18;
b. a second pharmaceutically active compound selected from the group consisting of cholinesterase inhibitor, NMDA receptor modulator, β amyloid modulator, β amyloid inhibitor, nicotinic cholinergic agent, nicotine receptor partial agonist, estrogenic agent, selective estrogen receptor modulator, muscarinic agonist, neurotransmitter precursor, neurotransmitter reuptake inhibitor, Coenzyme Q10, *Ginkgo biloba,* phosphatidylserine, vitamin E, and combinations thereof; and
c. a pharmaceutically acceptable carrier.
